# EUROPEAN PATENT SPECIFICATION

(11) **EP 4 563 579 B1**
(45) Date of publication and mention of the grant of the patent: **18.11.2026**
(21) Application number: 23845496.1
(22) Date of filing: 24.07.2023
(51) Int. Cl.: C07D 498/22, A61K 31/395, A61K 31/519, A61P 35/00

(54) **MACROCYCLIC EGFR INHIBITOR, AND PREPARATION METHOD THEREFOR AND PHARMACEUTICAL USE THEREOF**
MAKROCYCLISCHER EGFR-HEMMER UND HERSTELLUNGSVERFAHREN DAFÜR UND PHARMAZEUTISCHE VERWENDUNG DAVON
INHIBITEUR D'EGFR MACROCYCLIQUE, SON PROCÉDÉ DE PRÉPARATION ET SON UTILISATION PHARMACEUTIQUE

(30) Priority: 27.07.2022 CN 202210890232; 23.12.2022 CN 202211663752
(43) Date of publication of application: 04.06.2025
(73) Proprietor: Abbisko Therapeutics Co., Ltd., Shanghai 201203 (CN)
(72) Inventor: YANG, Fei, Shanghai 201203 (CN); DENG, Haibing, Shanghai 201203 (CN); YU, Yong, Shanghai 201203 (CN); YU, Hongping, Shanghai 201203 (CN); CHEN, Zhui, Shanghai 201203 (CN); XU, Yaochang, Shanghai 201203 (CN)
(74) Representative: Potter Clarkson
(86) International application number: PCT/CN2023/108872
(87) International publication number: WO 2024/022286

(56) References cited:
- WO-A1-2021/168074
- WO-A1-2021/216440
- WO-A1-2022/098992
- WO-A1-2022/133037
- US-A1- 2016 257 682

## Description

### TECHNICAL FIELD

The present invention belongs to the field of pharmaceutical synthesis, and particularly relates to a a macrocyclic EGFR inhibitor, preparation method therefor, and pharmaceutical use thereof.

### BACKGROUND

Lung cancer is a malignant tumor with the highest mortality rate worldwide, posing a serious threat to human health. Non-small-cell Lung Cancer (NSCLC) accounts for approximately 85% of all lung cancer cases. Currently, molecular mechanisms such as multiple gene mutations and abnormal gene expression have been confirmed to be related to the pathogenesis of NSCLC, among which, EGFR is the primary driver gene. EGFR activating mutations occur in approximately 17% of NSCLC patients worldwide and are more prevalent in East Asian populations, with a mutation frequency of about 50%. EGFR activating mutations typically occur in exons 18-21. The most common mutation subtypes are deletion mutations in exon 19 (Del19) and L858R point mutations in exon 21, accounting for more than 80% of all mutation types. These mutations can lead to ligand-independent receptor activation and promote tumor cell survival and proliferation. The first- and second-generation EGFR Tyrosine Kinase Inhibitors (TKIs) are mainly targeted at these two activating mutations. Compared with platinum-based chemotherapy, the first- and second-generation EGFR TKIs can significantly prolong progression-free survival, but resistance tends to emerge after 10-12 months of treatment. The EGFR T790M mutation is the main mechanism of resistance to the first- and second-generation EGFR TKIs, and subsequently, the third-generation EGFR TKI, Osimertinib, was developed to selectively inhibit the EGFR T790M mutation and other common EGFR mutations. The results of the phase III FLAURA clinical trial demonstrated that Osimertinib exhibited superior efficacy compared with the first-generation EGFR TKIs, significantly improving progression-free survival (18.9 months vs. 10.2 months) and overall survival (38.6 months vs. 31.8 months), which directly promoted Osimertinib to be included in the first-line treatment options.

Despite the significant efficacy of Osimertinib, resistance inevitably emerges and leads to disease progression. Currently, no targeted drugs are approved for patients who are resistant to Osimertinib. Several EGFR-dependent and -independent mechanisms of resistance have been reported in both preclinical and clinical studies. The C797X (with the C797S mutation being the most frequent) mutation in exon 20 has emerged as the most common EGFR-dependent mechanism of resistance to Osimertinib. The C797X mutation is located in the tyrosine kinase region of EGFR, making it impossible for Osimertinib to form a covalent bond within the ATP binding domain of EGFR, which results in resistance. In the phase 3 AURA3 clinical trial, the probability of C797X mutation in patients receiving Osimertinib in the second-line setting was 15%, while in the phase 3 FLAURA clinical trial, the probability of C797X mutation in patients receiving Osimertinib in the first-line setting was 7%. Therefore, it is crucial to develop a next-generation EGFR inhibitor with improved activity and selectivity, targeting both a double mutation consisting of an EGFR activating mutation/C797S and a triple mutation consisting of an EGFR activating mutation/T790M/C797S, for patients who have developed resistance to the first-line or second-line Osimertinib therapy.

### SUMMARY

The objective of the present invention is to provide a macrocyclic EGFR inhibitor, a preparation method therefor, and a pharmaceutical use thereof. The series of compounds of the present invention have a strong inhibitory effect on cytological activity of EGFR L858R/C797S double mutation, EGFR Del 19/C797S double mutation, L858R/T790M/C797S triple mutation and Del19/T790M/C797S triple mutation, and can be widely used in the preparation of drugs for treatment and/or prevention of tumors or metastatic diseases associated at least partially with EGFR L858R/C797S double mutation, EGFR Del19/C797S double mutation, L858R/T790M/C797S triple mutation and Del19/T790M/C797S triple mutation, especially in the preparation of drugs for treating hyperproliferative diseases and disorders inducing cell death, thereby promising the development of a new-generation EGFR inhibitor.

The invention is as defined in the appended claims.

The first aspect of the present invention provides a compound of formula (III), a stereoisomer or pharmaceutically acceptable salt thereof: 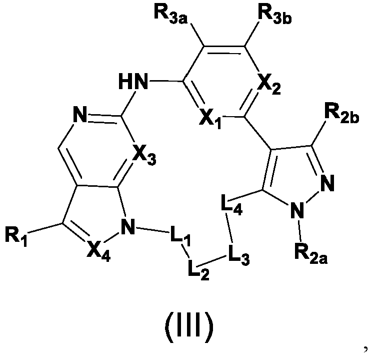
wherein, X₁ is CRₐ or N; X₂ is CR_{b} or N; X₃ is CR_{c} or N; X₄ is CR_{d} or N;
L₁ is CR₅ₐR_{5b}; L₂ is (CR_{5c}R_{5d})ₚ; L₃ is a bond, CR₅ₑR_{5f} or C(O); L₄ is CR_{5g}R₅ₕ, O, NR_{6c} or C(O);
R₁ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-8 membered heterocyclyl, C₆₋₈ aryl, 5-8 membered heteroaryl, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, and the above groups are each independently optionally further substituted by one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₁₋₄ alkyl substituted by C₃₋₆ cycloalkyl, C₁₋₄ alkyl substituted by 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -N(R₁₃)-S(O)₂R₁₀, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₇)R₈, -C₀₋₄ alkyl-N=S(O)R₈R₉, -C₀₋₄ alkyl-N=SR₈R₉, -C₀₋₄ alkyl-N(R₁₃)-S(O)₂R₁₀, -C₀₋₄ alkyl-O-S(O)₂R₁₀, -C₀₋₄ alkyl-S(O)ᵣR₁₀, -C₀₋₄ alkyl-O-R₁₁, -C₀₋₄ alkyl-C(O)OR₁₁, -C₀₋₄ alkyl-C(O)SR₁₁, -C₀₋₄ alkyl-S-C(O)R₁₂, -C₀₋₄ alkyl-C(O)R₁₂, -C₀₋₄ alkyl-O-C(O)R₁₂, -C₀₋₄ alkyl-P(O)(R₁₂)₂, -C₀₋₄ alkyl-NR₁₃R₁₄, -C₀₋₄ alkyl-C(=NR₁₃)R₁₂, -C₀₋₄ alkyl-N(R₁₃)-C(=NR₁₄)R₁₂, -C₀₋₄ alkyl-C(O)NR₁₃R₁₄ and -C₀₋₄ alkyl-N(R₁₃)-C(O)R₁₂;
R₂ₐ is selected from the group consisting of hydrogen, deuterium, hydroxy, C₁₋₄ alkyl, C₂₋₄ alkenyl, C₂₋₄ alkynyl, C₃₋₆ cycloalkyl and 3-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R_{2b} is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₃ₐ, R_{3b}, Rₐ and R_{b} are each independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅;
R_{c} is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅;
R_{d} is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅;
R₅ₐ and R_{5b} are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅, or, R₅ₐ and R_{5b}, together with the carbon atom to which they are directly attached, form a C(O), C₃₋₆ cycloalkyl, 4-6 membered heterocyclyl, C₆₋₈ aryl or 5-8 membered heteroaryl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
when p is 1, R_{5c} and R_{5d}, together with the carbon atom to which they are directly attached, form a C₃₋₆ cycloalkyl or 4-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, or,
when p is 2, one group of R_{5c} and R_{5d}, together with the carbon atom to which they are directly attached, form a C₃₋₆ cycloalkyl or 4-6 membered heterocyclyl, and when one group of R_{5c} and R_{5d}, together with the carbon atom to which they are directly attached, form a C₃₋₆ cycloalkyl or 4-6 membered heterocyclyl, the other group of R_{5c} and R_{5d} are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₅ₑ and R_{5f} are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅, or, R₅ₑ and R_{5f}, together with the carbon atom to which they are directly attached, form a C₃₋₆ cycloalkyl, 4-6 membered heterocyclyl, C₆₋₈ aryl or 5-8 membered heteroaryl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R_{5g} and R₅ₕ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅, or, R_{5g} and R₅ₕ, together with the carbon atom to which they are directly attached, form a C₃₋₆ cycloalkyl, 4-6 membered heterocyclyl, C₆₋₈ aryl or 5-8 membered heteroaryl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R_{6c} is selected from the group consisting of hydrogen, deuterium, C₁₋₄ alkyl, C₃₋₆ cycloalkyl, 3-6 membered heterocyclyl and -O-R₁₁, and the above groups are each independently optionally further substituted by one or more substituents selected from the group consisting of deuterium, halogen, cyano, hydroxy, =O, C₁₋₄ alkyl, C₁₋₄ haloalkyl, C₁₋₄ deuterioalkyl, C₂₋₄ alkenyl, C₂₋₄ alkynyl, C₁₋₄ alkoxy, C₃₋₆ cycloalkyl, C₃₋₆ cycloalkoxy, 3-6 membered heterocyclyl, 3-6 membered heterocyclyloxy and -NR₁₃R₁₄; and
each R₇ is independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₀, -C₀₋₈ alkyl-C(O)OR₁₁, -C₀₋₈ alkyl-C(O)R₁₂ or -C₀₋₈ alkyl-C(O)NR₁₃R₁₄, and the above groups are optionally further substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ or -C₀₋₈ alkyl-N(R₁₃)-C(O)R₁₂;
each R₈ and each R₉ are independently selected from the group consisting of hydrogen, deuterium, hydroxy, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl and 5-10 membered heteroaryl, or, R₈ and R₉, together with the sulfur atom to which they are directly attached, form a 3-10 membered heterocyclyl, and the above groups are optionally further substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂;
each R₁₀ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₁₃R₁₄, and the above groups are each independently optionally further substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₃R₁₄;
each R₁₁ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl and 5-10 membered heteroaryl, and the above groups are each independently optionally further substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₃R₁₄;
each R₁₂ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₃R₁₄, and the above groups are each independently optionally further substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₃R₁₄;
each R₁₃ and each R₁₄ are independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₁₀ alkanoyl, and the above groups are each independently optionally further substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, monoC₁₋₁₀ alkylamino, diC₁₋₁₀ alkylamino and C₁₋₁₀ alkanoyl; or
R₁₃ and R₁₄, together with the nitrogen atom to which they are directly attached, form a 4-10 membered heterocyclyl or 5-10 membered heteroaryl and the 4-10 membered heterocyclyl or 5-10 membered heteroaryl is optionally further substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, methanesulfonylmethyl, amino, monoC₁₋₁₀ alkylamino, diC₁₋₁₀ alkylamino and C₁₋₁₀ alkanoyl;
p is 1 or 2; and
each r is independently 0, 1 or 2.

As a further preferred embodiment, in the compound of formula (III), the stereoisomer or pharmaceutically acceptable salt thereof, the compound of formula (III) is a compound having formula (IV) as shown below: 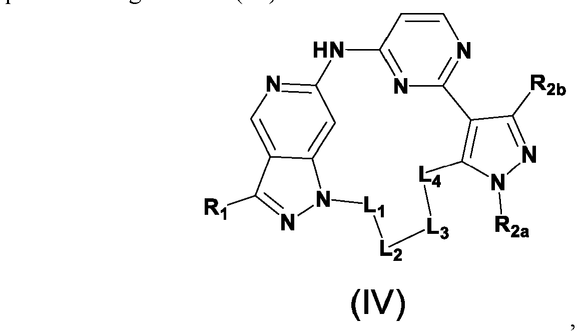
wherein, L₁ is CR₅ₐR_{5b}; L₂ is CR_{5c}R_{5d}; L₃ is CR₅ₑR_{5f}; L₄ is O;
R₁ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-8 membered heterocyclyl, C₆₋₈ aryl, 5-8 membered heteroaryl and -SF₅, and the above groups are each independently optionally further substituted by one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₁₋₄ alkyl substituted by C₃₋₆ cycloalkyl, C₁₋₄ alkyl substituted by 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -N(R₁₃)-S(O)₂R₁₀, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, and the above groups are each independently optionally further more substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -N(R₁₃)-S(O)₂R₁₀, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₂ₐ is selected from the group consisting of hydrogen, deuterium, hydroxy, C₁₋₄ alkyl, C₂₋₄ alkenyl, C₂₋₄ alkynyl, C₃₋₆ cycloalkyl and 3-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅;
R_{2b} is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl and -SF₅, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅;
R₅ₐ and R_{5b} are each independently selected from the group consisting of hydrogen, deuterium, halogen, cyano, C₁₋₄ alkyl, C₃₋₆ cycloalkyl and 3-6 membered heterocyclyl, or, R₅ₐ and R_{5b}, together with the carbon atom to which they are directly attached, form a C₃₋₆ cycloalkyl or 4-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅;
R_{5c} and R_{5d}, together with the carbon atom to which they are directly attached, form a C₃₋₆ cycloalkyl or 4-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅;
R₅ₑ and R_{5f} are each independently selected from the group consisting of hydrogen, deuterium, halogen, cyano, C₁₋₄ alkyl, C₃₋₆ cycloalkyl and 3-6 membered heterocyclyl, or, R₅ₑ and R_{5f}, together with the carbon atom to which they are directly attached , form a C₃₋₆ cycloalkyl or 4-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅; and
R₇, R₈, R₉, R₁₀, R₁₁, R₁₂, R₁₃, R₁₄ and r are as defined in the compound of formula (III).

As a more further preferred embodiment, in the compound of formula (III), the stereoisomer or pharmaceutically acceptable salt thereof, L₁ is CR₅ₐR_{5b}; L₂ is CR₅ₑR_{5d}; L₃ is CR₅ₑR_{5f}; L₄ is O;
R₅ₐ and R_{5b} are each independently selected from the group consisting of hydrogen, deuterium, fluorine, chlorine, bromine, cyano, methyl, ethyl, propyl, isopropyl, cyclopropyl, cyclobutyl, oxacyclopropyl, oxacyclobutyl, azacyclopropyl and azacyclobutyl, and the above groups are each independently optionally further substituted by one or more substituents selected from the group consisting of deuterium, fluorine, chlorine, bromine, cyano, methyl, ethyl, propyl, isopropyl, monofluoromethyl, difluoromethyl, trifluoromethyl, monodeuteriomethyl, dideuteriomethyl, trideuteriomethyl, vinyl, ethynyl, cyclopropyl, cyclobutyl, oxacyclopropyl, oxacyclobutyl, azacyclopropyl, azacyclobutyl, =O, =S and -SF₅;
R_{5c} and R_{5d}, together with the carbon atom to which they are directly attached, form a C₃₋₆ cycloalkyl or 4-6 membered heterocyclyl, and the above groups are each independently optionally further substituted by one or more substituents selected from the group consisting of deuterium, fluorine, chlorine, bromine, cyano, methyl, ethyl, propyl, isopropyl, monofluoromethyl, difluoromethyl, trifluoromethyl, monodeuteriomethyl, dideuteriomethyl, trideuteriomethyl, vinyl, ethynyl, cyclopropyl, cyclobutyl, oxacyclopropyl, oxacyclobutyl, azacyclopropyl, azacyclobutyl, =O, =S and -SF₅; and
R₅ₑ and R_{5f} are each independently selected from the group consisting of hydrogen, deuterium, fluorine, chlorine, bromine, cyano, methyl, ethyl, propyl, isopropyl, cyclopropyl, cyclobutyl, oxacyclopropyl, oxacyclobutyl, azacyclopropyl and azacyclobutyl, and the above groups are each independently optionally further substituted by one or more substituents selected from the group consisting of deuterium, fluorine, chlorine, bromine, cyano, methyl, ethyl, propyl, isopropyl, monofluoromethyl, difluoromethyl, trifluoromethyl, monodeuteriomethyl, dideuteriomethyl, trideuteriomethyl, vinyl, ethynyl, cyclopropyl, cyclobutyl, oxacyclopropyl, oxacyclobutyl, azacyclopropyl, azacyclobutyl, =O, =S and -SF₅.

As a more further preferred embodiment, in the compound of formula (III), the stereoisomer or pharmaceutically acceptable salt thereof, R₂ₐ is selected from the group consisting of hydrogen, deuterium, hydroxy, methyl, ethyl, propyl, isopropyl, vinyl, ethynyl, cyclopropyl, cyclobutyl, oxacyclopropyl, oxacyclobutyl, azacyclopropyl and azacyclobutyl, and the above groups are each independently optionally further substituted by one or more substituents selected from the group consisting of deuterium, fluorine, chlorine, bromine, cyano, methyl, ethyl, propyl, isopropyl, monofluoromethyl, difluoromethyl, trifluoromethyl, monodeuteriomethyl, dideuteriomethyl, trideuteriomethyl, vinyl, ethynyl, cyclopropyl, cyclobutyl, oxacyclopropyl, oxacyclobutyl, azacyclopropyl, azacyclobutyl, =O, =S and -SF₅; and
R_{2b} is selected from the group consisting of hydrogen, deuterium, fluorine, chlorine, bromine, cyano, methyl, ethyl, propyl, isopropyl, vinyl, ethynyl, cyclopropyl, cyclobutyl, oxacyclopropyl, oxacyclobutyl, azacyclopropyl, azacyclobutyl and -SF₅, and the above groups are each independently optionally further substituted by one or more substituents selected from the group consisting of deuterium, fluorine, chlorine, bromine, cyano, methyl, ethyl, propyl, isopropyl, monofluoromethyl, difluoromethyl, trifluoromethyl, monodeuteriomethyl, dideuteriomethyl, trideuteriomethyl, vinyl, ethynyl, cyclopropyl, cyclobutyl, oxacyclopropyl, oxacyclobutyl, azacyclopropyl, azacyclobutyl, =O, =S and -SF₅.

As a further preferred embodiment, in the compound of formula (III), the stereoisomer or pharmaceutically acceptable salt thereof, the compound of formula (III) is a compound having formula (V) as shown below: 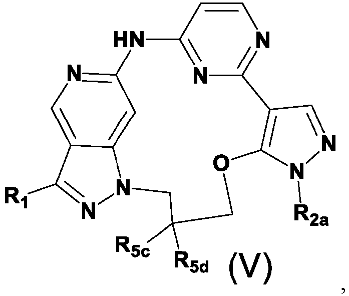
wherein, R₁ is selected from C₂₋₄ alkenyl, C₂₋₄ alkynyl and 3-8 membered heterocyclyl, and the above groups are each independently optionally further substituted by one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₁₋₄ alkyl substituted by C₃₋₆ cycloalkyl, C₁₋₄ alkyl substituted by 3-6 membered heterocyclyl, =O, =S, -SF₅, -N(R₁₃)-S(O)₂R₁₀, -O-S(O)₂R₁₀, -S(O)ᵣR₁₀, -O-R₁₁, -C(O)OR₁₁, -S-C(O)R₁₂, -C(O)R₁₂, -O-C(O)R₁₂, -NR₁₃R₁₄, -C(O)NR₁₃R₁₄ and -N(R₁₃)-C(O)R₁₂, and the above groups are each independently optionally further more substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -N(R₁₃)-S(O)₂R₁₀, -O-S(O)₂R₁₀, -S(O)ᵣR₁₀, -O-R₁₁, -C(O)OR₁₁, -C(O)R₁₂, -O-C(O)R₁₂, -NR₁₃R₁₄, -C(O)NR₁₃R₁₄ and -N(R₁₃)-C(O)R₁₂;
R₂ₐ is selected from the group consisting of hydrogen, deuterium, C₁₋₄ alkyl, C₃₋₆ cycloalkyl and 3-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₃₋₆ cycloalkyl and 3-6 membered heterocyclyl;
R_{5c} and R_{5d}, together with the carbon atom to which they are directly attached, form a C₃₋₆ cycloalkyl or 4-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅;
R₁₀, R₁₁, R₁₂, R₁₃, R₁₄ and r are as defined in the compound of formula (III).

As a more further preferred embodiment, in the compound of formula (III), the stereoisomer or pharmaceutically acceptable salt thereof, R_{5c} and R_{5d}, together with the carbon atom to which they are directly attached, form a C₃₋₆ cycloalkyl or 4-6 membered heterocyclyl.

As a more further preferred embodiment, in the compound of formula (III), the stereoisomer or pharmaceutically acceptable salt thereof, R_{5c} and R_{5d}, together with the carbon atom to which they are directly attached, form a cyclopropyl, cyclobutyl, cyclopentyl, cyclohexyl, oxacyclobutyl, oxacyclopentyl or oxacyclohexyl.

As a more further preferred embodiment, in the compound of formula (III), the stereoisomer or pharmaceutically acceptable salt thereof, R₁ is C₂₋₄ alkynyl, and the C₂₋₄ alkynyl is optionally further substituted by one or more substituents selected from the group consisting of deuterium, C₁₋₄ alkyl, C₃₋₆ cycloalkyl and 3-6 membered heterocyclyl, and the above groups are each independently optionally further more substituted by one or more substituents selected from the group consisting of deuterium, halogen, cyano, C₁₋₄ alkyl, C₁₋₄ haloalkyl, C₁₋₄ deuterioalkyl, C₃₋₆ cycloalkyl, 3-6 membered heterocyclyl.

As a more further preferred embodiment, in the compound of formula (III), the stereoisomer or pharmaceutically acceptable salt thereof, R₁ is ethynyl, and the ethynyl is optionally further substituted by one or more substituents selected from the group consisting of deuterium, methyl, ethyl, propyl, isopropyl, butyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl, and the above groups are each independently optionally further more substituted by one or more substituents selected from the group consisting of deuterium, fluorine, bromine, cyano, methyl, ethyl, propyl, isopropyl, butyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl.

As a more further preferred embodiment, in the compound of formula (III), the stereoisomer or pharmaceutically acceptable salt thereof, R₁ is ethynyl, and the ethynyl is optionally further substituted by one or more substituents selected from the group consisting of cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl, and the above groups are each independently optionally further more substituted by one or more substituents selected from the group consisting of deuterium, fluorine, bromine, cyano, methyl, ethyl, propyl, isopropyl, monofluoromethyl, difluoromethyl, trifluoromethyl, monodeuteriomethyl, dideuteriomethyl and trideuteriomethyl.

As a more further preferred embodiment, in the compound of formula (III), the stereoisomer or pharmaceutically acceptable salt thereof, R₁ is 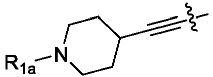 or 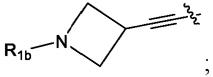
R₁ₐ is selected from hydrogen, methyl, ethyl, propyl, isopropyl, monofluoromethyl, difluoromethyl, trifluoromethyl, monodeuteriomethyl, dideuteriomethyl and trideuteriomethyl; and
R_{1b} is selected from hydrogen, methyl, ethyl, propyl, isopropyl, monofluoromethyl, difluoromethyl, trifluoromethyl, monodeuteriomethyl, dideuteriomethyl and trideuteriomethyl.

As a more further preferred embodiment, in the compound of formula (III), the stereoisomer or pharmaceutically acceptable salt thereof, R₁ is 4-8 membered nitrogen-containing heterocyclyl, and the 4-8 membered nitrogen-containing heterocyclyl is optionally further substituted by one or more substituents selected from the group consisting of deuterium, halogen, cyano, C₁₋₄ alkyl, =O, -N(R₁₃)-S(O)₂R₁₀, -S(O)ᵣR₁₀, -O-R₁₁, -O-C(O)R₁₂, -NR₁₃R₁₄ and -N(R₁₃)-C(O)R₁₂, and the C₁₋₄ alkyl is more optionally further substituted by one or more substituents selected from the group consisting of deuterium, halogen, cyano, C₁₋₄ alkyl, C₁₋₄ haloalkyl, C₁₋₄ deuterioalkyl, -N(R₁₃)-S(O)₂R₁₀, -O-S(O)₂R₁₀, -S(O)ᵣR₁₀, -O-R₁₁, -O-C(O)R₁₂, -NR₁₃R₁₄ and -N(R₁₃)-C(O)R₁₂; and
R₁₀, R₁₁, R₁₂, R₁₃, R₁₄ and r are as defined in the compound of formula (III).

As a more further preferred embodiment, in the compound of formula (III), the stereoisomer or pharmaceutically acceptable salt thereof, R₁ is of the following structure: 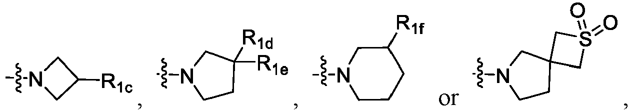 wherein,
R_{1c} is selected from the group consisting of hydrogen, deuterium, halogen, C₁₋₄ alkyl, -N(R₁₃ₐ)-S(O)₂R₁₀ₐ, -O-R₁₁ₐ and -N(R₁₃ₐ)-C(O)R₁₂ₐ, and the C₁₋₄ alkyl is more optionally further substituted by one or more substituents selected from the group consisting of deuterium, C₁₋₄ alkyl, C₁₋₄ haloalkyl, C₁₋₄ deuterioalkyl, -S(O)ᵣR_{10b} and -O-R₁₁ₐ;
R_{1d} is selected from the group consisting of hydrogen, deuterium, halogen, C₁₋₄ alkyl, -N(R₁₃ₐ)-S(O)₂R₁₀ₐ, -O-R₁₁ₐ and -N(R₁₃ₐ)-C(O)R₁₂ₐ;
R₁ₑ is selected from the group consisting of hydrogen, deuterium, halogen, C₁₋₄ alkyl, -N(R₁₃ₐ)-S(O)₂R₁₀ₐ, -O-R₁₁ₐ and -N(R₁₃ₐ)-C(O)R₁₂ₐ, and the C₁₋₄ alkyl is more optionally further substituted by one or more substituents selected from the group consisting of deuterium, C₁₋₄ alkyl, C₁₋₄ haloalkyl, C₁₋₄ deuterioalkyl, -S(O)ᵣR_{10b} and -O-R₁₁ₐ;
R_{1f} is selected from the group consisting of hydrogen, deuterium, halogen, C₁₋₄ alkyl, -N(R₁₃ₐ)-S(O)₂R₁₀ₐ, -O-R₁₁ₐ and -N(R₁₃ₐ)-C(O)R₁₂ₐ, and the C₁₋₄ alkyl is more optionally further substituted by one or more substituents selected from the group consisting of deuterium, C₁₋₄ alkyl, C₁₋₄ haloalkyl, C₁₋₄ deuterioalkyl, -S(O)ᵣR_{10b} and -O-R₁₁ₐ;
each R₁₀ₐ is independently selected from the group consisting of hydrogen, C₁₋₄ alkyl, C₃₋₆ cycloalkyl and 3-6 membered heterocyclyl;
each R_{10b} is independently selected from the group consisting of hydrogen, C₁₋₄ alkyl, C₃₋₆ cycloalkyl and 3-6 membered heterocyclyl, and the above groups are each independently optionally further substituted by one or more substituents selected from the group consisting of deuterium, halogen, hydroxy, C₁₋₄ alkyl and C₁₋₄ alkoxy;
each R₁₁ₐ is independently hydrogen or C₁₋₄ alkyl;
each R₁₂ₐ is independently selected from the group consisting of hydrogen, C₁₋₄ alkyl, C₁₋₄ alkoxy and C₃₋₆ cycloalkyl, and the above groups are each independently optionally further substituted by one or more substituents selected from the group consisting of deuterium, halogen, hydroxy and cyano;
each R₁₃ₐ is independently hydrogen or C₁₋₄ alkyl; and
each r is independently 0, 1 or 2.

As a more further preferred embodiment, in the compound of formula (III), the stereoisomer or pharmaceutically acceptable salt thereof, R₁ is of the following structure: 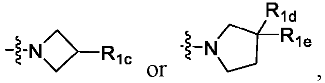
wherein, R_{1c} is selected from the group consisting of hydrogen, deuterium, fluorine, bromine, methyl, ethyl, propyl, isopropyl, butyl, -N(R₁₃ₐ)-S(O)₂R₁₀ₐ, -O-R₁₁ₐ and -N(R₁₃ₐ)-C(O)R₁₂ₐ, and the methyl, ethyl, propyl, isopropyl or butyl is more independently optionally further substituted by one or more substituents selected from the group consisting of deuterium, methyl, ethyl, propyl, isopropyl, butyl, monofluoromethyl, difluoromethyl, trifluoromethyl, monodeuteriomethyl, dideuteriomethyl, trideuteriomethyl, -S(O)ᵣR_{10b} and -O-R₁₁ₐ;
R_{1d} is selected from the group consisting of hydrogen, deuterium, fluorine, bromine, methyl, ethyl, propyl, isopropyl and butyl;
R₁ₑ is selected from the group consisting of hydrogen, deuterium, fluorine, bromine, methyl, ethyl, propyl, isopropyl, butyl, -N(R₁₃ₐ)-S(O)₂R₁₀ₐ, -O-R₁₁ₐ and -N(R₁₃ₐ)-C(O)R₁₂ₐ, and the methyl, ethyl, propyl, isopropyl or butyl is more independently optionally further substituted by one or more substituents selected from the group consisting of deuterium, methyl, ethyl, propyl, isopropyl, butyl, monofluoromethyl, difluoromethyl, trifluoromethyl, monodeuteriomethyl, dideuteriomethyl, trideuteriomethyl, -S(O)ᵣR_{10b} and -O-R₁₁ₐ;
each R₁₀ₐ is independently selected from the group consisting of hydrogen, methyl, ethyl, propyl, isopropyl, butyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl;
each R_{10b} is independently selected from the group consisting of hydrogen, methyl, ethyl, propyl, isopropyl, butyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl, and the above groups are each independently optionally further substituted by one or more substituents selected from the group consisting of deuterium, fluorine, chlorine, hydroxy, methyl, ethyl, propyl, isopropyl, butyl, methoxy, ethoxy and propoxy;
each R₁₁ₐ is independently selected from the group consisting of hydrogen, methyl, ethyl, propyl, isopropyl and butyl;
each R₁₂ₐ is independently selected from the group consisting of hydrogen, methyl, ethyl, propyl, isopropyl, butyl, methoxy, ethoxy, propoxy, tert-butoxy, cyclopropyl, cyclobutyl, cyclopentyl and cyclohexyl, and the above groups are each independently optionally further substituted by one or more substituents selected from the group consisting of deuterium, fluorine, chlorine, hydroxy and cyano;
each R₁₃ₐ is independently hydrogen; and
each r is independently 0, 1 or 2.

As a more further preferred embodiment, in the compound of formula (III), the stereoisomer or pharmaceutically acceptable salt thereof, R₁ is of the following structure: 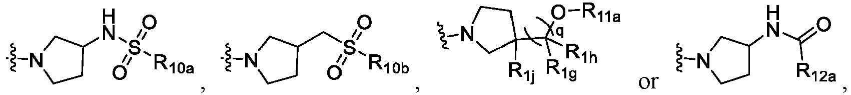 wherein,
R_{1g} is hydrogen, deuterium or methyl; R₁ₕ is hydrogen, deuterium or methyl; R₁ⱼ is hydrogen, deuterium or methyl; q is 0, 1 or 2;
R₁₀ₐ is selected from the group consisting of hydrogen, methyl, ethyl, propyl, isopropyl, butyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl;
R_{10b} is selected from the group consisting of hydrogen, methyl, ethyl, propyl, isopropyl, butyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl, and the above groups are each independently optionally further substituted by one or more substituents selected from the group consisting of deuterium, fluorine, chlorine, hydroxy, methyl, ethyl, propyl, isopropyl, butyl, methoxy, ethoxy and propoxy;
each R₁₁ₐ is independently selected from the group consisting of hydrogen, methyl, ethyl, propyl, isopropyl and butyl;
R₁₂ₐ is selected from the group consisting of hydrogen, methyl, ethyl, propyl, isopropyl, butyl, methoxy, ethoxy, propoxy, tert-butoxy, cyclopropyl, cyclobutyl, cyclopentyl and cyclohexyl, and the above groups are each independently optionally further substituted by one or more substituents selected from the group consisting of deuterium, fluorine, chlorine, hydroxy and cyano.

As a more further preferred embodiment, in the compound of formula (III), the stereoisomer or pharmaceutically acceptable salt thereof, R₂ₐ is selected from the group consisting of hydrogen, deuterium, C₁₋₄ alkyl, C₁₋₄ haloalkyl, C₁₋₄ deuterioalkyl, C₃₋₆ cycloalkyl and 3-6 membered heterocyclyl.

As a more further preferred embodiment, in the compound of formula (III), the stereoisomer or pharmaceutically acceptable salt thereof, R₂ₐ is selected from the group consisting of hydrogen, deuterium, methyl, ethyl, propyl, isopropyl, butyl, monofluoromethyl, difluoromethyl, trifluoromethyl, monodeuteriomethyl, dideuteriomethyl, trideuteriomethyl, cyclopropyl, cyclobutyl, oxacyclopropyl, oxacyclobutyl, azacyclopropyl and azacyclobutyl.

As the most preferred embodiment, the compound of formula (III), the stereoisomer or pharmaceutically acceptable salt thereof includes, but is not limited to, the following compounds: 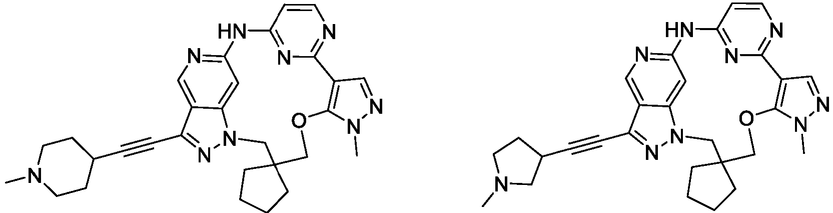 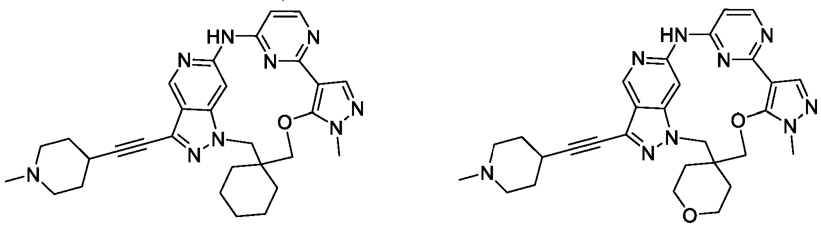 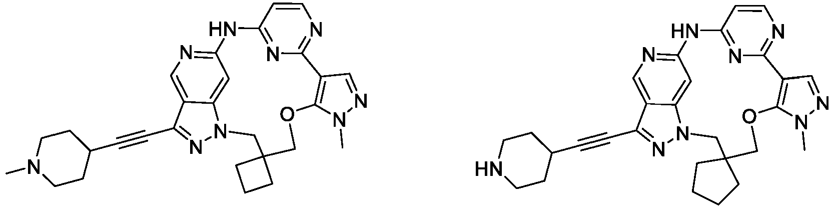 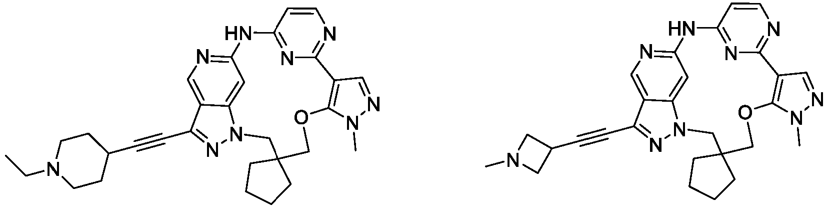 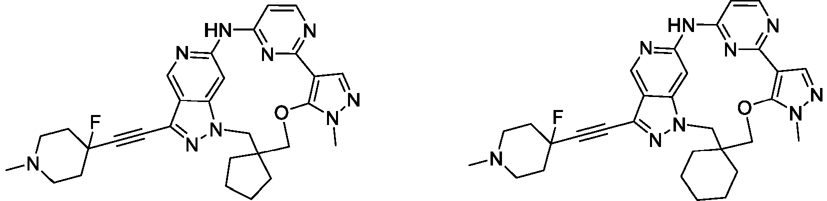 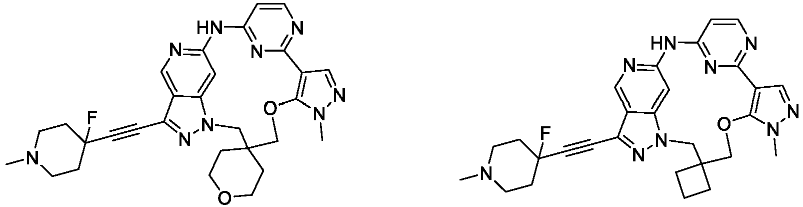 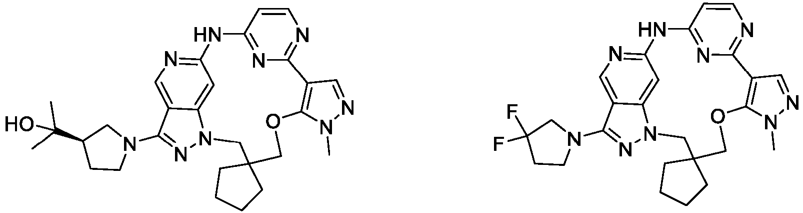 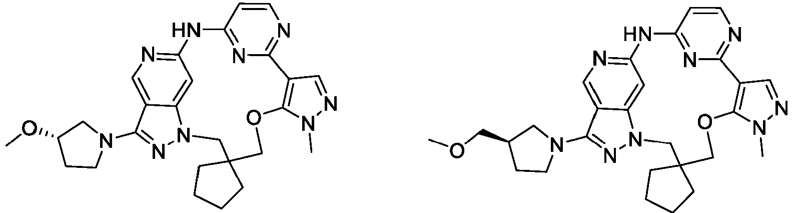 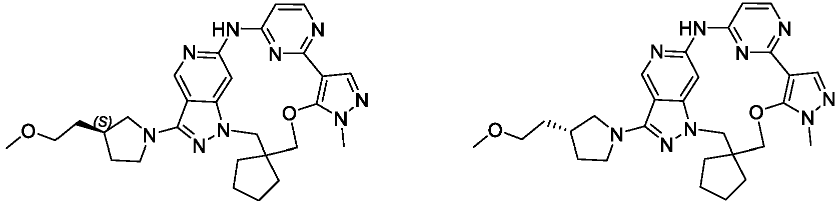 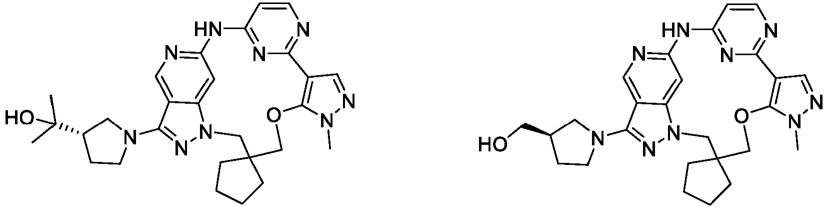 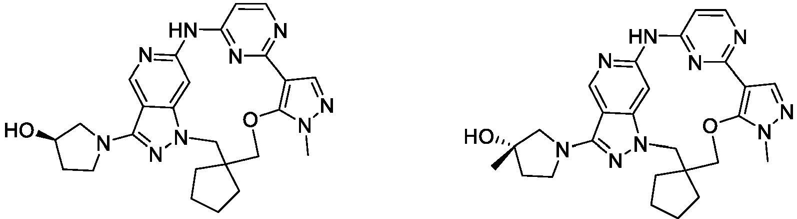 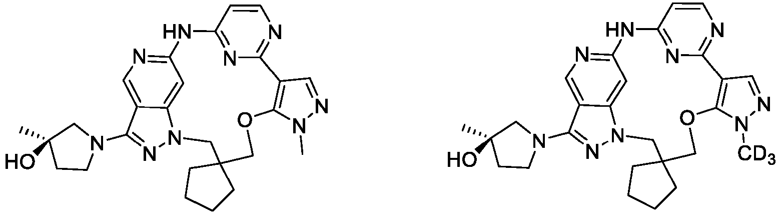 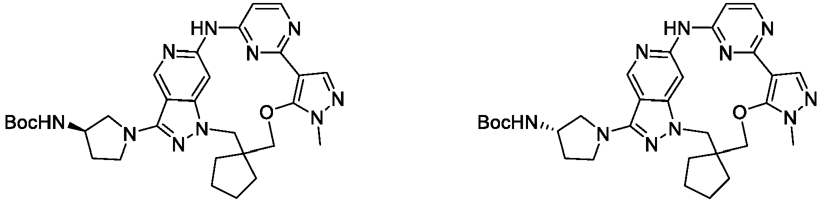 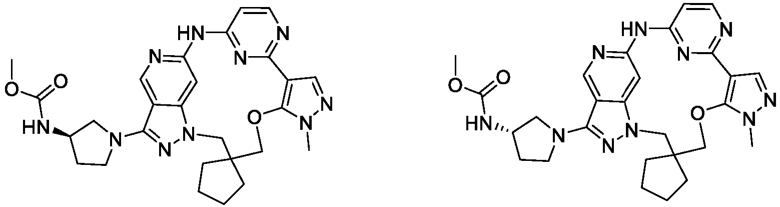 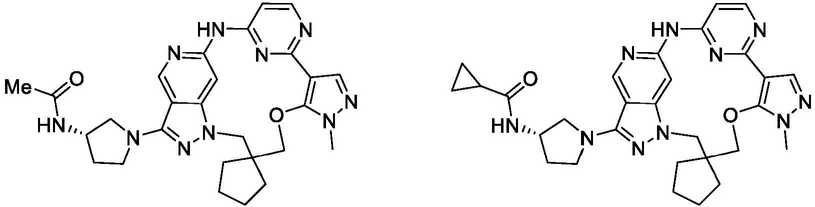 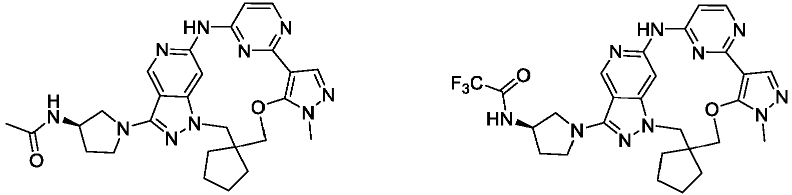 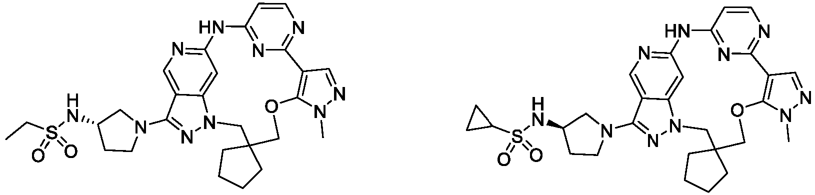 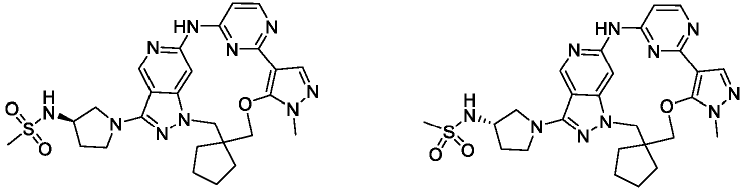 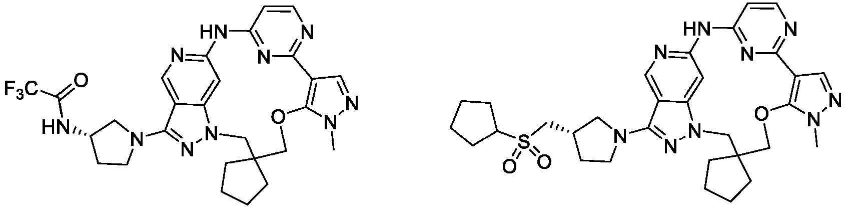 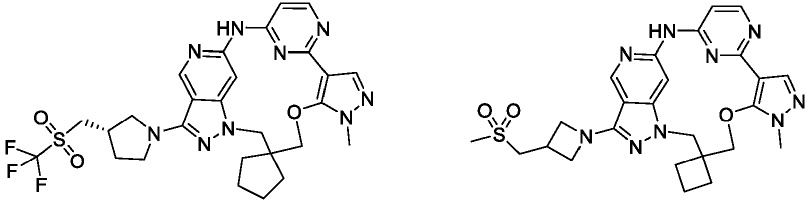 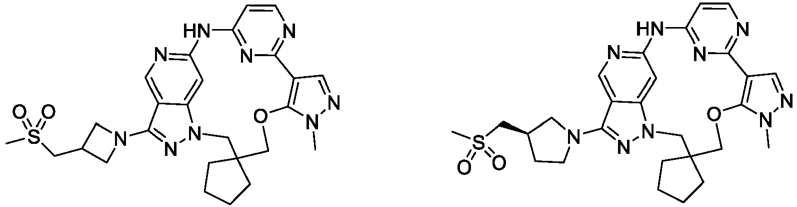 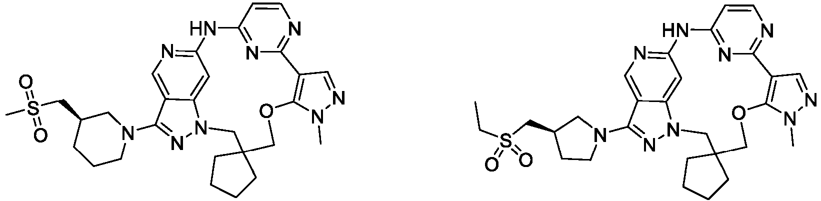 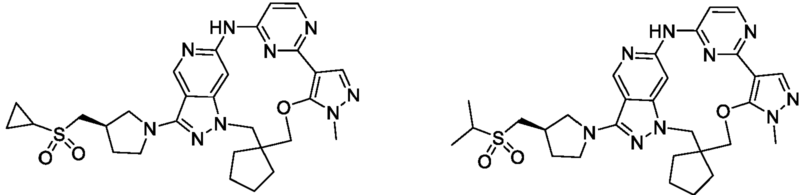 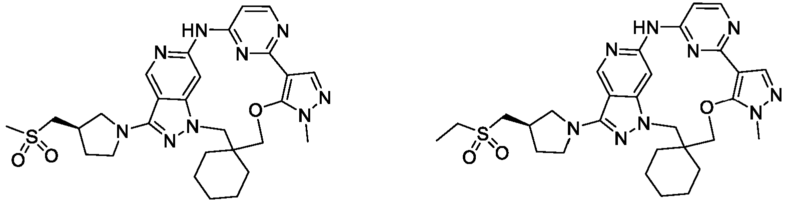 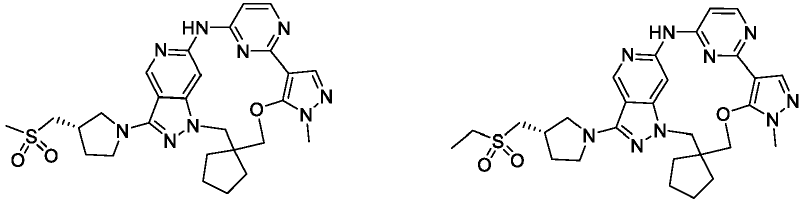 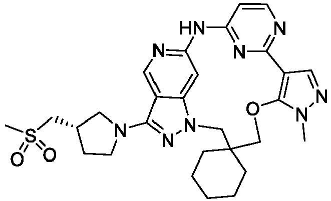 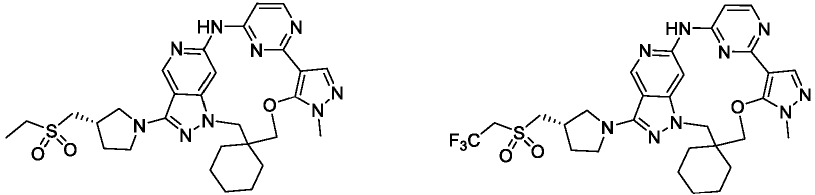 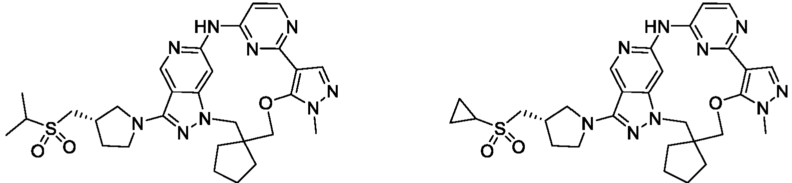 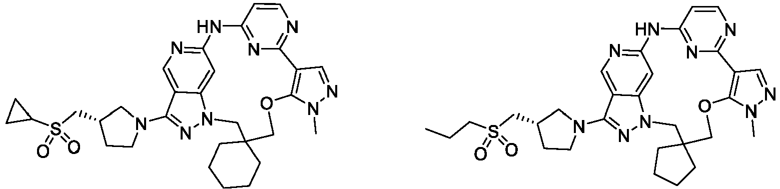 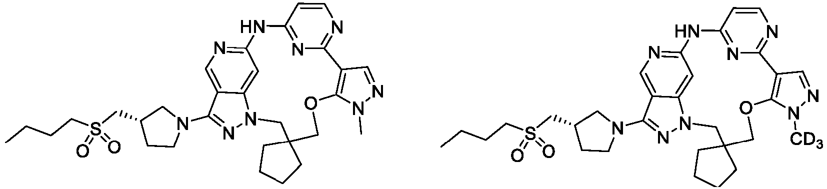 and

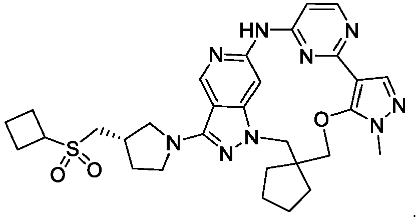

Also disclosed herein is a process for preparing the compound of formula (III), the stereoisomer or pharmaceutically acceptable salt thereof, comprising the following step:

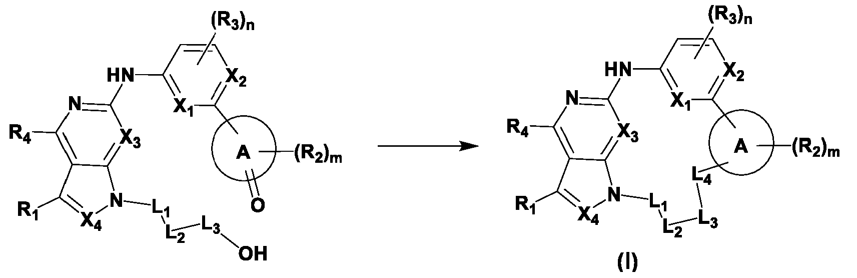

Ring A, X₁, X₂, X₃, X₄, L₁, L₂, L₃, L₄, R₁, R₂, R₃, R₄, m and n are as defined in the compound of formula (III).

The third aspect of the present invention provides a pharmaceutical composition comprising the compound of formula (III), the stereoisomer or pharmaceutically acceptable salt thereof, and a pharmaceutically acceptable carrier.

The present invention also relates to use of the compound of formula (III), the stereoisomer or pharmaceutically acceptable salt thereof in the preparation of a medicament for treating and/or preventing tumors or metastatic diseases associated at least partially with EGFR L858R/C797S double mutation, EGFR Del19/C797S double mutation, L858R/T790M/C797S triple mutation and Del19/T790M/C797S triple mutation.

The present invention also relates to a use of the compound of formula (III), the stereoisomer or pharmaceutically acceptable salt thereof in the preparation of drugs for preventing and/or treating tumors and/or metastatic diseases caused by hyperproliferation diseases and dysfunction in cell death induction.

The present invention also relates to a use of the aforementioned compound of formula (III), the stereoisomer or pharmaceutically acceptable salt thereof in the preparation of drugs for preventing and/or treating lung cancer, colon cancer, pancreatic cancer, head and neck cancer, breast cancer, ovarian cancer, uterine cancer, gastric cancer, non-small cell lung cancer, leukemia, myelodysplastic syndrome, malignant lymphoma, head and neck tumor, thoracic tumor, gastrointestinal tumor, endocrine tumor, breast and other gynecological tumor, urological tumor, skin tumor, sarcoma, sinonasal inverted papilloma or sinonasal squamous cell carcinoma associated with sinonasal inverted papilloma, all of which are associated at least partially with EGFR L858R/C797S double mutation, EGFR Del19/C797S double mutation, L858R/T790M/C797S triple mutation and Del19/T790M/C797S triple mutation.

The present invention also relates to the compound of formula (III), the stereoisomer or pharmaceutically acceptable salt thereof, for use as a drug.

The present invention also relates to the compound of formula (III), the stereoisomer or pharmaceutically acceptable salt thereof, for use in treating and/or preventing tumors or metastatic diseases associated at least partially with EGFR L858R/C797S double mutation, EGFR Del19/C797S double mutation, L858R/T790M/C797S triple mutation and Del19/T790M/C797S triple mutation.

The present invention also relates to the compound of formula (III), the stereoisomer or pharmaceutically acceptable salt thereof, for use in preventing and/or treating tumors and/or metastatic diseases caused by hyperproliferation diseases and disorders inducing cell death.

The present invention also relates to the compound of formula (III), the stereoisomer or pharmaceutically acceptable salt thereof, for use in treating and/or preventing lung cancer, colon cancer, pancreatic cancer, head and neck cancer, breast cancer, ovarian cancer, uterine cancer, gastric cancer, non-small cell lung cancer, leukemia, myelodysplastic syndrome, malignant lymphoma, head and neck tumor, thoracic tumor, gastrointestinal tumor, endocrine tumor, breast and other gynecological tumor, urological tumor, skin tumor, sarcoma, sinonasal inverted papilloma or sinonasal squamous cell carcinoma associated with inverted papilloma of the sinonasal cavity, all of which are associated at least partially with EGFR L858R/C797S double mutation, EGFR Del19/C797S double mutation, L858R/T790M/C797S triple mutation and Del19/T790M/C797S triple mutation.

Also disclosed herein is a method for treating and/or preventing tumors or metastatic diseases associated at least partially with EGFR L858R/C797S double mutation, EGFR Del19/C797S double mutation, L858R/T790M/C797S triple mutation and Del19/T790M/C797S triple mutation, comprising administering to a patient in need a therapeutically effective amount of the compound of formula (III), the stereoisomer or pharmaceutically acceptable salt thereof.

Further disclosed herein is a method for preventing and/or treating tumors and/or metastatic diseases caused by hyperproliferation diseases and disorders inducing cell death, comprising administering to a patient in need a therapeutically effective amount of the compound of formula (III), the stereoisomer or pharmaceutically acceptable salt thereof.

Also disclosed herein is a a method for treating and/or preventing lung cancer, colon cancer, pancreatic cancer, head and neck cancer, breast cancer, ovarian cancer, uterine cancer, gastric cancer, non-small cell lung cancer, leukemia, myelodysplastic syndrome, malignant lymphoma, head and neck tumor, thoracic tumor, gastrointestinal tumor, endocrine tumor, breast and other gynecological tumor, urological tumor, skin tumor, sarcoma, inverted papilloma of the sinonasal cavity or squamous cell carcinoma of the sinonasal cavity associated with inverted papilloma of the sinonasal cavity, all of which are associated at least partially with EGFR L858R/C797S double mutation, EGFR Del19/C797S double mutation, L858R/T790M/C797S triple mutation and Del19/T790M/C797S triple mutation, comprising administering to a patient in need a therapeutically effective amount of the compound of formula (I), the stereoisomer or pharmaceutically acceptable salt thereof.

### DETAILED DESCRIPTION OF EMBODIMENTS

After an extensive and intensive research, the inventors of the present invention develop an EGFR inhibitor with the structure of formula (III) for the first time. The series of compounds of the present invention can be widely used in the preparation of drugs for treating and/or preventing tumors or metastatic diseases associated at least partially with EGFR L858R/C797S double mutation, EGFR Del19/C797S double mutation, L858R/T790M/C797S triple mutation and Del19/T790M/C797S triple mutation, and are expected to be developed into a new-generation EGFR inhibitor. The present invention is achieved on this basis.

Detailed description: unless otherwise stated or specified, the following terms used in the specification and claims have the following meanings.

"Alkyl" refers to linear or branched saturated aliphatic alkyl groups, preferably including a linear or branched alkyl having 1 to 10 or 1 to 6 carbon atoms or 1 to 4 carbon atoms, including but not limited to methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, etc. "C₁₋₁₀ alkyl" means a linear or branched alkyl containing 1 to 10 carbon atoms, "C₁₋₄ alkyl" means a linear or branched alkyl containing 1 to 4 carbon atoms, "C₀₋₈ alkyl" means a linear or branched alkyl containing 0 to 8 carbon atoms, and "C₀₋₄ alkyl" means a linear or branched alkyl containing 0 to 4 carbon atoms.

Alkyl can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₇)R₈, -C₀₋₈ alkyl-N=S(O)R₈R₉, -C₀₋₈ alkyl-N=SR₈R₉, -C₀₋₈ alkyl-O-S(O)₂R₁₀, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)SR₁₁, -C₀₋₈ alkyl-S-C(O)R₁₂, -C₀₋₈ alkyl-C(O)R₁₂, -C₀₋₈ alkyl-O-C(O)R₁₂, -C₀₋₈ alkyl-P(O)(R₁₂)₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Cycloalkyl" or "carbocyclic ring" refers to monocyclic or polycyclic hydrocarbon substituents that are saturated or partially unsaturated, wherein the partially unsaturated cyclic hydrocarbon refers to a cyclic hydrocarbon that may contain one or more (preferably 1, 2 or 3) double bonds, but none of the rings have a fully conjugated π-electron system. Cycloalkyl may be monocyclic cycloalkyl or polycyclic cycloalkyl, preferably including a cycloalkyl containing 3 to 12 or 3 to 8 or 3 to 6 carbon atoms. For example, "C₃₋₁₂ cycloalkyl" means a cycloalkyl having 3 to 12 carton atoms, "C₄₋₁₂ cycloalkyl" means a cycloalkyl having 4 to 12 carton atoms, "C₄₋₈ cycloalkyl" means a cycloalkyl having 4 to 8 carton atoms, "C₃₋₈ cycloalkyl" means a cycloalkyl having 3 to 8 carton atoms, "C₃₋₆ cycloalkyl" means a cycloalkyl having 3 to 6 carton atoms, and "C₄₋₆ cycloalkyl" means a cycloalkyl having 4 to 6 carton atoms, wherein:
Monocyclic cycloalkyl includes but is not limited to cyclopropyl, cyclobutyl, cyclopentyl, cyclopentenyl, cyclohexyl, cyclohexenyl, cyclohexadienyl, cycloheptyl, cycloheptarienyl, cyclooctyl, etc.

Polycyclic cycloalkyl includes spirocycloalkyl, fused cycloalkyl and bridged cycloalkyl. "Spirocycloalkyl" refers to a polycyclic group in which a carton atom (called spiro-atom) is shared among monocyclic rings, wherein these rings may contain one or more (preferably 1, 2 or 3) double bonds, but none of them have a fully conjugated π-electron system. According to the number of the spiro-atoms shared among the rings, the spirocycloalkyl may be monospirocycloalkyl, bispirocycloalkyl or polyspirocycloalkyl, including but not limited to:

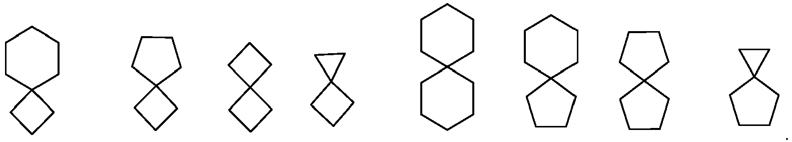

"Fused cycloalkyl" refers to an all-carbon polycyclic group in which each ring shares a pair of adjacent carbon atoms with other rings in the system, wherein one or more of the rings may contain one or more (preferably 1, 2 or 3) double bonds, but none of them have a fully conjugated π-electron system. According to the number of formed rings, the fused cycloalkyl may be bicyclic, tricyclic, tetracyclic or polycyclic, including but not limited to:

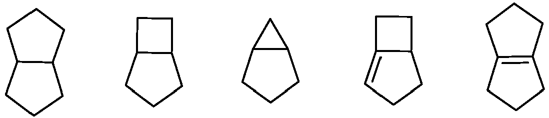

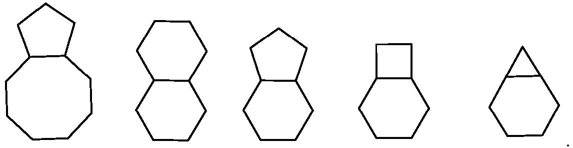

"Bridged cycloalkyl" refers to an all-carbon polycyclic group in which any two rings share two carbon atoms that are not directly connected to each other, wherein these rings may contain one or more (preferably 1, 2 or 3) double bonds, but none of them have a fully conjugated π-electron system. According to the number of formed rings, the bridged cycloalkyl may be bicyclic, tricyclic, tetracyclic or polycyclic, including but not limited to:

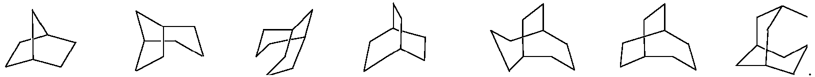

The cycloalkyl ring may be fused to an aryl, heteroaryl or heterocycloalkyl ring, wherein the ring attached to the parent structure is cycloalkyl, which includes, but is not limited to, indenyl, tetrahydronaphthyl, benzocycloheptyl, etc.

Cycloalkyl can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₇)R₈, -C₀₋₈ alkyl-N=S(O)R₈R₉, -C₀₋₈ alkyl-N=SR₈R₉, -C₀₋₈ alkyl-O-S(O)₂R₁₀, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)SR₁₁, -C₀₋₈ alkyl-S-C(O)R₁₂, -C₀₋₈ alkyl-C(O)R₁₂, -C₀₋₈ alkyl-O-C(O)R₁₂, -C₀₋₈ alkyl-P(O)(R₁₂)₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Heterocyclyl" or "heterocycle" refers to a monocyclic or polycyclic hydrocarbon substituent that is saturated or partially unsaturated, wherein the partially unsaturated cyclic hydrocarbon refers to a cyclic hydrocarbon that may contain one or more (preferably 1, 2 or 3) double bonds, but none of the rings have a fully conjugated π-electron system, wherein one or more (preferably 1, 2, 3 or 4) of the rings atoms in heterocyclyl are heteroatoms selected from N, O, N•O or S(O)ᵣ (wherein r is an integer of 0, 1 or 2), excluding ring portions of -O-O-, -O-S- or -S-S-, and the remaining ring atoms are carton atoms. It preferably includes heterocyclyl containing 3 to 12 or 3 to 8 or 3 to 6 ring atoms. For example, "3-6 membered heterocyclyl" means a heterocyclyl containing 3 to 6 ring atoms, "3-8 membered heterocyclyl" means a heterocyclyl containing 3 to 8 ring atoms, "4-6 membered heterocyclyl" means a heterocyclyl containing 4 to 6 ring atoms, "4-8 membered heterocyclyl" means a heterocyclyl containing 4 to 8 ring atoms, "4-10 membered heterocyclyl" means a heterocyclyl containing 4 to 10 ring atoms, "4-12 membered heterocyclyl" means a heterocyclyl containing 4 to 12 ring atoms, "5-8 membered heterocyclyl" means a heterocyclyl containing 5 to 8 ring atoms, and "3-12 membered heterocyclyl" means a heterocyclyl containing 3 to 12 ring atoms.

Monocyclic heterocyclyl includes but is not limited to pyrrolidinyl, piperidinyl, piperazinyl, morpholinyl, thiomorpholinyl, homopiperazinyl, oxacyclobutyl, tetrahydrofuranyl, etc.

Polycyclic heterocyclyl includes spiroheterocyclyl, fused heterocyclyl and bridged heterocyclyl. "Spiroheterocyclyl" refers to a polycyclic heterocyclyl group in which an atom (called spiro-atom) is shared among monocyclic rings, wherein one or more (preferably 1, 2, 3 or 4) of the ring atoms are heteroatoms selected from N, O, N•O or S(O)ᵣ (wherein r is an integer of 0, 1 or 2), and the remaining ring atoms are carbon atoms. These rings may contain one or more (preferably 1, 2 or 3) double bonds, but none of them have a fully conjugated π-electronic system. According to the number of sprio-atoms shared among the rings, spiroheterocyclyl may be monospiroheterocyclyl, bispiroheterocyclyl or polyspiroheterocyclyl. Spiroheterocyclyl includes but is not limited to:

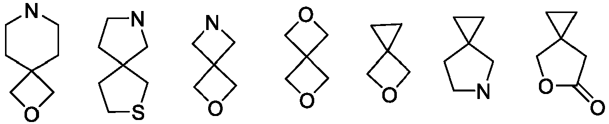

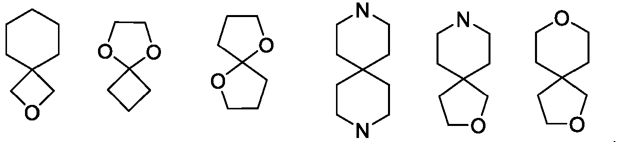

"Fused heterocyclyl" refers to a polycyclic heterocyclyl in which each ring shares a pair of adjacent atoms with the other rings in the system, where one or more (preferably 1, 2, 3 or 4) of the rings may contain one or more (preferably 1, 2 or 3) double bonds, but none of them have a fully conjugated π-electron system, where one or more (preferably 1, 2, 3 or 4) of the ring atoms are heteroatoms selected from N, O, N•O or S(O)ᵣ (where r is an integer of 0, 1 or 2), and the remaining ring atoms are carbon atoms. According to the number of formed rings, the fused heterocyclyl may be bicyclic, tricyclic, tetracyclic or polycyclic, including but not limited to:

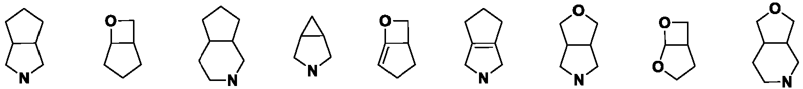

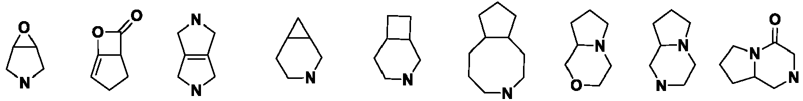

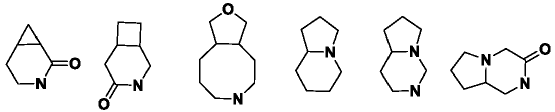

"Bridged heterocyclyl" refers to a polycyclic heterocyclyl in which any two rings share two carton atoms that are not directly attached to each other, where these rings may contain one or more (preferably 1, 2 or 3) double bonds, but none of them have a fully conjugated π-electron system, where one or more (preferably 1, 2, 3 or 4) of the ring atoms are heteroatoms selected from N, O, N•O or S(O)ᵣ (where r is an integer of 0, 1 or 2), and the remaining ring atoms are carbon atoms. According to the number of formed rings, the bridged heterocyclyl may be bicyclic, tricyclic, tetracyclic or polycyclic, including but not limited to:

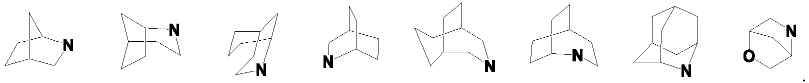

The heterocyclyl ring may be fused to an aryl, heteroaryl or cycloalkyl ring, wherein the ring attached to the parent structure is heterocyclyl, which includes but is not limited to:

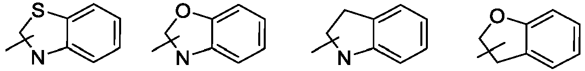

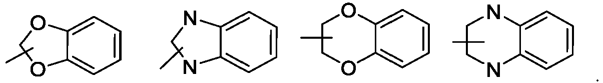

Heterocyclyl can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₇)R₈, -C₀₋₈ alkyl-N=S(O)R₈R₉, -C₀₋₈ alkyl-N=SR₈R₉, -C₀₋₈ alkyl-O-S(O)₂R₁₀, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)SR₁₁, -C₀₋₈ alkyl-S-C(O)R₁₂, -C₀₋₈ alkyl-C(O)R₁₂, -C₀₋₈ alkyl-O-C(O)R₁₂, -C₀₋₈ alkyl-P(O)(R₁₂)₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Aryl" or "aromatic ring" means an all-carbon monocyclic or fused-polycyclic group (i.e., rings that share a pair of adjacent carbon atoms) and a polycyclic group having a conjugated π-electron system (i.e., rings with adjacent pairs of carbon atoms), preferably including all-carbon aryl containing 6-10 or 6-8 carbon atoms. For example, "C₆₋₁₀ aryl" means an all-carbon aryl containing 6-10 carbon atoms, including but not limited to phenyl and naphthyl, and "C₆₋₈ aryl" means an all-carbon aryl containing 6-8 carbon atoms. The aryl ring may be fused to an heteroaryl, heterocyclyl or cycloalkyl ring, wherein the ring attached to the parent structure is aryl ring, including but not limited to:

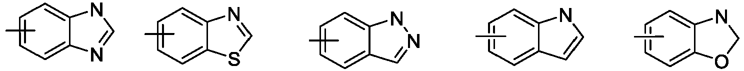

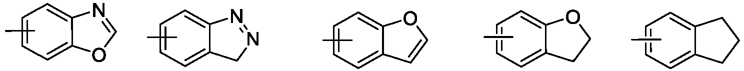

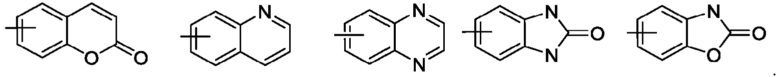

"Aryl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₇)R₈, -C₀₋₈ alkyl-N=S(O)R₈R₉, -C₀₋₈ alkyl-N=SR₈R₉, -C₀₋₈ alkyl-O-S(O)₂R₁₀, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)SR₁₁, -C₀₋₈ alkyl-S-C(O)R₁₂, -C₀₋₈ alkyl-C(O)R₁₂, -C₀₋₈ alkyl-O-C(O)R₁₂, -C₀₋₈ alkyl-P(O)(R₁₂)₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Heteroaryl" refers to a heteroaromatic system containing one or more (preferably 1, 2, 3 or 4) of the heteroatoms, where the heteroatoms include heteroatoms selected from N, O, N•O and S(O)r (where r is an integer of 0, 1 or 2), preferably including a heteroaromatic system containing 5-10 or 5-8 or 5-6 ring atoms. For example, "5-8 membered heteroaryl" means a heteroaromatic system containing 5-8 ring atoms, and "5-10 membered heteroaryl" means a heteroaromatic system containing 5-10 ring atoms, including but not limited to furyl, thiophenyl, pyridyl, pyrrolyl, N-alkylpyrrolyl, pyrimidinyl, pyrazinyl, imidazolyl, tetrazolyl, etc. The heteroaryl ring may be fused to an aryl, heterocyclyl or cycloalkyl ring, wherein the ring attached to the parent structure is heteroaryl ring, including but not limited to:

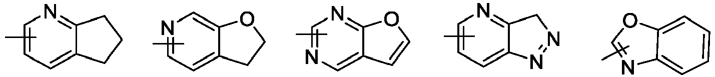

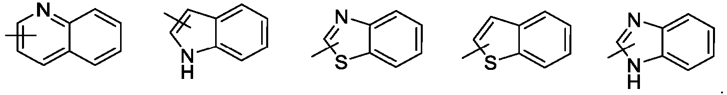

"Heteroaryl" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₇)R₈, -C₀₋₈alkyl -N=S(O)R₈R₉, -C₀₋₈ alkyl-N=SR₈R₉, -C₀₋₈ alkyl-O-S(O)₂R₁₀, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)SR₁₁, -C₀₋₈ alkyl-S-C(O)R₁₂, -C₀₋₈ alkyl-C(O)R₁₂, -C₀₋₈ alkyl-O-C(O)R₁₂, -C₀₋₈ alkyl-P(O)(R₁₂)₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Alkenyl" refers to an alkyl defined as above consisting of at least two carbon atoms and at least one carbon-carbon double bond, preferably including a linear or branched alkenyl containing 2-10 or 2-4 carbon atoms. For example, "C₂₋₁₀ alkenyl" means a linear or branched alkenyl containing 2-10 carbon atoms, and "C₂₋₄ alkenyl" means a linear or branched alkenyl containing 2-4 carbon atoms. The alkenyl includes, but is not limited to, vinyl, 1-propenyl, 2-propenyl, 1-, 2- or 3-butenyl, etc.

"Alkenyl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₇)R₈, -C₀₋₈alkyl -N=S(O)R₈R₉, -C₀₋₈ alkyl-N=SR₈R₉, -C₀₋₈ alkyl-O-S(O)₂R₁₀, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)SR₁₁, -C₀₋₈ alkyl-S-C(O)R₁₂, -C₀₋₈ alkyl-C(O)R₁₂, -C₀₋₈ alkyl-O-C(O)R₁₂, -C₀₋₈ alkyl-P(O)(R₁₂)₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Alkynyl" refers to an alkyl defined as above consisting of at least two carbon atoms and at least one carbon-carbon triple bond, preferably including a linear or branched alkynyl containing 2-10 or 2-4 carbon atoms. For example, "C₂₋₁₀ alkynyl" means a linear or branched alkynyl containing 2-10 carbon atoms, and "C₂₋₄ alkynyl" means a linear or branched alkynyl containing 2-4 carbon atoms. The alkynyl includes, but is not limited to, ethynyl, 1-propynyl, 2-propynyl, 1-, 2- or 3-butynyl, etc.

"Alkynyl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₇)R₈, -C₀₋₈alkyl -N=S(O)R₈R₉, -C₀₋₈ alkyl-N=SR₈R₉, -C₀₋₈ alkyl-O-S(O)₂R₁₀, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)SR₁₁, -C₀₋₈ alkyl-S-C(O)R₁₂, -C₀₋₈ alkyl-C(O)R₁₂, -C₀₋₈ alkyl-O-C(O)R₁₂, -C₀₋₈ alkyl-P(O)(R₁₂)₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Alkoxy" refers to an -O-alkyl group, wherein the alkyl is defined as above. For example, "C₁₋₁₀ alkoxy" means an alkyloxy containing 1-10 carbon atoms, "C₁₋₄ alkoxy" means an alkyloxy containing 1-4 carbon atoms, and "C₁₋₂ alkoxy" means an alkyloxy containing 1-2 carbon atoms, including but not limited to, methoxy, ethoxy, propoxy, butoxy, etc.

"Alkoxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₇)R₈, -C₀₋₈alkyl -N=S(O)R₈R₉, -C₀₋₈ alkyl-N=SR₈R₉, -C₀₋₈ alkyl-O-S(O)₂R₁₀, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)SR₁₁, -C₀₋₈ alkyl-S-C(O)R₁₂, -C₀₋₈ alkyl-C(O)R₁₂, -C₀₋₈ alkyl-O-C(O)R₁₂, -C₀₋₈ alkyl-P(O)(R₁₂)₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Cycloalkoxy" or "cycloalkyloxy" refers to an -O-cycloalkyl group, wherein cycloalkyl is defined as above. For example, "C₃₋₁₂ cycloalkoxy" means a cycloalkyloxy containing 3-12 carbon atoms, and "C₃₋₆ cycloalkoxy" means a cycloalkyloxy containing 3-6 carbon atoms, including but not limited to cyclopropoxy, cyclobutoxy, cyclopentoxy, cyclohexoxy, etc.

"Cycloalkoxy" or "cycloalkyloxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₇)R₈, -C₀₋₈alkyl -N=S(O)R₈R₉, -C₀₋₈ alkyl-N=SR₈R₉, -C₀₋₈ alkyl-O-S(O)₂R₁₀, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)SR₁₁, -C₀₋₈ alkyl-S-C(O)R₁₂, -C₀₋₈ alkyl-C(O)R₁₂, -C₀₋₈ alkyl-O-C(O)R₁₂, -C₀₋₈ alkyl-P(O)(R₁₂)₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Heterocycloxy" or "heterocyclyloxy" refers to an -O-heterocyclyl group, wherein the heterocyclyl is defined as above, including but not limited to, azetidyloxy, oxetanyloxy, azetidyloxy, nitrogen, oxetanyloxy, etc.

"Heterocycloxy" or "heterocyclyloxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₇)R₈, -C₀₋₈alkyl -N=S(O)R₈R₉, -C₀₋₈ alkyl-N=SR₈R₉, -C₀₋₈ alkyl-O-S(O)₂R₁₀, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)SR₁₁, -C₀₋₈ alkyl-S-C(O)R₁₂, -C₀₋₈ alkyl-C(O)R₁₂, -C₀₋₈ alkyl-O-C(O)R₁₂, -C₀₋₈ alkyl-P(O)(R₁₂)₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"C₁₋₁₀ alkanoyl" refers to a monovalent atomic group which is obtained after a hydroxy is removed from the C₁₋₁₀ alkyl acid, and is also generally referred to as "C₀₋₉ alkyl-C(O)-". For example, "C₁ alkyl-C(O)-" refers to acetyl; "C₂ alkyl-C(O)-" refers to propanoyl; "C₃ alkyl-C(O)-" refers to butanoyl or isobutanoyl.

"-C₀₋₈ alkyl-S(O)(=N-R₇)R₈" refers to the sulfur atom in -S(O)(=N-R₇)R₈ attached to C₀₋₈ alkyl, where the C₀₋₈ alkyl is defined as above.

"-C₀₋₈ alkyl-N=S(O)R₈R₉" refers to the nitrogen atom in -N=S(O)R₈R₉ attached to C₀₋₈ alkyl, where the C₀₋₈ alkyl is defined as above.

"-C₀₋₈ alkyl-N=SR₈R₉" refers to the nitrogen atom in -N=SR₈R₉ attached to C₀₋₈ alkyl, where the C₀₋₈ alkyl is defined as above.

"-C₀₋₈ alkyl-N(R₁₃)-S(O)₂R₁₀" refers to the nitrogen atom in -N(R₁₃)-S(O)₂R₁₀ attached to C₀₋₈ alkyl, where the C₀₋₈ alkyl is defined as above.

"-C₀₋₈ alkyl-O-S(O)₂R₁₀" refers to the oxygen atom in -O-S(O)₂R₁₀ attached to C₀₋₈ alkyl, where the C₀₋₈ alkyl is defined as above.

"-C₀₋₈ alkyl-S(O)ᵣR₁₀" refers to the sulfur atom in -S(O)ᵣR₁₀ attached to C₀₋₈ alkyl, where the C₀₋₈ alkyl is defined as above.

"-C₀₋₈ alkyl-P(O)(R₁₂)₂" refers to the phosphorus atom in -P(O)(R₁₂)₂ attached to C₀₋₈ alkyl, where the C₀₋₈ alkyl is defined as above.

"-C₀₋₈ alkyl-O-R₁₁" refers to the oxygen atom in -O-R₁₁ attached to C₀₋₈ alkyl, where the C₀₋₈ alkyl is defined as above.

"-C₀₋₈ alkyl-C(O)OR₁₁" refers to the carbonyl in -C(O)OR₁₁ attached to C₀₋₈ alkyl, where the C₀₋₈ alkyl is defined as above.

"-C₀₋₈ alkyl-C(O)SR₁₁" refers to the carbonyl in -C(O)SR₁₁ attached to C₀₋₈ alkyl, where the C₀₋₈ alkyl is defined as above.

"-C₀₋₈ alkyl-S-C(O)R₁₂" refers to the sulfur atom in -S-C(O)R₁₂ attached to C₀₋₈ alkyl, where the C₀₋₈ alkyl is defined as above.

"-C₀₋₈ alkyl-C(O)R₁₂" refers to the carbonyl in -C(O)R₁₂ attached to C₀₋₈ alkyl, where the C₀₋₈ alkyl is defined as above.

"-C₀₋₈ alkyl-O-C(O)R₁₂" refers to the oxygen atom in -O-C(O)R₁₂ attached to C₀₋₈ alkyl, where the C₀₋₈ alkyl is defined as above.

"-C₀₋₈ alkyl-NR₁₃R₁₄" refers to the nitrogen atom in -NR₁₃R₁₄ attached to C₀₋₈ alkyl, where the C₀₋₈ alkyl is defined as above.

"-C₀₋₈ alkyl-C(=NR₁₃)R₁₂" refers to the carbon atom in -C(=NR₁₃)R₁₂ attached to C₀₋₈ alkyl, where the C₀₋₈ alkyl is defined as above.

"-C₀₋₈ alkyl-N(R₁₃)-C(=NR₁₄)R₁₂" refers to the nitrogen atom in -N(R₁₃)-C(=NR₁₄)R₁₂ attached to C₀₋₈ alkyl, where the C₀₋₈ alkyl is defined as above.

"-C₀₋₈ alkyl-C(O)NR₁₃R₁₄" refers to the carbonyl in -C(O)NR₁₃R₁₄ attached to C₀₋₈ alkyl, where the C₀₋₈ alkyl is defined as above.

"-C₀₋₈ alkyl-N(R₁₃)-C(O)R₁₂" refers to the nitrogen atom in -N(R₁₃)-C(O)R₁₂ attached to C₀₋₈ alkyl, where the C₀₋₈ alkyl is defined as above.

"C₁₋₁₀ haloalkyl" refers to an alkyl having 1-10 carbon atoms in which hydrogens on the alkyl are optionally substituted by a fluorine, chlorine, bromine and iodine atom, including but not limited to difluoromethyl, dichloromethyl, dibromomethyl, trifluoromethyl, trichloromethyl, tribromomethyl, etc.

"C₁₋₁₀ haloalkoxy" refers to an alkoxy having 1-10 carbon atoms in which hydrogens on the alkyl are optionally substituted by a fluorine, chlorine, bromine and iodine atom, including but not limited to difluoromethoxy, dichloromethoxy, dibromomethoxy, trifluoromethoxy, trichloromethoxy, tribromomethoxy, etc.

"C₁₋₁₀ deuterioalkyl" refers to an alkyl having 1-10 carbon atoms in which hydrogens on the alkyl are optionally substituted by a deuterium atom, including but not limited to monodeuterium, dideuterium, trideuterium, etc.

"Halogen" refers to fluorine, chlorine, bromine or iodine.

The term "optional" or "optionally" means that the event or circumstance subsequently described may, but not necessarily, occur, and that the description includes instances where the event or circumstance occurs or dose not occur, i.e., both substituted or unsubstituted instances. For example, "heterocyclyl group optionally substituted by alkyl" means that alkyl may be, but not necessarily, present, and that the description includes instances where the heterocyclyl group is and is not substituted by alkyl.

The term "substituted" means that one or more "hydrogen atoms" in a group are each independently substituted by a corresponding number of substituents. It goes without saying that a substituent is only in its possible chemical position and consistent with the valence bond theory in chemistry, and those skilled in the art will be able to determine (by experiments or theories) possible or impossible substitution without undue efforts. For example, it may be unstable when an amino or hydroxy having a free hydrogen is bound to a carbon atom having an unsaturated bond (such as olefin).

"Stereoisomer" refers to isomers produced by different spatial arrangements of atoms in the molecules. They can be divided into cis-trans isomers and enantiomers and can also be divided into two categories: enantiomers and diastereomers. A stereoisomer produced by the rotation of a single bond is referred to as a conformational stereo-isomer, sometimes also referred to as a rotamer. A stereoisomer produced by bond length, bond angle, double bonds in the molecule, ring and other reasons is referred to as a configuration stereo-isomer, which can be divided into two categories. An isomer produced by the inability of a double bond or a single bond of ring carbon atoms to rotate freely becomes a geometric isomer, also known as a cis-trans isomer, which can be divided into Z and E configurations. For example: cis-2-butene and trans-2-butene are a pair of geometric isomers. Stereoisomers with different optical properties produced by the absence of anti-axial symmetry in the molecules are called optical isomers, which can be divided into R and S configurations. "Stereoisomers" as described in the present invention, if not specifically indicated, are to be understood to include one or more of the enantiomers, configurational isomers, and conformational isomers described above.

"Pharmacologically acceptable salt" in the present invention refers to pharmaceutically acceptable acid addition salts, including inorganic and organic acid salts, which may be prepared by methods known in the art.

"Pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities.

The present invention is further explained in detail below with reference to examples, which are not intended to limit the present invention, and the present invention is not merely limited to the contents of the examples.

The compound structure of the present invention is determined by nuclear magnetic resonance (NMR) or/and liquid chromatography-mass spectrometry (LC-MS). The NMR chemical shift (δ) is given in parts per million (ppm). The NMR determination is conducted by using a Bruker AVANCE-400/500 nuclear magnetic resonance apparatus, with hexadeuterodimethyl sulfoxide (DMSO-*d₆*), tetradeuteromethanol (CD₃OD) and deuterated chloroform (CDCl₃) as determination solvents, and tetramethylsilane (TMS) as internal standard.

The LC-MS determination is conducted by using an Agilent 6120 mass spectrometer. The HPLC determination is conducted by using an Agilent 1200 DAD high pressure liquid chromatography (Sunfire C18 150 × 4.6 mm chromatographic column) and a Waters 2695-2996 high pressure liquid chromatography (Gimini C18 150 × 4.6 mm chromatographic column).

Yantai Yellow Sea HSGF254 or Qingdao GF254 silica gel plate is adopted as a thin layer chromatography (TLC) silica gel plate. The specification adopted by the TLC is 0.15-0.20 mm, and the specification adopted by the thin layer chromatography for the separation and purification of products is 0.4-0.5 mm. The Yantai Yellow Sea silica gel of 200-300 mesh is generally utilized as a carrier in column chromatography.

Starting materials in the examples of the present invention are known and commercially available, or may be synthesized by using or according to methods known in the art.

Unless otherwise stated, all reactions of the present invention are carried out under a dry nitrogen or argon atmosphere with continuous magnetic stirring, wherein the solvent is a dry solvent and the reaction temperature is in degree centigrade (°C).

### I. Preparation of Intermediates

### Intermediate 1-1: Preparation of (S)-3-bromo-1-(4-((tert-butyldimethylsilyl)oxy)butan-2-yl)-6-chloro-1H-pyrazolo[4, 3-c]pyridine

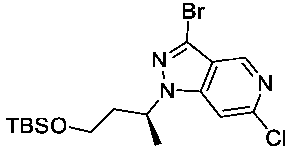

### Step 1: Synthesis of 3-bromo-6-chloro-1H-pyrazolo[4,3-c]pyridine

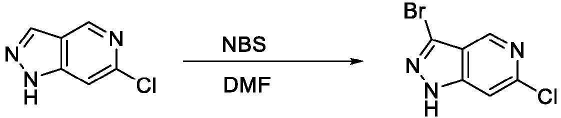

6-chloro-1H-pyrazolo[4,3-c]pyridine (5.0 g, 32.56 mmol) was dissolved in DMF (30 mL), and NBS (6.95 g, 39.07 mmol) was added. The mixture was stirred at room temperature for 2 hours. CH₂Cl₂ and saturated aqueous NaHCO₃ solution were added for extraction, the organic phase was washed with saturated aqueous NaCl solution, dried over anhydrous Na₂SO₄, filtered and concentrated, and the residue was separated by using silica gel column chromatography to obtain 3-bromo-6-chloro-1H-pyrazolo[4,3-c]pyridine (7.4 g, yield: 97.8%).

### Step 2: Synthesis of (R)-4-((tert-butyldimethylsilyl)oxy)butan-2-ol

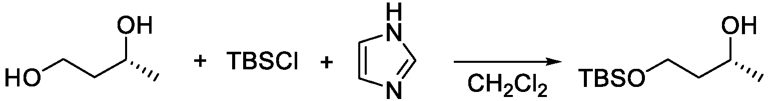

(R)-butan-1,3-diol (4.98 mL, 55.48 mmol) was dissolved in CH₂Cl₂ (80 mL), and imidazole (4.16 g, 61.03 mmol) was added. The mixture was cooled to 0°C, and then TBSCl (8.36 g, 55.48 mmol) was added. The mixture was stirred at 10°C for 3 hours. The reaction solution was diluted with CH₂Cl₂ and water, and the organic phase was separated by extraction, washed with saturated aqueous NaCl solution, dried over anhydrous Na₂SO₄, filtered and concentrated. The residue was separated by using silica gel column chromatography to obtain (R)-4-((tert-butyldimethylsilyl)oxy)butan-2-ol (9.0 g, yield: 79.4%).

### Step 3: Synthesis of (R)-4-((tert-butyldimethylsilyl)oxy)butan-2-yl 4-methylbenzene-1-sulfonate

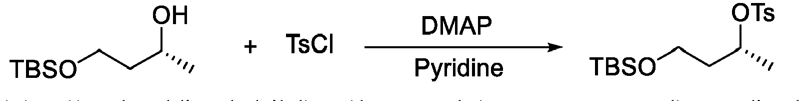

(R)-4-((tert-butyldimethylsilyl)oxy)butan-2-ol (6.0 g, 29.36 mmol) was dissolved in pyridine (60 mL), and then DMAP (1.08 g, 8.81 mmol) was added, and TsCl (12.31 g, 64.59 mmol) was added under cooling to 0°C. After the addition, the mixture was stirred at room temperature for 18 hours. The reaction solution was diluted with CH₂Cl₂ and water, and the organic phase was separated by extraction, washed with saturated aqueous NaCl solution, dried over anhydrous Na₂SO₄, filtered and concentrated. The residue was separated by using silica gel column chromatography to obtain (R)-4-((tert-butyldimethylsilyl)oxy)butan-2-yl 4-methylbenzene-1-sulfonate (6.4 g, yield: 60.8%).

### Step 4: Synthesis of (S)-3-bromo-1-(4-((tert-butyldimethylsilyl)oxy)butan-2-yl)-6-chloro-1H-pyrazolo[4, 3-c]pyridine

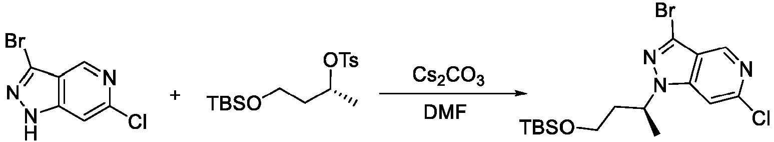

3-bromo-6-chloro-1H-pyrazolo[4,3-c]pyridine (1.0 g, 4.30 µmol) was dissolved in DMF (20 mL), and then (R)-4-((tert-butyldimethylsilyl)oxy)butan-2-yl 4-methylbenzene-1-sulfonate (1.85 g, 5.16 mmol) and Cs₂CO₃ (2.80 g, 8.60 mmol) were added in sequence. The mixture was heated to 45°C and stirred for 18 hours. The mixture was cooled to room temperature, and EtOAc and water were added for extraction. The organic phase was washed with saturated aqueous NaCl solution, dried over anhydrous Na₂SO₄, filtered and concentrated. The residue was separated by using silica gel column chromatography to obtain (S)-3-bromo-1-(4-((tert-butyldimethylsilyl)oxy)butan-2-yl)-6-chloro-1H-pyrazolo[4,3-c ]pyridine (1.5 g, yield: 83.3%). MS m/z (ESI): 418.2/420.2 [M+H]⁺.

**Intermediates 1-2 to 1-6 can be prepared by selecting the corresponding raw materials with reference to the synthesis methods of intermediate 1-1.**

| **Intermediate No.** | **Structural Formula** | **Chemical Name** | **MS: *m*/*z* [M+H]⁺** |
|---|---|---|---|
| **1-2** | 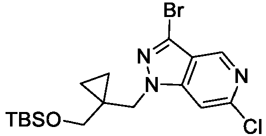 | 3-bromo-1-((1-(((tert-but yldimethylsilyl)oxy)meth yl)cyclopropyl)methyl)-6 -chloro-1H-pyrazolo[4,3-c]pyridine | 431 |
| **1-3** | 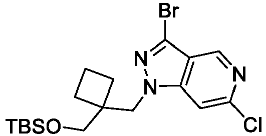 | 3-bromo-1-((1-(((tert-but yldimethylsilyl)oxy)meth yl)cyclobutyl)methyl)-6-c hloro-1H-pyrazolo[4,3-c] pyridine | 445 |
| **1-4** | 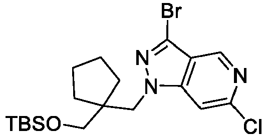 | 3-bromo-1-((1-(((tert-but yldimethylsilyl)oxy)meth yl)cyclopentyl)methyl)-6-chloro-1H-pyrazolo[4,3-c ]pyridine | 459 |
| **1-5** | 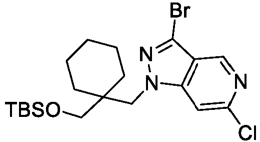 | 3-bromo-1-((1-(((tert-but yldimethylsilyl)oxy)meth yl)cyclohexyl)methyl)-6-chloro-1H-pyrazolo[4,3-c ]pyridine | 474 |
| **1-6** | 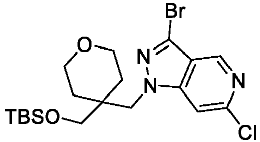 | 3-bromo-1-((4-(((tert-but yldimethylsilyl)oxy)meth yl)tetrahydro-2H-pyran-4 -yl)methyl)-6-chloro-1H-pyrazolo[4,3-c]pyridine | 476 |

### Intermediate 1-7: Preparation of (1-((3-bromo-6-chloro-1H-pyrazolo[4,3-c]pyridin-1-yl)methyl)cyclopentyl)methano l

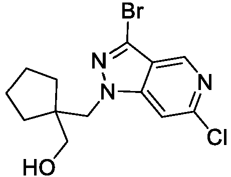

### Step 1: Synthesis of methyl 1-(iodomethyl)cyclopentane-1-carboxylate

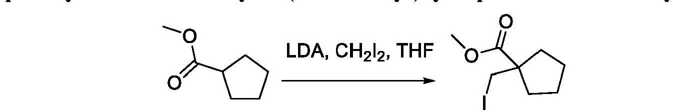

Methyl cyclopentanecarboxylate (11.7 g, 91.285 mmol) was dissolved in THF (120 mL). Under nitrogen protection, LDA/THF solution (54.8 mL, 109.542 mmol, 2M) was slowly added dropwise at -78°C, and then CH₂I₂ (8.837 mL, 109.542 mmol) was added. After the addition, the mixture was warmed to room temperature and reacted for 1.5 hours. The reaction was completed as detected by TLC. The solution was quenched by adding saturated aqueous NH₄Cl solution (150 mL). The solution was extracted three times with EtOAc (100 mL), and the organic phase was combined and dried over anhydrous Na₂SO₄. It was filtered and concentrated, and the residue was separated by using silica gel column chromatography [eluent: PE/EtOAc 0-15%] to obtain methyl 1-(iodomethyl)cyclopentane-1-carboxylate (23.5 g, 78.891 mmol, yield: 86.4%).

¹H NMR (400 MHz, CDCl₃) δ 3.72 (d, J = 0.9 Hz, 3H), 3.41 (d, J = 0.9 Hz, 2H), 2.26 - 2.16 (m,2H), 1.79 - 1.61 (m, 6H).

### Step 2: Synthesis of methyl 1-((3-bromo-6-chloro-1H-pyrazolo[4,3-c]pyridin-1-yl)methyl)cyclopentane-1-carbo xylate

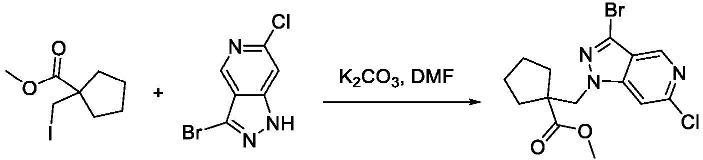

3-bromo-6-chloro-1H-pyrazolo[4,3-c]pyridine (37.46 g, 161.140 mmol) and methyl 1-(iodomethyl)cyclopentane-1-carboxylate (43.2 g, 161.14 mmol) were dissolved in DMF (800 mL), and K₂CO₃ (44.54 g, 322.28 mmol) was added, and then the mixture was heated to 100°C and stirred for 16 hours. The reaction was completed as detected by TLC. The reaction solution was cooled to room temperature and concentrated, and the residue was separated by using silica gel column chromatography [eluent: PE/EA 5/1] to obtain methyl 1-((3-bromo-6-chloro-1H-pyrazolo[4,3-c]pyridin-1-yl)methyl)cyclopentane-1-carboxyla te (38.5 g, 103.31 mmol, yield: 64.1 %). MS m/z (ESI): 372.0,374.0 [M+H]⁺.

### Step 3: Synthesis of (1-((3-bromo-6-chloro-1H-pyrazolo[4,3-c]pyridin-1-yl)methyl)cyclopentyl)methano l

Methyl

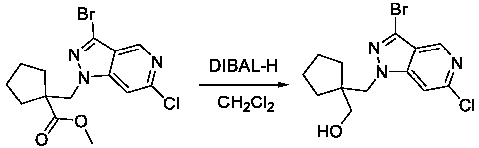

1-((3-bromo-6-chloro-1H-pyrazolo[4,3-c]pyridin-1-yl)methyl)cyclopentane-1-carboxyla te (8.5 g, 22.81 mmol) was dissolved in CH₂Cl₂ (100 mL), the mixture was cooled to -78°C, DIBAL-H (45.62 mL, 45.62 mmol) was slowly added at the low temperature, and the mixture was stirred at -78°C for 2 hours. The reaction was completed as detected by TLC. Saturated brine was added to quench the reaction mixture, and then aqueous potassium sodium tartrate solution (50 mL) was added, and the mixture was stirred at room temperature for 10 minutes. Then, the mixture was extracted three times with CH₂Cl₂ (50 mL). The organic phase was combined, dried over anhydrous sodium sulfate, filtered and concentrated, and the resulting residue was separated by using silica gel column chromatography [eluent: PE/EA = 2/1] to obtained (1-((3-bromo-6-chloro-1H-pyrazolo[4,3-c]pyridin-1-yl)methyl)cyclopentyl)methanol (5.8 g, 16.83 mmol, yield: 73.78%). MS m/z (ESI): 344.0, 346.0 [M+H]⁺.

**Intermediates 1-8 to 1-10 can be prepared by selecting the corresponding raw materials with reference to the synthesis routes of intermediate 1-7.**

| **Intermediate No.** | **Structural Formula** | **Chemical Name** | **MS: *m*/*z* [M+H]⁺** |
|---|---|---|---|
| **1-8** | 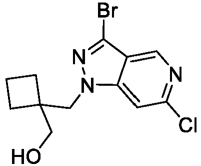 | (1-((3-bromo-6-chloro-1 H-pyrazolo[4,3-c]pyridin -1-yl)methyl)cyclobutyl) methanol | 330.0 |
| **1-9** | 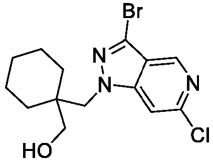 | (1-((3-bromo-6-chloro-1 H-pyrazolo[4,3-c]pyridin -1-yl)methyl)cyclohexyl) methanol | 358.0 |
| **1-10** | 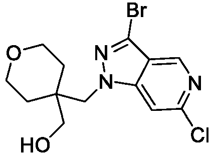 | (4-((3-bromo-6-chloro-1 H-pyrazolo[4,3-c]pyridin -1-yl)methyl)tetrahydro-2H-pyran-4-yl)methanol | 360.0 |

### Intermediate 2-1: Preparation of (S)-1-(4-((tert-butyldimethylsilyl)oxy)butan-2-yl)-6-chloro-3-(prop-1-yn-1-yl)-1H-p yrazolo[4,3-c]pyridine

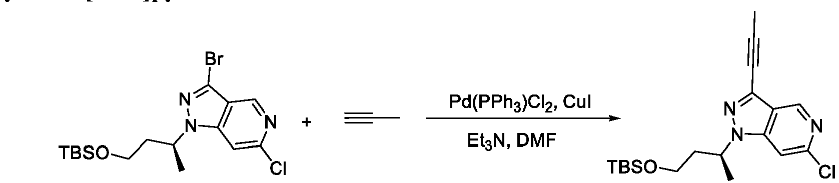

(S)-3-bromo-1-(4-((tert-butyldimethylsilyl)oxy)butan-2-yl)-6-chloro-1H-pyrazolo[ 4,3-c]pyridine (0.90 g, 2.14 mmol) was dissolved in DMF (10 mL), and Pd(PPh₃)₂Cl₂ (0.31 g, 0.43 mmol), CuI (164 mg, 0.86 mmol) and Et₃N (3 mL) were added. Under nitrogen protection, propyne solution (10 mL g, 10 mmol) was added with a syringe, and the mixture was heated to 70°C and reacted overnight. After the reaction was completed, EtOAc was added to dilute the mixture, and the organic phase was washed with water and saturated brine in sequence, and dried over anhydrous sodium sulfate. It was filtered, and the filtrate was concentrated and then separated by using column chromatography to obtain (S)-1-(4-((tert-butyldimethylsilyl)oxy)butan-2-yl)-6-chloro-3-(prop-1-yn-1-yl)-1H-pyra zolo[4,3-c]pyridine (0.77 g, yield: 80%). MS m/z (ESI): 378.2 [M+H]⁺.

### Intermediate 2-2: Preparation of 1-((S)-4-((tert-butyldimethylsilyl)oxy)butan-2-yl)-6-chloro-3-(((S)-1-methylpyrrolid in-2-yl)ethynyl)-1H-pyrazolo[4,3-c]pyridine

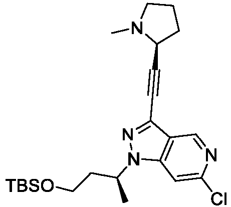

### Step 1: Synthesis of tert-butyl (S)-2-ethynylpyrrolidine-1-carboxylate

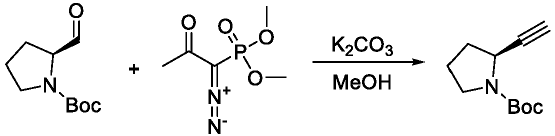

Tert-butyl (S)-2-formylpyrrolidine-1-carboxylate (5.0 g, 25.09 mmol) was dissolved in MeOH (50 mL), then K₂CO₃ (6.94 g, 50.19 mmol) was added, the mixture was cooled to 0°C, and dimethyl(1-diazo-2-oxopropyl)phosphate (5.65 mL, 37.64 mmol) was added dropwise. The mixture was reacted at room temperature for 18 hours. After the reaction was completed, the mixture was filtered, and the filtrate was concentrated and then separated by using column chromatography to obtain tert-butyl (S)-2-ethynylpyrrolidine-1-carboxylate (4.4 g, yield: 89.8%). MS m/z (ESI): 195.8 [M+H]⁺.

### Step 2: Synthesis of tert-butyl (S)-2-((1-((S)-4-((tert-butyldimethylsilyl)oxy)butan-2-yl)-6-chloro-1H-pyrazolo[4,3-c]pyridin-3-yl)ethynyl)pyrrolidin-1-carboxylate

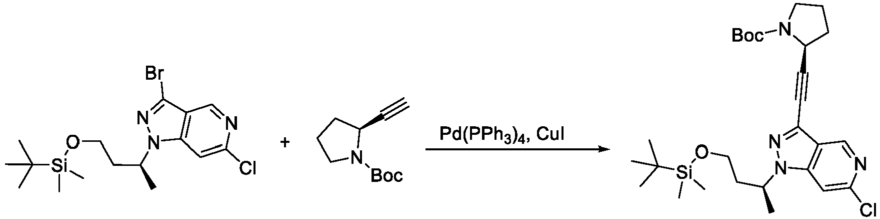

(S)-3-bromo-1-(4-((tert-butyldimethylsilyl)oxy)butan-2-yl)-6-chloro-1H-pyrazolo[ 4,3-c]pyridine (0.6 g, 1.43 mmol) was dissolved in DMF (20 mL), and tert-butyl (S)-2-ethynylpyrrolidin-1-carboxylate (1119 mg, 5.73 mmol), CuI (0.27 g, 1.43 mmol), Et₃N (1.0 mL, 7.16 mmol) and Pd(PPh₃)₄ were added (0.50 g, 0.43 mmol). The mixture was heated to 80°C and reacted for 2 hours under nitrogen protection. The mixture was cooled to room temperature, filtered, and the filtrate was poured into water, extracted with EtOAc, and the organic phase was washed with saturated brine and dried over anhydrous sodium sulfate. It was filtered, and the filtrate was concentrated and then separated by using column chromatography to obtain tert-butyl (S)-2-((1-((S)-4-((tert-butyldimethylsilyl)oxy)butan-2-yl)-6-chloro-1H-pyrazolo[4,3-c]p yridin-3-yl)ethynyl)pyrrolidin-1-carboxylate (720 mg, yield: 94.3%). MS m/z (ESI): 533.4 [M+H]⁺.

### Step 3: Synthesis of 1-((S)-4-((tert-butyldimethylsilyl)oxy)butan-2-yl)-6-chloro-3-(((S)-pyrrolidin-2-yl)e thynyl)-1H-pyrazolo[4,3-c]pyridine

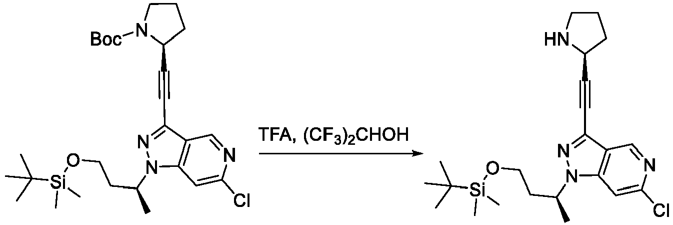

Tert-butyl (S)-2-((1-((S)-4-((tert-butyldimethylsilyl)oxy)butan-2-yl)-6-chloro-1H-pyrazolo[4,3-c]p yridin-3-yl)ethynyl)pyrrolidin-1-carboxylate (720 mg, 1.35 mmol) was dissolved in hexafluoroisopropanol (20 mL), and then TFA (1.0 mL, 13.50 mmol) was added dropwise. The mixture was reacted at room temperature for 1 hour. After the reaction was completed, CH₂Cl₂ was added for dilution, and the mixture was washed with saturated aqueous NaHCO₃ solution, pure water, and saturated brine in sequence, and dried over anhydrous sodium sulfate. It was filtered, and the filtrate was concentrated to obtain a crude product, which was directly used for the next reaction. MS m/z (ESI): 433.2 [M+H]⁺.

### Step 4: Synthesis of 1-((S)-4-((tert-butyldimethylsilyl)oxy)butan-2-yl)-6-chloro-3-(((S)-1-methylpyrrolid in-2-yl)ethynyl)-1H-pyrazolo[4,3-c]pyridine

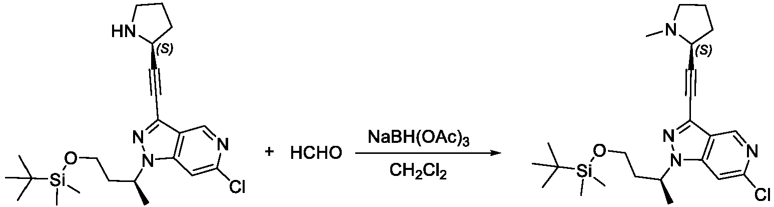

1-((S)-4-((tert-butyldimethylsilyl)oxy)butan-2-yl)-6-chloro-3-(((S)-pyrrolidin-2-yl) ethynyl)-1H-pyrazolo[4,3-c]pyridine (550 mg, 1.27 mmol) was dissolved in CH₂Cl₂ (20 mL), and then aqueous formaldehyde solution (1271 mg, 12.7 mmol) and NaBH(OAc)₃ (1346 mg, 6.35 mmol) were added. The mixture was reacted at room temperature for 3 hours. After the reaction was completed, CH₂Cl₂ was added for dilution, and the mixture was washed with saturated aqueous NaHCO₃ solution, pure water, and saturated brine in sequence, and dried over anhydrous sodium sulfate. It was filtered, and the filtrate was concentrated and then separated by using column chromatography to obtain 1-((S)-4-((tert-butyldimethylsilyl)oxy)butan-2-yl)-6-chloro-3-(((S)-1-methylpyrrolidin-2-yl)ethynyl)-1H-pyrazolo[4,3-c]pyridine (350 mg, yield: 61.6%). MS m/z (ESI): 447.2 [M+H]⁺.

### Intermediate 2-3: Preparation of (1-((6-chloro-3-((1-methylpiperidin-4-yl)ethynyl)-1H-pyrazolo[4,3-c]pyridin-1-yl)m ethyl)cyclopentyl)methanol

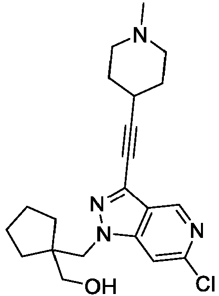

### Step 1: Synthesis of tert-butyl 4-((1-((1-(((tert-butyldimethylsilyl)oxy)methyl)cyclopentyl)methyl)-6-chloro-1H-py razolo[4,3-c]pyridin-3-yl)ethynyl)piperidine-1-carboxylate

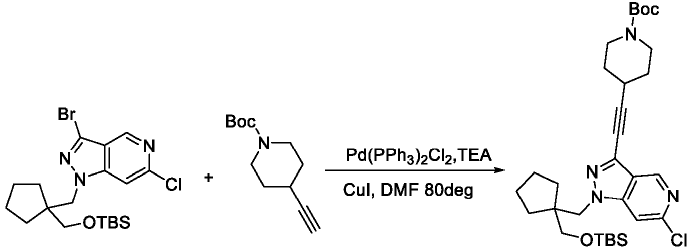

3-bromo-1-((1-(((tert-butyldimethylsilyl)oxy)methyl)cyclopentyl)methyl)-6-chloro -1H-pyrazolo[4,3-c]pyridine (375 mg, 0.817 mmol) was dissolved in DMF (20 mL), and then tert-butyl 4-ethynylpiperidin-1-carboxylate (256.5 mg, 1.226 mmol), CuI (62.3 mg, 0.327 mmol), Et₃N (1.14 mL, 8.172 mmol) and Pd(PPh₃)₂Cl₂ (114.7 mg, 0.163 mmol) were added. The mixture was heated to 80°C and stirred overnight under nitrogen protection. After the reaction was completed, the reaction solution was concentrated and separated by using column chromatography to obtain tert-butyl 4-((1-((1-(((tert-butyldimethylsilyl)oxy)methyl)cyclopentyl)methyl)-6-chloro-1H-pyraz olo[4,3-c]pyridin-3-yl)ethynyl)piperidine-1-carboxylate (450 mg, 0.766 mmol, yield: 93.8%). MS m/z (ESI): 587.4 [M+H]⁺.

### Step 2: Synthesis of (1-((6-chloro-3-(piperidin-4-ylethynyl)-1H-pyrazolo [4,3-c] pyridin-1-yl)methyl)cycl opentyl)methanol

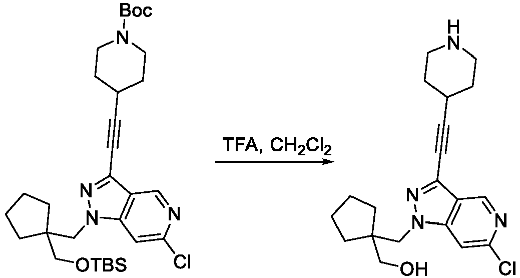

Tert-butyl 4-((1-((1-(((tert-butyldimethylsilyl)oxy)methyl)cyclopentyl)methyl)-6-chloro-1H-pyraz olo[4,3-c]pyridin-3-yl)ethynyl)piperidine-1-carboxylate (450 mg, 0.766 mmol) was dissolved in CH₂Cl₂ (15 mL), and TFA (0.57 mL, 7.662 mmol) was added dropwise. The mixture was stirred at room temperature for 2 hours. After the reaction was completed, saturated aqueous sodium bicarbonate solution was added to the reaction solution and the reaction solution was extracted with dichloromethane. The organic phase was combined, washed with water and saturated brine in sequence, and dried over anhydrous sodium sulfate. It was filtered and concentrated to obtain crude (1-((6-chloro-3-(piperidin-4-ylethynyl)-1H-pyrazolo[4,3-c]pyridin-1-yl)methyl)cyclope ntyl)methanol (260 mg, 0.697 mmol, yield: 91.0%). MS m/z (ESI): 373.2 [M+H]⁺.

### Step 3: Synthesis of (1-((6-chloro-3-((1-methylpiperidin-4-yl)ethynyl)-1H-pyrazolo[4,3-c]pyridin-1-yl)m ethyl)cyclopentyl)methanol

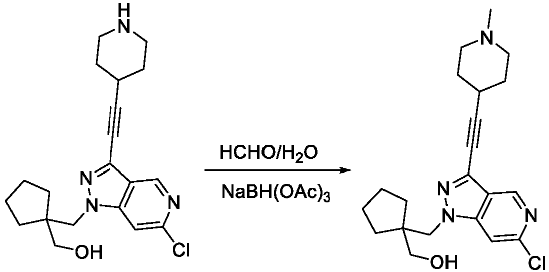

(1-((6-chloro-3-(piperidin-4-ylethynyl)-1H-pyrazolo[4,3-c]pyridin-1-yl)methyl)cyc lopentyl)methanol (260 mg, 0.697 mmol) was dissolved in CH₂Cl₂ (20 mL), and then 33% formaldehyde aqueous solution (299 mg, 3.486 mmol) and NaBH(OAc)₃ (739 mg, 3.486 mmol) were added. The mixture was stirred at room temperature for 18 hours. After the reaction was completed, saturated aqueous NaHCO₃ solution was added to quench the reaction solution, and the reaction solution was extracted with dichloromethane three times. The organic phase was combined, concentrated, and then separated by using column chromatography to obtain (1-((6-chloro-3-((1-methylpiperidin-4-yl)ethynyl)-1H-pyrazolo[4,3-c]pyridin-1-yl)meth yl)cyclopentyl)methanol (260 mg, 0.672 mmol, yield: 96.4%). MS m/z (ESI): 387.2 [M+H]⁺.

**Intermediates 2-4 to 2-23 can be prepared by selecting the corresponding raw materials with reference to the synthesis methods of intermediate 2-2 or 2-3.**

| **Intermediate No.** | **Structural Formula** | **Chemical Name** | **MS: *m*/*z* [M+H]⁺** |
|---|---|---|---|
| **2-4** | 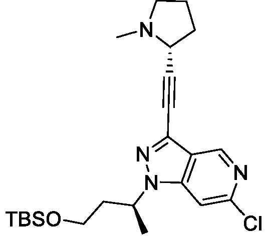 | 1-((S)-4-((tert-butyldimeth ylsilyl)oxy)butan-2-yl)-6-chloro-3-(((R)-1-methylpy rrolidin-2-yl)ethynyl)-1H-pyrazolo[4,3-c]pyridine | 448 |
| **2-5** | 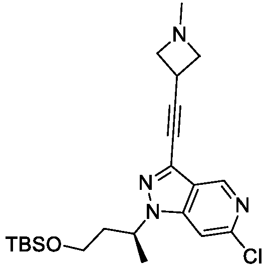 | (S)-1-(4-((tert-butyldimeth ylsilyl)oxy)butan-2-yl)-6-chloro-3-((1-methylazetidi n-3-yl)ethynyl)-1H-pyrazo lo[4,3-c]pyridine | 434 |
| **2-6** | 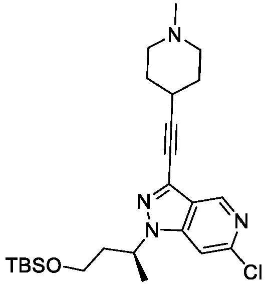 | (S)-1-(4-((tert-butyldimeth ylsilyl)oxy)butan-2-yl)-6-chloro-3-((1-methylpiperi din-4-yl)ethynyl)-1H-pyra zolo[4,3-c]pyridine | 462 |
| **2-7** | 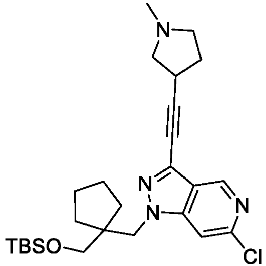 | 1-((1-((tert-butyldimethyls ilyl)oxy)methyl)cyclopent yl)methyl)-6-chloro-3-((1-methylpyrrolidin-3-yl)eth ynyl)-1H-pyrazolo[4,3-c]p yridine | 487 |
| **2-8** | 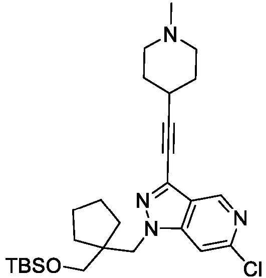 | 1-((1-((tert-butyldimethyls ilyl)oxy)methyl)cyclopent yl)methyl)-6-chloro-3-((1-methylpiperidin-4-yl)ethy nyl)-1H-pyrazolo[4,3-c]py ridine | 501 |
| **2-9** | 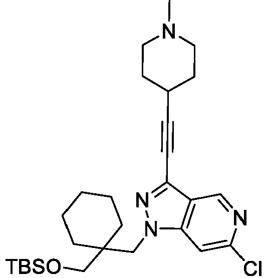 | 1-((1-((tert-butyldimethyls ilyl)oxy)methyl)cyclohexy 1)methyl)-6-chloro-3-((1-methylpiperidin-4-yl)ethy nyl)-1H-pyrazolo[4,3-c]py ridine | 515 |
| **2-10** | 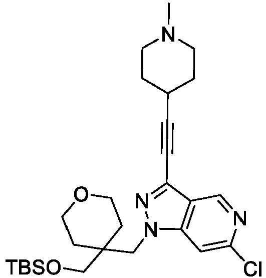 | 1-((4-(((tert-butyldimethyl silyl)oxy)methyl)tetrahydr o-2H-pyran-4-yl)methyl)-6-chloro-3-((1-methylpipe ridin-4-yl)ethynyl)-1H-pyr azolo[4,3-c]pyridine | 517 |
| **2-11** | 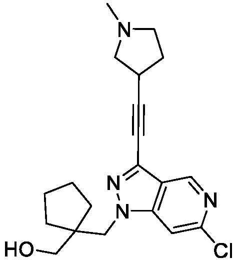 | (1-((6-chloro-3-((1-methyl pyrrolidin-3-yl)ethynyl)-1 H-pyrazolo[4,3-c]pyridin-1-yl)methyl)cyclopentyl) methanol | 373 |
| **2-12** | 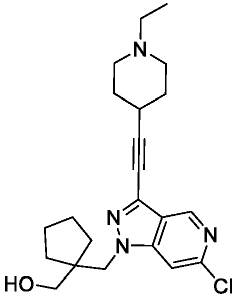 | (1-((6-chloro-3-((1-ethylpi peridin-4-yl)ethynyl)-1H-pyrazolo[4,3-c]pyridin-1-y l)methyl)cyclopentyl)meth anol | 401 |
| **2-13** | 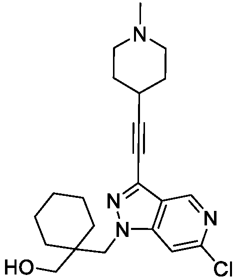 | (1-((6-chloro-3-((1-methyl piperidin-4-yl)ethynyl)-1 H-pyrazolo[4,3-c]pyridin-1-yl)methyl)cyclohexyl)m ethanol | 401 |
| **2-14** | 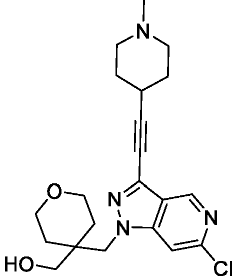 | (4-((6-chloro-3-((1-methyl piperidin-4-yl)ethynyl)-1 H-pyrazolo[4,3-c]pyridin-1-yl)methyl)tetrahydro-2H -pyran-4-yl)methanol | 403 |
| **2-15** | 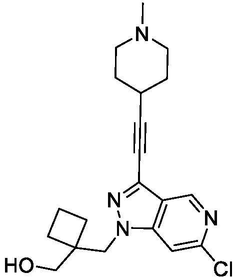 | (1-((6-chloro-3-((1-methyl piperidin-4-yl)ethynyl)-1 H-pyrazolo[4,3-c]pyridin-1-yl)methyl)cyclobutyl)m ethanol | 373 |
| **2-16** | 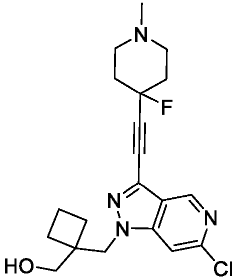 | (1-((6-chloro-3-((4-fluoro-1-methylpiperidin-4-yl)eth ynyl)-1H-pyrazolo[4,3-c]p yridin-1-yl)methyl)cyclob utyl)methanol | 391 |
| **2-17** | 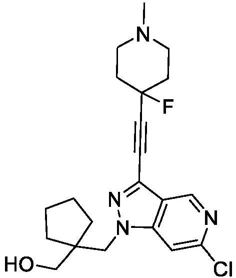 | (1-((6-chloro-3-((4-fluoro-1-methylpiperidin-4-yl)eth ynyl)-1H-pyrazolo[4,3-c]p yridin-1-yl)methyl)cyclop entyl)methanol | 405 |
| **2-18** | 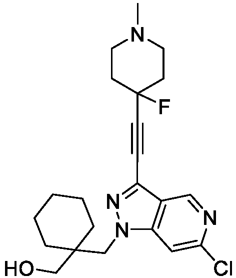 | (1-((6-chloro-3-((4-fluoro-1-methylpiperidin-4-yl)eth ynyl)-1H-pyrazolo[4,3-c]p yridin-1-yl)methyl)cycloh exyl)methanol | 419 |
| **2-19** | 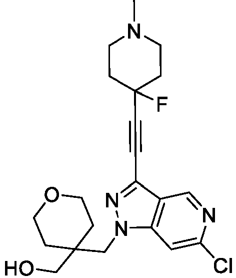 | (4-((6-chloro-3-((4-fluoro-1-methylpiperidin-4-yl)eth ynyl)-1H-pyrazolo[4,3-c]p yridin-1-yl)methyl)tetrahy dro-2H-pyran-4-yl)methan ol | 421 |
| **2-20** | 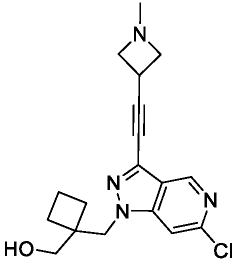 | (1-((6-chloro-3-((1-methyl azetidin-3-yl)ethynyl)-1H-pyrazolo[4,3-c]pyridin-1-y l)methyl)cyclobutyl)metha nol | 345 |
| **2-21** | 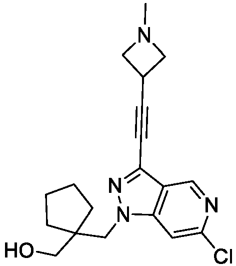 | (1-((6-chloro-3-((1-methyl azetidin-3-yl)ethynyl)-1H-pyrazolo[4,3-c]pyridin-1-y l)methyl)cyclopentyl)meth anol | 359 |
| **2-22** | 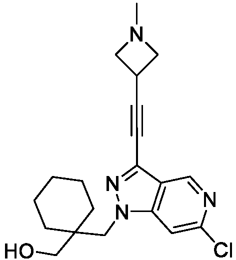 | (1-((6-chloro-3-((1-methyl azetidin-3-yl)ethynyl)-1H-pyrazolo[4,3-c]pyridin-1-y l)methyl)cyclohexyl)meth anol | 373 |
| **2-23** | 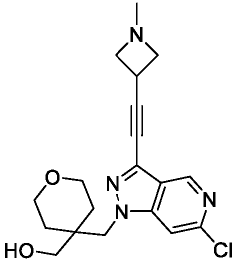 | (4-((6-chloro-3-((1-methyl azetidin-3-yl)ethynyl)-1H-pyrazolo[4,3-c]pyridin-1-y 1)methyl)tetrahydro-2H-py ran-4-yl)methanol | 375 |

### Intermediate 3-1: Preparation of tert-butyl 3-((methylsulfonyl)methyl)azetidine-1-carboxylate

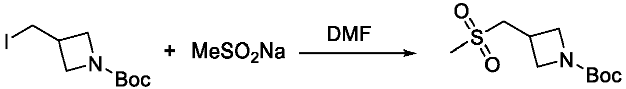

Tert-butyl 3-iodoazetidine-1-carboxylate (5.00 g, 16.83 mmol) was dissolved in DMF (30 mL), and sodium methanesulfinate (2.58 g, 25.24 mmol) was added. The mixture was heated to 70°C and stirred for 3 hours. After the reaction was completed, ethyl acetate was added to dilute the reaction solution, and the mixture was washed with water and saturated brine in sequence and dried over anhydrous sodium sulfate. It was filtered, and the filtrate was concentrated and then separated by using column chromatography to obtain tert-butyl 3-((methylsulfonyl)methyl)azetidine-1-carboxylate (3.20 g, yield: 68%).

¹HNMR (400 MHz, DMSO-*d*₆) δ 3.97 (t, 2H), 3.70 (d, 2H), 3.48 (d, *J* = 7.5 Hz, 2H), 3.04 - 2.99 (m, 1H), 2.95 (s, 3H), 1.37 (s, 9H).

### Intermediate 3-2: Preparation of tert-butyl (S)-3-(((trifluoromethyl)sulfonyl)methyl)pyrrolidine-1-carboxylate

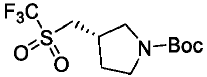

### Step 1: Synthesis of tert-butyl (S)-3-(((trifluoromethyl)thio)methyl)pyrrolidine-1-carboxylate

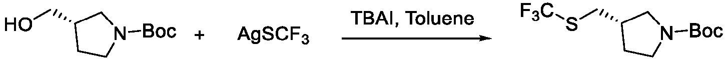

Tetrabutylammonium iodide (132.14 g, 357.73 mmol) and AgSCF₃ (25.03 g, 119.24 mmol) were added to toluene (300 mL), and tert-butyl (S)-3-(hydroxymethyl)pyrrolidine-1-carboxylate (6.0 g, 29.81 mmol) was added under nitrogen protection. The mixture was heated to 120°C and reacted overnight. The reaction solution was filtered with silica gel and washed with ethyl acetate three times. The organic layer was washed with saturated brine, dried over sodium sulfate and concentrated. The product was separated by using column chromatography [petroleum ether/ethyl acetate = 0-20%] to obtain tert-butyl (S)-3-(((trifluoromethyl)thio)methyl)pyrrolidine-1-carboxylate (5.0 g, purity: 93.0%, yield: 54.6%). MS m/z (ESI): 230.1 [M-56]⁺.

¹HNMR (400 MHz, CDCl₃) δ 3.67 - 3.38 (m, 2H), 3.38 - 3.25 (m, 1H), 3.04 (q, J = 10.0, 9.6 Hz, 1H), 2.91 (t, J = 7.6 Hz, 2H), 2.47 (q, J = 7.6 Hz, 1H), 2.10 (tt, J = 7.6, 3.6 Hz, 1H), 1.69 - 1.56 (m, 1H), 1.45 (s, 9H).

### Step 2: Synthesis of tert-butyl (S)-3-(((trifluoromethyl)sulfonyl)methyl)pyrrolidine-1-carboxylate

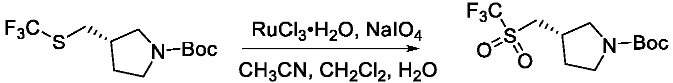

Ruthenium trichloride hydrate (0.79 g, 3.51 mmol) and sodium periodate (11.24 g, 52.57 mmol) were added in sequence at room temperature to a mixed solution of tert-butyl (S)-3-(((trifluoromethyl)thio)methyl)pyrrolidine-1-carboxylate (5.0 g, 17.52 mmol) in dichloromethane (20 mL), acetonitrile (20 mL) and water (20 mL), and the mixture was reacted at room temperature for 2 hours. The reaction solution was diluted with water, extracted three times with dichloromethane, and the organic phase was combined, washed with saturated brine and dried over anhydrous sodium sulfate in sequence. It was filtered, concentrated and separated by using column chromatography to obtain tert-butyl (S)-3-(((trifluoromethyl)sulfonyl)methyl)pyrrolidine-1-carboxylate (2.9 g, yield: 52.1%). MS m/z (ESI): 262.0 [M-56]⁺.

### Intermediate 3-3: Preparation of tert-butyl (S)-3-((ethylsulfonyl)methyl)pyrrolidine-1-carboxylate

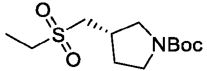

### Step 1: Synthesis of tert-butyl (S)-3-((tosyloxy)methyl)pyrrolidine-1-carboxylate

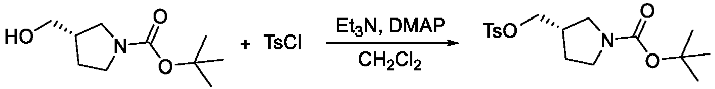

Tert-butyl (S)-3-(hydroxymethyl)pyrrolidine-1-carboxylate (15.0 g, 74.53 mmol) was dissolved in CH₂Cl₂ (200 mL), and then DMAP (4.55 g, 37.26 mmol) and Et₃N (31 mL, 223.58 mmol) were added. TsCl (21.31 g, 111.79 mmol) was added at 0°C and the mixture was reacted at room temperature for 3 hours. Water and dichloromethane were added to the reaction solution, the solution were separated, and the aqueous phase was extracted twice with dichloromethane. The organic phase was combined, washed sequentially with saturated brine, and dried over anhydrous sodium sulfate. It was filtered, concentrated and separated by using column chromatography to obtain tert-butyl (S)-3-((tosyloxy)methyl)pyrrolidine-1-carboxylate (23.5 g, yield: 88.7%).

¹HNMR (400 MHz, CDCl₃) δ 7.78 (d, J = 8.3 Hz, 2H), 7.35 (d, J = 7.9 Hz, 2H), 3.95 (qd, J = 9.8, 7.0 Hz, 2H), 3.45 (dd, J = 11.2, 7.5 Hz, 1H), 3.40 - 3.22 (m, 2H), 3.00 (dd, J = 11.2, 6.9 Hz, 1H), 2.53 (dq, J = 14.4, 7.2 Hz, 1H), 2.45 (s, 3H), 1.94 (s, 1H), 1.65 (s, 1H), 1.43 (s, 9H).

### Step 2: Synthesis of tert-butyl (S)-3-((ethylsulfonyl)methyl)pyrrolidine-1-carboxylate

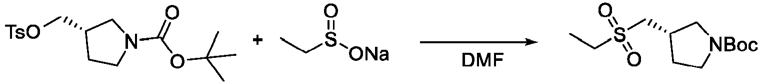

Tert-butyl (S)-3-((tosyloxy)methyl)pyrrolidine-1-carboxylate (8.0 g, 22.51 mmol) was dissolved in DMF (60 mL), potassium iodide (11.21 g, 67.52 mmol) and sodium ethanesulfinate (7.84 g, 67.52 mmol) were added in sequence, and the mixture was heated to 120°C and reacted for 2 hours. The reaction solution was cooled to room temperature, concentrated, and separated by using column chromatography to obtain tert-butyl (S)-3-((ethylsulfonyl)methyl)pyrrolidine-1-carboxylate (5.4 g, yield: 86.5%).

### Intermediate 3-4: Preparation of tert-butyl (S)-3-((cyclobutylsulfonyl)methyl)pyrrolidine-1-carboxylate

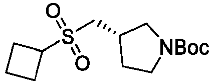

### Step 1: Synthesis of cyclobutanesulfonyl chloride

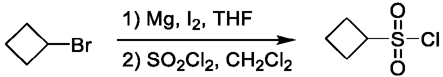

Magnesium chips (0.41 g, 8.33 mmol) were added to anhydrous tetrahydrofuran (5 mL), and cyclobutyl bromide (0.30 g, 2.22 mmol) and iodine (0.14 g, 0.56 mmol) were added. The mixture was heated with a hair dryer while being stirred until the reaction triggered the system to become colorless. A solution of the remaining cyclobutyl bromide (1.20 g, 8.89 mmol) in tetrahydrofuran (15 mL) was slowly added dropwise. After the addition, the mixture was refluxed for 1 hour and cooled to room temperature for use as a suspension. Under nitrogen, a solution of chlorosulfonyl chloride (2.70 mL, 33.34 mmol) in anhydrous dichloromethane (10 mL) was cooled to 0°C, and the above suspension was slowly added. After the addition, the mixture was warmed to room temperature and reacted for 1 hour. The reaction solution was concentrated to dryness, and n-heptane (25 mL) was added for slurrying. It was filtered, and the filtrate was concentrated to dryness to obtain crude cyclobutanesulfonyl chloride (0.95 g, yield: 55.2%), which was directly used for the next reaction.

### Step 2: Synthesis of sodium cyclobutanesulfinate

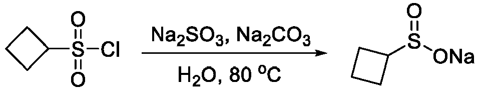

A solution of sodium sulfite (1.55 g, 12.29 mmol) in water (10 mL) was heated to 80°C, and the crude cyclobutanesulfonyl chloride (0.95 g, 6.14 mmol) and sodium carbonate (1.30 g, 12.29 mmol) were added in sequence. The mixture was kept at 80°C for 1 hour and then concentrated to dryness. Methanol was added for slurrying, and the mixture was filtered. The filtrate was concentrated to dryness to obtain crude sodium cyclobutanesulfinate (0.86 g, yield: 98.5%), which was directly used for the next reaction.

### Step 3: Synthesis of tert-butyl (S)-3-((cyclobutylsulfonyl)methyl)pyrrolidine-1-carboxylate

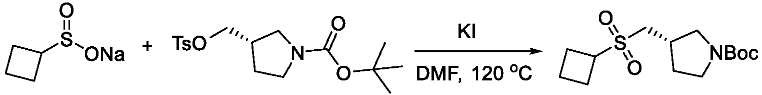

Potassium iodide (1.0 g, 6.02 mmol) and crude sodium cyclobutanesulfinate (0.86 g, 6.08 mmol) were added to a solution of tert-butyl (S)-3-((tosyloxy)methyl)pyrrolidine-1-carboxylate (0.80 g, 2.25 mmol) in N,N-dimethylformamide (10 mL) in sequence at room temperature, and the mixture was heated to 120°C and reacted for 2 hours. The reaction solution was concentrated and separated by using column chromatography [petroleum ether/ethyl acetate = 0-15%] to obtain tert-butyl (S)-3-((cyclobutylsulfonyl)methyl)pyrrolidine-1-carboxylate (0.50 g, yield: 73.2%).

¹HNMR (400 MHz, CDCl₃) δ 3.80 - 3.65 (m, 2H), 3.47 (ddd, J = 14.0, 7.3, 3.0 Hz, 1H), 3.30 (ddt, J = 11.0, 8.9, 5.4 Hz, 1H), 3.05 (dd, J = 10.9, 8.1 Hz, 1H), 2.89 (s, 1H), 2.82 - 2.66 (m, 1H), 2.65 - 2.51 (m, 2H), 2.35 - 2.17 (m, 3H), 2.07 (tt, J = 9.9, 3.7 Hz, 2H), 1.84 - 1.60 (m, 2H), 1.44 (s, 9H).

**Intermediates 3-5 to 3-15 can be prepared by selecting the corresponding raw materials with reference to the partial routes of intermediate 3-3 or 3-4.**

| **Intermediate No.** | **Structural Formula** | **Chemical Name** | **MS: *m*/*z* [M+H]⁺** |
|---|---|---|---|
| **3-5** | 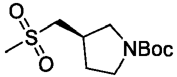 | tert-butyl (R)-3-((methylsulfonyl)methy l)pyrrolidine-1-carboxylate | 264 |
| **3-6** | 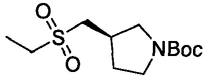 | tert-butyl (R)-3-((ethylsulfonyl)methyl) pyrrolidine-1-carboxylate | 278 |
| **3-7** | 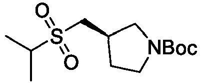 | tert-butyl (R)-3-((isopropylsulfonyl)met hyl)pyrrolidine-1-carboxylate | 292 |
| **3-8** | 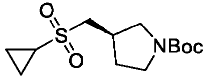 | tert-butyl (R)-3-((cyclopropylsulfonyl) methyl)pyrrolidine-1-carboxy late | 290 |
| **3-9** | 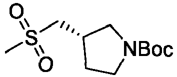 | tert-butyl (S)-3-((methylsulfonyl)methy l)pyrrolidine-1-carboxylate | 264 |
| **3-10** | 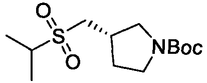 | tert-butyl (S)-3-((isopropylsulfonyl)met hyl)pyrrolidine-1-carboxylate | 292 |
| **3-11** | 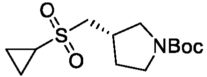 | tert-butyl (S)-3-((cyclopropylsulfonyl) methyl)pyrrolidine-1-carboxy late | 290 |
| **3-12** | 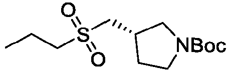 | tert-butyl (S)-3-((propylsulfonyl)methyl )pyrrolidine-1-carboxylate | 292 |
| **3-13** | 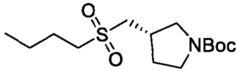 | tert-butyl (S)-3-((butylsulfonyl)methyl) pyrrolidine-1-carboxylate | 306 |
| **3-14** | 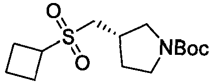 | tert-butyl (S)-3-((cyclobutylsulfonyl)m ethyl)pyrrolidine-1-carboxylate | 304 |
| **3-15** | 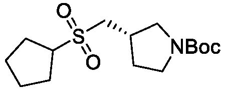 | tert-butyl (S)-3-((cyclopentylsulfonyl) methyl)pyrrolidine-1-carboxy late | 318 |

### Intermediate 4-1: Preparation of 1-((1-(((tert-butyldimethylsilyl)oxy)methyl)cyclobutyl)methyl)-6-chloro-3-(3-((met hylsulfonyl)methyl)azetidin-1-yl)-1H-pyrazolo[4,3-c]pyridine

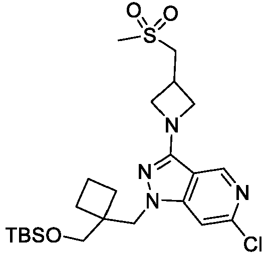

### Step 1: Synthesis of 3-((methylsulfonyl)methyl)azetidine hydrochloride

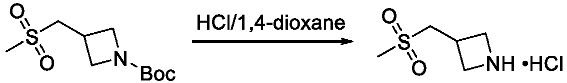

Tert-butyl 3-((methylsulfonyl)methyl)azetidine-1-carboxylate (3.90 g, 15.64 mmol) was dissolved in 1,4-dioxane (4.0 mL), hydrochloric acid (4.0 mL, 16.0 mmol) was added, and the mixture was stirred at room temperature for 1 hour. After the reaction was completed, the reaction solution was concentrated to obtain 3-((methylsulfonyl)methyl)azetidine hydrochloride (3.30 g, yield: 90%).

¹HNMR (400 MHz, MeOH-*d*₄) δ 4.07 - 3.97 (m, 2H), 3.89 - 3.79 (m, 2H), 3.60 (d, *J* = 7.6 Hz, 2H), 3.31 - 3.23 (m, 1H), 3.00 (s, 3H).

### Step 2: Synthesis of 1-((1-(((tert-butyldimethylsilyl)oxy)methyl)cyclobutyl)methyl)-6-chloro-3-(3-((meth ylsulfonyl)methyl)azetidin-1-yl)-1H-pyrazolo[4,3-c]pyridine

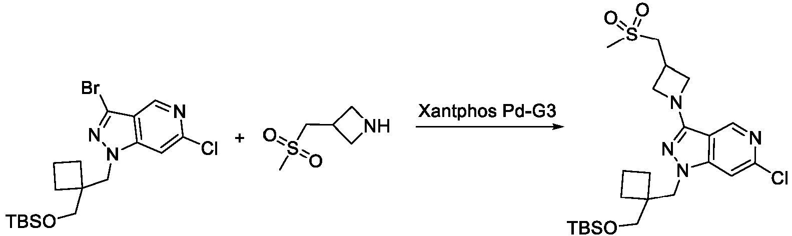

3-bromo-1-((1-(((tert-butyldimethylsilyl)oxy)methyl)cyclobutyl)methyl)-6-chloro-1H-pyrazolo[4,3-c]pyridine (0.60 g, 1.24 mmol) was added to 1,4-dioxane (15.0 mL), and then 3-((methylsulfonyl)methyl)azetidine hydrochloride (0.35 g, 1.86 mmol), Cs₂CO₃ (1.21 g, 3.72 mmol) and Xantphos Pd-G3 (0.35 g, 0.37 mmol) were added in sequence. Under nitrogen, the mixture was heated to 80°C under nitrogen and reacted overnight. After the reaction was completed, the reaction solution was cooled to room temperature, quenched by adding saturated brine, and extracted with EtOAc. The organic layer was washed with saturated brine and dried over anhydrous sodium sulfate. It was filtered and concentrated, and the residue was separated by using column chromatography to obtain 1-((1-(((tert-butyldimethylsilyl)oxy)methyl)cyclobutyl)methyl)-6-chloro-3-(3-((methyls ulfonyl)methyl)azetidine-1-yl)-1H-pyrazolo[4,3-c]pyridine (0.50 g, purity: 58.5%, yield: 45.7%). MS m/z (ESI): 513.2 [M+H]⁺.

### Intermediate 4-2: Preparation of (S)-(1-((6-chloro-3-(3-((ethylsulfonyl)methyl)pyrrolidin-1-yl)-1H-pyrazolo[4,3-c]py ridin-1-yl)methyl)cyclopentyl)methanol

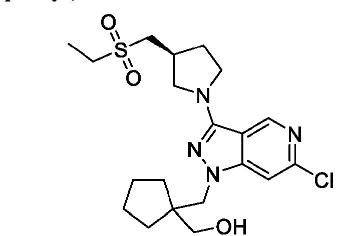

### Step 1: Synthesis of (S)-3-((ethylsulfonyl)methyl)pyrrolidine hydrochloride

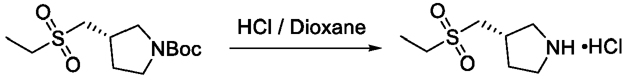

Tert-butyl (S)-3-((ethylsulfonyl)methyl)pyrrolidine-1-carboxylate (5.4 g, 19.47 mmol) was dissolved in 1,4-dioxane (15 mL), and then HCl/1,4-dioxane solution (14.6 mL, 4M, 58.40 mmol) was slowly added dropwise and reacted at room temperature for 1 hour. After the reaction was completed, the reaction solution was concentrated to obtain crude (S)-3-((ethylsulfonyl)methyl)pyrrolidine hydrochloride (3.75 g, yield: 90.1%), which was directly used in the next reaction.

### Step 2: Synthesis of methyl (S)-1-((6-chloro-3-(3-((ethylsulfonyl)methyl)pyrrolidin-1-yl)-1H-pyrazolo[4,3-c]pyr idin-1-yl)methyl)cyclopentane-1-carboxylate

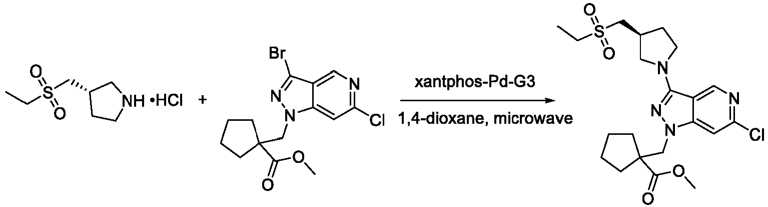

(S)-3-((ethylsulfonyl)methyl)pyrrolidine hydrochloride (0.34 g, 1.61 mmol) and methyl 1-((3-bromo-6-chloro-1H-pyrazolo[4,3-c]pyridin-1-yl)methyl)cyclopentane-1-carboxyla te (0.40 g, 1.07 mmol) were dissolved in 1,4-dioxane (8 mL), and then Cs₂CO₃ (1.40 g, 4.29 mmol) and xantphos Pd-G3 (0.15 g, 0.16 mmol) were added. After the nitrogen substitution was carried out, the mixture was heated in a microwave oven at 85°C for 4 hours. After the reaction solution was cooled, ethyl acetate was added to dilute it, and then the reaction solution was washed once with water and saturated brine respectively. The organic phase was washed with saturated brine and dried over anhydrous sodium sulfate. It was filtered and concentrated, and the crude product was separated by using column chromatography to obtain methyl (S)-1-((6-chloro-3-(3-((ethylsulfonyl)methyl)pyrrolidin-1-yl)-1H-pyrazolo[4,3-c]pyridin -1-yl)methyl)cyclopentane-1-carboxylate (0.33 g, purity: 77.5%, yield: 51.2%). MS m/z (ESI): 469.2 [M+H]⁺.

### Step 3: Synthesis of (S)-(1-((6-chloro-3-(3-((ethylsulfonyl)methyl)pyrrolidin-1-yl)-1H-pyrazolo[4,3-c]py ridin-1-yl)methyl)cyclopentyl)methanol

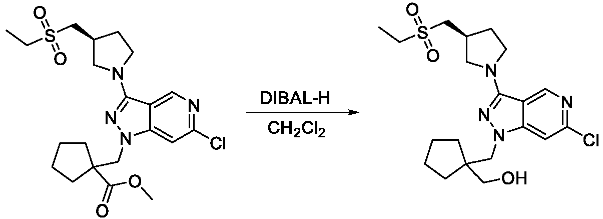

(S)-1-((6-chloro-3-(3-((ethylsulfonyl)methyl)pyrrolidin-1-yl)-1H-pyrazolo[4,3-c]p yridin-1-yl)methyl)cyclopentane-1-carboxylate (0.60 g, 0.99 mmol) was dissolved in dichloromethane (8 mL), the mixture was cooled to -78°C, and DIBAL-H solution (4.06 mL, 1.0 M, 4.06 mmol) was slowly added dropwise. The mixture was maintained at the low temperature and reacted for 1 hour. Saturated aqueous ammonium chloride solution was added to the reaction solution to quench the reaction, and then the solution was extracted twice with dichloromethane. The organic phase was combined, washed sequentially with saturated brine, and dried over anhydrous sodium sulfate. It was filtered and concentrated, and the crude product was separated by using column chromatography (eluent: CH₂Cl₂/MeOH 0-3%) to obtain (S)-(1-((6-chloro-3-(3-((ethylsulfonyl)methyl)pyrrolidin-1-yl)-1H-pyrazolo[4,3-c]pyridi n-1-yl)methyl)cyclopentyl)methanol (0.43 g, purity: 87.0%, yield: 85.6%). MS m/z (ESI): 441.2 [M+H]⁺.

**Intermediates 4-3 to 4-23 can be prepared by selecting the corresponding raw materials with reference to the synthesis methods of intermediate 4-1 or 4-2.**

| **Intermediate No.** | **Structural Formula** | **Chemical Name** | **MS: *m*/*z* [M+H]⁺** |
|---|---|---|---|
| **4-3** | 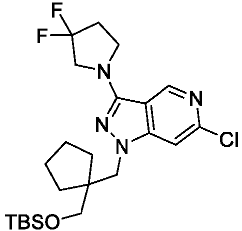 | 1-((1-(((tert-butyldimethyl silyl)oxy)methyl)cyclopent yl)methyl)-6-chloro-3-(3,3 -difluoropyrrolidin-1-yl)-1 H-pyrazolo[4,3-c]pyridine | 485 |
| **4-4** | 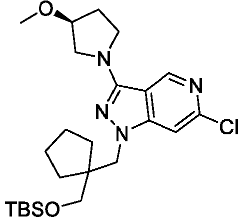 | (S)-1-((1-(((tert-butyldimet hylsilyl)oxy)methyl)cyclo pentyl)methyl)-6-chloro-3-(3-methoxypyrrolidin-1-yl )-1H-pyrazolo[4,3-c]pyridine | 479 |
| **4-5** | 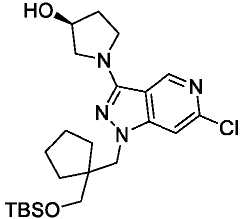 | (S)-1-(1-((1-(((tert-butyldi methylsilyl)oxy)methyl)cy clopentyl)methyl)-6-chloro -1H-pyrazolo[4,3-c]pyridin-3-yl)pyrrolidin-3-ol | 465 |
| **4-6** | 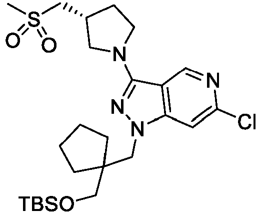 | (R)-1-((1-(((tert-butyldime thylsilyl)oxy)methyl)cyclo pentyl)methyl)-6-chloro-3-(3-((methylsulfonyl)methy l)pyrrolidin-1-yl)-1H-pyrazolo[4,3-c]pyridine | 541 |
| **4-7** | 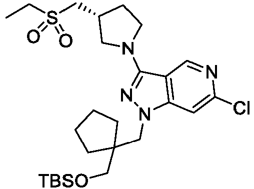 | (R)-1-((1-(((tert-butyldime thylsilyl)oxy)methyl)cyclo pentyl)methyl)-6-chloro-3-(3-((ethylsulfonyl)methyl) pyrrolidin-1-yl)-1H-pyrazolo[4,3-c]pyridine | 555 |
| **4-8** | 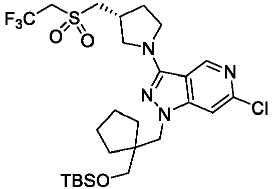 | (R)-1-((1-(((tert-butyldime thylsilyl)oxy)methyl)cyclo pentyl)methyl)-6-chloro-3-(3-(((2,2,2-trifluoroethyl)s ulfonyl)methyl)pyrrolidin-1-yl)-1H-pyrazolo[4,3-c]pyridine | 609 |
| **4-9** | 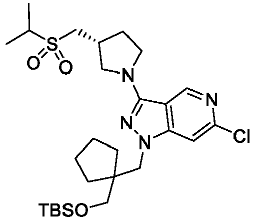 | (R)-1-((1-(((tert-butyldime thylsilyl)oxy)methyl)cyclo pentyl)methyl)-6-chloro-3-(3-((isopropylsulfonyl)met hyl)pyrrolidin-1-yl)-1H-pyrazolo[4,3-c]pyridine | 569 |
| **4-10** | 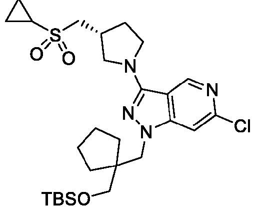 | (R)-1-((1-(((tert-butyldime thylsilyl)oxy)methyl)cyclo pentyl)methyl)-6-chloro-3-(3-((cyclopropylsulfonyl) methyl)pyrrolidin-1-yl)-1 H-pyrazolo[4,3-c]pyridine | 567 |
| **4-11** | 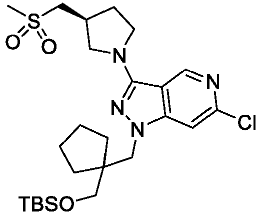 | (S)-1-((1-(((tert-butyldimet hylsilyl)oxy)methyl)cyclo pentyl)methyl)-6-chloro-3-(3-((methylsulfonyl)methy l)pyrrolidin-1-yl)-1H-pyrazolo[4,3-c]pyridine | 541 |
| **4-12** | 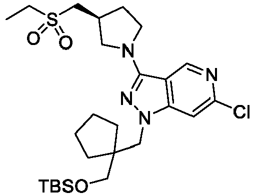 | (S)-1-((1-(((tert-butyldimet hylsilyl)oxy)methyl)cyclo pentyl)methyl)-6-chloro-3-(3-((ethylsulfonyl)methyl) pyrrolidin-1-yl)-1H-pyrazolo[4,3-c]pyridine | 555 |
| **4-13** | 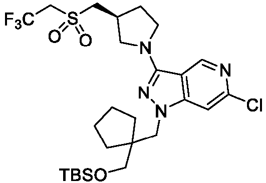 | (S)-1-((1-(((tert-butyldimet hylsilyl)oxy)methyl)cyclo pentyl)methyl)-6-chloro-3-(3-(((2,2,2-trifluoroethyl)s ulfonyl)methyl)pyrrolidin-1-yl)-1H-pyrazolo[4,3-c]pyridine | 609 |
| **4-14** | 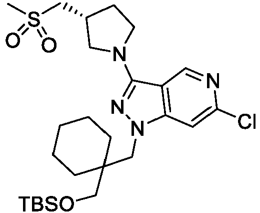 | (R)-1-((1-(((tert-butyldime thylsilyl)oxy)methyl)cyclo hexyl)methyl)-6-chloro-3-( 3-((methylsulfonyl)methyl )pyrrolidin-1-yl)-1H-pyrazolo[4,3-c]pyridine | 555 |
| **4-15** | 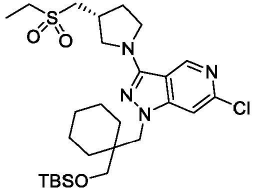 | (R)-1-((1-(((tert-butyldime thylsilyl)oxy)methyl)cyclo hexyl)methyl)-6-chloro-3-( 3-((ethylsulfonyl)methyl)p yrrolidin-1-yl)-1H-pyrazolo[4,3-c]pyridine | 569 |
| **4-16** | 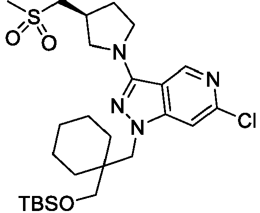 | (S)-1-((1-(((tert-butyldimet hylsilyl)oxy)methyl)cyclo hexyl)methyl)-6-chloro-3-( 3-((methylsulfonyl)methyl )pyrrolidin-1-yl)-1H-pyrazolo[4,3-c]pyridine | 555 |
| **4-17** | 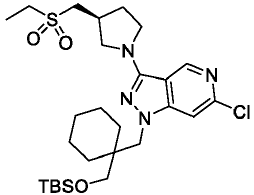 | (S)-1-((1-(((tert-butyldimet hylsilyl)oxy)methyl)cyclo hexyl)methyl)-6-chloro-3-( 3-((ethylsulfonyl)methyl)p yrrolidin-1-yl)-1H-pyrazolo[4,3-c]pyridine | 569 |
| **4-18** | 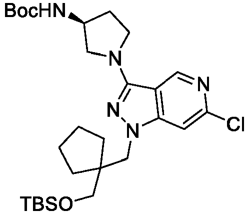 | tert-butyl-(S)-(1-(1-((1-(((t ert-butyldimethylsilyl)oxy) methyl)cyclopentyl)methyl )-6-chloro-1H-pyrazolo[4, 3-c]pyridin-3-yl)pyrrolidin -3-yl)carbamate | 564 |
| **4-19** | 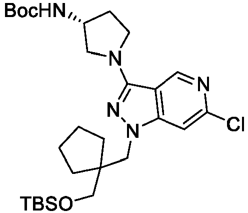 | tert-butyl-(R)-(1-(1-((1-(((t ert-butyldimethylsilyl)oxy) methyl)cyclopentyl)methyl )-6-chloro-1H-pyrazolo[4, 3-c]pyridin-3-yl)pyrrolidin -3-yl)carbamate | 564 |
| **4-20** | 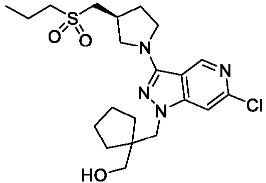 | (S)-(1-((6-chloro-3-(3-((pr opylsulfonyl)methyl)pyrro lidin-1-yl)-1H-pyrazolo[4, 3-c]pyridin-1-yl)methyl)cyclopentyl)methanol | 455 |
| **4-21** | 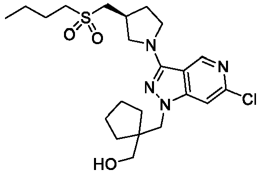 | (S)-(1-((6-chloro-3-(3-((bu tylsulfonyl)methyl)pyrroli din-1-yl)-1H-pyrazolo[4,3-c]pyridin-1-yl)methyl)cyclopentyl)methanol | 469 |
| **4-22** | 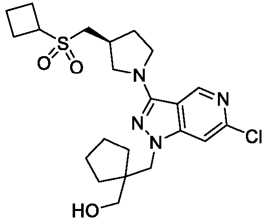 | (S)-(1-((6-chloro-3-(3-((cy clobutylsulfonyl)methyl)p yrrolidin-1-yl)-1H-pyrazol o[4,3-c]pyridin-1-yl)methy l)cyclopentyl)methanol | 467 |
| **4-23** | 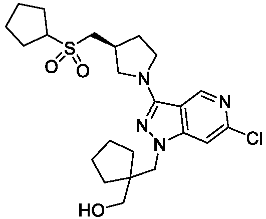 | (S)-(1-((6-chloro-3-(3-((cy clopentylsulfonyl)methyl) pyrrolidin-1-yl)-1H-pyrazo lo[4,3-c]pyridin-1-yl)meth yl)cyclopentyl)methanol | 481 |

### Intermediate 5-1: Preparation of 4-(4-aminopyrimidin-2-yl)-2-methyl-1-((2-(trimethylsilyl)ethoxy)methyl)-1,2-dihyd ro-3H-pyrazol-3-one

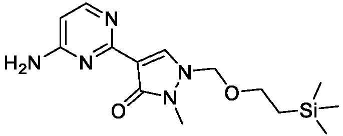

### Step 1: Synthesis of 2-methyl-1-((2-(trimethylsilyl)ethoxy)methyl)-1,2-dihydro-3H-pyrazol-3-one

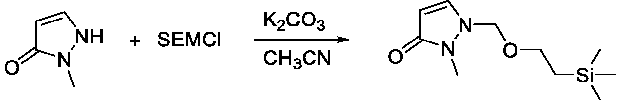

2-methyl-2,3-dihydro-1H-pyrazol-3-one (6.0 g, 61.16 mmol) was dissolved in CH₃CN (80 mL), and then K₂CO₃ (36.0 g, 260.52 mmol) and SEMCl (22.0 mL, 124.15 mmol) were added. The reaction was stirred at room temperature for 18 hours. It was filtered, and the filterate was concentrated. The solid was added to a PE/EA (10:1) mixture for slurrying. The mixture was filtered, and the solid was dried in vacuum to obtain 2-methyl-1-((2-(trimethylsilyl)ethoxy)methyl)-1,2-dihydro-3H-pyrazol-3-one (12.1 g, yield: 82.3%).

### Step 2: Synthesis of 4-bromo-2-methyl-1-((2-(trimethylsilyl)ethoxy)methyl)-1,2-dihydro-3H-pyrazol-3-o ne

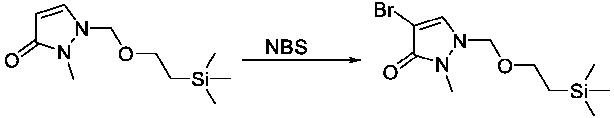

2-methyl-1-((2-(trimethylsilyl)ethoxy)methyl)-1,2-dihydro-3H-pyrazol-3-one (12.1 g, 52.98 mmol) was dissolved in CH₃CN (250 mL). The mixture was cooled to 0°C, and NBS (14.15 g, 79.48 mmol) was added. The mixture was stirred at room temperature for 3 hours. After the reaction was completed, saturated aqueous Na₂S₂O₃ solution was added to quench the reaction, and the mixture was extracted with CH₂Cl₂. The organic phase was washed with saturated aqueous NaCl solution, dried over anhydrous Na₂SO₄, filtered and concentrated. The residue was added to the PE/EA (10:1) mixture and slurried, filtered, and the solid was dried in vacuum to obtain 4-bromo-2-methyl-1-((2-(trimethicone)ethoxy)methyl)-1,2-dihydro-3H-pyrazol-3-one (12.00 g, yield: 70%).

### Step 3: Synthesis of tert-butyl (tert-butyloxycarbonyl)(2-chloropyrimidin-4-yl)carbamate

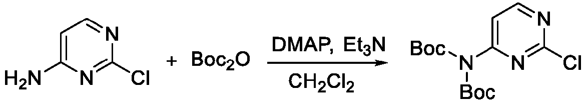

2-chloropyrimidin-4-amine (8.00 g, 61.75 mmol) was dissolved in THF (100 mL), and DMAP (0.75 g, 6.18 mmol), Et₃N (34.3 mL, 247.0 mmol) and Boc₂O (40.43 g, 185.26 mmol) were added. The mixture was stirred at room temperature and reacted for 18 hours. After the reaction was completed, EtOAc and water were added for dilution and extraction, and the organic phase was washed with saturated aqueous NaCl solution, dried over anhydrous Na₂SO₄, filtered and concentrated. The residue was slurried in petroleum ether, filtered, and the filter cake was dried in vacuum to obtain tert-butyl (tert-butoxycarbonyl)(2-chloropyrimidin-4-yl)carbamate (19.72 g, yield: 92%).

### Step 4: Synthesis of tert-butyl (tert-butyloxycarbonyl)(2-(2-methyl-3-oxy-1-((2-(trimethylsilyl)ethoxy)methyl)-2,3-dihydro-1H-pyrazol-4-yl)pyrimidin-4-yl)carbamate

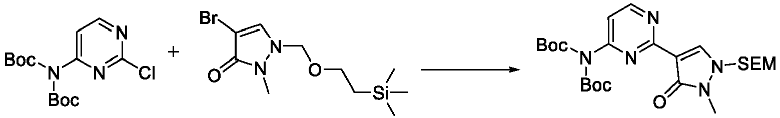

4-bromo-2-methyl-1-((2-(trimethylsilyl)ethoxy)methyl)-1,2-dihydro-3H-pyrazol-3-one (0.5 g, 1.63 mmol) was dissolved in a CH₃CN/H₂O mixture (25/2.5 mL), and tert-butyl (tert-butyloxycarbonyl)(2-chloropyrimidin-4-yl)carbamate (2.68 g, 8.14 mmol), B₂Pin₂ (2.11 mL, 8.14 mmol), Na₂CO₃ (0.86 g, 8.14 mmol) and Pd(Amphos)₂Cl₂ (0.23 g, 0.33 mmol) were added. Under nitrogen protection, the mixture was heated to 100°C and reacted for 4 hours. After the reaction was completed, the reaction solution was cooled to room temperature, and EtOAc and H₂O were added for extraction. The organic phase was washed with saturated aqueous NaCl solution, dried over anhydrous Na₂SO₄, filtered, and concentrated. The residue was separated by using column chromatography to obtain tert-butyl (tert-butoxycarbonyl)(2-(2-methyl-3-oxy-1-((2-(trimethylsilyl)ethoxy)methyl)-2,3-dihy dro-1H-pyrazol-4-yl)pyrimidin-4-yl)carbamate (580 mg, yield: 47.8%).

### Step 5: Synthesis of 4-(4-aminopyrimidin-2-yl)-2-methyl-1-((2-(trimethylsilyl)ethoxy)methyl)-1,2-dihyd ro-3H-pyrazol-3-one

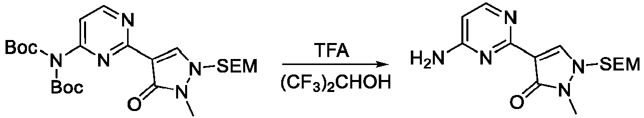

Tert-butyl (tert-butoxycarbonyl)(2-(2-methyl-3-oxy-1-((2-(trimethylsilyl)ethoxy)methyl)-2,3-dihy dro-1H-pyrazol-4-yl)pyrimidin-4-yl)carbamate (1.4 g, 2.68 mmol) was dissolved in hexafluoroisopropanol (15 mL), and TFA (2.8 mL, 37.57 mmol) was added. The mixture was stirred at room temperature and reacted for 3 hours. Saturated aqueous NaHCO₃ solution was added for neutralization, and the mixture was extracted with EtOAc. The organic phase was washed with saturated aqueous NaCl solution, dried over anhydrous Na₂SO₄, filtered and concentrated. The residue was separated by using column chromatography to obtain 4-(4-aminopyrimidin-2-yl)-2-methyl-1-((2-(trimethylsilyl)ethoxy)methyl)-1,2-dihydro-3 H-pyrazol-3-one (0.60 g, yield: 69.6%).

### II. Preparation of Compounds of Examples

### Example A1: Preparation of (S)-1¹,5-dimethyl-4³-(prop-1-yn-1-yl)-1¹H,4¹H-8-oxa-3-aza-4(6,1)-pyrazolo[4,3-c]py ridina-2(2,4)-pyrimidina-1(4,5)-pyrazolacyclooctaphane

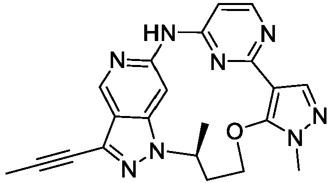

### Step 1: Synthesis of (S)-4-(4-((1-(4-((tert-butyldimethylsilyl)oxy)butan-2-yl)-3-(prop-1-yn-1-yl)-1H-pyra zolo[4,3-c]pyridin-6-yl)amino)pyrimidin-2-yl)-2-methyl-1-((2-(trimethylsilyl)ethoxy )methyl)-1,2-dihydro-3H-pyrazol-3-one

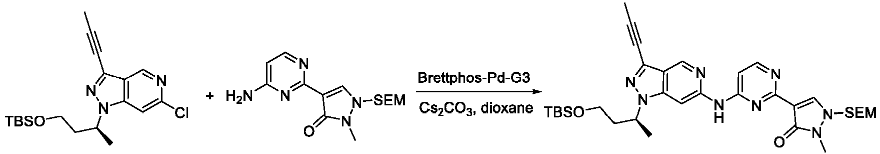

(S)-1-(4-((tert-butyldimethylsilyl)oxy)butan-2-yl)-6-chloro-3-(prop-1-yn-1-yl)-1H-pyrazolo[4,3-c]pyridine (353 mg, 0.93 mmol) and 4-(4-aminopyrimidin-2-yl)-2-methyl-1-((2-(trimethylsilyl)ethoxy)methyl)-1,2-dihydro-3 H-pyrazol-3-one (300 mg, 0.93 mmol) were dissolved in 1,4-dioxane (10 mL), and Cs₂CO₃ (608 mg, 1.86 mmol) and XantPhos Pd-G3 (169 mg, 0.19 mmol) were added. Under nitrogen protection, the mixture was heated to 120°C and stirred overnight. After the reaction was completed, the mixture was cooled to room temperature. Ethyl acetate was added to dilute the solution, and then the solution was washed with saturated brine and dried over anhydrous sodium sulfate. It was filtered and the filtrate was concentrated and separated by using column chromatography to obtain (S)-4-(4-((1-(4-((tert-butyldimethylsilyl)oxy)butan-2-yl)-3-(prop-1-yn-1-yl)-1H-pyrazol o[4,3-c]pyridin-6-yl)amino)pyrimidin-2-yl)-2-methyl-1-((2-(trimethylsilyl)ethoxy)meth yl)-1,2-dihydro-3H-pyrazol-3-one (0.32 g, yield: 51%). MS m/z (ESI): 663.4 [M+H]⁺.

### Step 2: Synthesis of (S)-4-(4-((1-(4-hydroxybutan-2-yl)-3-(prop-1-yn-1-yl)-1H-pyrazolo[4,3-c]pyridin-6-yl)amino)pyrimidin-2-yl)-2-methyl-1,2-dihydro-3H-pyrazol-3-one

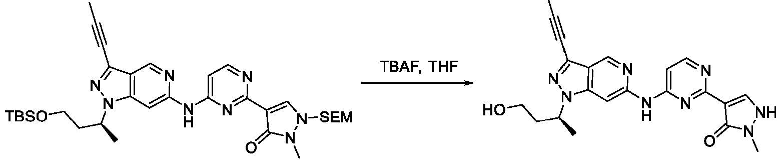

(S)-4-(4-((1-(4-((tert-butyldimethylsilyl)oxy)butan-2-yl)-3-(prop-1-yn-1-yl)-1H-py razolo[4,3-c]pyridin-6-yl)amino)pyrimidin-2-yl)-2-methyl-1-((2-(trimethylsilyl)ethoxy) methyl)-1,2-dihydro-3H-pyrazol-3-one (320 mg, 0.48 mmol) was dissolved in THF (5 mL), and then 1M TBAF/THF solution (2.9 mL, 2.90 mmol) was added. Under nitrogen protection, the mixture was heated to 70°C and stirred for 2 hours. After the reaction was completed, the reaction solution was cooled to room temperature and concentrated. The residue was separated by using column chromatography to obtain (S)-4-(4-((1-(4-hydroxybutan-2-yl)-3-(prop-1-yn-1-yl)-1H-pyrazolo[4,3-c]pyridin-6-yl) amino)pyrimidin-2-yl)-2-methyl-1,2-dihydro-3H-pyrazol-3-one (98 mg, yield: 48%). MS m/z (ESI): 419.2 [M+H]⁺.

### Step 3: Synthesis of (S)-1¹,5-dimethyl-4³-(prop-1-yn-1-yl)-1¹H,4¹H-8-oxa-3-aza-4(6,1)-pyrazolo[4,3-c]py ridina-2(2,4)-pyrimidina-1(4,5)-pyrazolacyclooctaphane

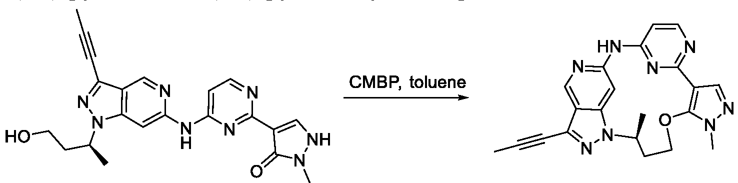

(S)-4-(4-((1-(4-hydroxybutan-2-yl)-3-(prop-1-yn-1-yl)-1H-pyrazolo[4,3-c]pyridin-6-yl)amino)pyrimidin-2-yl)-2-methyl-1,2-dihydro-3H-pyrazol-3-one (98 mg, 0.23 mmol) was dissolved in toluene (5 mL), and CMBP (339 mg, 1.41 mmol) was added. Under nitrogen protection, the mixture was heated to 130°C and reacted for 24 hours. After the reaction was completed, the reaction solution was cooled to room temperature, diluted with CH₂Cl₂, washed with saturated aqueous NaHCO₃, dried over anhydrous sodium sulfate, filtered, and the filtrate was concentrated. The residue was separated by using column chromatography to obtain (S)-1¹,5-dimethyl-4³-(prop-1-yn-1-yl)-1¹*H*,4¹*H*8-oxa-3-aza-4(6,1)-pyrazolo[4,3-c]pyridi na-2(2,4)-pyrimidina-1(4,5)-pyrazolacyclooctaphane-(8.2 mg, yield: 9%). MS m/z (ESI): 401.2 [M+H]⁺.

¹HNMR (400 MHz, DMSO-*d*₆) δ 10.33 (s, 1H), 9.09 (d, *J* = 1.1Hz, 1H), 8.75 (d, *J* = 1.0 Hz, 1H), 8.31 (d, *J* = 5.8 Hz, 1H), 7.93 (s, 1H), 6.74 (d, *J* = 5.8 Hz, 1H), 5.14 (dd, *J* = 10.4, 6.8 Hz, 1H), 4.37 (t, *J* = 11.4 Hz, 1H), 4.01 - 3.87 (m, 1H), 3.76 (s, 3H), 2.17 (s, 3H), 1.99 (t, *J* = 7.6 Hz, 1H), 1.75 (d, *J* = 6.8 Hz, 3H).

**Examples A2 to A3 can be prepared by selecting the corresponding raw materials with reference to the synthesis methods of Example A1:**

| **Example No.** | **Structural Formula** | **Chemical Name** | **MS: *m*/*z* [M+H]⁺** |
|---|---|---|---|
| **A2** | 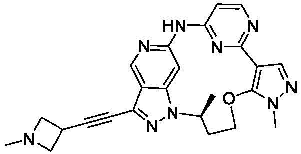 | (S)-1¹,5-dimethyl-4³-((1-me thylazetidin-3-yl)ethynyl)-1¹ *H*,4¹*H*-8-oxa-3-aza-4(6,1)-p yrazolo[4,3-c]pyridina-2(2,4 )-pyrimidina-1(4,5)-pyrazolacyclooctaphane | 456.2 |
| **A3** | 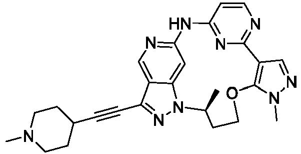 | (S)-1¹,5-dimethyl-4³-((1-me thylpiperidin-4-yl)ethynyl)-1¹*H*,4¹*H*-8-oxa-3-aza-4(6,1) -pyrazolo[4,3-c]pyridina-2( 2,4)-pyrimidina-1(4,5)-pyrazolacyclooctaphane | 484.2 |

### Example 1: Preparation of 1'-methyl-3'-((1-methylpiperidin-4-yl)ethynyl)spiro(cyclopentane-1,6'-8-oxa-3-aza-4(6,1)-pyrazolo[4,3-c]pyridina-2(2,4)-pyrimidina-1(4,5)-pyrazolacyclooctaphane)

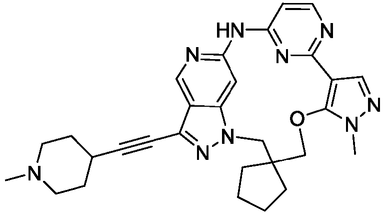

### Step 1: Synthesis of 4-(4-(1-((1-(hydroxymethyl)cyclopentyl)methyl)-3-((1-methylpiperidin-4-yl)ethynyl )-1H-pyrazol[4,3-c]pyridin-6-yl)amino)pyrimidin-2-yl)-2-methyl-1-((2-(trimethylsil yl)ethoxy)methyl)-1,2-dihydro-3H-pyrazol-3-one

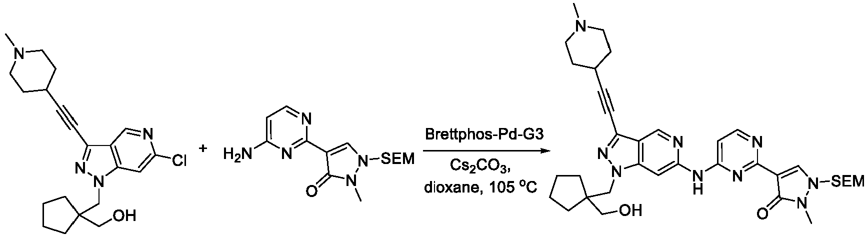

(1-((6-chloro-3-((1-methylpiperidin-4-yl)ethynyl)-1H-pyrazolo[4,3-c]pyridin-1-yl) methyl)cyclopentyl)methanol (245 mg, 0.633 mmol) was dissolved in 1,4-dioxane (15 mL), and then 4-(4-aminopyrimidin-2-yl)-2-methyl-1-((2-(trimethylsilyl)ethoxy)methyl)-1,2-dihydro-3 H-pyrazol-3-one (224 mg, 0.697 mmol), Cs₂CO₃ (413 mg, 1.266 mmol) and Brettphos-Pd-G3 (115 mg, 0.127 mmol) were added. The mixture was heated to 105°C and stirred overnight under nitrogen protection. After the reaction was completed, the reaction solution was cooled to room temperature, concentrated, and separated by using column chromatography to obtain 4-(4-((1-((1-(hydroxymethyl)cyclopentyl)methyl)-3-((1-methylpiperidin-4-yl)ethynyl)-1 H-pyrazolo[4,3-c]pyridin-6-yl)amino)pyrimidin-2-yl)-2-methyl-1-((2-(trimethylsilyl)eth oxy)methyl)-1,2-dihydro-3H-pyrazol-3-one (210 mg, 0.219 mmol, yield: 34.55%). MS m/z (ESI): 672.6 [M+H]⁺.

### Step 2: Synthesis of 4-(4-((1-((1-(hydroxymethyl)cyclopentyl)methyl)-3-((1-methylpiperidin-4-yl)ethyny l)-1H-pyrazolo[4,3-c]pyridin-6-yl)amino)pyrimidin-2-yl)-2-methyl-1,2-dihydro-3H-pyrazol-3-one

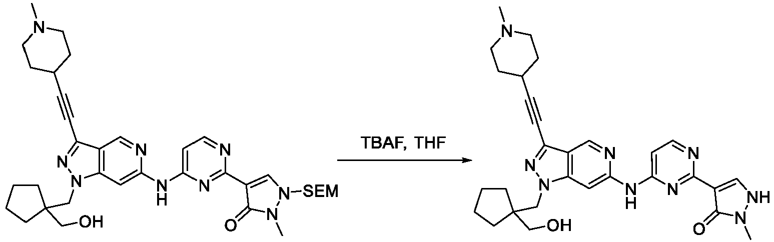

4-(4-((1-((1-(hydroxymethyl)cyclopentyl)methyl)-3-((1-methylpiperidin-4-yl)ethyn yl)-1H-pyrazolo[4,3-c]pyridin-6-yl)amino)pyrimidin-2-yl)-2-methyl-1-((2-(trimethylsil yl)ethoxy)methyl)-1,2-dihydro-3H-pyrazol-3-one (210 mg, 0.219 mmol) was dissolved in THF (12 mL), and TBAF/THF solution (0.46 mL, 1 M, 0.459 mmol) was added. The mixture was heated to 70°C and stirred for 1 hour. After the reaction was completed, the reaction solution was concentrated, the crude product was slurried with methanol, filtered and dried to obtain 4-(4-((1-((1-(hydroxymethyl)cyclopentyl)methyl)-3-((1-methylpiperidin-4-yl)ethynyl)-1 H-pyrazolo[4,3-c]pyridin-6-yl)amino)pyrimidin-2-yl)-2-methyl-1,2-dihydro-3H-pyrazol -3-one (65 mg, 0.120 mmol, yield: 54.9%). MS m/z (ESI): 542.4 [M+H]⁺.

### Step 3: Synthesis of 1'-methyl-3'-((1-methylpiperidin-4-yl)ethynyl)spiro(cyclopentane-1,6'-8-oxa-3-aza-4(6,1)-pyrazolo[4,3-c]pyridina-2(2,4)-pyrimidina-1(4,5)-pyrazolacyclooctaphane)

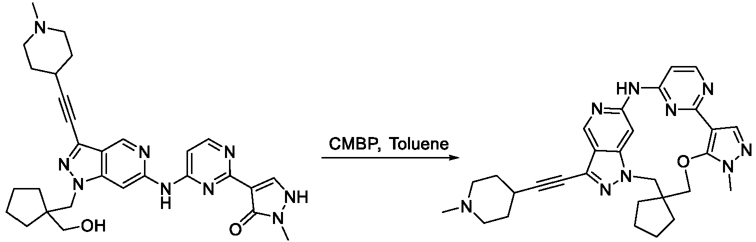

4-(4-((1-((1-(hydroxymethyl)cyclopentyl)methyl)-3-((1-methylpiperidin-4-yl)ethyn yl)-1H-pyrazolo[4,3-c]pyridin-6-yl)amino)pyrimidin-2-yl)-2-methyl-1,2-dihydro-3H-py razol-3-one (60 mg, 0.111 mmol) was dissolved in toluene (15 mL), and then CMBP (214 mg, 0.886 mmol) was added. Under nitrogen protection, the mixture was heated to 130°C and reacted overnight. After the reaction was completed, the mixture was cooled to room temperature, quenched by adding saturated aqueous sodium bicarbonate solution, and then extracted twice with dichloromethane. The organic phase was concentrated and separated by using column chromatography to obtain a crude product, which was then separated by preparative HPLC to obtain 1'-methyl-3'-((1-methylpiperidin-4-yl)ethynyl)spiro(cyclopentane-1,6'-8-oxa-3-aza-4(6 ,1)-pyrazolo[4,3-c]pyiidina-2(2,4)-pyrimidina-1(4,5)-pyrazolacyclooctaphane) (14 mg, 0.024 mmol, yield: 21.7%). MS m/z (ESI): 524.4 [M+H]⁺.

¹H NMR (400 MHz, DMSO-*d*₆) δ 10.51 (s, 1H), 9.54 (s, 1H), 8.74 (s, 1H), 8.34 (d, J = 5.8 Hz, 1H), 8.02 (s, 1H), 6.80 (d, J = 5.8 Hz, 1H), 3.82 (s, 3H), 2.77 - 2.60 (m, 4H), 2.18 (s, 3H), 2.15 - 2.08 (m, 3H), 1.96 - 1.83 (m, 6H), 1.75-1.67 (m, 4H).

**Examples 2 to 12 can be prepared by selecting the corresponding raw materials with reference to the synthesis methods of Example 1 or A1:**

| **Example No.** | **Structural Formula** | **Chemical Name** | **MS: *m*/*z* [M+H]⁺** |
|---|---|---|---|
| **2** | 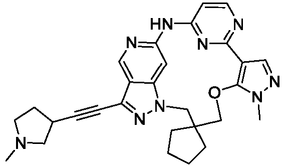 | 1'-methyl-3'-((1-methylpyrr olidin-3-yl)ethynyl)spiro(cyc lopentane-1,6'-8-oxa-3-aza-4(6,1)-pyrazolo[4,3-c]pyridi na-2(2,4)-pyrimidina-1(4,5)-pyrazolacyclooctaphane) | 510.2 |
| **3** | 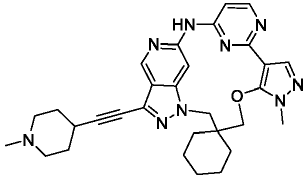 | 1'-methyl-3'-((1-methylpipe ridin-4-yl)ethynyl)spiro(cycl ohexane-1,6'-8-oxa-3-aza-4( 6,1)-pyrazolo[4,3-c]pyridina -2(2,4)-pyrimidina-1(4,5)-pyrazolacyclooctaphane) | 538.4 |
| **4** | 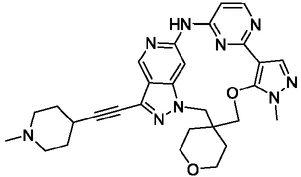 | 1'-methyl-3'-((1-methylpipe ridin-4-yl)ethynyl)-2,3,5,6-te trahydrospiro(pyran-4,6'-8-o xa-3-aza-4(6,1)-pyrazolo[4,3 -c]pyridina-2(2,4)-pyrimidin a-1(4,5)-pyrazolacyclooctaphane) | 540.2 |
| **5** | 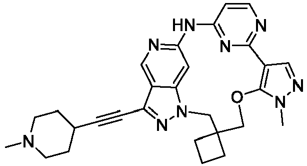 | 1'-methyl-3'-((1-methylpipe ridin-4-yl)ethynyl)spiro(cycl obutane-1,6'-8-oxa-3-aza-4( 6,1)-pyrazolo[4,3-c]pyridina -2(2,4)-pyrimidina-1(4,5)-pyrazolacyclooctaphane) | 510.4 |
| **6** | 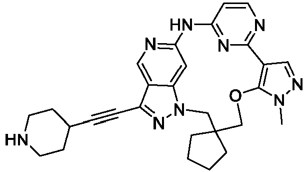 | 1'-methyl-3'-(piperidin-4-yl ethynyl)spiro(cyclopentane-1,6'-8-oxa-3-aza-4(6,1)-pyra zolo[4,3-c]pyridina-2(2,4)-p yrimidina-1(4,5)-pyrazolacyclooctaphane) | 510.4 |
| **7** | 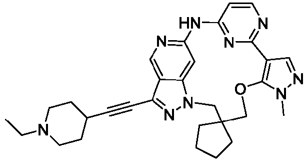 | 3'-((1-ethylpiperidin-4-yl)et hynyl)-1'-methylspiro(cyclo pentane-1,6'-8-oxa-3-aza-4( 6,1)-pyrazolo[4,3-c]pyridina -2(2,4)-pyrimidina-1(4,5)-pyrazolacyclooctaphane) | 538.4 |
| **8** | 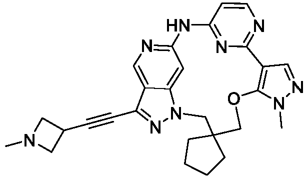 | 1'-methyl-3'-((1-methylazeti din-3-yl)ethynyl)spiro(cyclo pentane-1,6'-8-oxa-3-aza-4( 6,1)-pyrazolo[4,3-c]pyridina -2(2,4)-pyrimidina-1(4,5)-pyrazolacyclooctaphane) | 496.4 |
| **9** | 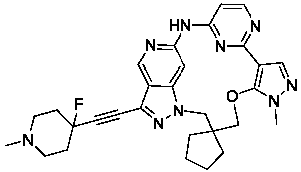 | 3'-((4-fluoro-1-methylpiperi din-4-yl)ethynyl)-1'-methyls piro(cyclopentane-1,6'-8-ox a-3-aza-4(6,1)-pyrazolo[4,3-c]pyridina-2(2,4)-pyrimidina -1(4,5)-pyrazolacyclooctaphane) | 542.4 |
| **10** | 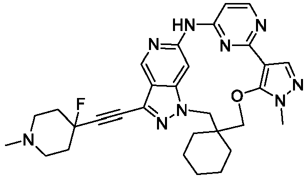 | 3'-((4-fluoro-1-methylpiperi din-4-yl)ethynyl)-1'-methyls piro(cyclohexane-1,6'-8-oxa -3-aza-4(6,1)-pyrazolo[4,3-c ]pyridina-2(2,4)-pyrimidina-1(4,5)-pyrazolacyclooctaphane) | 556.4 |
| **11** | 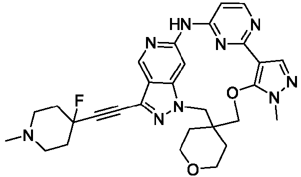 | 3'-((4-fluoro-1-methylpiperi din-4-yl)ethynyl)-1'-methyl-2,3,5,6-tetrahydrospiro(pyra n-4,6'-8-oxa-3-aza-4(6,1)-py razolo[4,3-c]pyridina-2(2,4)-pyrimidina-1(4,5)-pyrazolacyclooctaphane) | 558.4 |
| **12** | 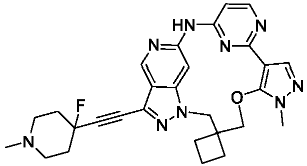 | 3'-((4-fluoro-1-methylpiperi din-4-yl)ethynyl)-1'-methyls piro(cyclobutane-1,6'-8-oxa-3-aza-4(6,1)-pyrazolo[4,3-c] pyridina-2(2,4)-pyrimidina-1 (4,5)-pyrazolacyclooctaphane) | 528.4 |

### Example 13: Preparation of (R)-2-(1-(1'-methylspiro[cyclopentane-1,6'-8-oxa-3-aza-4(6,1)-pyrazolo[4,3-c]pyrid ina-2(2,4)-pyrimidina-1(4,5)-pyrazolacyclooctaphane]-3'-yl)pyrrolidin-3-yl)propan -2-ol

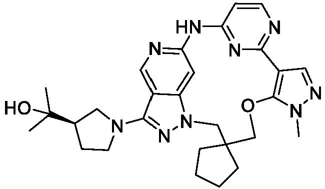

### Step 1: Synthesis of (R)-2-(1-(6-chloro-1-((1-(hydroxymethyl)cyclopentyl)methyl)-1H-pyrazolo[4,3-c]py ridin-3-yl)pyrrolidin-3-yl)propan-2-ol

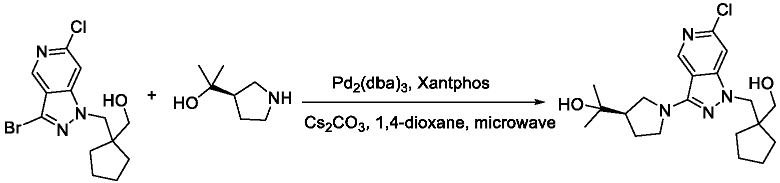

(R)-2-(pyrrolidin-3-yl)propan-2-ol hydrochloride (0.31 g, 1.86 mmol) and (1-((3-bromo-6-chloro-1H-pyrazolo[4,3-c]pyridin-1-yl)methyl)cyclopentyl)methanol (0.40 g, 1.16 mmol) were dissolved in 1,4-dioxane (8 mL), and then Cs₂CO₃ (1.13 g, 3.48 mmol), Pd₂(dba)₃ (0.16 g, 0.17 mmol) and Xantphos (0.20 g, 0.35 mmol) were added. After the nitrogen substitution was carried out, the mixture was heated in a microwave to 80°C and reacted for 2 hours. After the reaction was completed, ethyl acetate was added to dilute the reaction solution, and it was washed once with water and once with saturated brine respectively. The organic layer was separated, washed with saturated brine, and dried over anhydrous sodium sulfate. It was filtered and concentrated, and the resulting crude product was separated by using column chromatography (eluent: CH₂Cl₂/MeOH 0-5%) to obtain (R)-2-(1-(6-chloro-1-((1-(hydroxymethyl)cyclopentyl)methyl)-1H-pyrazolo[4,3-c]pyridi n-3-yl)pyrrolidin-3-yl)propan-2-ol (0.28 g, purity: 72.4%, yield: 44.1%). MS m/z (ESI): 393.2 [M+H]⁺.

### Step 2: Synthesis of (R)-4-(4-((1-((1-(hydroxymethyl)cyclopentyl)methyl)-3-(3-(2-hydroxypropan-2-yl)p yrrolidin-1-yl)-1H-pyrazolo[4,3-c]pyridin-6-yl)amino)pyrimidin-2-yl)-2-methyl-1-(( 2-(trimethylsilyl)ethoxy)methyl)-1,2-dihydro-3H-pyrazol-3-one

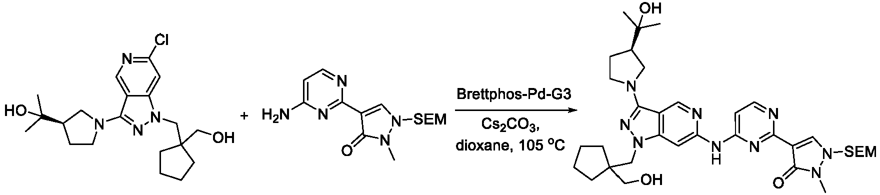

(R)-2-(1-(6-chloro-1-((1-(hydroxymethyl)cyclopentyl)methyl)-1H-pyrazolo[4,3-c] pyridin-3-yl)pyrrolidin-3-yl)propan-2-ol (0.40 g, 0.74 mmol) and 4-(4-aminopyrimidin-2-yl)-2-methyl-1-((2-(trimethylsilyl)ethoxy)methyl)-1,2-dihydro-3 H-pyrazol-3-one (0.24 g, 0.74 mmol) were dissolved in 1,4-dioxane (8 mL), and then Cs₂CO₃ (0.48 g, 1.47 mmol) and Brettphos-Pd-G3 (0.13 g, 0.15 mmol) were added. After the nitrogen substitution was carried out, the mixture was heated in a microwave to 130°C and reacted for 2 hours. After the reaction solution was cooled, ethyl acetate was added to dilute it, and the mixture was washed once with water and once with saturated brine, respectively. The organic layer was separated, dried over anhydrous sodium sulfate and then concentrated. The resulting crude product was separated by using column chromatography (eluent: CH₂Cl₂/MeOH 0-6%) to obtain (R)-4-(4-((1-((1-(hydroxymethyl)cyclopentyl)methyl)-3-(3-(2-hydroxypropan-2-yl)pyrr olidin-1-yl)-1H-pyrazolo[4,3-c]pyridin-6-yl)amino)pyrimidin-2-yl)-2-methyl-1-((2-(tri methylsilyl)ethoxy)methyl)-1,2-dihydro-3H-pyrazol-3-one (0.38 g, purity: 62.6%, yield: 47.6%). MS m/z (ESI): 678.6 [M+H]⁺.

### Step 3: Synthesis of (R)-4-(4-((1-((1-(hydroxymethyl)cyclopentyl)methyl)-3-(3-(2-hydroxypropan-2-yl)p yrrolidin-1-yl)-1H-pyrazolo[4,3-c]pyridin-6-yl)amino)pyrimidin-2-yl)-1-methyl-1H -pyrazol-5-ol

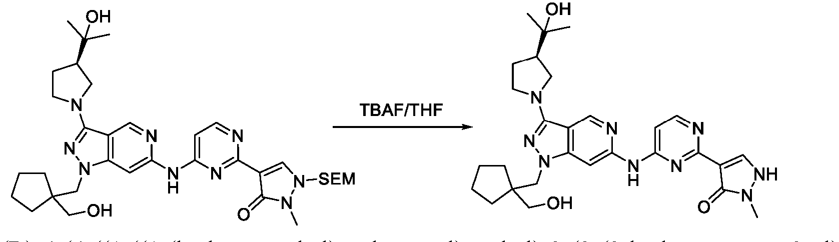

(R)-4-(4-((1-((1-(hydroxymethyl)cyclopentyl)methyl)-3-(3-(2-hydroxypropan-2-yl) pyrrolidin-1-yl)-1H-pyrazolo[4,3-c]pyridin-6-yl)amino)pyrimidin-2-yl)-2-methyl-1-((2-(trimethylsilyl)ethoxy)methyl)-1,2-dihydro-3H-pyrazol-3-one (370 mg, purity: 62.6%, 0.35 mmol) was dissolved in THF (5.0 mL), and TBAF/THF solution (2.11 mL, 1.0 M, 2.11 mmol) was added. The mixture was heated to 70°C and reacted for 1 hour. After the reaction was completed, the reaction solution was cooled to room temperature, and then concentrated and separated by using column chromatography to obtain (R)-4-(4-((1-((1-(hydroxymethyl)cyclopentyl)methyl)-3-(3-(2-hydroxypropan-2-yl)pyrr olidin-1-yl)-1H-pyrazolo[4,3-c]pyridin-6-yl)amino)pyrimidin-2-yl)-1-methyl-1H-pyraz ol-5-ol (200 mg, purity: 85.4%, yield: 89.9%). MS m/z (ESI): 548.4 [M+H]⁺.

### Step 4: Synthesis of (R)-2-(1-(1'-methylspiro[cyclopentane-1,6'-8-oxa-3-aza-4(6,1)-pyrazolo[4,3-c]pyrid ina-2(2,4)-pyrimidina-1(4,5)-pyrazolacyclooctaphane]-3'-yl)pyrrolidin-3-yl)propan -2-ol

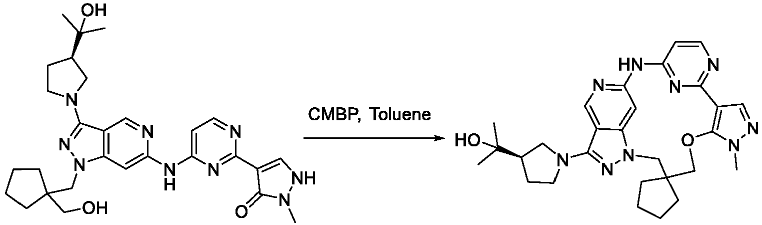

(R)-4-(4-((1-((1-(hydroxymethyl)cyclopentyl)methyl)-3-(3-(2-hydroxypropan-2-yl) pyrrolidin-1-yl)-1H-pyrazolo[4,3-c]pyridin-6-yl)amino)pyrimidin-2-yl)-1-methyl-1H-py razol-5-ol (180 mg, purity: 85.4%, 0.28 mmol) was dissolved in toluene (5 mL), and CMBP (542 mg, 2.25 mmol) was added. The mixture was heated to 130°C and reacted for 24 hours. After the reaction was completed, the reaction solution was cooled to room temperature and saturated aqueous NaHCO₃ solution was added to quench the reaction. The mixture was extracted twice with ethyl acetate, and the organic layer was combined, washed with saturated brine, and dried over anhydrous sodium sulfate. It was filtered, concentrated, and separated by using column chromatography (eluent: CH₂Cl₂/MeOH 0-5%) to obtain (R)-2-(1-(1'-methylspiro[cyclopentane-1,6'-8-oxa-3-aza-4(6,1)-pyrazolo[4,3-c]pyridina -2(2,4)-pyrimidina-1(4,5)-pyrazolacyclooctaphane]-3'-yl)pyrrolidin-3-yl)propan-2-ol (34.4 mg, yield: 22.9%). MS m/z (ESI): 530.4 [M+H]⁺.

¹HNMR (400 MHz, DMSO-*d*₆) δ 10.26 (s, 1H), 9.18 (s, 1H), 8.71 (s, 1H), 8.29 (d, *J =* 5.8 Hz, 1H), 8.00 (s, 1H), 6.77 (d, *J =* 5.9 Hz, 1H), 4.37 (s, 1H), 3.80 (s, 3H), 3.71 - 3.61 (m, 1H), 3.60 - 3.38 (m, 3H), 2.41 - 2.28 (m, 1H), 1.92 (q, *J* = 9.4 Hz, 4H), 1.70 (brs, 2H), 1.17 (brs, 3H), 1.15 (s, 3H).

### Example 14: Preparation of (S)-1'-methyl-3'-(3-(((trifluoromethyl)sulfonyl)methyl)pyrrolidin-1-yl)spiro[cyclop entane-1,6'-8-oxa-3-aza-4(6,1)-pyrazolo[4,3-c]pyridina-2(2,4)-pyrimidina-1(4,5)-py razolacyclooctaphane]

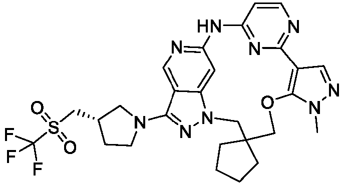

### Step 1: Synthesis of (S)-4-(4-((1-((1-(hydroxymethyl)cyclopentyl)methyl)-3-(3-(((trifluoromethyl)sulfon yl)methyl)pyrrolidin-1-yl)-1H-pyrazolo[4,3-c]pyridin-6-yl)amino)pyrimidin-2-yl)-2 -methyl-1-((2-(trimethylsilyl)ethoxy)methyl)-1,2-dihydro-3H-pyrazol-3-one

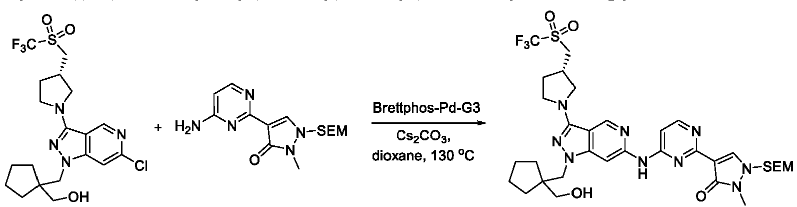

(S)-(1-((6-chloro-3-(3-(((trifluoromethyl)sulfonyl)methyl)pyrrolidin-1-yl)-1H-pyra zolo[4,3-c]pyridin-1-yl)methyl)cyclopentyl)methanol (0.48 g, 0.98 mmol) and 4-(4-aminopyrimidin-2-yl)-2-methyl-1-((2-(trimethylsilyl)ethoxy)methyl)-1,2-dihydro-3 H-pyrazol-3-one (330 mg, 1.03 mmol) were dissolved in 1,4-dioxane (8 mL), and then Cs₂CO₃ (0.64 g, 1.95 mmol) and Brettphos-Pd-G3 (0.18 g, 0.195 mmol) were added. After the nitrogen substitution was carried out, the mixture was heated in a microwave to 130°C and reacted for 2 hours. The reaction solution was cooled and diluted with ethyl acetate, and then washed once with water and saturated brine in sequence. The organic layer was separated and dried over anhydrous sodium sulfate. It was filtered and concentrated, and the resulting crude product was separated by using column chromatography to obtain (S)-4-(4-((1-((1-(hydroxymethyl)cyclopentyl)methyl)-3-(3-(((trifluoromethyl)sulfonyl) methyl)pyrrolidin-1-yl)-1H-pyrazolo[4,3-c]pyridin-6-yl)amino)pyrimidin-2-yl)-2-methy 1-1-((2-(trimethylsilyl)ethoxy)methyl)-1,2-dihydro-3H-pyrazol-3-one (0.32 g, purity: 73.6%, yield: 31.5%). MS m/z (ESI): 766.4 [M+H]⁺.

### Step 2: Synthesis of (S)-4-(4-((1-((1-(hydroxymethyl)cyclopentyl)methyl)-3-(3-(((trifluoromethyl)sulfon yl)methyl)pyrrolidin-1-yl)-1H-pyrazolo[4,3-c]pyridin-6-yl)amino)pyrimidin-2-yl)-2 -methyl-1,2-dihydro-3H-pyrazol-3-one

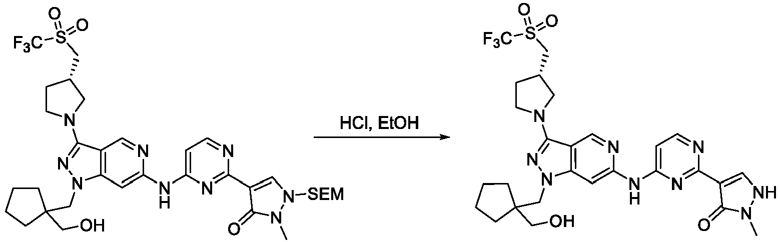

(S)-4-(4-((1-((1-(hydroxymethyl)cyclopentyl)methyl)-3-(3-(((trifluoromethyl)sulfo nyl)methyl)pyrrolidin-1-yl)-1H-pyrazolo[4,3-c]pyridin-6-yl)amino)pyrimidin-2-yl)-2-m ethyl-1-((2-(trimethylsilyl)ethoxy)methyl)-1,2-dihydro-3H-pyrazol-3-one (320 mg, purity: 73.6%, 0.31 mmol) was dissolved in ethanol (5.0 mL), and concentrated hydrochloric acid (0.50 mL, 12 M, 6.00 mmol) was added. The mixture was heated to 70°C and reacted for 2 hours. After the reaction was completed, it was cooled to room temperature. The reaction solution was concentrated and separated by using column chromatography (eluent: CH₂Cl₂/MeOH 0-5%) to obtain (S)-4-(4-((1-((1-(hydroxymethyl)cyclopentyl)methyl)-3-(3-(((trifluoromethyl)sulfonyl) methyl)pyrrolidin-1-yl)-1H-pyrazolo[4,3-c]pyridin-6-yl)amino)pyrimidin-2-yl)-2-methy l-1,2-dihydro-3H-pyrazol-3-one (150 mg, purity: 86.8%, yield: 65.1%). MS m/z (ESI): 636.4 [M+H]⁺.

### Step 3: Synthesis of (S)-1'-methyl-3'-(3-(((trifluoromethyl)sulfonyl)methyl)pyrrolidin-1-yl)spiro[cyclop entane-1,6'-8-oxa-3-aza-4(6,1)-pyrazolo[4,3-c]pyridina-2(2,4)-pyrimidina-1(4,5)-py razolacyclooctaphane]

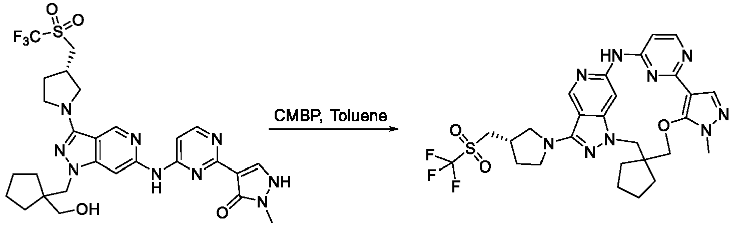

(S)-4-(4-((1-((1-(hydroxymethyl)cyclopentyl)methyl)-3-(3-(((trifluoromethyl)sulfo nyl)methyl)pyrrolidin-1-yl)-1H-pyrazolo[4,3-c]pyridin-6-yl)amino)pyrimidin-2-yl)-2-m ethyl-1,2-dihydro-3H-pyrazol-3-one (150 mg, purity: 86.8%, 0.21 mmol) was dissolved in dry toluene (20 mL), and CMBP (400 mg, 1.64 mmol) was added. The mixture was heated to 130°C and reacted for 24 hours. After the reaction was completed, the reaction solution was cooled to room temperature, quenched by adding saturated aqueous NaHCO₃ solution, and extracted twice with ethyl acetate. The organic phase was combined, washed sequentially with saturated brine, and dried over anhydrous sodium sulfate. It was filtered and concentrated. The resulting crude product was separated by preparative HPLC to obtain (S)-1'-methyl-3'-(3-(((trifluoromethyl)sulfonyl)methyl)pyrrolidin-1-yl)spiro[cyclopenta ne-1,6'-8-oxa-3-aza-4(6,1)-pyrazolo[4,3-c]pyridina-2(2,4)-pyrimidina-1(4,5)-pyrazolac yclooctaphane] (26.8 mg, vield: 20.7%. MS m/z (ESI): 618.4 [M+H]⁺.

¹HNMR (400 MHz, DMSO-*d*₆) δ 10.28 (s, 1H), 9.21 (s, 1H), 8.69 (s, 1H), 8.30 (d, *J* = 5.8 Hz, 1H), 8.00 (s, 1H), 6.76 (d, *J* = 5.9 Hz, 1H), 4.60 (brs, 2H), 4.11 (d, *J* = 6.7 Hz, 2H), 3.89 - 3.69 (m, 5H), 3.61 (q, *J* = 8.3 Hz, 1H), 3.38 (t, *J* = 9.0 Hz, 1H), 2.90 (p, *J =* 7.6 Hz, 1H), 2.33 - 2.29 (m, 1H), 2.05 - 1.84 (m, 4H), 1.70 (brs, 2H).

**Examples 15 to 47 and Example A4 can be prepared by selecting** the **corresponding raw materials with reference to the routes of Example A1, 1, 13** or **14:**

| **Example No.** | **Structural Formula** | **Chemical Name** | **MS: m/z [M+H]⁺** |
|---|---|---|---|
| **15** | 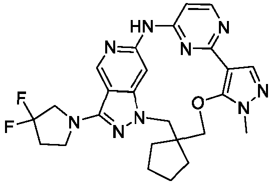 | 3'-(3,3-difluoropyrrolidin-1 -yl)-1'-methylspiro(cyclope ntane-1,6'-8-oxa-3-aza-4(6, 1)-pyrazolo[4,3-c]pyridina-2(2,4)-pyrimidina-1(4,5)-py razolacyclooctaphane) | 508.2 |
| **16** | 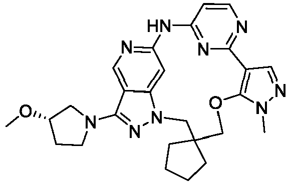 | (S)-3'-(3-methoxypyrrolidin -1-yl)-1'-methylspiro(cyclo pentane-1,6'-8-oxa-3-aza-4( 6,1)-pyrazolo[4,3-c]pyridin a-2(2,4)-pyrimidina-1(4,5)-pyrazolacyclooctaphane) | 502.2 |
| **17** | 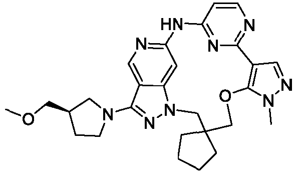 | (R)-3'-(3-(methoxymethyl) pyrrolidin-1-yl)-1'-methyls piro(cyclopentane-1,6'-8-ox a-3-aza-4(6,1)-pyrazolo[4,3 -c]pyridina-2(2,4)-pyrimidi na-1(4,5)-pyrazolacyclooctaphane) | 516.4 |
| **18** | 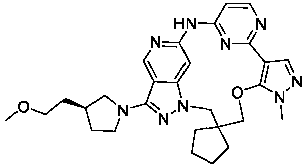 | (S)-3'-(3-(2-methoxyethyl)p yrrolidin-1-yl)-1'-methylspi ro(cyclopentane-1,6'-8-oxa-3-aza-4(6,1)-pyrazolo[4,3-c ]pyridina-2(2,4)-pyrimidina -1(4,5)-pyrazolacyclooctaphane) | 530.4 |
| **19** | 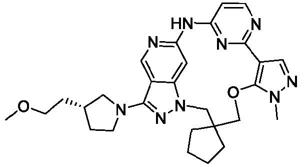 | (R)-3'-(3-(2-methoxyethyl) pyrrolidin-1-yl)-1'-methyls piro(cyclopentane-1,6'-8-ox a-3-aza-4(6,1)-pyrazolo[4,3 -c]pyridina-2(2,4)-pyrimidi na-1(4,5)-pyrazolacyclooctaphane) | 530.4 |
| **20** | 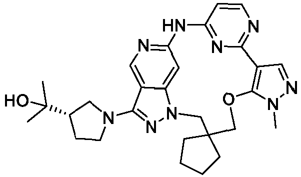 | (S)-2-(1-(1'-methylspiro[cy clopentane-1,6'-8-oxa-3-aza -4(6,1)-pyrazolo[4,3-c]pyrid ina-2(2,4)-pyrimidina-1(4,5 )-pyrazolacyclooctaphane]-3'-yl)pyrrolidin-3-yl)propan -2-ol | 530.4 |
| **21** | 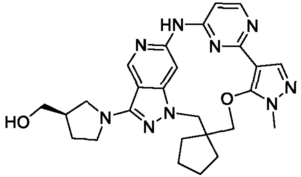 | (R)-(1-(1'-methylspiro(cycl opentane-1,6'-8-oxa-3-aza-4(6,1)-pyrazolo[4,3-c]pyridi na-2(2,4)-pyrimidina-1 (4,5) -pyrazolacyclooctaphane)-3 '-yl)pyrrolidin-3-yl)methanol | 502.4 |
| **22** | 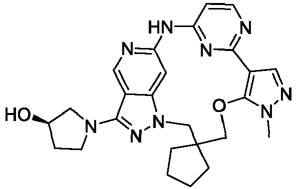 | (R)-1-(1'-methylspiro(cyclo pentane-1,6'-8-oxa-3-aza-4( 6,1)-pyrazolo[4,3-c]pyridin a-2(2,4)-pyrimidina-1(4,5)-pyrazolacyclooctaphane)-3' -yl)pyrrolidin-3-ol | 488.4 |
| **23** | 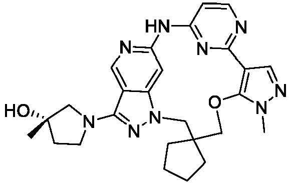 | (S)-3-methyl-1-(1'-methyls piro(cyclopentane-1,6'-8-ox a-3-aza-4(6,1)-pyrazolo[4,3 -c]pyridina-2(2,4)-pyrimidi na-1(4,5)-pyrazolacycloocta phane)-3'-yl)pyrrolidin-3-ol | 502.4 |
| **24** | 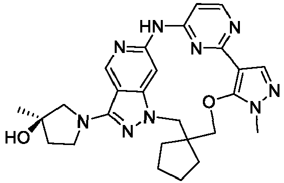 | (R)-3-methyl-1-(1'-methyls piro(cyclopentane-1,6'-8-ox a-3-aza-4(6,1)-pyrazolo[4,3 -c]pyridina-2(2,4)-pyrimidi na-1(4,5)-pyrazolacycloocta phane)-3'-yl)pyrrolidin-3-ol | 502.4 |
| **25** | 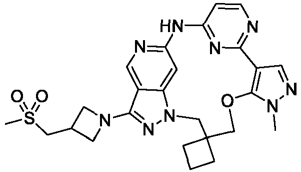 | 1'-methyl-3'-(3-((methylsul fonyl)methyl)azetidin-1-yl)s piro(cyclobutane-1,6'-8-oxa -3-aza-4(6,1)-pyrazolo[4,3-c]pyridina-2(2,4)-pyrimidin a-1(4,5)-pyrazolacyclooctaphane) | 536.2 |
| **26** | 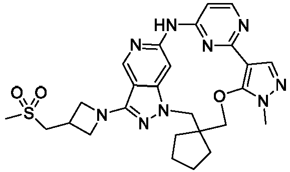 | 1'-methyl-3'-(3-((methylsul fonyl)methyl)azetidin-1-yl)s piro(cyclopentane-1,6'-8-ox a-3-aza-4(6,1)-pyrazolo[4,3 -c]pyridina-2(2,4)-pyrimidi na-1(4,5)-pyrazolacyclooctaphane) | 550.2 |
| **27** | 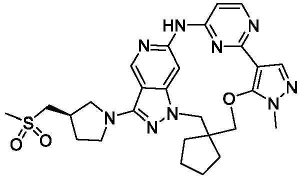 | (R)-1'-methyl-3'-(3-((meth ylsulfonyl)methyl)pyrrolidi n-1-yl)spiro(cyclopentane-1 ,6'-8-oxa-3-aza-4(6,1)-pyra zolo[4,3-c]pyridina-2(2,4)-p yrimidina-1(4,5)-pyrazolacyclooctaphane) | 564.3 |
| **28** | 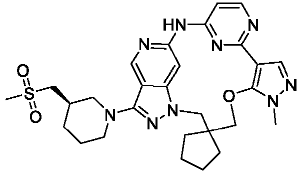 | (R)-1'-methyl-3'-(3-((meth ylsulfonyl)methyl)piperidin -1-yl)spiro(cyclopentane-1, 6'-8-oxa-3-aza-4(6,1)-pyraz olo[4,3-c]pyridina-2(2,4)-py rimidina-1(4,5)-pyrazolacyc looctaphane) | 578.4 |
| **29** | 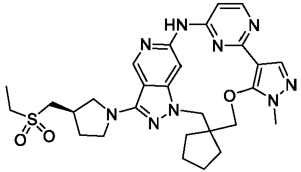 | (R)-3'-(3-((ethylsulfonyl)m ethyl)pyrrolidin-1-yl)-1'-me thylspiro(cyclopentane-1,6' -8-oxa-3-aza-4(6,1)-pyrazol o[4,3-c]pyridina-2(2,4)-pyri midina-1(4,5)-pyrazolacyclooctaphane) | 578.4 |
| **30** | 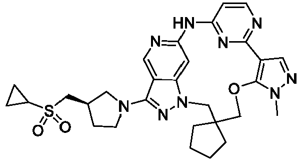 | R)-3'-(3-((cyclopropylsulfo nyl)methyl)pyrrolidin-1-yl)-1'-methylspiro(cyclopentan e-1,6'-8-oxa-3-aza-4(6,1)-p yrazolo[4,3-c]pyridina-2(2, 4)-pyrimidina-1(4,5)-pyrazo lacyclooctaphane) | 590.4 |
| **31** | 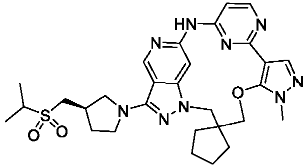 | (R)-3'-(3-((isopropylsulfon yl)methyl)pyrrolidin-1-yl)-1 '-methylspiro(cyclopentane-1,6'-8-oxa-3-aza-4(6,1)-pyr azolo[4,3-c]pyridina-2(2,4)-pyrimidina-1(4,5)-pyrazolac yclooctaphane) | 592.4 |
| **32** | 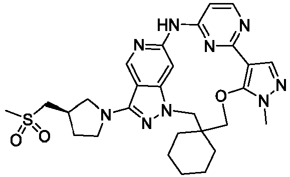 | (R)-1'-methyl-3'-(3-((meth ylsulfonyl)methyl)pyrrolidi n-1-yl)spiro(cyclohexane-1, 6'-8-oxa-3-aza-4(6,1)-pyraz olo[4,3-c]pyridina-2(2,4)-py rimidina-1(4,5)-pyrazolacyclooctaphane) | 578.4 |
| **33** | 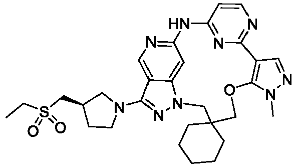 | (R)-1'-methyl-3'-(3-((ethyls ulfonyl)methyl)pyrrolidin-1 -yl)spiro(cyclohexane-1,6'-8-oxa-3-aza-4(6,1)-pyrazolo [4,3-c]pyridina-2(2,4)-pyri midina-1(4,5)-pyrazolacyclooctaphane) | 592.4 |
| **34** | 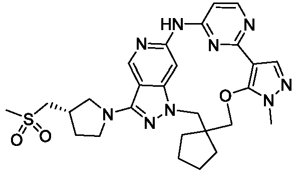 | (S)-1'methyl-3'-(3-((methyl sulfonyl)methyl)pyrrolidin-1-yl)spiro(cyclopentane-1,6 '-8-oxa-3-aza-4(6,1)-pyrazo lo[4,3-c]pyridina-2(2,4)-pyr imidina-1(4,5)-pyrazolacyclooctaphane) | 564.4 |
| **35** | 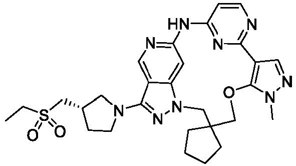 | (S)-3'-(3-((ethylsulfonyl)me thyl)pyrrolidin-1-yl)-1'-met hylspiro(cyclopentane-1,6'-8-oxa-3-aza-4(6,1)-pyrazolo [4,3-c]pyridina-2(2,4)-pyri midina-1(4,5)-pyrazolacyclooctaphane) | 578.4 |
| **36** | 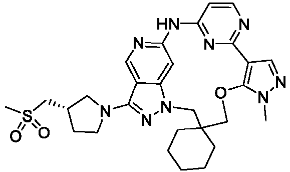 | (S)-1'-methyl-3'-(3-((methy lsulfonyl)methyl)pyrrolidin-1-yl)spiro(cyclohexane-1,6' -8-oxa-3-aza-4(6,1)-pyrazol o[4,3-c]pyridina-2(2,4)-pyri midina-1(4,5)-pyrazolacyclooctaphane) | 578.4 |
| **37** | 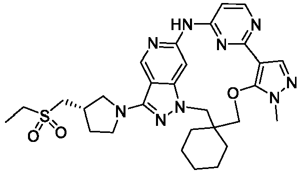 | (S)-1'-methyl-3'-(3-((ethyls ulfonyl)methyl)pyrrolidin-1 -yl)spiro(cyclohexane-1,6'-8-oxa-3-aza-4(6,1)-pyrazolo [4,3-c]pyridina-2(2,4)-pyri midina-1(4,5)-pyrazolacyclooctaphane) | 592.4 |
| **38** | 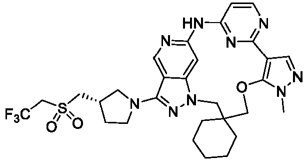 | (S)-1'-methyl-3'-(3-(((triflu oromethyl)sulfonyl)methyl) pyrrolidin-1-yl)spiro(cycloh exane-1,6'-8-oxa-3-aza-4(6, 1)-pyrazolo[4,3-c]pyridina-2(2,4)-pyrimidina-1(4,5)-py razolacyclooctaphane) | 632.4 |
| **39** | 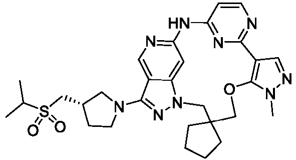 | (S)-3'-(3-((isopropylsulfony l)methyl)pyrrolidin-1-yl)-1' -methylspiro(cyclopentane-1,6'-8-oxa-3-aza-4(6,1)-pyr azolo[4,3-c]pyridina-2(2,4)-pyrimidina-1(4,5)-pyrazolacyclooctaphane) | 592.4 |
| **40** | 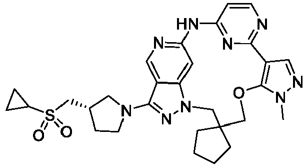 | (S)-3'-(3-((cyclopropylsulfo nyl)methyl)pyrrolidin-1-yl)-1'-methylspiro(cyclopentan e-1,6'-8-oxa-3-aza-4(6,1)-p yrazolo[4,3-c]pyridina-2(2, 4)-pyrimidina-1(4,5)-pyrazolacyclooctaphane) | 590.4 |
| **41** | 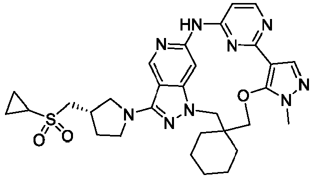 | (S)-3'-(3-((cyclopropylsulfo nyl)methyl)pyrrolidin-1-yl)-1'-methylspiro(cyclohexane -1,6'-8-oxa-3-aza-4(6,1)-py razolo[4,3-c]pyridina-2(2,4) -pyrimidina-1(4,5)-pyrazolacyclooctaphane) | 604.4 |
| **42** | 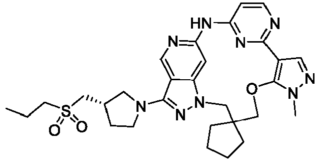 | (S)-3'-(3-((propylsulfonyl) methyl)pyrrolidin-1-yl)-1'-methylspiro(cyclopentane-1 ,6'-8-oxa-3-aza-4(6,1)-pyra zolo[4,3-c]pyridina-2(2,4)-p yrimidina-1(4,5)-pyrazolacyclooctaphane) | 592.4 |
| **43** | 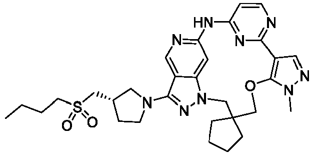 | (S)-3'-(3-((butylsulfonyl)m ethyl)pyrrolidin-1-yl)-1'-me thylspiro(cyclopentane-1,6' -8-oxa-3-aza-4(6,1)-pyrazol o[4,3-c]pyridina-2(2,4)-pyri midina-1(4,5)-pyrazolacyclooctaphane) | 606.4 |
| **44** | 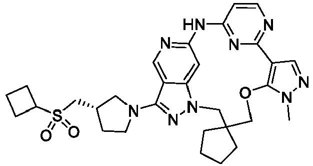 | (S)-3'-(3-((cyclobutylsulfon yl)methyl)pyrrolidin-1-yl)-1 '-methylspiro(cyclopentane-1,6'-8-oxa-3-aza-4(6,1)-pyr azolo[4,3-c]pyridina-2(2,4)-pyrimidina-1(4,5)-pyrazolacyclooctaphane) | 604.4 |
| **45** | 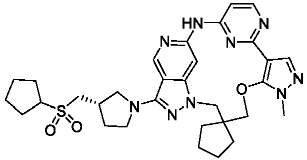 | (S)-3'-(3-((cyclopentylsulfo nyl)methyl)pyrrolidin-1-yl)-1'-methylspiro(cyclopentan e-1,6'-8-oxa-3-aza-4(6,1)-p yrazolo[4,3-c]pyridina-2(2, 4)-pyrimidina-1(4,5)-pyrazolacyclooctaphane) | 618.4 |
| **46** | 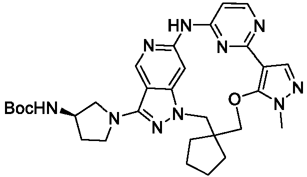 | Tert-butyl (R)-(1-(1'-methylspiro(cycl opentane-1,6'-8-oxa-3-aza-4(6,1)-pyrazolo[4,3-c]pyridi na-2(2,4)-pyrimidina-1(4,5) -pyrazolacyclooctaphane)-3 '-yl)pyrrolidin-3-yl)carbamate | 587.4 |
| **47** | 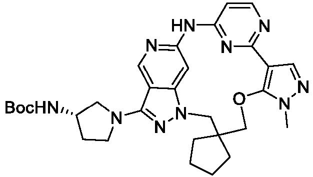 | Tert-butyl (S)-(1-(1'-methylspiro(cycl opentane-1,6'-8-oxa-3-aza-4(6,1)-pyrazolo[4,3-c]pyridi na-2(2,4)-pyrimidina-1(4,5) -pyrazolacyclooctaphane)-3 '-yl)pyrrolidin-3-yl)carbamate | 587.4 |
| **A4** | 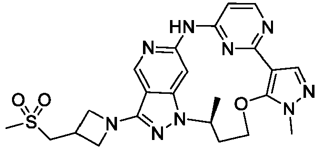 | (S)-1',5-dimethyl-4*-(3-((m ethylsulfonyl)methyl)azetidi n-1-yl)-1¹H,4¹H-8-oxa-3-az a-4(6,1)-pyrazolo[4,3-c]pyri dina-2(2,4)-pyrimidina-1(4, 5)-pyrazolacyclooctaphane | 510.4 |

### Example 48: Preparation of (R)-N-(1-(1'-methylspiro(cyclopentane-1,6'-8-oxa-3-aza-4(6,1)-pyrazolo[4,3-c]pyri dina-2(2,4)-pyrimidina-1(4,5)-pyrazolacyclooctaphane)-3'-yl)pyrrolidin-3-yl)metha nesulfonamide

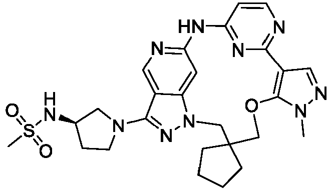

### Step 1: Synthesis of (R)-1-(1'-methylspiro(cyclopentane-1,6'-8-oxa-3-aza-4(6,1)-pyrazolo[4,3-c]pyridina -2(2,4)-pyrimidina-1(4,5)-pyrazolacyclooctaphane)-3'-yl)pyrrolidin-3-amine

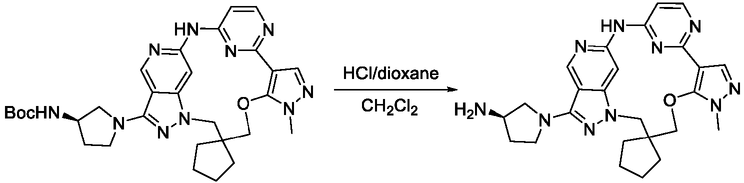

Tert-butyl (R)-(1-(1'-methylspiro(cyclopentane-1,6'-8-oxa-3-aza-4(6,1)-pyrazolo[4,3-c]pyridin-2( 2,4)-pyrimidin-1(4,5)-pyrazolyl)-3'-yl)pyrrolidin-3-yl)carbamate (prepared with reference to Example 1) (70 mg, purity: 77.1%, 0.10 mmol) was dissolved in CH₂Cl₂ (3.0 mL), and HCl/dioxane solution (0.46 mL, 4.0 M, 1.84 mmol) was added under cooling in an ice-water bath, and the mixture was reacted at room temperature for 1 hour. After the reaction was completed, the reaction solution was concentrated to obtain (R)-1-(1'-methylspiro(cyclopentane-1,6'-8-oxa-3-aza-4(6,1)-pyrazolo[4,3-c]pyridina-2( 2,4)-pyrimidina-1(4,5)-pyrazolacyclooctaphane)-3'-yl)pyrrolidin-3-amine, which was directly used for the next reaction. MS m/z (ESI): 487.3 [M+H]⁺.

### Step 2: Synthesis of (R)-N-(1-(1'-methylspiro(cyclopentane-1,6'-8-oxa-3-aza-4(6,1)-pyrazolo[4,3-c]pyri dina-2(2,4)-pyrimidina-1(4,5)-pyrazolacyclooctaphane)-3'-yl)pyrrolidin-3-yl)metha nesulfonamide

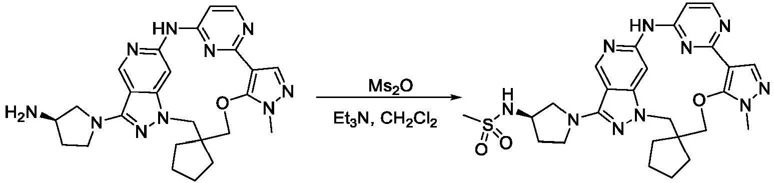

The crude product obtained in the previous step was dissolved in dry CH₂Cl₂ (3.0 mL), and triethylamine (0.10 mL, 0.74 mmol) and methanesulfonic anhydride (32 mg, 0.18 mmol) were added under cooling in an ice-water bath, and the mixture was reacted at room temperature for 1 hour. The reaction was quenched by adding saturated aqueous NaHCO₃ solution, and then extracted twice with CH₂Cl₂. The organic phase was combined, washed sequentially with saturated brine, and dried over anhydrous sodium sulfate. It was filtered, concentrated and separated by using column chromatography to obtain (R)-N-(1-(1'-methylspiro(cyclopentane-1,6'-8-oxa-3-aza-4(6,1)-pyrazolo[4,3-c]pyridina -2(2,4)-pyrimidina-1(4,5)-pyrazolacyclooctaphane)-3'-yl)pyrrolidin-3-yl)methanesulfon amide (7.3 mg, yield: 13%). MS m/z (ESI): 565.3 [M+H]⁺.

¹H NMR (400 MHz, DMSO-*d*₆) δ 10.26 (s, 1H), 9.21 (s, 1H), 8.71 (d, *J* = 0.9 Hz, 1H), 8.30 (d, *J* = 5.8 Hz, 1H), 8.00 (s, 1H), 6.77 (dd, J = 5.8, 1.3 Hz, 1H), 4.61 (brs, 2H), 4.08 (p, *J* = 6.3 Hz, 1H), 3.80 (s, 4H), 3.68 (dd, *J* = 8.7, 5.9 Hz, 1H), 3.58 (q, *J* = 7.8 Hz, 1H), 3.41 (dd, *J* = 9.9, 5.5 Hz, 1H), 3.00 (s, 3H), 2.35 - 2.24 (m, 2H), 2.04 - 1.85 (m, 4H), 1.70 (s, 2H).

### Example 49: Preparation of (R)-N-(1-(1'-methylspiro(cyclopentane-1,6'-8-oxa-3-aza-4(6,1)-pyrazolo[4,3-c]pyri dina-2(2,4)-pyrimidina-1(4,5)-pyrazolacyclooctaphane-3'-yl)pyrrolidin-3-yl)acetam ide

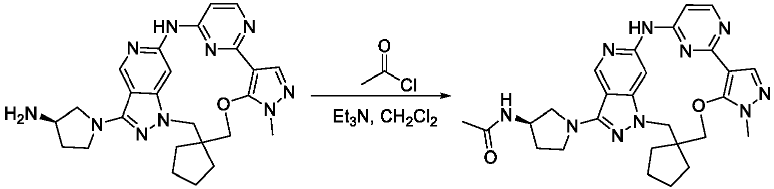

(R)-1-(1'-methylspiro(cyclopentane-1,6'-8-oxa-3-aza-4(6,1)-pyrazolo[4,3-c]pyridi na-2(2,4)-pyrimidina-1(4,5)-pyrazolacyclooctaphane)-3'-yl)pyrrolidin-3-amine (0.010 mmol) was dissolved in CH₂Cl₂ (3.0 mL). Triethylamine (0.10 mL, 0.74 mmol) was added under cooling in an ice-water bath, and acetyl chloride (0.03 mL, 0.46 mmol) was slowly added dropwise. The mixture was maintained at 0°C and reacted for 1 hour. After the reaction was completed, saturated aqueous NaHCO₃ solution was added to quench the reaction, and the mixture was extracted twice with CH₂Cl₂. The organic phase was combined, washed successively with saturated brine, and dried over anhydrous sodium sulfate. It was filtered, concentrated and separated by using column chromatography to obtain (R)-N-(1-(1'-methylspiro(cyclopentane-1,6'-8-oxa-3-aza-4(6,1)-pyrazolo[4,3-c]pyridina -2(2,4)-pyrimidina-1(4,5)-pyrazolacyclooctaphane)-3'-yl)pyrrolidin-3-yl)acetamide (14.1 mg, yield: 26%). MS m/z (ESI): 529.4 [M+H]⁺.

¹HNMR (400 MHz, DMSO-*d*₆) δ 10.26 (s, 1H), 9.21 (d, J = 1.0 Hz, 1H), 8.71 (d, J = 1.0 Hz, 1H), 8.30 (d, J = 5.8 Hz, 1H), 8.18 (d, J = 6.7 Hz, 1H), 8.00 (s, 1H), 6.76 (dd, J = 5.8, 1.5 Hz, 1H), 4.65 (brs, 2H), 4.37 (q, J = 5.8 Hz, 1H), 3.80 (s, 3H), 3.75 (dd, J = 10.0, 6.5 Hz, 1H), 3.67 (q, J = 5.6 Hz, 1H), 3.59 (q, J = 8.1 Hz, 1H), 3.37 - 3.35 (m, 1H), 2.20 (q, J = 6.1 Hz, 2H), 1.94 - 1.87 (m, 3H), 1.82 (s, 3H), 1.70 (s, 2H).

### Example 50: Preparation of (R)-2,2,2-trifluoro-N-(1-(1'-methylspiro(cyclopentane-1,6'-8-oxa-3-aza-4(6,1)-pyra zolo[4,3-c]pyridina-2(2,4)-pyrimidina-1(4,5)-pyrazolacyclooctaphane-3'-yl)pyrroli din-3-yl)acetamide

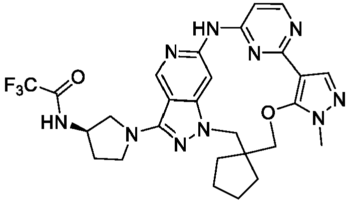

### Step 1: Synthesis of (R)-1-(1'-methylspiro(cyclopentane-1,6'-8-oxa-3-aza-4(6,1)-pyrazolo[4,3-c]pyridina -2(2,4)-pyrimidina-1(4,5)-pyrazolacyclooctaphane)-3'-yl)pyrrolidin-3-amine

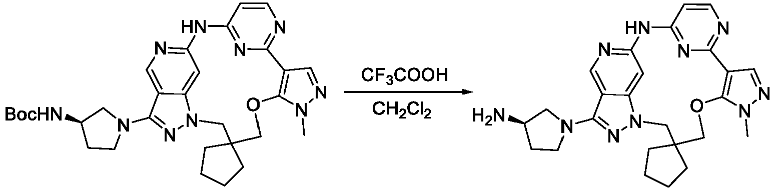

Tert-butyl (R)-(1-(1'-methylspiro(cyclopentane-1,6'-8-oxa-3-aza-4(6,1)-pyrazolo[4,3-c]pyridina-2 (2,4)-pyrimidina-1(4,5)-pyrazolacyclooctaphane)-3'-yl)pyrrolidin-3-yl)carbamate (50 mg, 0.08 mmol) was dissolved in CH₂Cl₂ (3.0 mL). CF₃COOH (0.24 mL, 3.28 mmol) was added under cooling in an ice-water bath. After the mixture was stirred for 1 hour, it was heated to room temperature and reacted for 3 hours. After it was concentrated to remove the solvent, the resulting crude product was directly used for the next reaction. MS m/z (ESI): 487.2 [M+H]⁺.

### Step 2: Synthesis of (R)-2,2,2-trifluoro-N-(1-(1'-methylspiro(cyclopentane-1,6'-8-oxa-3-aza-4(6,1)-pyra zolo[4,3-c]pyridina-2(2,4)-pyrimidina-1(4,5)-pyrazolacyclooctaphane-3'-yl)pyrroli din-3-yl)acetamide

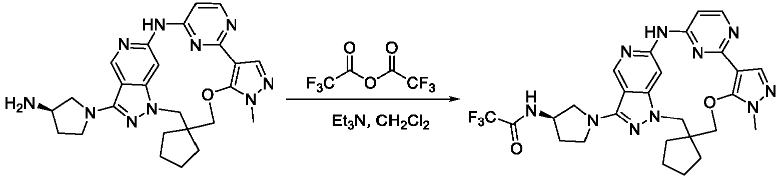

(R)-1-(1'-methylspiro(cyclopentane-1,6'-8-oxa-3-aza-4(6,1)-pyrazolo[4,3-c]pyridi na-2(2,4)-pyrimidina-1(4,5)-pyrazolacyclooctaphane)-3'-yl)pyrrolidin-3-amine (0.08 mmol) was dissolved in CH₂Cl₂ (3.0 mL), triethylamine (0.03 mL, 0.25 mmol) was added under cooling in an ice-water bath, and TFAA (0.02 mL, 0.12 mmol) was slowly added dropwise. The mixture was maintained at 0°C and reacted for 1 hour. After the reaction was completed, saturated aqueous NaHCO₃ solution was added to quench the reaction, and the mixture was extracted twice with CH₂Cl₂. The organic phase was combined, washed successively with saturated brine, and dried over anhydrous sodium sulfate. It was filtered, concentrated and separated by using column chromatography to obtain (R)-2,2,2-trifluoro-N-(1-(1'-methylspiro(cyclopentane-1,6'-8-oxa-3-aza-4(6,1)-pyrazolo [4,3-c]pyridina-2(2,4)-pyrimidina-1(4, 5)-pyrazolacyclooctaphane)-3'-yl)pyrrolidin-3-yl )acetamide (13.3 mg, yield: 27%). MS m/z (ESI): 583.4 [M+H]⁺.

¹H NMR (400 MHz, DMSO-*d*₆) δ 10.29 (s, 1H), 9.75 (d, *J* = 6.8 Hz, 1H), 9.22 (s, 1H), 8.74 (s, 1H), 8.30 (d, *J* = 5.8 Hz, 1H), 8.00 (s, 1H), 6.76 (dd, *J* = 5.9, 1.5 Hz, 1H), 4.65 (brs, 2H), 4.51 (s, 1H), 3.87 - 3.81 (m, 1H), 3.80 (s, 3H), 3.77 - 3.67 (m, 1H), 3.61 *(d, J=* 7.6 Hz, 1H), 3.53 (dd, J = 10.2, 4.6 Hz, 1H), 2.37 - 2.23 (m, 2H), 2.05 (dq, *J* = 13.0, 6.6 Hz, 2H), 1.91 (s, 3H), 1.70 (s, 2H).

**Examples 51 to 58 can be prepared by selecting the corresponding raw materials with reference to the synthesis methods of Example 48, 49 or 50.**

| **Example No.** | **Structural Formula** | **Chemical Name** | **MS: *m*/*z* [M+H]⁺** |
|---|---|---|---|
| **51** | 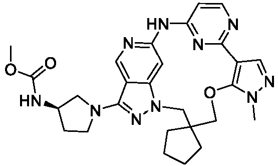 | Methyl (R)-(1-(1'-methylspiro(cycl opentane-1,6'-8-oxa-3-aza-4 (6,1)-pyrazolo[4,3-c]pyridin a-2(2,4)-pyrimidina-1(4,5)-p yrazolacyclooctaphane)-3'-y l)pyrrolidin-3-yl)carbamate | 545.4 |
| **52** | 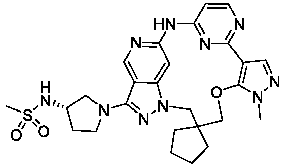 | (R)-N-(1-(1'-methylspiro(cy clopentane-1,6'-8-oxa-3-aza -4(6,1)-pyrazolo[4,3-c]pyrid ina-2(2,4)-pyrimidina-1(4,5) -pyrazolacyclooctaphane)-3' -yl)pyrrolidin-3-yl)methanesulfonamide | 565.3 |
| **53** | 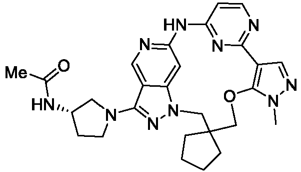 | (S)-N-(1-(1'-methylspiro(cy clopentane-1,6'-8-oxa-3-aza -4(6,1)-pyrazolo[4,3-c]pyrid ina-2(2,4)-pyrimidina-1(4,5) -pyrazolacyclooctaphane-3'-yl)pyrrolidin-3-yl)acetamide | 529.4 |
| **54** | 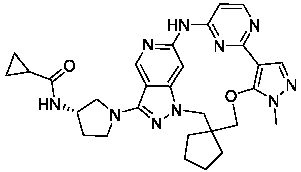 | (S)-N-(1-(1'-methylspiro(cy clopentane-1,6'-8-oxa-3-aza -4(6,1)-pyrazolo[4,3-c]pyrid ina-2(2,4)-pyrimidina-1(4,5) -pyrazolacyclooctaphane)-3' -yl)pyrrolidin-3-yl)cyclopro panecarboxamide | 555.4 |
| **55** | 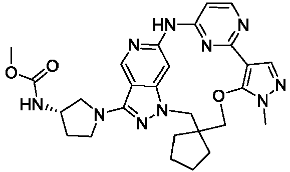 | Methyl (S)-(1-(1'-methylspiro(cyclo pentane-1,6'-8-oxa-3-aza-4( 6,1)-pyrazolo[4,3-c]pyridina -2(2,4)-pyrimidina-1(4,5)-p yrazolacyclooctaphane)-3'-y l)pyrrolidin-3-yl)carbamate | 545.4 |
| **56** | 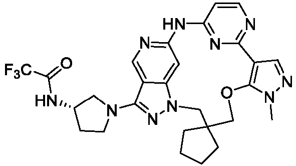 | (S)-2,2,2-trifluoro-N-(1-(1'-methylspiro(cyclopentane-1, 6'-8-oxa-3-aza-4(6,1)-pyraz olo[4,3-c]pyridina-2(2,4)-py rimidina-1(4,5)-pyrazolacyc looctaphane-3'-yl)pyrrolidin -3-yl)acetamide | 583.4 |
| **57** | 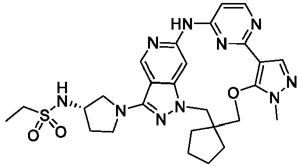 | (S)-N-(1-(1'-methylspiro(cy clopentane-1,6'-8-oxa-3-aza -4(6,1)-pyrazolo[4,3-c]pyrid ina-2(2,4)-pyrimidina-1(4,5) -pyrazolacyclooctaphane-3'-yl)pyrrolidin-3-yl)ethanesulfonamide | 579.4 |
| **58** | 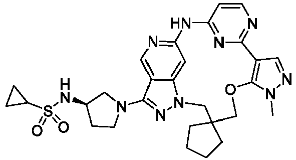 | (R)-N-(1-(1'-methylspiro(cy clopentane-1,6'-8-oxa-3-aza -4(6,1)-pyrazolo[4,3-c]pyrid ina-2(2,4)-pyrimidina-1(4,5) -pyrazolacyclooctaphane)-3' -yl)pyrrolidin-3-yl)cyclopropanesulfonamide | 591.7 |

¹HNMR data for the compounds prepared in the above examples are as follows:

| **Example No.** | **¹H NMR (400MHz)** |
|---|---|
| **2** | ¹H NMR (400 MHz, DMSO-*d*₆) δ 10.47 (s, 1H), 9.49 (s, 1H), 8.73 (s,1H), 8.31 (d, *J* = 8.0 Hz, 1H), 7.99 (s, 1H), 6.77 (d, *J* = 4.0 Hz, 1H), 4.79 (s, 1H), 3.87 - 3.72 (m, 4H), 2.98 - 2.81 (m, 3H), 2.32 (s, 3H), 2.27 (s, 3H), 1.98 - 1.72 (m, 6H), 1.59 - 1.27 (m, 3H), 0.94 - 0.73 (m, 2H). |
| **3** | ¹H NMR (400 MHz, DMSO-*d*₆) δ 10.51 (s, 1H), 9.55 (s, 1H), 8.72 (s, 1H), 8.33 (d, *J* = 5.8 Hz, 1H), 8.02 (s, 1H), 6.79 (d, *J* = 5.8 Hz, 1H), 4.93 - 4.23 (m, 3H), 3.81 (s, 3H), 2.78 - 2.56 (m, 3H), 2.17 (s, 3H), 2.10 (s, 2H), 1.97 - 1.89 (m, 2H), 1.85 - 1.53 (m, 7H), 1.24 (s, 2H), 0.64 (brs, 2H). |
| **4** | ¹H NMR (400 MHz, DMSO-*d*₆) δ 10.53 (s, 1H), 9.54 (s, 1H), 8.73 (s, 1H), 8.34 (d, *J* = 5.8 Hz, 1H), 8.02 (s, 1H), 6.80 (d, *J* = 5.8 Hz, 1H), 4.68 (brs, 3H), 3.81 (s, 7H), 3.60 (d, *J* = 27.1 Hz, 1H), 2.91 (brs, 1H), 2.79 - 2.61 (m, 3H), 2.18 (s, 3H), 2.16 - 2.06 (m, 2H), 1.99 - 1.88 (m, 3H), 1.70 (dtd, J = 13.0, 9.5, 3.5 Hz, 2H), 1.00 (brs, 1H), 0.42 (brs, 1H). |
| **5** | ¹H NMR (400 MHz, DMSO-*d*₆) δ 10.47 (s, 1H), 9.45 (s, 1H), 8.75 (s, 1H), 8.33 (d, *J* = 5.8 Hz, 1H), 8.01 (s, 1H), 6.79 (d, *J* = 5.9 Hz, 1H), 4.77 (brs, 2H), 4.30 (brs, 2H), 3.81 (s, 3H), 2.94 (brs, 4H), 2.43 (s, 3H), 2.26 - 2.12 (m, 2H), 2.11 - 1.98 (m, 3H), 1.98 - 1.76 (m, 5H). |
| **6** | ¹H NMR (400 MHz, DMSO-*d*₆) δ 10.52 (s, 1H), 9.54 (s, 1H), 8.77 (s, 1H), 8.35 - 8.32 (m, 2H), 8.02 (s, 1H), 6.80 (d, *J* = 5.9 Hz, 1H), 3.82 (s, 3H), 3.17-3.08 (m, 2H), 3.04-2.96 (m, 1H), 2.84 (t, *J* = 10.3 Hz, 2H), 2.48-2.42 (m, 8H), 2.05 - 1.97 (m, 2H), 1.92 - 1.83 (m, 3H), 1.79 - 1.70 (m, 3H). |
| **7** | ¹H NMR (400 MHz, DMSO-*d*₆) δ 10.51 (s, 1H), 9.53 (s, 1H), 8.74 (s, 1H), 8.34 (d, *J* = 5.8 Hz, 1H), 8.02 (s, 1H), 6.80 (d, *J* = 5.8 Hz, 1H), 3.82 (s, 4H), 2.81 - 2.71 (m, 4H), 2.67 (s, 1H), 2.39-2.31 (m, 4H), 2.21-2.11 (m, 2H), 2.01 - 1.92 (m, 3H), 1.91 (s, 1H), 1.87 (s, 3H), 1.75 - 1.64 (m, 4H), 1.01 (t, *J* = 7.2 Hz, 3H). |
| **8** | ¹H NMR (400 MHz, DMSO-*d*₆) δ 10.50 (s, 1H), 9.51 (s, 1H), 8.77 (s, 1H), 8.33 (d, *J* = 5.8 Hz, 1H), 8.01 (s, 1H), 6.79 (d, *J* = 5.8 Hz, 1H), 3.80 (s, 3H), 3.66 (t, *J* = 7.1 Hz, 3H), 3.58 - 3.52 (m, 2H), 3.47 - 3.39 (m, 2H), 3.23 (t, *J* = 6.8 Hz, 3H), 2.30 (s, 3H), 1.87 (s, 3H), 1.80 - 1.61 (m, 2H), 1.03 - 0.62 (s, 2H). |
| **9** | ¹H NMR (400 MHz, DMSO-*d*₆) δ 10.56 (s, 1H), 9.58 (s, 1H), 8.82 (s, 1H), 8.34 (d, *J* = 5.8 Hz, 1H), 8.02 (s, 1H), 6.80 (d, *J* = 5.9 Hz, 1H), 5.23 - 4.01 (m, 5H), 3.82 (s, 3H), 2.93 (s, 1H), 2.22 (s, 3H), 2.21 - 2.07 (m, 4H), 1.88 - 1.72 (m, 5H). |
| **11** | ¹H NMR (400 MHz, DMSO-*d*₆) δ 10.58 (s, 1H), 9.59 (s, 1H), 8.81 (d, *J* = 1.0 Hz, 1H), 8.35 (d, *J=* 5.8 Hz, 1H), 8.03 (s, 1H), 6.81 (d, *J=* 5.8 Hz, 1H), 4.74 (s, 2H), 3.82 - 3.57 (m, 9H), 2.94 - 2.83 (m, 4H), 2.22 (s, 3H), 2.20 - 1.80 (m, 8H). |
| **12** | ¹H NMR (400 MHz, DMSO-*d*₆) δ 10.53 (d, J = 5.3 Hz, 1H), 9.49 (d, J = 5.3 Hz, 1H), 8.81 (d, J = 5.5 Hz, 1H), 8.34 (d, J = 5.7 Hz, 1H), 8.01 (d, J = 5.4 Hz, 1H), 6.79 (d, J = 5.8 Hz, 1H), 4.81 (brs, 2H), 4.29 (brs, 3H), 3.81 (s, 3H), 2.93 (s, 1H), 2.26 - 2.07 (m, 9H), 1.93 (brs, 5H), 1.23 (s, 1H). |
| **15** | ¹H NMR (400 MHz, DMSO-*d*₆) δ 10.31 (s, 1H), 9.23 (d, *J* = 1.0 Hz, 1H), 8.79 (d, *J* = 1.0 Hz, 1H), 8.30 (d, *J* = 5.9 Hz, 1H), 8.00 (s, 1H), 6.77 (d, *J* = 5.8 Hz, 1H), 3.95 (t, *J* = 13.2 Hz, 2H), 3.80 (s, 3H), 3.77 (d, *J* = 7.2 Hz, 2H), 2.57 (td, *J* = 14.5, 7.3 Hz, 2H), 1.90 (s, 3H), 1.70 (s, 3H). |
| **16** | ¹H NMR (400 MHz, DMSO-*d*₆) δ 10.25 (s, 1H), 9.20 (d, *J* = 1.0 Hz, 1H), 8.74 (d, *J* = 0.9 Hz, 1H), 8.30 (d, *J* = 5.8 Hz, 1H), 8.00 (s, 1H), 6.77 (d, *J* = 5.9 Hz, 1H), 4.11 (s, 1H), 3.80 (s, 3H), 3.68 (dd, *J* = 10.8, 4.8Hz, 1H), 3.63 - 3.53 (m, 3H), 3.28 (s, 3H), 2.13 - 2.04 (m, 2H), 1.91 (s, 2H), 1.70 (s, 2H), 1.62 - 1.53 (m, 1H), 1.35 (dd, J = 14.9, 7.7 Hz, 1H), 0.88 (t, *J* = 7.1 Hz, 1H). |
| **17** | ¹H NMR (400 MHz, DMSO-*d*₆) δ 10.26 (s, 1H), 9.19 (s, 1H), 8.70 (s, 1H), 8.30 (d, *J* = 5.8 Hz, 1H), 8.00 (s, 1H), 6.76 (d, *J* = 5.8 Hz, 1H), 3.80 (s, 3H), 3.69 - 3.59 (m, 2H), 3.54 (q, *J* = 8.0 Hz, 1H), 3.39 (dd, *J* = 6.8, 2.9 Hz, 3H), 3.29 (s, 5H), 2.60 (p, *J* = 7.1 Hz, 1H), 2.09 (h, *J* = 7.0 Hz, 1H), 1.91 (s, 3H), 1.81 - 1.66 (m, 3H). |
| **18** | ¹H NMR (400 MHz, DMSO-*d*₆) δ 10.22 (s, 1H), 9.18 (s, 1H), 8.71 (s, 1H), 8.29 (d, *J* = 5.8 Hz, 1H), 8.00 (s, 1H), 6.76 (d, *J* = 5.8 Hz, 1H), 4.50 (brs, 2H), 3.79 (s, 3H), 3.72 (dd, *J* = 9.5, 7.5 Hz, 1H), 3.62 (dt, *J* = 8.8, 5.1 Hz, 1H), 3.51 *(q, J=* 8.5 Hz, 1H), 3.42 (t, *J* = 6.5 Hz, 2H), 3.26 (s, 3H), 3.14 (t, *J* = 8.8 Hz, 1H), 2.92 (s, 1H), 2.34 (p, *J* = 7.6 Hz, 1H), 2.13 (d, *J=* 11.8 Hz, 1H), 1.90 (s, 2H), 1.81 - 1.48 (m, 6H). |
| **19** | ¹H NMR (400 MHz, DMSO-*d*₆) δ 10.23 (s, 1H), 9.18 (s, 1H), 8.71 (s, 1H), 8.29 (d, *J* = 5.9 Hz, 1H), 8.00 (s, 1H), 6.76 (d, *J* = 5.8 Hz, 1H), 4.50 (brs, 2H), 3.79 (s, 3H), 3.71 (dd, *J* = 9.4, 7.4 Hz, 1H), 3.63 (td, *J* = 8.7, 3.1 Hz, 1H), 3.56 - 3.46 (m, 1H), 3.42 (t, *J* = 6.5 Hz, 2H), 3.26 (s, 3H), 3.14 (t, *J* = 8.8 Hz, 1H), 2.89 (s, 1H), 2.33 (p, *J* = 7.4 Hz, 1H), 2.12 (s, 1H), 1.90 (s, 2H), 1.79 - 1.45 (m, 6H). |
| **20** | ¹H NMR (400 MHz, DMSO-*d*₆) δ 10.25 (s, 1H), 9.19 (s, 1H), 8.71 (s, 1H), 8.30 (d, *J* = 5.8 Hz, 1H), 8.00 (s, 1H), 6.77 (d, *J* = 5.9 Hz, 1H), 4.38 (s, 1H), 3.80 (s, 3H), 3.66 (t, *J* = 8.6 Hz, 1H), 3.58 - 3.39 (m, 3H), 2.45 - 2.27 (m, 2H), 1.92 (m, 5H), 1.70 (s, 3H), 1.16 (d, *J* = 7.6 Hz, 6H). |
| **21** | ¹H NMR (400 MHz, DMSO-*d*₆) δ 10.25 (s, 1H), 9.19 (s, 1H), 8.71 (s, 1H), 8.30 (d, *J* = 5.8 Hz, 1H), 8.00 (s, 1H), 6.76 (d, *J* = 5.9 Hz, 1H), 4.72 (t, *J* = 5.3 Hz, 1H), 3.80 (s, 3H), 3.61 (dt, *J* = 15.5, 8.2 Hz, 2H), 3.52 *(d, J* = 8.5 Hz, 1H), 3.46 (q, *J* = 5.6 Hz, 2H), 2.05 (dd, *J* = 13.5, 6.6 Hz, 1H), 1.91 (s, 2H), 1.81 - 1.59 (m, 4H). |
| **22** | ¹H NMR (400 MHz, DMSO-*d*₆) δ 10.24 (s, 1H), 9.19 (s, 1H), 8.71 (s, 1H), 8.30 (d, J = 5.8 Hz, 1H), 8.00 (s, 1H), 6.76 (d, J = 5.8 Hz, 1H), 4.98 (s, 1H), 4.43 (s, 1H), 3.80 (s, 3H), 3.74 - 3.53 (m, 3H), 3.40 (d, J = 10.1 Hz, 1H), 2.15 - 1.99 (m, 1H), 1.99 - 1.83 (m, 4H), 1.71 (s, 3H). |
| **23** | ¹H NMR (400 MHz, DMSO-*d*₆) δ 10.24 (s, 1H), 9.18 (s, 1H), 8.69 (s, 1H), 8.29 (d, *J* = 5.8 Hz, 1H), 8.00 (s, 1H), 6.76 (d, *J* = 5.9 Hz, 1H), 4.80 (s, 1H), 4.50 (brs, 3H), 3.80 (s, 3H), 3.74 - 3.54 (m, 3H), 3.52 - 3.41 (m, 2H), 2.93 (brs, 1H), 1.93 (dd, *J* = 12.8, 5.8 Hz, 4H), 1.70 (brs, 2H), 1.38 (s, 3H). |
| **24** | ¹H NMR (400 MHz, DMSO-*d*₆) δ 10.24 (s, 1H), 9.19 (s, 1H), 8.69 (s, 1H), 8.29 (d, *J* = 5.8 Hz, 1H), 8.00 (s, 1H), 6.76 (d, *J* = 5.8 Hz, 1H), 4.80 (s, 1H), 3.80 (s, 3H), 3.72 - 3.57 (m, 2H), 3.52 - 3.42 (m, 2H), 1.98 - 1.87 (m, 4H), 1.70 (s, 2H), 1.38 (s, 3H). |
| **25** | ¹H NMR (400 MHz, DMSO-*d*₆) δ 10.27 (s, 1H), 9.19 (d, *J* = 1.1 Hz, 1H), 8.55 (d, *J* = 1.0 Hz, 1H), 8.30 (d, *J* = 5.8 Hz, 1H), 7.99 (s, 1H), 6.76 (d, *J =* 5.9 Hz, 1H), 4.54 (s, 2H), 4.32 (t, *J* = 7.7 Hz, 2H), 3.96 (t, *J* = 7.0 Hz, 2H), 3.80 (s, 3H), 3.57 (d, *J* = 7.5 Hz, 2H), 3.00 (s, 3H), 2.24 - 2.05 (m, 2H), 1.94 (s, 4H). |
| **26** | ¹H NMR (400 MHz, DMSO-*d*₆) δ 10.30 (s, 1H),9.26 (d, *J* = 1.0 Hz, 1H), 8.57 (s, 1H), 8.30 (d, *J* = 5.8 Hz, 1H), 8.00 (s, 1H), 6.77 (dd, *J* = 5.9, 1.5 Hz, 1H), 4.30 (t, *J* = 7.6 Hz, 2H), 3.94 (t, *J* = 7.0 Hz, 2H), 3.80 (s, 3H), 3.57 (d, *J* = 7.5 Hz, 2H), 3.33 (m, 8H), 3.00 (s, 3H), 1.89 (m, 2H), 1.70 (m, 2H). |
| **27** | ¹H NMR (400 MHz, DMSO-*d*₆) δ10.26 (s, 1H), 9.20 (s, 1H), 8.68 (d, J = 0.9 Hz, 1H), 8.30 (d, J = 5.8 Hz, 1H), 8.00 (s, 1H), 6.76 (dd, J = 5.8, 1.5 Hz, 1H), 4.50 (brs, 3H), 3.87 - 3.81 (m, 1H), 3.79 (s, 3H), 3.68 (td, J = 8.8, 3.1 Hz, 1H), 3.62 - 3.51 (m, 1H), 3.41 (h, J = 6.7 Hz, 2H), 3.05 (s, 3H), 2.81 (dt, J = 14.7, 7.5 Hz, 1H), 2.33 - 2.22 (m, 1H), 1.98 - 1.78 (m, 4H), 1.69 (s, 2H). |
| **28** | ¹H NMR (400 MHz, DMSO-*d*₆) δ 10.29 (s, 1H), 9.21 (s, 1H), 8.82 (s, 1H), 8.30 (d, J = 5.8 Hz, 1H), 8.00 (s, 1H), 6.77 (d, J = 5.9 Hz, 1H), 4.75 (brs, 2H), 4.03 (s, 1H), 3.79 (s, 3H), 3.75 (s, 1H), 3.31 - 3.25 (m, 2H), 3.23 - 3.13 (m, 1H), 3.04 (s, 3H), 3.02 - 2.82 (m, 3H), 2.34 (s, 2H), 1.91 (s, 4H), 1.81 - 1.53 (m, 5H), 1.46 - 1.30 (m, 2H). |
| **29** | ¹H NMR (400 MHz, DMSO-*d*₆) δ 10.26 (s, 1H), 9.20 (d, *J* = 1.0 Hz, 1H), 8.68 (d, *J* = 0.9 Hz, 1H), 8.30 (d, J = 5.8 Hz, 1H), 8.00 (s, 1H), 6.76 (dd, J = 5.9, 1.4 Hz, 1H), 4.67 (brs, 3H), 3.87 - 3.82 (m, 1H), 3.79 (s, 3H), 3.68 (s, 1H), 3.57 (p, J = 8.2 Hz, 1H), 3.35 (d, J = 13.6 Hz, 5H), 3.15 (q, J = 7.4 Hz, 2H), 2.80 (dt, J = 14.7, 7.4 Hz, 1H), 2.34 - 2.24 (m, 1H), 2.00 - 1.79 (m, 4H), 1.70 (s, 2H), 1.25 (t, J = 7.4 Hz, 3H). |
| **30** | ¹H NMR (400 MHz, DMSO-*d*₆) δ 10.26 (s, 1H), 9.20 (d, *J=* 1.0 Hz, 1H), 8.68 (d, *J* = 0.9 Hz, 1H), 8.30 (d, *J* = 5.8 Hz, 1H), 8.00 (s, 1H), 6.76 (dd, *J* = 5.9, 1.5 Hz, 1H), 4.63 (brs, 3H), 3.87 - 3.82 (m, 1H), 3.79 (s, 3H), 3.69 (dt, *J* = 9.8, 4.9 Hz, 1H), 3.58 (p, *J* = 7.7 Hz, 1H), 3.49 - 3.39 (m, 2H), 3.34 (s, 4H), 2.91 - 2.73 (m, 2H), 2.33 - 2.25 (m, 1H), 1.88 (dt, *J* = 12.4, 8.4 Hz, 4H), 1.70 (s, 2H), 1.10 - 0.97 (m, 4H). |
| **31** | ¹H NMR (400 MHz, DMSO-*d*₆) δ 10.26 (s, 1H), 9.20 (s, 1H), 8.70 - 8.66 (m, 1H), 8.30 (d, *J* = 5.8 Hz, 1H), 8.00 (s, 1H), 6.77 (dd, *J* = 5.8, 1.5 Hz, 1H), 4.64 (brs, 3H), 3.80 (s, 4H), 3.73 - 3.64 (m, 1H), 3.57 (q, *J=* 8.3 Hz, 1H), 3.33 (s, 8H), 2.80 (p, *J* = 7.4 Hz, 1H), 2.33 - 2.26 (m, 1H), 1.98 - 1.79 (m, 4H), 1.28 (d, *J* = 6.8 Hz, 6H). |
| **32** | ¹H NMR (400 MHz, DMSO-*d*₆) δ 10.35 (s, 1H), 9.21 (s, 1H), 8.68 (s, 1H), 8.30 (d, *J* = 5.9 Hz, 1H), 8.03 (s, 1H), 6.78 (d, *J* = 5.8 Hz, 1H), 4.46 - 4.25 (m, 3H), 3.80 (s, 4H), 3.68 (s, 1H), 3.56 (d, *J* = 8.4 Hz, 1H), 3.41 *(q, J=* 6.9 Hz, 3H), 3.05 (s, 3H), 2.82 (q, *J=* 7.7 Hz, 1H), 2.29 (t, *J=* 10.5 Hz, 1H), 1.85 (dd, *J* = 12.1, 8.7 Hz, 2H), 1.65 - 1.52 (m, 3H), 1.37 - 1.25 (m, 3H), 1.24 (d, *J* = 7.2 Hz, 2H). |
| **34** | ¹H NMR (400 MHz, DMSO-*d*₆) δ 10.26 (s, 1H), 9.21 (s, 1H), 8.69 (s, 1H), 8.30 (dd, *J* = 5.8, 1.5 Hz, 1H), 8.00 (d, *J* = 1.5 Hz, 1H), 6.77 (d,*J* = 5.9 Hz, 1H), 4.58 (brs, 3H), 3.84 (d, *J* = 8.9 Hz, 1H), 3.80 (s, 3H), 3.68 (t, *J* = 5.3 Hz, 1H), 3.57 (q, *J* = 8.3 Hz, 1H), 3.40 (t, *J* = 6.5 Hz, 2H), 3.30 (s, 3H), 3.05 (s, 3H), 2.81 (p, *J* = 7.5 Hz, 1H), 2.30 (d, *J* = 18.7 Hz, 1H), 1.97 - 1.80 (m, 4H), 1.70 (s, 3H). |
| **35** | ¹H NMR (400 MHz, DMSO-*d*₆) δ10.26 (s, 1H), 9.20 (s, 1H), 8.68 (s, 1H), 8.29 (d, J = 5.8 Hz, 1H), 8.00 (s, 1H), 6.76 (dd, J = 5.8, 1.5 Hz, 1H), 4.62 (brs, 2H), 3.79 (s, 4H), 3.73 - 3.63 (m, 1H), 3.56 (q, J = 8.4 Hz, 1H), 3.37 (s, 1H), 3.32 (s, 6H), 3.15 (q, J = 7.4 Hz, 2H), 2.80 (p, J = 7.3 Hz, 1H), 2.29 (td, J = 10.0, 5.4 Hz, 1H), 2.01 - 1.79 (m, 4H), 1.69 (s, 2H), 1.25 (t, J = 7.4 Hz, 3H). |
| **36** | ¹H NMR (400 MHz, DMSO-*d*₆) δ 10.27 (s, 1H), 9.23 (s, 1H), 8.68 (s, 1H), 8.30 (d, *J* = 5.9 Hz, 1H), 8.01 (s, 1H), 6.77 (d, *J* = 5.9 Hz, 1H), 3.81 (s, 3H), 3.69 (m, 1H), 3.61 - 3.53 (m, 1H), 3.19 - 3.10 (m, 2H), 3.05 (s, 3H), 2.95 (m, 2H), 2.81 (p, *J* = 7.5 Hz, 2H), 2.28 (m, 2H), 2.03 - 1.96 (m, 1H), 1.86 (dd, *J* = 12.3, 8.8 Hz, 3H), 1.62 (s, 3H). |
| **37** | ¹H NMR (400 MHz, DMSO-*d*₆) δ 10.28 (s, 1H), 9.23 (s, 1H), 8.68 (s, 1H), 8.30 (d, J = 5.8 Hz, 1H), 8.01 (s, 1H), 6.77 (d, J = 5.7 Hz, 1H), 4.51 (brs, 2H), 3.81 (s, 4H), 3.69 (s, 1H), 3.58 (s, 1H), 3.38 (s, 1H), 3.31 (s, 6H), 3.15 (q, J = 7.4 Hz, 2H), 2.81 (q, J = 7.9 Hz, 1H), 1.88 (d, J = 10.5 Hz, 2H), 1.62 (s, 1H), 1.37 (d, J = 6.3 Hz, 2H), 1.24 (d, J = 7.4 Hz, 6H), 0.86 (dt, J = 12.2, 7.5 Hz, 3H). |
| **39** | ¹H NMR (400 MHz, DMSO-*d*₆) δ10.25 (s, 1H), 9.20 (s, 1H), 8.68 (s, 1H), 8.30 (d, J = 5.8 Hz, 1H), 8.00 (s, 1H), 6.77 (dd, J = 5.8, 1.4 Hz, 1H), 4.50 (brs, 3H), 3.80 (s, 4H), 3.73 - 3.64 (m, 1H), 3.57 (d, J = 8.3 Hz, 1H), 3.34 (d, J = 7.2 Hz, 3H), 2.80 (t, J = 7.9 Hz, 1H), 2.29 (dq, J = 7.4, 3.5 Hz, 1H), 1.89 (q, J = 10.4 Hz, 3H), 1.70 (s, 2H), 1.28 (d, J = 6.8 Hz, 6H). |
| **40** | ¹H NMR (400 MHz, DMSO-*d*₆) δ 10.27 (s, 1H), 9.20 (s, 1H), 8.69 (s, 1H), 8.30 (d, *J* = 5.9 Hz, 1H), 8.00 (s, 1H), 6.77 (d, *J* = 5.9 Hz, 1H), 4.65 (brs, 3H), 3.83 (dd, *J* = 9.7, 7.3 Hz, 1H), 3.80 (s, 3H), 3.69 (t, *J* = 8.7 Hz, 1H), 3.57 (q, *J* = 8.4 Hz, 1H), 3.44 (d, *J* = 7.0 Hz, 2H), 2.89 - 2.75 (m, 2H), 2.34 - 2.25 (m, 1H), 1.98 - 1.81 (m, 4H), 1.70 (s, 2H), 1.07 - 0.98 (m, 4H). |
| **41** | 1H NMR (400 MHz, CDCl₃) δ 9.34 (s, 1H), 8.59 (s, 1H), 8.36 (d, *J* = 5.7 Hz, 2H), 8.21 (s, 1H), 6.48 (d, *J* = 5.8 Hz, 1H), 4.50 (s, 2H), 3.99 (t, *J=* 8.5 Hz, 1H), 3.87 (s, 3H), 3.82 - 3.74 (m, 1H), 3.66 (q, *J* = 8.2 Hz, 1H), 3.47 (t, *J* = 8.7 Hz, 1H), 3.27 (d, *J* = 7.0 Hz, 2H), 3.06 (p, *J* = 7.3 Hz, 1H), 2.46 (tt, *J* = 8.0, 4.7 Hz, 2H), 2.06 - 1.92 (m, 2H), 1.81 (s, 2H), 1.37 - 1.24 (m, 4H), 1.13 - 1.07 (m, 2H). |
| **42** | ¹H NMR (400 MHz, DMSO-*d*₆) δ 10.26 (s, 1H), 9.20 (s, 1H), 8.68 (s, 1H), 8.30 (d, *J* = 5.8 Hz, 1H), 8.00 (s, 1H), 6.77 (dd, *J* = 5.9, 1.4 Hz, 1H), 4.50 (brs, 3H), 3.87 - 3.81 (m, 1H), 3.79 (s, 3H), 3.68 (dt, *J* = 9.0, 4.9 Hz, 1H), 3.56 (q, *J* = 8.4 Hz, 1H), 3.35 (d, *J* = 7.0 Hz, 2H), 3.17 - 3.09 (m, 2H), 2.80 (p, *J* = 7.3 Hz, 1H), 2.29 (td, *J* = 10.1, 3.5 Hz, 1H), 1.99 - 1.58 (m, 8H), 1.01 (t, *J* = 7.4 Hz, 3H). |
| **43** | ¹H NMR (400 MHz, DMSO-*d*₆) δ 10.27 (s, 1H), 9.22 - 9.18 (m, 1H), 8.68 (d, *J* = 0.9 Hz, 1H), 8.29 (d, *J* = 5.8 Hz, 1H), 8.00 (s, 1H), 6.76 (dd, *J* = 5.8, 1.4 Hz, 1H), 4.62 (brs, 3H), 3.87 - 3.81 (m, 1H), 3.79 (s, 3H), 3.72 - 3.64 (m, 1H), 3.56 (q, J = 8.3 Hz, 1H), 3.41 - 3.34 (m, 2H), 3.19 - 3.11 (m, 2H), 2.80 (p, *J =* 7.4 Hz, 1H), 2.34 - 2.21 (m, 1H), 2.02 - 1.81 (m, 4H), 1.69 (ddd, *J* = 12.1, 10.3, 6.6 Hz, 4H), 1.41 (hept, *J* = 7.1 Hz, 3H), 0.91 (t, J = 7.3 Hz, 3H). |
| **44** | ¹H NMR (400 MHz, DMSO-*d*₆) δ 11.05 (s, 1H), 9.10 (s, 1H), 8.75 (s, 1H), 8.33 (d, *J* = 6.4 Hz, 1H), 8.17 (s, 1H), 6.89 (d, *J* = 6.5 Hz, 1H), 4.61 (brs, 3H), 4.05 (p, *J* = 8.2 Hz, 2H), 3.82 (s, 4H), 3.68 (t, *J* = 8.7 Hz, 1H), 3.56 (q, *J* = 8.5 Hz, 1H), 3.35 - 3.19 (m, 3H), 2.76 (p, *J* = 7.8 Hz, 1H), 2.40 - 2.21 (m, 6H), 2.07 - 1.80 (m, 6H), 1.70 (s, 2H). |
| **45** | ¹H NMR (400 MHz, DMSO-*d*₆) δ 10.35 (s, 1H), 9.18 (s, 1H), 8.68 (s, 1H), 8.30 (d, *J* = 5.8 Hz, 1H), 8.02 (s, 1H), 6.78 (d, *J* = 5.9 Hz, 1H), 4.49 (brs, 3H), 3.80 (s, 5H), 3.62 (ddt, *J* = 23.7, 16.0, 9.5 Hz, 4H), 2.85 - 2.77 (m, 1H), 2.36 - 2.20 (m, 2H), 1.90 (dq, *J* = 20.7, 9.1 Hz, 8H), 1.78 - 1.54 (m, 8H). |
| **47** | ¹H NMR (400 MHz, CDCl₃) δ 9.32 (s, 1H), 8.59 (s, 1H), 8.35 (d, *J* = 5.8 Hz, 1H), 8.20 (s, 1H), 8.00 (s, 1H), 6.46 (d, *J* = 5.8 Hz, 1H), 4.81 (d, *J* = 7.5 Hz, 1H), 4.43 (s, 1H), 3.87 (s, 3H), 3.85 (m, 1H), 3.76 (dt, *J* = 9.7, 7.4 Hz, 1H), 3.67 (td, *J =* 8.7, 5.3 Hz, 1H), 3.49 (dd, *J =* 10.0, 4.0 Hz, 1H), 2.35 (dq, *J* = 13.5, 6.8 Hz, 1H), 1.98-2.04 (m, 4H), 1.47 (s, 9H). |
| **51** | ¹H NMR (400 MHz, DMSO-*d*₆) δ 10.26 (s, 1H), 9.19 (s, 1H), 8.70 (s, 1H), 8.29 (d, *J* = 5.6 Hz, 1H), 8.00 (d, *J* = 1.1 Hz, 1H), 7.53 (d, *J* = 6.6 Hz, 1H), 6.76 (d, *J* = 5.7 Hz, 1H), 4.60 (brs, 2H), 4.23 - 4.15 (m, 1H), 3.79 (s, 3H), 3.76 - 3.64 (m, 2H), 3.61 - 3.52 (m, 4H), 2.20 (dq, *J* = 13.1, 6.7 Hz, 1H), 1.91 (s, 4H), 1.69 (s, 2H). |
| **52** | ¹H NMR (400 MHz, DMSO-*d*₆) δ 10.21 (s, 1H), 9.14 (s, 1H), 8.65 (s, 1H), 8.23 (d, *J* = 5.8 Hz, 1H), 7.93 (s, 1H), 7.40 (d, *J* = 6.8 Hz, 1H), 6.70 (dd, *J* = 5.8, 1.5 Hz, 1H), 4.04 - 3.98 (m, 1H), 3.73 (m, 5H), 3.65 - 3.59 (m, 1H), 3.51 (q, *J* = 7.7 Hz, 1H), 3.37 - 3.33 (m, 1H), 2.94 (s, 3H), 2.23 - 2.16 (m, 2H), 1.95 - 1.88 (m, 2H), 1.84 (s, 3H), 1.63 (s, 3H). |
| **54** | ¹H NMR (400 MHz, DMSO-*d*₆) δ 10.27 (s, 1H), 9.21 (s, 1H), 8.72 (s, 1H), 8.41 (d, *J* = 6.8 Hz, 1H), 8.30 (d, *J* = 5.8 Hz, 1H), 8.00 (s, 1H), 6.76 (d, *J* = 5.9 Hz, 1H), 4.41 (q, *J=* 5.8 Hz, 1H), 3.80 (s, 3H), 3.78 - 3.68 (m, 2H), 3.60 (q, *J* = 7.9 Hz, 1H), 3.40 - 3.35 (m, 1H), 2.20 (dt, *J* = 13.9, 6.9 Hz, 1H), 1.92 (d, *J* = 6.0 Hz, 3H), 1.70 (s, 2H), 1.57 (td, *J* = 7.6, 3.8 Hz, 1H), 0.71 - 0.62 (m, 4H). |
| **55** | ¹H NMR (400 MHz, DMSO-*d*₆) δ 10.27 (s, 1H), 9.20 (s, 1H), 8.70 (s, 1H), 8.30 (d, *J* = 5.8 Hz, 1H), 8.00 (s, 1H), 7.54 (d, *J* = 6.7 Hz, 1H), 6.76 (d, *J* = 5.8 Hz, 1H), 4.20 (q, *J* = 6.1 Hz, 1H), 3.80 (s, 3H), 3.76 (dd,*J* = 9.9, 6.6 Hz, 1H), 3.68 (q, *J* = 7.6 Hz, 1H), 3.59 (t, *J* = 7.9 Hz, 1H), 3.55 (s, 3H), 3.39 (d, *J* = 4.8 Hz, 1H), 2.19 (dt, *J* = 13.7, 6.6 Hz, 1H), 2.04 - 1.94 (m, 1H), 1.92 (d, *J*= 7.5 Hz, 3H), 1.70 (s, 2H). |
| **56** | ¹H NMR (400 MHz, DMSO-*d*₆) δ 10.28 (s, 1H), 9.75-9.73 (d, 1H), 9.22 (s, 1H), 8.74 (s, 1H), 8.30 (d, *J* = 5.8 Hz, 1H), 8.00 (s, 1H), 6.77 (d, *J=* 5.7 Hz, 1H), 4.51 (q, *J* = 5.9 Hz, 1H), 3.88 - 3.82 (m, 1H), 3.80 (s, 3H), 3.72 (q, *J* = 7.9 Hz, 1H), 3.61 (q, *J* = 7.9 Hz, 1H), 3.53 (dd, *J* = 10.0, 4.7 Hz, 1H), 3.34 (s, 3H), 2.36 - 2.24 (m, 2H), 2.05 (dp, *J* = 11.5, 5.8 Hz, 2H), 1.91 (d, *J* = 7.7 Hz, 3H), 1.70 (s, 2H). |
| **57** | ¹H NMR (400 MHz, DMSO-*d*₆) δ 10.28 (s, 1H), 9.21 (s, 1H), 8.72 (s, 1H), 8.30 *(d, J=* 5.9 Hz, 1H), 8.00 (s, 1H), 7.51-7.49 (d, 1H),6.77 *(d, J=* 5.8 Hz, 1H), 4.06 - 4.02 (m, 1H), 3.81 (m, *J* = 2.1 Hz, 4H), 3.70 (m, 1H), 3.58 (m, 1H), 3.41 (m, 1H), 3.09 (t, *J* = 7.3 Hz, 2H), 2.29 - 2.24 (m, 2H), 1.99 (m, 3H), 1.92 (s, 3H), 1.71 (s, 2H), 1.24 (m, 5H). |
| **58** | ¹H NMR (400 MHz, DMSO-*d*₆) δ 10.26 (s, 1H), 9.20 (s, 1H), 8.71 (s, 1H), 8.30 (d, J = 5.8 Hz, 1H), 8.00 (s, 1H), 6.77 (d, J = 5.8 Hz, 1H), 5.32 (t, J = 4.8 Hz, 1H), 4.66 (brs, 2H), 4.15 - 4.08 (m, 1H), 3.80 (s, 4H), 3.70 (q, J = 7.9 Hz, 1H), 3.59 (t, J = 8.0 Hz, 1H), 3.44 (dd, J = 10.0, 5.5 Hz, 1H), 2.70 - 2.64 (m, 1H), 2.33 - 2.24 (m, 2H), 2.02 - 1.91 (m, 4H), 1.74 - 1.68 (m, 1H), 1.46 (d, J = 7.7 Hz, 1H), 0.97 (t, J = 6.9 Hz, 4H). |
| **A2** | ¹H NMR (400 MHz, DMSO-*d*₆) δ 10.35 (s, 1H), 9.09 (s, 1H), 8.77 (s, 1H), 8.31 (d, *J* = 5.8 Hz, 1H), 7.93 (s, 1H), 6.74 (d, *J* = 5.8 Hz, 1H), 5.14 (dt, *J* = 14.0, 7.0 Hz, 1H), 4.37 (t, *J* = 11.3 Hz, 1H), 3.95 (d, *J* = 10.2 Hz, 1H), 3.76 (s, 3H), 3.64 - 3.55 (m, 2H), 3.49 (p, *J* = 7.2 Hz, 1H), 3.12 (td, *J* = 6.2, 1.4 Hz, 2H), 2.24 (s, 3H), 2.00 (t, *J* = 12.9 Hz, 1H), 1.76 (d, *J* = 6.8 Hz, 3H). |
| **A3** | ¹H NMR (400 MHz, DMSO-*d*₆) δ 10.27 (s, 1H), 9.01 (s, 1H), 8.67 (s, 1H), 8.24 (d, *J* = 5.8 Hz, 1H), 7.86 (s, 1H), 6.67 (d, *J* = 5.9 Hz, 1H), 5.07 (dd, *J* = 10.4, 6.6 Hz, 1H), 4.30 (t, *J* = 11.4 Hz, 1H), 3.88 (d, *J* = 10.2 Hz, 1H), 3.69 (s, 3H), 2.66 (m, 3H), 2.11 (s, 3H), 2.05 (m, 2H), 1.94 (t, *J* = 12.8 Hz, 2H), 1.89 - 1.79 (m, 2H), 1.69 (d, *J* = 6.9 Hz, 3H), 1.66 - 1.56 (m, 2H). |
| **A4** | ¹H NMR (400 MHz, DMSO-*d*₆) δ 8.67 (d, *J* = 1.0 Hz, 1H), 8.42 (d, *J* = 6.1 Hz, 1H), 8.27 (d, *J* = 1.1 Hz, 1H), 7.87 (s, 1H), 6.79 (d, *J* = 6.1 Hz, 1H), 4.96 - 4.82 (m, 1H), 4.37 - 4.28 (m, 2H), 4.03 - 3.94 (m, 2H), 3.84 (d, *J* = 8.3 Hz, 2H), 3.70 (s, 3H), 3.61 - 3.53 (m, 5H), 3.42-3.35 (m, 1H), 3.00 (s, 3H), 2.42 - 2.31 (m, 1H), 1.91 (t, *J* = 12.9 Hz, 1H), 1.67 (d, *J* = 6.7 Hz, 3H). |

### III. Biological Test Evaluation

### (Cell Proliferation Assay)

### (I) Reagents and Consumables

DMEM Medium (Gibco, Cat#11965118)
RPMI1640 Medium (Gibco, Cat#11875119)
Fetal Bovine Serum (FBS) (GBICO, Cat#10099-141)
CellTiter-Glo^{®} Luminescent Cell Viability Assay Kit (Promega, Cat#G7572)
96-well, Black, Clear Flat Bottom Plate (Corning^{®}, Cat# 3603)

### (II) Instruments

SpectraMax Multimode Microplate Reader MD, 2104-0010A;
CO2 Incubator, Thermo Scientific 3100 Series;
Biosafety Cabinet, Thermo Scientific, 1300 Series A2;
Inverted Microscope, Olympus, CKX41SF;
Siemens Refrigerator, KK25E76TI.

### (III) Cell Lines and Culture Conditions

| No. | Cell Line | Cell Medium | Cell Density |
|---|---|---|---|
| 1 | A431 WT | DMEM + 15% FBS | 5000 |
| 2 | Ba/F3 EGFR-L858R-C797S (LC) | RPMI1640 + 10% FBS | 3000 |
| 3 | Ba/F3 EGFR-DEL19-C797S (DC) | RPMI1640 + 10% FBS | 3000 |
| 4 | Ba/F3 EGFR-L858R-T790M-C797S (LTC) | RPMI1640 + 10% FBS | 3000 |
| 5 | Ba/F3 EGFR-DEL19-T790M-C797S (DTC) | RPMI1640 + 10% FBS | 3000 |

### (IV) Experiment Steps

### 1. Cell Culture and Inoculation:

(1) Harvest cells in a logarithmic growth phase and count the cells using a platelet counter. Test cell viability using a trypan blue exclusion method to ensure that cell viability is above 90%.
(2) Adjust the cell concentration to desired final density; and add 90 µL of cell suspension to a 96-well plate.
(3) Incubate the cells overnight at 37°C, under 5% CO₂ and 95% humidity in the 96-well plate.

### 2. T0 Reference Data:

(1) Add 10 µL PBS to each well of the T0 plate containing cells.
(2) Thaw the CTG reagent and equilibrate the plate to room temperature for 30 minutes.
(3) Add an equal volume of CTG solution to each well.
(4) Lyse cells by shaking on an orbital shaker for 5 minutes.
(5) Leave the plate at room temperature for 20 minutes to stabilize the luminescence signal.
(6) Read the T0 fluorescence signal.

### 3. Compound Dilution and Addition

(1) Add a corresponding volume of DMSO to a corresponding compound powder according to the compound information sheet to prepare a 10 mM stock solution.
(2) Prepare 1000-fold and 3.16-fold diluted compound solutions.
(3) Dilute the 1000× diluted compound solution 100-fold with PBS to prepare a 10-fold compound solution, with a maximum concentration of 10 µM, nine concentrations and 3.16-fold dilution. Then add 10 µL of the drug solution concentration to individual wells of the 96-well plate seeded with cells. Test each compound concentration in triplicate wells, and the final DMSO concentration is 0.1%.
(4) Incubate the cells in the 96-well plate containing the drug compounds at 37°C, under 5% CO₂, and 95% humidity for an additional 72 hour period prior to performing the CTG analysis.

### 4. Fluorescence Signal Reading

(1) Thaw the CTG reagent and equilibrate the plate to room temperature for 30 minutes.
(2) Add an equal volume of CTG solution to each well.
(3) Lyse cells by shaking on an orbital shaker for 5 minutes.
(4) Leave the plate at room temperature for 20 minutes to stabilize the fluorescence signal.
(5) Read fluorescence value.

### 5. Data Processing

Analyze the data by using GraphPad Prism 7.0 software, and use Non-linear S-curve regression to fit the dose-response curves, to calculate the IC₅₀ values (in nM). The specific experiment results are presented in Table 1: 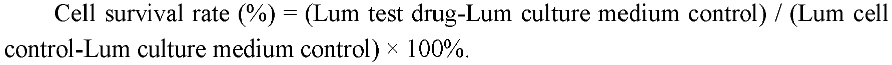 Cell survival rate (%) = (Lum test drug-Lum culture medium control) / (Lum cell control-Lum culture medium control) × 100%.

**Table 1: Biology Test Results**

| **Example No.** | **A431 WT** | **BaF3 LTC** | **BaF3 DTC** | **BaF3 LC** | **BaF3 DC** |
|---|---|---|---|---|---|
| **1** | 322.8 | 10.5 | 7.2 | 17.4 | 7.9 |
| **2** | 602.9 | 12.8 | 17.5 | 21.1 | 30.4 |
| **3** | 439.4 | 10.4 | 6.8 | 30.6 | 19.4 |
| **4** | 822.1 | 12.4 | 15.6 | 50.3 | 26.8 |
| **5** | 1084.3 | 9.2 | 4.7 | 14.0 | 19.7 |
| **6** | 1358.0 | 28.3 | NT | 35.6 | NT |
| **7** | 1660.0 | 24.6 | NT | 43.5 | NT |
| **8** | 499.2 | 39.4 | 27.8 | 63.5 | 31.5 |
| **9** | 1923.4 | 47.0 | 18.4 | 62.7 | 32.8 |
| **11** | 806.9 | 133.2 | 92.2 | 187.2 | 127.7 |
| **12** | 629.0 | 61.9 | NT | 123.5 | NT |
| **13** | 794.4 | 14.9 | 8.4 | 25.5 | 14.8 |
| **14** | 1716 | 3.6 | 3.5 | 5.0 | 2.8 |
| **15** | 1047.0 | 32.4 | NT | 84.4 | NT |
| **16** | 481.6 | 13.3 | NT | 23.8 | NT |
| **17** | 390.9 | 7.8 | NT | 32.1 | NT |
| **18** | 765.0 | 10.9 | NT | 25.4 | NT |
| **19** | 770.9 | 11.3 | NT | 19.5 | NT |
| **20** | 511.5 | 16.0 | NT | 38.0 | NT |
| **21** | 171.4 | 4.7 | 3.4 | 15.8 | 7.1 |
| **22** | 408.8 | 5.9 | NT | 13.2 | NT |
| **23** | 770.6 | 8.1 | 3.5 | 19.5 | 7.8 |
| **24** | 378.1 | 6.1 | 0.7 | NT | 4.4 |
| **25** | 570.7 | 106 | 42.6 | 123 | 66.5 |
| **26** | 645.4 | 28.5 | 16.3 | 33.5 | 11.4 |
| **27** | 587.2 | 5.2 | 1.0 | 13.4 | 1.1 |
| **28** | 682.4 | 86.9 | 12.4 | 45.7 | 12.8 |
| **29** | 621.8 | 4.0 | 1.8 | 9.6 | 1.6 |
| **30** | 743.5 | 5.8 | 2.4 | 11.8 | 2.5 |
| **31** | 366.3 | 4.3 | 4.8 | 10.8 | 3.5 |
| **32** | 543.0 | 12.1 | 10.5 | 14.9 | 5.1 |
| **34** | 375.3 | 1.4 | 1.6 | 2.7 | 0.7 |
| **35** | 345.0 | 1.0 | 0.7 | 2.2 | 0.9 |
| **36** | 614.0 | 2.0 | 1.5 | 5.1 | 2.5 |
| **37** | 553.9 | 6.4 | 3.2 | 15.8 | 6.1 |
| **39** | 445.7 | 0.8 | 1.5 | 2.1 | 2.3 |
| **40** | 320.5 | 0.7 | NT | 2.1 | NT |
| **41** | 564.0 | 3.1 | 5.2 | 12.9 | 15.9 |
| **42** | 592.9 | 1.2 | 2.3 | 1.9 | 3.5 |
| **43** | 699.6 | 1.3 | 3.6 | 2.9 | 5.0 |
| **44** | 1132.0 | 1.9 | 4.0 | 5.3 | 10.1 |
| **45** | 738.1 | 1.1 | 3.9 | 2.9 | 4.0 |
| **47** | 2574.0 | 35.2 | NT | 82.0 | NT |
| **48** | 357.0 | 4.5 | 2.0 | 7.6 | 2.5 |
| **49** | 926.0 | 29.6 | NT | 59.9 | NT |
| **50** | 1061.0 | 20.7 | NT | 86.5 | NT |
| **51** | 424.1 | 9.0 | 10.1 | 17.6 | 15.0 |
| **52** | 260.9 | 16.4 | NT | 31.0 | NT |
| **54** | 654.2 | 12.0 | 8.7 | 31.6 | 12.1 |
| **55** | 329.2 | 12.0 | 8.5 | 33.1 | 12.1 |
| **56** | 842.9 | 12.5 | NT | 36.5 | NT |
| **57** | 251.2 | 7.9 | 3.1 | 22.8 | 5.3 |
| **58** | 911.4 | 2.1 | 2.8 | 6.9 | 7.2 |
| **A1** | 2701 | 262.0 | 64.9 | 299 | 40.9 |
| **A2** | 2492 | 167.3 | NT | 176.5 | NT |
| **A3** | 1843 | 62.1 | NT | 53.3 | NT |
| **A4** | 2681.0 | 186.4 | 243.8 | 379.7 | 175.9 |
| **Positive Compoun d** | 449.5 | 8.0 | 4.2 | 11.5 | 4.9 |
| **Notes** | "NT" is an abbreviation of "Not Tested", which means that it has not been tested for the time being. | | | | |

The "positive compound" used in Biological Test Evaluation and Pharmacokinetic Assay in Mice of the present invention is compound 101 from WO2021168074A1, which is one of the most potent active compounds described in that prior art. The chemical structure of compound 101 is as follows:

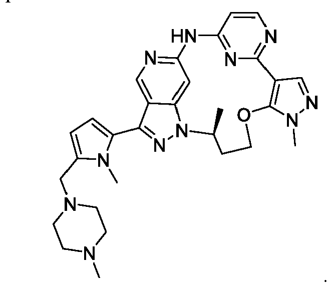

From the bioactivity data of the compounds in specific examples, it can be seen that the series of compounds of the present invention had a strong inhibitory effect on various resistant EGFR C797S mutations at the cellular level, including the EGFR L858R/C797S double mutation, EGFR Del19/C797S double mutation, L858R/T790M/C797S triple mutation and Del19/T790M/C797S triple mutation, and had high selectivity for EGFR WT.

### IV. Pharmacokinetic Experiment in Mice

### (I) Study Purpose

The purpose of this trial is to study the pharmacokinetic behavior of some compounds of the present invention, and the administration method is: Single oral administration (PO) to ICR mice.

### (II) Trial Protocol

### 1. Test Drug

The compounds used in this trial are from the compounds in the specific examples of the present invention and the listed positive compounds.

### 2. Test Animals

ICR mice male N=9 original source: Shanghai Sippr-Bk Lab Animal Co., Ltd.

### 3. Drug Preparation and Administration

The compounds are weighed and dissolved in the corresponding solution (10% PG + 10% Solutol^{®} + 80% pH3 citrate buffer). The mixture is then shaken well and sonicated to prepare a 1 mg/mL stock solution. Nine mice are orally administered at a dose of 10 mg/kg (or IV 2 mg/kg) after an overnight fast.

### 4. Sample Collection

About 90 µL of blood is collected via the submandibular vein per time point, heparin sodium anticoagulant is added, the sample is placed on ice after collection, and plasma is centrifuged within 1 hour (centrifugation conditions: 8,000 rpm, 6 minutes, 2-8°C). The blood collection timepoints are 0, 0.25, 0.5, 1, 2, 4, 6, 8 and 24 h. The samples are stored in a -20°C refrigerator.

The plasma sample is 40 µL, 160 µL of ice-cold acetonitrile containing internal standard is added, vortexed for 3 minutes, and centrifuged at 11,000 rpm for 5 minutes. 100 µL of the supernatant is taken and added to 100 µL of water, 5 µL of the sample is taken and injected into LC/MS/MS for analysis.

### 5. Test Results

**Table 2: Pharmacokinetic Experiment Results**

| **Example No.** | **Dose** | **Cₘₐₓ (ng/mL)** | **AUCₗₐₛₜ (hr*ng/mL)** | **T_{1/2} (h)** |
|---|---|---|---|---|
| **Example 1** | 10 mg/kg | 88.2 | 1029.9 | 3.5 |
| **Example 3** | 10 mg/kg | 307.1 | 3916.8 | 5.6 |
| **Example 7** | 10 mg/kg | 193.4 | 2515.9 | 5.6 |
| **Example 13** | 10 mg/kg | 1542.3 | 8867.0 | 2.2 |
| **Example 14** | 10 mg/kg | 882.3 | 5321.2 | 2.4 |
| **Example 22** | 10 mg/kg | 502.8 | 2336.1 | 3.0 |
| **Example 29** | 10 mg/kg | 1399.1 | 4540.4 | 0.66 |
| **Example 34** | 10 mg/kg | 693.6 | 2388.8 | 4.7 |
| **Example 35** | 10 mg/kg | 1498.7 | 5574.9 (Oral F, 81.5%) | 2.6 |
| **Example 37** | 10 mg/kg | 2071.5 | 10058.2 | 3.1 |
| **Example 39** | 10 mg/kg | 1559.2 | 6082.1 | 3.0 |
| **Example 40** | 10 mg/kg | 1352.4 | 4943.3 | 3.5 |
| **Example 41** | 10 mg/kg | 1918.8 | 7105.9 | 2.3 |
| **Example 42** | 10 mg/kg | 1059.2 | 4286.4 | 3.0 |
| **Example 51** | 10 mg/kg | 595.5 | 2691.9 | 2.8 |
| **Example 54** | 10 mg/kg | 412.3 | 1730.4 | 1.3 |
| **Positive Compounds** | 10 mg/kg | 32.7 | 204.4 | 4.8 |

From the pharmacokinetic data of the compounds in the above specific examples, this series of compounds of the present invention were absorbed into the mice body via a single oral dose (10 mg/kg), and showed very good PK data. Compared to the positive compounds, the AUC values of this series of compounds of the present invention were increased by at least 5-fold. Furthermore, some of the compounds in the examples exhibited AUC increases of up to 10-fold or more. The experiment results demonstrate that this series of compounds of the present invention exhibit significantly increased oral exposure, have good oral bioavailability, and are likely to solve the problem that pharmacokinetic limitations that make the positive compounds unsuitable for oral administration.

## Claims

1. A compound of formula (III), a stereoisomer or pharmaceutically acceptable salt thereof: 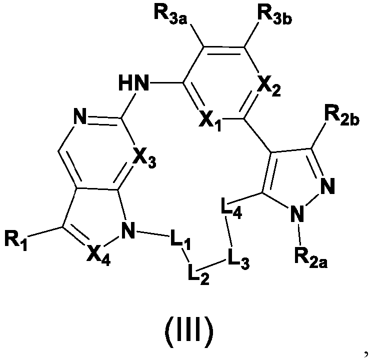 wherein,
X₁ is CRₐ or N;
X₂ is CR_{b} or N;
X₃ is CR_{c} or N;
X₄ is CR_{d} or N;
L₁ is CR₅ₐR_{5b};
L₂ is (CR_{5c}R₅ₐ)ₚ;
L₃ is a bond, CR₅ₑR_{5f} or C(O);
L₄ is CR_{5g}R₅ₕ, O, NR_{6c} or C(O);
R₁ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-8 membered heterocyclyl, C₆₋₈ aryl, 5-8 membered heteroaryl, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, - P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, and the above groups are each independently optionally further substituted by one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₁₋₄ alkyl substituted by C₃₋₆ cycloalkyl, C₁₋₄ alkyl substituted by 3-6 membered heterocyclyl, C₆₋₈ aryl, 5-8 membered heteroaryl, =O, =S, -SF₅, -S(O)(=N-R₇)R₈, -N=S(O)R₈R₉, - N=SR₈R₉, -N(R₁₃)-S(O)₂R₁₀, -O-S(O)₂R₁₀, -S(O)ᵣR₁₀, -O-R₁₁, -C(O)OR₁₁, -C(O)SR₁₁, - S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, and the above groups are each independently optionally further more substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₇)R₈, -C₀₋₄ alkyl-N=S(O)R₈R₉, -C₀₋₄ alkyl-N=SR₈R₉, -C₀₋₄ alkyl-N(R₁₃)-S(O)₂R₁₀, -C₀₋₄ alkyl-O-S(O)₂R₁₀, -C₀₋₄ alkyl-S(O)ᵣR₁₀, -C₀₋₄ alkyl-O-R₁₁, -C₀₋₄ alkyl-C(O)OR₁₁, -C₀₋₄ alkyl-C(O)SR₁₁, -C₀₋₄ alkyl-S-C(O)R₁₂, -C₀₋₄ alkyl-C(O)R₁₂, -C₀₋₄ alkyl-O-C(O)R₁₂, -C₀₋₄ alkyl-P(O)(R₁₂)₂, -C₀₋₄ alkyl-NR₁₃R₁₄, -C₀₋₄ alkyl-C(=NR₁₃)R₁₂, -C₀₋₄ alkyl-N(R₁₃)-C(=NR₁₄)R₁₂, -C₀₋₄ alkyl-C(O)NR₁₃R₁₄ and -C₀₋₄ alkyl-N(R₁₃)-C(O)R₁₂;
R₂ₐ is selected from the group consisting of hydrogen, deuterium, hydroxy, C₁₋₄ alkyl, C₂₋₄ alkenyl, C₂₋₄ alkynyl, C₃₋₆ cycloalkyl and 3-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, - N=SR₈R₉, -O-S(O)₂R₁₀, -S(O)ᵣR₁₀, -O-R₁₁, -C(O)OR₁₁, -C(O)SR₁₁, -S-C(O)R₁₂, - C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, - C(O)NR₁₃R₁₄ and -N(R₁₃)-C(O)R₁₂;
R_{2b} is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₃ₐ, R_{3b}, Rₐ and R_{b} are each independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅;
R_{c} is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅;
R_{d} is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅;
R₅ₐ and R_{5b} are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅, or, R₅ₐ and R_{5b}, together with the carbon atom to which they are directly attached, form a C(O), C₃₋₆ cycloalkyl, 4-6 membered heterocyclyl, C₆₋₈ aryl or 5-8 membered heteroaryl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
when p is 1, R_{5c} and R_{5d}, together with the carbon atom to which they are directly attached, form a C₃₋₆ cycloalkyl or 4-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, - N=SR₈R₉, -O-S(O)₂R₁₀, -S(O)ᵣR₁₀, -O-R₁₁, -C(O)OR₁₁, -C(O)SR₁₁, -S-C(O)R₁₂, - C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, - C(O)NR₁₃R₁₄ and -N(R₁₃)-C(O)R₁₂, or
when p is 2, one group of R_{5c} and R_{5d}, together with the carbon atom to which they are directly attached, form a C₃₋₆ cycloalkyl or 4-6 membered heterocyclyl, and when one group of R_{5c} and R_{5d}, together with the carbon atom to which they are directly attached, form a C₃₋₆ cycloalkyl or 4-6 membered heterocyclyl, the other group of R_{5c} and R_{5d} are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₇)R₈, -N=S(O)R₈R₉, -N=SR₈R₉, - O-S(O)₂R₁₀, -S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₅ₑ and R_{5f} are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅, or, R₅ₑ and R_{5f}, together with the carbon atom to which they are directly attached, form a C₃₋₆ cycloalkyl, 4-6 membered heterocyclyl, C₆₋₈ aryl or 5-8 membered heteroaryl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, - N=SR₈R₉, -O-S(O)₂R₁₀, -S(O)ᵣR₁₀, -O-R₁₁, -C(O)OR₁₁, -C(O)SR₁₁, -S-C(O)R₁₂, - C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, - C(O)NR₁₃R₁₄ and -N(R₁₃)-C(O)R₁₂;
R_{5g} and R₅ₕ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl and -SF₅, or, R_{5g} and R₅ₕ, together with the carbon atom to which they are directly attached, form a C₃₋₆ cycloalkyl, 4-6 membered heterocyclyl, C₆₋₈ aryl or 5-8 membered heteroaryl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, - N=SR₈R₉, -O-S(O)₂R₁₀, -S(O)ᵣR₁₀, -O-R₁₁, -C(O)OR₁₁, -C(O)SR₁₁, -S-C(O)R₁₂, - C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, - C(O)NR₁₃R₁₄ and -N(R₁₃)-C(O)R₁₂;
R_{6c} is selected from the group consisting of hydrogen, deuterium, C₁₋₄ alkyl, C₃₋₆ cycloalkyl, 3-6 membered heterocyclyl and -O-R₁₁, and the above groups are each independently optionally further substituted by one or more substituents selected from the group consisting of deuterium, halogen, cyano, hydroxy, =O, C₁₋₄ alkyl, C₁₋₄ haloalkyl, C₁₋₄ deuterioalkyl, C₂₋₄ alkenyl, C₂₋₄ alkynyl, C₁₋₄ alkoxy, C₃₋₆ cycloalkyl, C₃₋₆ cycloalkoxy, 3-6 membered heterocyclyl, 3-6 membered heterocyclyloxy and -NR₁₃R₁₄;
each R₇ is independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₀, -C₀₋₈ alkyl-C(O)OR₁₁, -C₀₋₈ alkyl-C(O)R₁₂ or -C₀₋₈ alkyl-C(O)NR₁₃R₁₄, and the above groups are optionally further substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ or -C₀₋₈ alkyl-N(R₁₃)-C(O)R₁₂;
each R₈ and each R₉ are independently selected from the group consisting of hydrogen, deuterium, hydroxy, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl and 5-10 membered heteroaryl, or, R₈ and R₉, together with the sulfur atom to which they are directly attached, form a 3-10 membered heterocyclyl, and the above groups are optionally further substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂;
each R₁₀ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₁₃R₁₄, and the above groups are each independently optionally further substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₃R₁₄;
each R₁₁ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl and 5-10 membered heteroaryl, and the above groups are each independently optionally further substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₃R₁₄;
each R₁₂ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₃R₁₄, and the above groups are each independently optionally further substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₃R₁₄;
each R₁₃ and each R₁₄ are independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₁₀ alkanoyl, and the above groups are each independently optionally further substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, monoC₁₋₁₀ alkylamino, diC₁₋₁₀ alkylamino and C₁₋₁₀ alkanoyl; or
R₁₃ and R₁₄, together with the nitrogen atom to which they are directly attached, form a 4-10 membered heterocyclyl or 5-10 membered heteroaryl and the 4-10 membered heterocyclyl or 5-10 membered heteroaryl is optionally further substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, methanesulfonylmethyl, amino, monoC₁₋₁₀ alkylamino, diC₁₋₁₀ alkylamino and C₁₋₁₀ alkanoyl;
p is 1 or 2; and
each r is independently 0, 1 or 2.

2. The compound of formula (III), the stereoisomer or pharmaceutically acceptable salt thereof according to claim 1, wherein the compound of formula (III) is a compound of formula (IV) as shown below: 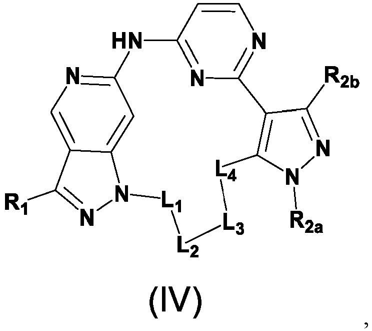 wherein,
L₁ is CR₅ₐR_{5b};
L₂ is CR_{5c}R_{5d};
L₃ is CR₅ₑR_{5f};
L₄ is O;
R₁ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-8 membered heterocyclyl, C₆₋₈ aryl, 5-8 membered heteroaryl and -SF₅, and the above groups are each independently optionally further substituted by one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₁₋₄ alkyl substituted by C₃₋₆ cycloalkyl, C₁₋₄ alkyl substituted by 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -N(R₁₃)-S(O)₂R₁₀, -O-S(O)₂R₁₀, - S(O)ᵣR₁₀, -O-R₁₁, -C(O)OR₁₁, -C(O)SR₁₁, -S-C(O)R₁₂, -C(O)R₁₂, -O-C(O)R₁₂, - P(O)(R₁₂)₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, and the above groups are each independently optionally further more substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₇)R₈, -N=S(O)R₈R₉, -N=SR₈R₉, -N(R₁₃)-S(O)₂R₁₀, -O-S(O)₂R₁₀, -S(O)ᵣR₁₀, - O-R₁₁, -C(O)OR₁₁, -C(O)SR₁₁, -S-C(O)R₁₂, -C(O)R₁₂, -O-C(O)R₁₂, -P(O)(R₁₂)₂, - NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₂ₐ is selected from the group consisting of hydrogen, deuterium, hydroxy, C₁₋₄ alkyl, C₂₋₄ alkenyl, C₂₋₄ alkynyl, C₃₋₆ cycloalkyl and 3-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅;
R_{2b} is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl and - SF₅, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅;
R₅ₐ and R_{5b} are each independently selected from the group consisting of hydrogen, deuterium, halogen, cyano, C₁₋₄ alkyl, C₃₋₆ cycloalkyl and 3-6 membered heterocyclyl, or, R₅ₐ and R_{5b}, together with the carbon atom to which they are directly attached, form a C₃₋₆ cycloalkyl or 4-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅; and
R_{5c} and R_{5d}, together with the carbon atom to which they are directly attached, form a C₃₋₆ cycloalkyl or 4-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅;
R₅ₑ and R_{5f} are each independently selected from the group consisting of hydrogen, deuterium, halogen, cyano, C₁₋₄ alkyl, C₃₋₆ cycloalkyl and 3-6 membered heterocyclyl, or, R₅ₑ and R_{5f}, together with the carbon atom to which they are directly attached, form a C₃₋₆ cycloalkyl or 4-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅; and R₇, R₈, R₉, R₁₀, R₁₁, R₁₂, R₁₃, R₁₄ and r are as defined in claim 1.

3. The compound of formula (III), the stereoisomer or pharmaceutically acceptable salt thereof according to claim 1, wherein:
L₁ is CR₅ₐR_{5b};
L₂ is CR_{5c}R_{5d};
L₃ is CR₅ₑR_{5f};
L₄ is O;
R₅ₐ and R_{5b} are each independently selected from the group consisting of hydrogen, deuterium, fluorine, chlorine, bromine, cyano, methyl, ethyl, propyl, isopropyl, cyclopropyl, cyclobutyl, oxacyclopropyl, oxacyclobutyl, azacyclopropyl and azacyclobutyl, and the above groups are each independently optionally further substituted by one or more substituents selected from the group consisting of deuterium, fluorine, chlorine, bromine, cyano, methyl, ethyl, propyl, isopropyl, monofluoromethyl, difluoromethyl, trifluoromethyl, monodeuteriomethyl, dideuteriomethyl, trideuteriomethyl, vinyl, ethynyl, cyclopropyl, cyclobutyl, oxacyclopropyl, oxacyclobutyl, azacyclopropyl, azacyclobutyl, =O, =S and -SF₅;
R_{5c} and R_{5d}, together with the carbon atom to which they are directly attached, form a C₃₋₆ cycloalkyl or 4-6 membered heterocyclyl, and the above groups are each independently optionally further substituted by one or more substituents selected from the group consisting of deuterium, fluorine, chlorine, bromine, cyano, methyl, ethyl, propyl, isopropyl, monofluoromethyl, difluoromethyl, trifluoromethyl, monodeuteriomethyl, dideuteriomethyl, trideuteriomethyl, vinyl, ethynyl, cyclopropyl, cyclobutyl, oxacyclopropyl, oxacyclobutyl, azacyclopropyl, azacyclobutyl, =O, =S and - SF₅; and
R₅ₑ and R_{5f} are each independently selected from the group consisting of hydrogen, deuterium, fluorine, chlorine, bromine, cyano, methyl, ethyl, propyl, isopropyl, cyclopropyl, cyclobutyl, oxacyclopropyl, oxacyclobutyl, azacyclopropyl and azacyclobutyl, and the above groups are each independently optionally further substituted by one or more substituents selected from the group consisting of deuterium, fluorine, chlorine, bromine, cyano, methyl, ethyl, propyl, isopropyl, monofluoromethyl, difluoromethyl, trifluoromethyl, monodeuteriomethyl, dideuteriomethyl, trideuteriomethyl, vinyl, ethynyl, cyclopropyl, cyclobutyl, oxacyclopropyl, oxacyclobutyl, azacyclopropyl, azacyclobutyl, =O, =S and -SF₅;
R₂ₐ is selected from the group consisting of hydrogen, deuterium, hydroxy, methyl, ethyl, propyl, isopropyl, vinyl, ethynyl, cyclopropyl, cyclobutyl, oxacyclopropyl, oxacyclobutyl, azacyclopropyl and azacyclobutyl, and the above groups are each independently optionally further substituted by one or more substituents selected from the group consisting of deuterium, fluorine, chlorine, bromine, cyano, methyl, ethyl, propyl, isopropyl, monofluoromethyl, difluoromethyl, trifluoromethyl, monodeuteriomethyl, dideuteriomethyl, trideuteriomethyl, vinyl, ethynyl, cyclopropyl, cyclobutyl, oxacyclopropyl, oxacyclobutyl, azacyclopropyl, azacyclobutyl, =O, =S and - SF₅; and
R_{2b} is selected from the group consisting of hydrogen, deuterium, fluorine, chlorine, bromine, cyano, methyl, ethyl, propyl, isopropyl, vinyl, ethynyl, cyclopropyl, cyclobutyl, oxacyclopropyl, oxacyclobutyl, azacyclopropyl, azacyclobutyl and -SF₅, and the above groups are each independently optionally further substituted by one or more substituents selected from the group consisting of deuterium, fluorine, chlorine, bromine, cyano, methyl, ethyl, propyl, isopropyl, monofluoromethyl, difluoromethyl, trifluoromethyl, monodeuteriomethyl, dideuteriomethyl, trideuteriomethyl, vinyl, ethynyl, cyclopropyl, cyclobutyl, oxacyclopropyl, oxacyclobutyl, azacyclopropyl, azacyclobutyl, =O, =S and - SF₅.

4. The compound of formula (III), the stereoisomer or pharmaceutically acceptable salt thereof according to claim 1, wherein the compound of formula (III) is a compound of formula (V) as shown below: 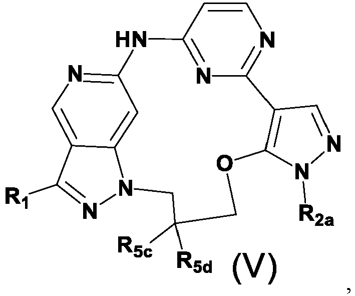 wherein,
R₁ is selected from C₂₋₄ alkenyl, C₂₋₄ alkynyl and 3-8 membered heterocyclyl, and the above groups are each independently optionally further substituted by one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₁₋₄ alkyl substituted by C₃₋₆ cycloalkyl, C₁₋₄ alkyl substituted by 3-6 membered heterocyclyl, =O, =S, -SF₅, -N(R₁₃)-S(O)₂R₁₀, -O-S(O)₂R₁₀, -S(O)ᵣR₁₀, -O-R₁₁, -C(O)OR₁₁, -S-C(O)R₁₂, - C(O)R₁₂, -O-C(O)R₁₂, -NR₁₃R₁₄, -C(O)NR₁₃R₁₄ and -N(R₁₃)-C(O)R₁₂, and the above groups are each independently optionally further more substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -N(R₁₃)-S(O)₂R₁₀, -O-S(O)₂R₁₀, -S(O)ᵣR₁₀, -O-R₁₁, -C(O)OR₁₁, -C(O)R₁₂, -O-C(O)R₁₂, -NR₁₃R₁₄, -C(O)NR₁₃R₁₄ and -N(R₁₃)-C(O)R₁₂;
R₂ₐ is selected from the group consisting of hydrogen, deuterium, C₁₋₄ alkyl, C₃₋₆ cycloalkyl and 3-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₃₋₆ cycloalkyl and 3-6 membered heterocyclyl;
R_{5c} and R_{5d}, together with the carbon atom to which they are directly attached, form a C₃₋₆ cycloalkyl or 4-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -SF₅;
R₁₀, R₁₁, R₁₂, R₁₃, R₁₄ and r are as defined in claim 1.

5. The compound of formula (III), the stereoisomer or pharmaceutically acceptable salt thereof according to claim 4, wherein R_{5c} and R_{5d}, together with the carbon atom to which they are directly attached, form a C₃₋₆ cycloalkyl or 4-6 membered heterocyclyl; optionally wherein R_{5c} and R_{5d}, together with the carbon atom to which they are directly attached, form a cyclopropyl, cyclobutyl, cyclopentyl, cyclohexyl, oxacyclobutyl, oxacyclopentyl or oxacyclohexyl.

6. The compound of formula (III), the stereoisomer or pharmaceutically acceptable salt thereof according to claim 4, wherein:
R₁ is C₂₋₄ alkynyl, and the C₂₋₄ alkynyl is optionally further substituted by one or more substituents selected from the group consisting of deuterium, C₁₋₄ alkyl, C₃₋₆ cycloalkyl and 3-6 membered heterocyclyl, and the above groups are each independently optionally further more substituted by one or more substituents selected from the group consisting of deuterium, halogen, cyano, C₁₋₄ alkyl, C₁₋₄ haloalkyl, C₁₋₄ deuterioalkyl, C₃₋₆ cycloalkyl and 3-6 membered heterocyclyl.

7. The compound of formula (III), the stereoisomer or pharmaceutically acceptable salt thereof according to claim 6, wherein:
R₁ is ethynyl, and the ethynyl is optionally further substituted by one or more substituents selected from the group consisting of deuterium, methyl, ethyl, propyl, isopropyl, butyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl, and the above groups are each independently optionally further more substituted by one or more substituents selected from the group consisting of deuterium, fluorine, bromine, cyano, methyl, ethyl, propyl, isopropyl, butyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl.

8. The compound of formula (III), the stereoisomer or pharmaceutically acceptable salt thereof according to claim 6, wherein:
R₁ is ethynyl, and the ethynyl is optionally further substituted by one or more substituents selected from the group consisting of cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl, and the above groups are each independently optionally further more substituted by one or more substituents selected from the group consisting of deuterium, fluorine, bromine, cyano, methyl, ethyl, propyl, isopropyl, monofluoromethyl, difluoromethyl, trifluoromethyl, monodeuteriomethyl, dideuteriomethyl and trideuteriomethyl.

9. The compound of formula (III), the stereoisomer or pharmaceutically acceptable salt thereof according to claim 6, wherein:
R₁ is 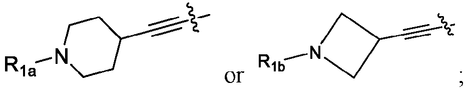
R₁ₐ is selected from the group consisting of hydrogen, methyl, ethyl, propyl, isopropyl, monofluoromethyl, difluoromethyl, trifluoromethyl, monodeuteriomethyl, dideuteriomethyl and trideuteriomethyl; and
R_{1b} is selected from the group consisting of hydrogen, methyl, ethyl, propyl, isopropyl, monofluoromethyl, difluoromethyl, trifluoromethyl, monodeuteriomethyl, dideuteriomethyl and trideuteriomethyl.

10. The compound of formula (III), the stereoisomer or pharmaceutically acceptable salt thereof according to claim 4, wherein R₁ is 4-8 membered nitrogen-containing heterocyclyl, and the 4-8 membered nitrogen-containing heterocyclyl is optionally further substituted by one or more substituents selected from the group consisting of deuterium, halogen, cyano, C₁₋₄ alkyl, =O, -N(R₁₃)-S(O)₂R₁₀, -S(O)ᵣR₁₀, -O-R₁₁, -O-C(O)R₁₂, - NR₁₃R₁₄ and -N(R₁₃)-C(O)R₁₂, and the C₁₋₄ alkyl is optionally further more substituted by one or more substituents selected from the group consisting of deuterium, halogen, cyano, C₁₋₄ alkyl, C₁₋₄ haloalkyl, C₁₋₄ deuterioalkyl, -N(R₁₃)-S(O)₂R₁₀, -O-S(O)₂R₁₀, - S(O)ᵣR₁₀, -O-R₁₁, -O-C(O)R₁₂, -NR₁₃R₁₄ and -N(R₁₃)-C(O)R₁₂; and
R₁₀, R₁₁, R₁₂, R₁₃, R₁₄ and r are as defined in claim 4.

11. The compound of formula (III), the stereoisomer or pharmaceutically acceptable salt thereof according to claim 10, wherein:
R₁ is of the following structure: 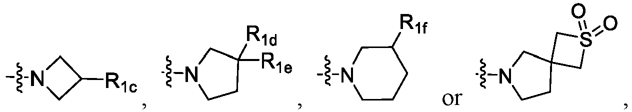 wherein,
R_{1c} is selected from the group consisting of hydrogen, deuterium, halogen, C₁₋₄ alkyl, -N(R₁₃ₐ)-S(O)₂R₁₀ₐ, -O-R₁₁ₐ and -N(R₁₃ₐ)-C(O)R₁₂ₐ, and the C₁₋₄ alkyl is optionally further more substituted by one or more substituents selected from the group consisting of deuterium, C₁₋₄ alkyl, C₁₋₄ haloalkyl, C₁₋₄ deuterioalkyl, -S(O)ᵣR_{10b} and -O-R₁₁ₐ;
R_{1d} is selected from the group consisting of hydrogen, deuterium, halogen, C₁₋₄ alkyl, -N(R₁₃ₐ)-S(O)₂R₁₀ₐ, -O-R₁₁ₐ and -N(R₁₃ₐ)-C(O)R₁₂ₐ;
R₁ₑ is selected from the group consisting of hydrogen, deuterium, halogen, C₁₋₄ alkyl, -N(R₁₃ₐ)-S(O)₂R₁₀ₐ, -O-R₁₁ₐ and -N(R₁₃ₐ)-C(O)R₁₂ₐ, and the C₁₋₄ alkyl is optionally further more substituted by one or more substituents selected from the group consisting of deuterium, C₁₋₄ alkyl, C₁₋₄ haloalkyl, C₁₋₄ deuterioalkyl, -S(O)ᵣR_{10b} and -O-R₁₁ₐ;
R_{1f} is selected from the group consisting of hydrogen, deuterium, halogen, C₁₋₄ alkyl, -N(R₁₃ₐ)-S(O)₂R₁₀ₐ, -O-R₁₁ₐ and -N(R₁₃ₐ)-C(O)R₁₂ₐ, and the C₁₋₄ alkyl is optionally further more substituted by one or more substituents selected from the group consisting of deuterium, C₁₋₄ alkyl, C₁₋₄ haloalkyl, C₁₋₄ deuterioalkyl, -S(O)ᵣR_{10b} and -O-R₁₁ₐ;
each R₁₀ₐ is independently selected from the group consisting of hydrogen, C₁₋₄ alkyl, C₃₋₆ cycloalkyl and 3-6 membered heterocyclyl;
each R_{10b} is independently selected from the group consisting of hydrogen, C₁₋₄ alkyl, C₃₋₆ cycloalkyl and 3-6 membered heterocyclyl, and the above groups are each independently optionally further substituted by one or more substituents selected from the group consisting of deuterium, halogen, hydroxy, C₁₋₄ alkyl and C₁₋₄ alkoxy;
each R₁₁ₐ is independently hydrogen or C₁₋₄ alkyl;
each R₁₂ₐ is independently selected from the group consisting of hydrogen, C₁₋₄ alkyl, C₁₋₄ alkoxy and C₃₋₆ cycloalkyl, and the above groups are each independently optionally further substituted by one or more substituents selected from the group consisting of deuterium, halogen, hydroxy and cyano;
each R₁₃ₐ is independently hydrogen or C₁₋₄ alkyl; and
each r is independently 0, 1 or 2.

12. The compound of formula (III), the stereoisomer or pharmaceutically acceptable salt thereof according to claim 10, wherein:
R₁ is of the following structure: 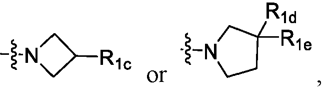 wherein,
R_{1c} is selected from the group consisting of hydrogen, deuterium, fluorine, bromine, methyl, ethyl, propyl, isopropyl, butyl, -N(R₁₃ₐ)-S(O)₂R₁₀ₐ, -O-R₁₁ₐ and -N(R₁₃ₐ)-C(O)R₁₂ₐ, and the methyl, ethyl, propyl, isopropyl or butyl is independently optionally further more substituted by one or more substituents selected from the group consisting of deuterium, methyl, ethyl, propyl, isopropyl, butyl, monofluoromethyl, difluoromethyl, trifluoromethyl, monodeuteriomethyl, dideuteriomethyl, trideuteriomethyl, -S(O)ᵣR_{10b} and -O-R₁₁ₐ;
R_{1d} is selected from the group consisting of hydrogen, deuterium, fluorine, bromine, methyl, ethyl, propyl, isopropyl and butyl;
R₁ₑ is selected from the group consisting of hydrogen, deuterium, fluorine, bromine, methyl, ethyl, propyl, isopropyl, butyl, -N(R₁₃ₐ)-S(O)₂R₁₀ₐ, -O-R₁₁ₐ and -N(R₁₃ₐ)-C(O)R₁₂ₐ, and the methyl, ethyl, propyl, isopropyl or butyl is independently optionally further more substituted by one or more substituents selected from the group consisting of deuterium, methyl, ethyl, propyl, isopropyl, butyl, monofluoromethyl, difluoromethyl, trifluoromethyl, monodeuteriomethyl, dideuteriomethyl, trideuteriomethyl, -S(O)ᵣR_{10b} and -O-R₁₁ₐ;
each R₁₀ₐ is independently selected from the group consisting of hydrogen, methyl, ethyl, propyl, isopropyl, butyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl;
each R_{10b} is independently selected from the group consisting of hydrogen, methyl, ethyl, propyl, isopropyl, butyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl, and the above groups are each independently optionally further substituted by one or more substituents selected from the group consisting of deuterium, fluorine, chlorine, hydroxy, methyl, ethyl, propyl, isopropyl, butyl, methoxy, ethoxy and propoxy;
each R₁₁ₐ is independently selected from the group consisting of hydrogen, methyl, ethyl, propyl, isopropyl and butyl;
each R₁₂ₐ is independently selected from the group consisting of hydrogen, methyl, ethyl, propyl, isopropyl, butyl, methoxy, ethoxy, propoxy, tert-butoxy, cyclopropyl, cyclobutyl, cyclopentyl and cyclohexyl, and the above groups are each independently optionally further substituted by one or more substituents selected from the group consisting of deuterium, fluorine, chlorine, hydroxy and cyano;
each R₁₃ₐ is independently hydrogen; and
each r is independently 0, 1 or 2.

13. The compound of formula (III), the stereoisomer or pharmaceutically acceptable salt thereof according to claim 10, wherein:
R₁ is of the following structure: 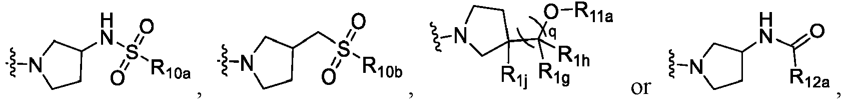 wherein,
R_{1g} is hydrogen, deuterium or methyl; R₁ₕ is hydrogen, deuterium or methyl; R₁ⱼ is hydrogen, deuterium or methyl; q is 0, 1 or 2;
R₁₀ₐ is selected from the group consisting of hydrogen, methyl, ethyl, propyl, isopropyl, butyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl;
R_{10b} is selected from the group consisting of hydrogen, methyl, ethyl, propyl, isopropyl, butyl, cyclopropyl, cyclobutyl, cyclopentyl, cyclohexyl, oxacyclopropyl, oxacyclobutyl, oxacyclopentyl, oxacyclohexyl, azacyclopropyl, azacyclobutyl, azacyclopentyl and azacyclohexyl, and the above groups are each independently optionally further substituted by one or more substituents selected from the group consisting of deuterium, fluorine, chlorine, hydroxy, methyl, ethyl, propyl, isopropyl, butyl, methoxy, ethoxy and propoxy;
each R₁₁ₐ is independently selected from the group consisting of hydrogen, methyl, ethyl, propyl, isopropyl and butyl;
R₁₂ₐ is selected from the group consisting of hydrogen, methyl, ethyl, propyl, isopropyl, butyl, methoxy, ethoxy, propoxy, tert-butoxy, cyclopropyl, cyclobutyl, cyclopentyl and cyclohexyl, and the above groups are each independently optionally further substituted by one or more substituents selected from the group consisting of deuterium, fluorine, chlorine, hydroxy and cyano.

14. The compound of formula (III), the stereoisomer or pharmaceutically acceptable salt thereof according to claim 4, wherein R₂ₐ is selected from the group consisting of hydrogen, deuterium, C₁₋₄ alkyl, C₁₋₄ haloalkyl, C₁₋₄ deuterioalkyl, C₃₋₆ cycloalkyl and 3-6 membered heterocyclyl.

15. The compound of formula (III), the stereoisomer or pharmaceutically acceptable salt thereof according to claim 14, wherein R₂ₐ is selected from the group consisting of hydrogen, deuterium, methyl, ethyl, propyl, isopropyl, butyl, monofluoromethyl, difluoromethyl, trifluoromethyl, monodeuteriomethyl, dideuteriomethyl, trideuteriomethyl, cyclopropyl, cyclobutyl, oxacyclopropyl, oxacyclobutyl, azacyclopropyl and azacyclobutyl.

16. The compound of formula (III), the stereoisomer or pharmaceutically acceptable salt thereof according to claim 1, wherein it is selected from the following compounds: 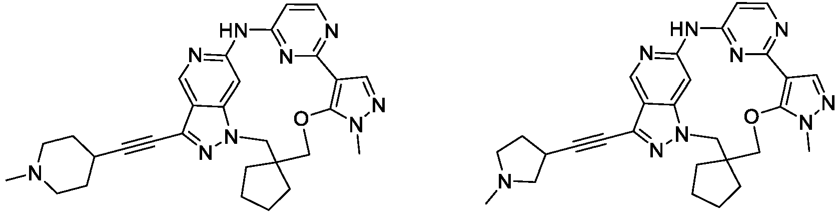 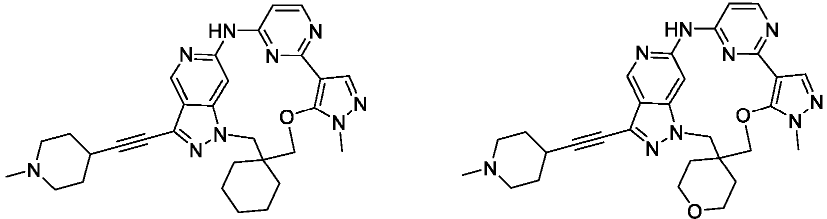 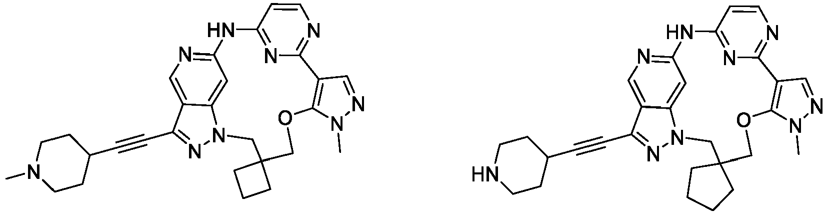 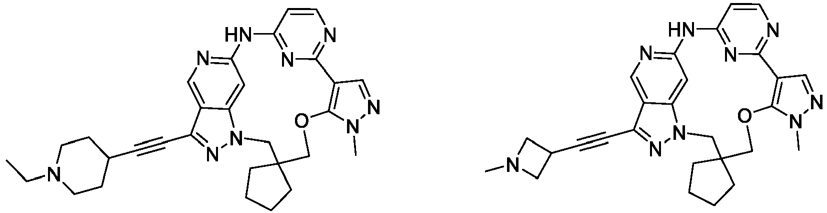 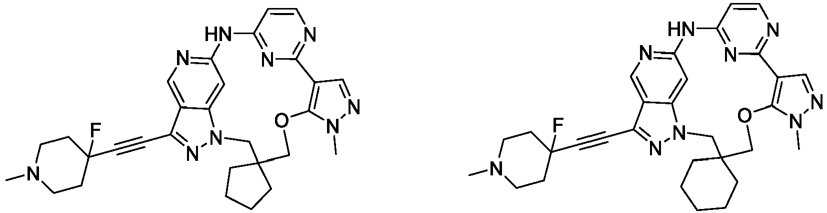 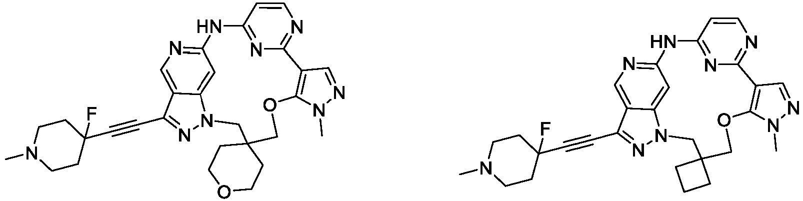 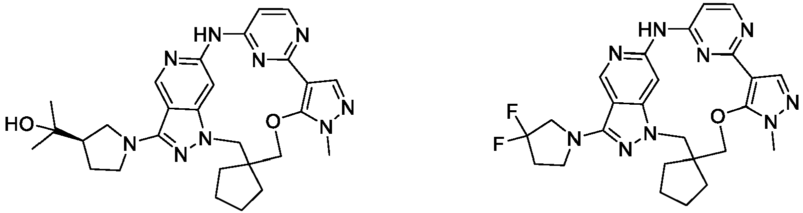 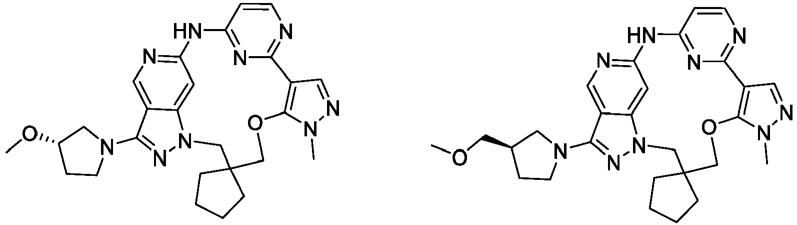 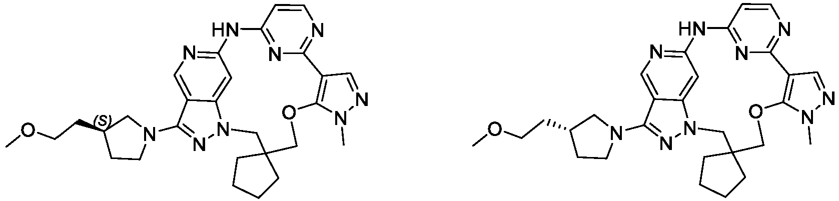 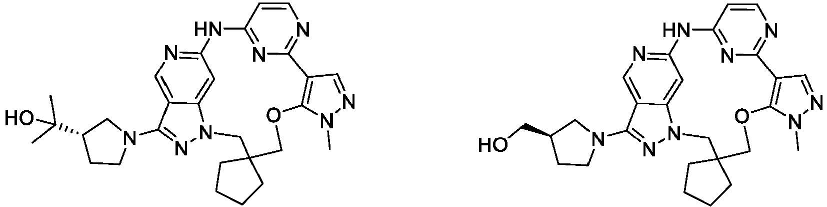 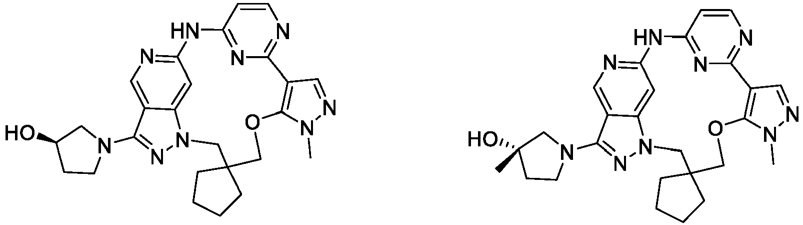 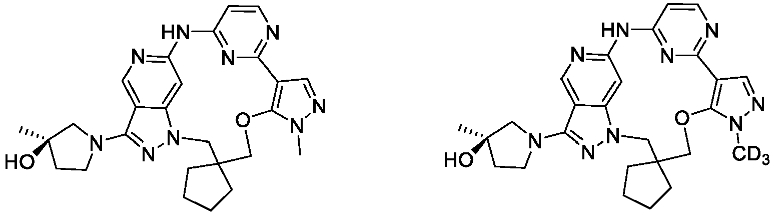 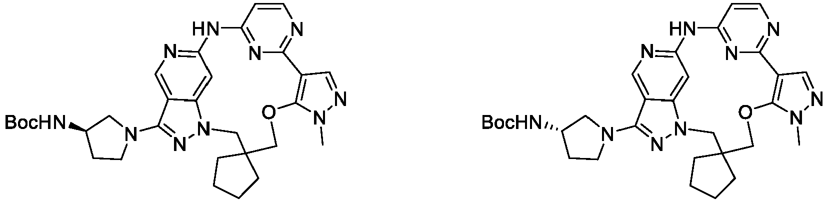 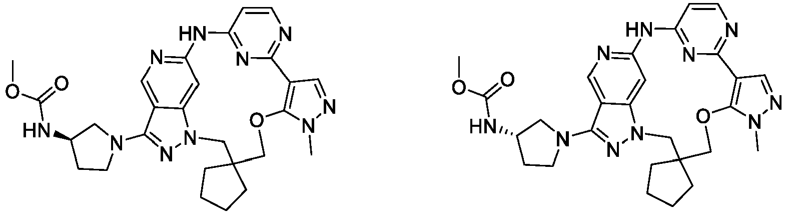 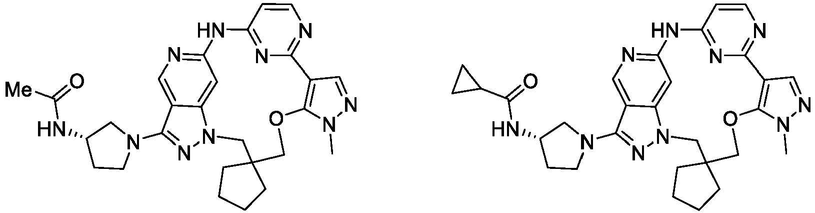 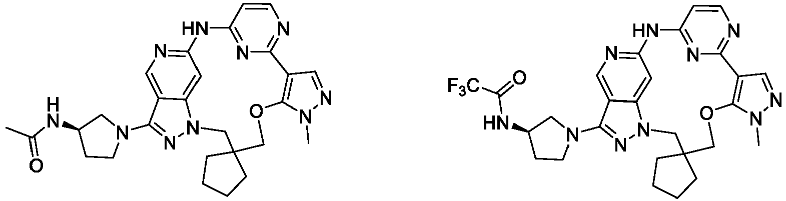 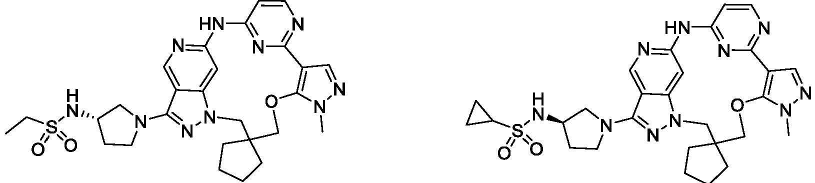 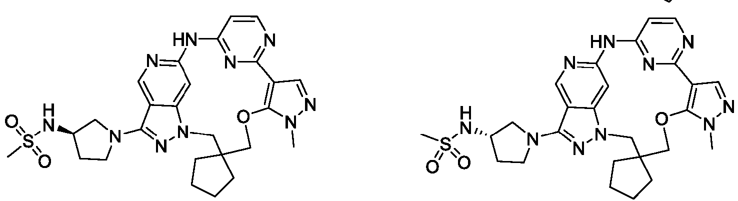 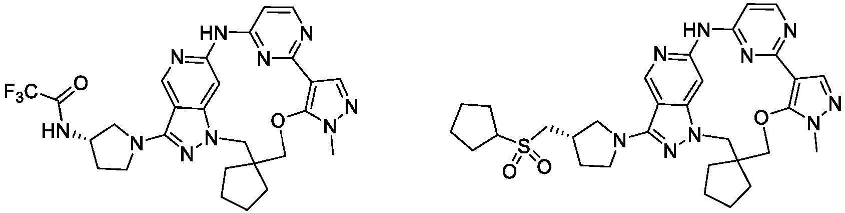 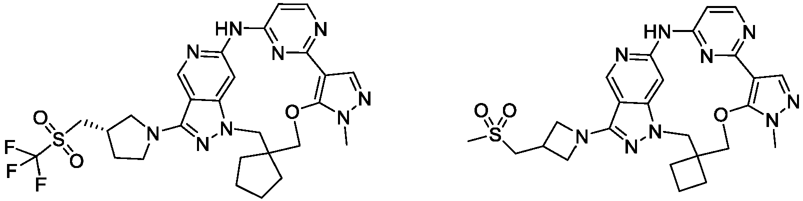 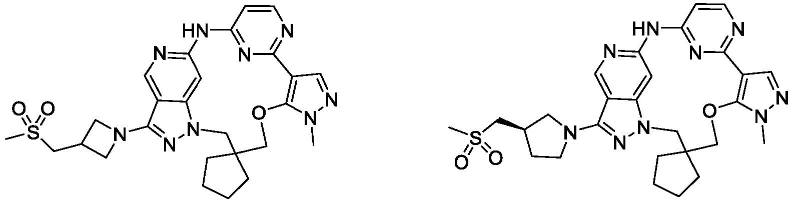 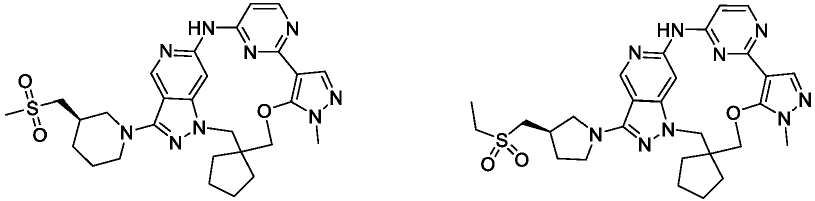 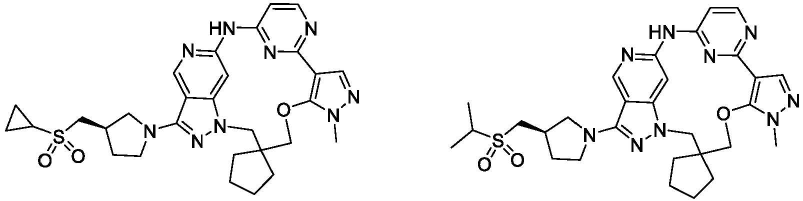 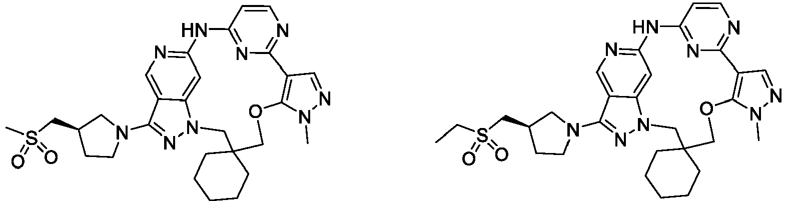 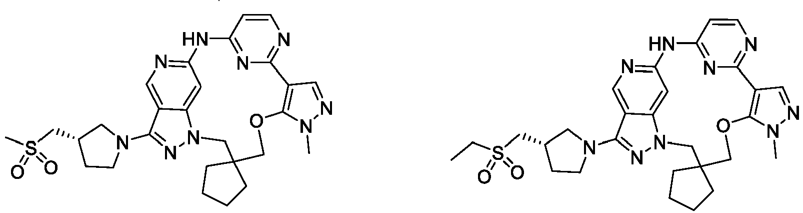 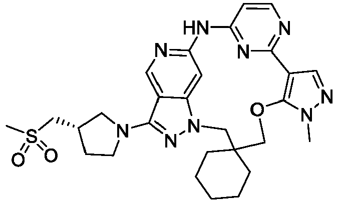 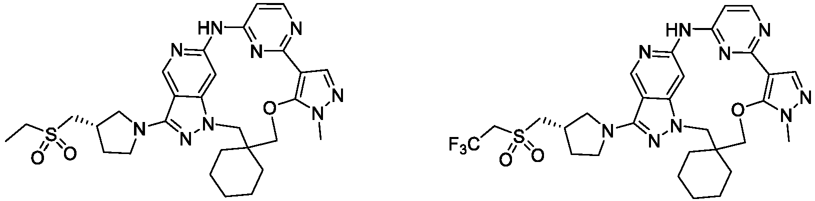 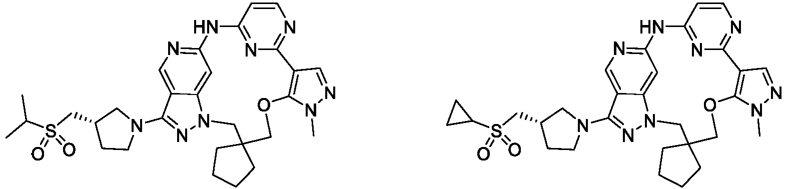 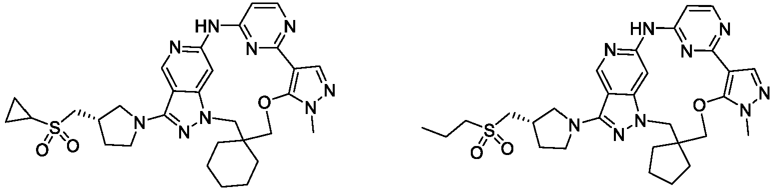 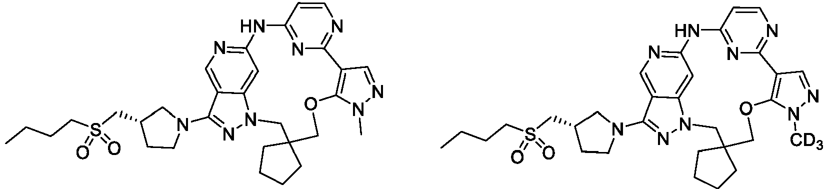 and

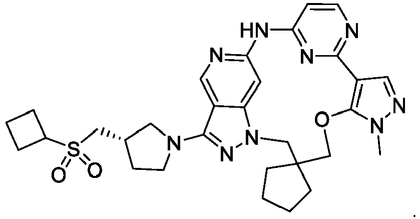

17. A pharmaceutical composition comprising the compound of formula (III), the stereoisomer or pharmaceutically acceptable salt thereof according to any one of claims 1-16, and a pharmaceutically acceptable carrier.

18. A compound of formula (III), the stereoisomer or pharmaceutically acceptable salt thereof according to any one of claims 1-16 for use in treating and/or preventing tumors or metastatic diseases associated at least partially with EGFR L858R/C797S double mutation, EGFR Del19/C797S double mutation, L858R/T790M/C797S triple mutation and Del19/T790M/C797S triple mutation; optionally wherein the tumors or metastatic diseases are tumors and/or metastatic diseases caused by hyperproliferation and dysfunction in cell death induction.

19. The compound for use according to claim 18, wherein the tumors or metastatic diseases are selected from the groups consisting of lung cancer, colon cancer, pancreatic cancer, head and neck cancer, breast cancer, ovarian cancer, uterine cancer, gastric cancer, non-small cell lung cancer, leukemia, myelodysplastic syndrome, malignant lymphoma, head and neck tumor, thoracic tumor, gastrointestinal tumor, endocrine tumor, breast and other gynecological tumor, urological tumor, skin tumor, sarcoma, sinonasal inverted papilloma or sinonasal squamous cell carcinoma associated with sinonasal inverted papilloma.

## Patentansprüche

1. Verbindung der Formel (III), ein Stereoisomer oder pharmazeutisch verträgliches Salz davon: 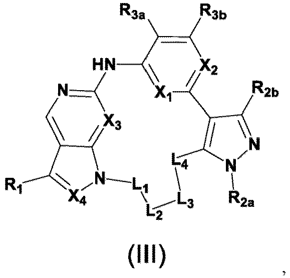 worin
X₁ CRₐ oder N ist;
X₂ CR_{b} oder N ist;
X₃ CR_{c} oder N ist;
X₄ CR_{d} oder N ist;
L₁ CR₅ₐR_{5b} ist;
L₂ (CR_{5c}R_{5d})ₚ ist;
L₃ eine Bindung, CR₅ₑR_{5f} oder C(O) ist;
L₄ CR_{5g}R₅ₕ, O, NR_{6c} oder C(O) ist;
R₁ ausgewählt ist aus der Gruppe bestehend aus Wasserstoff, Deuterium, Halogen, Cyan, C₁-₄-Alkyl, C₂₋₄-Alkenyl, C₂₋₄-Alkenyl, C₃₋₆-Cycloalkyl, 3- bis 8-gliedrigem Heterocyclyl, C₆₋₈-Aryl, 5- bis 8-gliedrigem Heteroaryl, -SF₅, -S(O)(=N-R₇)R₈, -N=S(O)R₈R₉, -N=SR₈R₉, -O-S(O)₂R₁₀, -S(O)rR₁₀, -O-R₁₁, -C(O)OR₁₁, - C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ und -N(R₁₃)-C(O)R₁₂, und die obigen Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₂₋₄-Alkenyl, C₂₋₄-Alkinyl, C₃₋₆-Cycloalkyl substituiert ist, 3- bis 6-gliedrigem Heterocyclyl, C₁₋₄-Alkyl, das durch C₃₋₆-Cycloalkyl, C₁₋₄-Alkyl, das durch 3- bis 6-gliedriges Heterocyclyl substituiert ist, C₆₋₈-Aryl, 5- bis 8-gliedrigem Heteroaryl, =O, =S, -SF₅, -S(O)(=N-R₇)R₈, -N=S(O)R₈R₉, -N=SR₈R₉, -N(R₁₃)-S(O)₂R₁₀, -O-S(O)₂R₁₀, -S(O)R₁₀, -O-R₁₁, -C(O)OR₁₁, -C(O)SR₁₁, -S-C(O)R₁₂, - C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, - C(O)NR₁₃R₁₄ und -N(R₁₃)-C(O)R₁₂, und die obigen Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuteroalkyl C₂₋₄-Alkenyl, C₂₋₄-Alkenyl, C₃₋₆-Cycloalkyl, 3- bis 6-gliedrigem Heterocyclyl, C₆₋₈-Aryl, 5- bis 8-gliedrigem Heteroaryl, =O, =S, -C₀₋₄-Alkyl-SFs, -C₀₋₄-Alkyl-S(O)(=N-R₇)R₈, -C₀₋₄-Alkyl-N=S(O)R₈R₉ -C₀₋₄-Alkyl-N=SR₈R₉, -C₀₋₄-Alkyl-N(R₁₃)-S(O)₂R₁₀, -C₀₋₄-Alkyl-O-S(O)₂R₁₀, -C₀₋₄-AlkylS(O)rR₁₀, -C₀₋₄-Alkyl-O-R₁₁, -C₀₋₄-Alkyl-C(O)OR₁₁, -C₀₋₄-Alkyl-1 C(O)SR₁₁, -C₀₋₄-Alkyl-S-C(O)R₁₂, -C₀₋₄-Alkyl-C(O)R₁₂, -C₀₋₄-Alkyl-O-C(O)R₁₂, -C₀₋₄-Alkyl-P(O)(R₁₂)₂, - C₀₋₄-Alkyl-NR₁₃R₁₄, -C₀₋₄-Alkyl-C(=NR₁₃)R₁₂, -C₀₋₄-Alkyl-N(R₁₃)-C(=NR₁₄)R₁₂, -C₀₋₄-Alkyl-C(O)NR₁₃R₁₄ und -C₀₋₄-Alkyl-N(R₁₃)-C(O)R₁₂;
R₂ₐ ausgewählt ist aus der Gruppe bestehend aus Wasserstoff, Deuterium, Hydroxy, C₁₋₄-Alkyl, C₂₋₄-Alkenyl, C₂₋₄-Alkinyl, C₃₋₆-Cycloalkyl und 3- bis 6-gliedrigem Heterocyclyl, und die obigen Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3-bis 6-gliedrigem Heterocyclyl, C₆₋₈-Aryl, 5- bis 8-gliedrigem Heteroaryl, =O, =S, -SF₅, - S(O)(=N-R₇)R₈, -N=S(O)R₈R₉, -N=SR₈R₉, -O-S(O)₂R₁₀,-S(O)rR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂, -NR₁₃R₁₄, - C(=NR₁₃)R₁₂, -N(R₁₃)-C(=NR₁₄)R₁₂, -C(O)NR₁₃R₁₄ und -N(R₁₃)-C(O)R₁₂;
R_{2b} ausgewählt ist aus der Gruppe bestehend aus Wasserstoff, Deuterium, Halogen, Cyan, C₁₋₄-Alkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl und - SF₅, und die obigen Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl, =O, =S,-SF₅,-S(O)(=N-R₇)R₈,-N=S(O)R₈R₉,-N=SR₈R₉,-O-S(O)₂R₁₀,-S(O)rR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ und -N(R₁₃)-C(O)R₁₂;
R₃ₐ, R_{3b}, Rₐ und R_{b} jeweils unabhängig voneinander ausgewählt sind aus der Gruppe bestehend aus Wasserstoff, Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3-bis 6-gliedrigem Heterocyclyl, C₆₋₈-Aryl, 5- bis 8-gliedrigem Heteroaryl und -SF₅;
R_{c} ausgewählt ist aus der Gruppe bestehend aus Wasserstoff, Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl und -SF₅;
R_{d} ausgewählt ist aus der Gruppe bestehend aus Wasserstoff, Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl und -SF₅;
R₅ₐ und R_{5b} jeweils unabhängig voneinander ausgewählt aus der Gruppe sind, bestehend aus Wasserstoff, Deuterium, Halogen, Cyan, C₁₋₄-Alkyl, C₂₋₄-Alkenyl, C₂₋₄-Alkinyl, C₃₋₆-Cycloalkyl, 3- bis 6-gliedrigem Heterocyclyl, C₆₋₈-Aryl, 5-bis 8-gliedrigem Heteroaryl und -SF₅, oder, R₅ₐ und R_{5b}, zusammen mit dem Kohlenstoffatom, an das sie direkt gebunden sind, ein C(O), C₃₋₆-Cycloalkyl, 4- bis 6-gliedriges Heterocyclyl, C₆₋₈-Aryl oder 5- bis 8-gliedriges Heteroaryl bilden, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl, =O, =S, -SF₅, - S(O)(=N-R₇)R₈, -N=S(O)R₈R₉, -N=SR₈R₉, -O-S(O)₂R₁₀,-S(O)rR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂,-NR₁₃R₁₄, - C(=NR₁₃)R₁₂, -N(R₁₃)-C(=NR₁₄)R₁₂, -C(O)NR₁₃R₁₄ und -N(R₁₃)-C(O)R₁₂;
wenn p 1 ist, R_{5c} und R_{5d} zusammen mit dem Kohlenstoffatom, an das sie direkt gebunden sind, ein C ₃₋₆-Cycloalkyl oder 4- bis 6-gliedriges Heterocyclyl bilden, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3-bis 6-gliedrigem Heterocyclyl, C₆₋₈-Aryl, 5- bis 8-gliedrigem Heteroaryl, =O, =S, - SF₅, -S(O)(=N-R₇)R₈, -N=S(O)R₈R₉-N=SR₈R₉, -O-S(O)₂R₁₀,-S(O)rR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂, -NR₁₃R₁₄, - C(=NR₁₃)R₁₂, -N(R₁₃)-C(=NR₁₄)R₁₂, -C(O)NR₁₃R₁₄ und -N(R₁₃)-C(O)R₁₂, oder
wenn p 2 ist, eine Gruppe aus R_{5c} und R_{5d} zusammen mit dem Kohlenstoffatom, an das sie direkt gebunden sind, ein C₃₋₆-Cycloalkyl oder 4- bis 6-gliedriges Heterocyclyl bildet, und wenn eine Gruppe aus R_{5c} und R_{5d} zusammen mit dem Kohlenstoffatom, an das sie direkt gebunden sind, ein C₃₋₆-Cycloalkyl oder 4- bis 6-gliedriges Heterocyclyl bildet, die andere Gruppe von R_{5c} und R_{5d} jeweils unabhängig voneinander aus der Gruppe ausgewählt ist, bestehend aus Wasserstoff, Deuterium, Halogen, Cyan, C₁₋₄-Alkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl, -SF₅, -S(O)(=N-R₇)R₈, -N=S(O)R₈R₉, -N=SR₈R₉, -O-S(O)₂R₁₀,-S(O)rR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂, -NR₁₃R₁₄, - C(=NR₁₃)R₁₂, -N(R₁₃)-C(=NR14)R₁₂, -C(O)NR₁₃R₁₄und -N(R₁₃)-C(O)R₁₂;
R₅ₑ und R_{5f} jeweils unabhängig voneinander ausgewählt sind, aus der Gruppe bestehend aus Wasserstoff, Deuterium, Halogen, Cyan, C₁₋₄-Alkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl und -SF₅, oder, R₅ₑ und R_{5f}, zusammen mit dem Kohlenstoffatom, an das sie direkt gebunden sind, ein C₃₋₆-Cycloalkyl, 4- bis 6-gliedriges Heterocyclyl, C₆₋₈-Aryl oder 5- bis 8-gliedriges Heteroaryl bilden, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3- bis 6-gliedrigem Heterocyclyl, C₆₋₈Aryl, 5- bis 8-gliedrigem Heteroaryl, =O, =S, -SF₅, -S(O)(=N-R₇)R₈, -N=S(O)R₈R₉, -N=SR₈R₉, -O-S(O)₂R₁₀,-S(O)rR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ und -N(R₁₃)-C(O)R₁₂;
R_{5g} und R₅ₕ jeweils unabhängig voneinander ausgewählt sind aus der Gruppe bestehend aus Wasserstoff, Deuterium, Halogen, Cyan, C₁₋₄-Alkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl und -SF₅, oder, R_{5g} und R₅ₕ, zusammen mit dem Kohlenstoffatom, an das sie direkt gebunden sind, ein C₃₋₆-Cycloalkyl, 4- bis 6-gliedriges Heterocyclyl, C₆₋₈-Aryl oder 5- bis 8-gliedriges Heteroaryl bilden, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl, =O, =S, -SF₅, -S(O)(=N-R₇)R₈, -N=S(O)R₈R₉,-N=SR₈R₉, -O-S(O)₂R₁₀,-S(O)rR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ und -N(R₁₃)-C(O)R₁₂;
R_{6c} ausgewählt ist aus der Gruppe bestehend aus Wasserstoff, Deuterium, C₁₋₄-Alkyl, C₃₋₆-Cycloalkyl, 3- bis 6-gliedrigem Heterocyclyl und -O-R₁₁, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, Hydroxy, =O, C₁₋₄-Alkyl, C₁₋₄-Halogenalkyl, C₁₋₄-Deuteroalkyl, C₂₋₄-Alkenyl, C₂₋₄-Alkinyl, C₁₋₄-Alkoxy, C₃₋₆-Cycloalkyl, C₃₋₆-Cycloalkoxy, 3- bis 6-gliedrigem Heterocyclyl, 3-bis 6-gliedrigem Heterocyclyloxy und -NR₁₃R₁₄;
jedes R₇ unabhängig voneinander ausgewählt ist aus der Gruppe bestehend aus Wasserstoff, Deuterium, C₁₋₁₀-Alkyl, C₂₋₁₀-Alkenyl, C₂₋₁₀-Alkinyl, C₃₋₁₀-Cycloalkyl, 3- bis 10-gliedrigem Heterocyclyl, C₆₋₁₀-Aryl, 5- bis 10-gliedrigem Heteroaryl, -C₀₋₈-Alkyl-S(O)ᵣR₁₀, -C₀₋₈-Alkyl-C(O)OR₁₁, -C₀₋₈-Alkyl-C(O)R₁₂ oder -C₀₋₈-Alkyl-C(O)NR₁₃R₁₄, und die oben genannten Gruppen jeweils unabhängig voneinander gegebenenfalls weiter durch einen oder mehrere Substituenten substituiert sind, ausgewählt aus der Gruppe bestehend aus Deuterium, Halogen, Cyan, Nitro, Azido, C₁₋₁₀-Alkyl, C₂₋₁₀-Alkenyl, C₂₋₁₀-Alkinyl, C₁₋₁₀-Halogenalkyl, C₁₋₁₀-Deuterioalkyl, C₃₋₁₀-Cycloalkyl, 3- bis 10-gliedrigem Heterocyclyl, C₆₋₁₀-Aryl, 5- bis 10-gliedrigem Heteroaryl, -C₀₋₈-Alkyl-S(O)ᵣR₁₀, -C₀₋₈-Alkyl-O-R₁₁, -C₀₋₈-Alkyl-C(O)OR₁₁, -C₀₋₈-Alkyl-C(O)R₁₂, -C₀₋₈-Alkyl-O-C(O)R₁₂, -C₀₋₈-Alkyl-NR₁₃R₁₄, -C₀₋₈-Alkyl-C(=NR₁₃)R₁₂, -C₀₋₈-Alkyl-N(R₁₃)-C(=NR₁₄)R₁₂, -C₀₋₈-Alkyl-C(O)NR₁₃R₁₄ oder -C₀₋₈-Alkyl-N(R₁₃)-C(O)R₁₂;
jedes R₈ und jedes R₉ unabhängig voneinander ausgewählt sind aus der Gruppe bestehend aus Wasserstoff, Deuterium, Hydroxy, C₁₋₁₀-Alkyl, C₂₋₁₀-Alkenyl, C₂₋₁₀-Alkinyl, C₃₋₁₀-Cycloalkyl, 3-bis 10-gliedrigem Heterocyclyl, C₆₋₁₀-Aryl und 5- bis 10-gliedrigem Heteroaryl, oder, R₈ und R₉ zusammen mit dem Schwefelatom, an das sie direkt gebunden sind, ein 3- bis 10-gliedriges Heterocyclyl bilden, und die oben genannten Gruppen unabhängig voneinander gegebenenfalls weiter substituiert sind durch einen oder mehrere Substituenten, ausgewählt aus der Gruppe bestehend aus Deuterium, Halogen, Cyan, Nitro, Azido, C₁₋₁₀-Alkyl, C₂₋₁₀-Alkenyl, C₂₋₁₀-Alkinyl, C₁₋₁₀-Halogenalkyl, C₁₋₁₀-Deuterioalkyl, C₃₋₁₀ -Cycloalkyl, 3- bis 10-gliedrigem Heterocyclyl, C₆₋₁₀-Aryl, 5- bis 10-gliedrigem Heteroaryl, -C₀₋₈-AlkylS(O)ᵣR₁₀, -C₀₋₈-Alkyl-O-R₁₁, -C₀₋₈ -Alkyl-C(O)OR₁₁, -C₀₋₈ -Alkyl-C(O)R₁₂, -C₀₋₈ -Alkyl-O-C(O)R₁₂, -C₀₋₈-Alkyl-NR₁₃R₁₄, -C₀₋₈-Alkyl-C(=NR₁₃)R₁₂, -C₀₋₈-Alkyl-N(R₁₃)-C(=NR₁₄)R₁₂, -C₀₋₈-Alkyl-C(O)NR₁₃R₁₄ und -C₀₋₈-Alkyl-N(R₁₃)-C(O)R₁₂;
jedes R₁₀ unabhängig ausgewählt ist aus der Gruppe bestehend aus Wasserstoff, Deuterium, Hydroxy, C₁₋₁₀-Alkyl, C₂₋₁₀-Alkenyl, C₃₋₁₂-Cycloalkyl, 3-bis 12-gliedrigem Heterocyclyl, C₆₋₁₀-Aryl, 5- bis 10-gliedrigem Heteroaryl und - NR₁₃R₁₄, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Hydroxy, =O, C₁₋₁₀-Alkyl, C₁₋₁₀-Alkoxy, C₃₋₁₂-Cycloalkyl, C₃₋₁₂-Cycloalkoxy, 3- bis 12-gliedrigem Heterocyclyl, 3- bis 12-gliedrigem Heterocyclyloxy, C₆₋₁₀-Aryl, C₆₋₁₀-Aryloxy, 5-bis 10-gliedrigem Heteroaryl, 5- bis 10-gliedrigem Heteroaryloxy und -NR₁₃R₁₄;
jedes R₁₁ unabhängig ausgewählt ist aus der Gruppe bestehend aus Wasserstoff, Deuterium, C₁₋₁₀-Alkyl, C₂₋₁₀-Alkenyl, C₃₋₁₂-Cycloalkyl, 3- bis 12-gliedrigem Heterocyclyl, C₆₋₁₀-Aryl und 5- bis 10-gliedrigem Heteroaryl, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Hydroxy, =O, Cyan, C₁₋₁₀-Alkyl, C₁₋₁₀-Alkoxy, C₃₋₁₂-Cycloalkyl, C₃₋₁₂-Cycloalkoxy, 3- bis 12-gliedrigem Heterocyclyl, 3- bis 12-gliedrigem Heterocyclyloxy, C₆₋₁₀-Aryl, C₆₋₁₀-Aryloxy, 5- bis 10-gliedrigem Heteroaryl, 5- bis 10-gliedrigem Heteroaryloxy und -NR₁₃R₁₄;
jedes R₁₂ unabhängig voneinander ausgewählt ist aus der Gruppe bestehend aus Wasserstoff, Deuterium, Hydroxy, C₁₋₁₀-Alkyl, C₁₋₁₀-Alkoxy, C₂₋₁₀-Alkenyl, C₂₋₁₀-Alkinyl, C₃₋₁₂-Cycloalkyl, C₃₋₁₂-Cycloalkoxy, 3- bis 12-gliedrigem Heterocyclyl, 3- bis 12-gliedrigem Heterocyclyloxy, C₆₋₁₀-Aryl, C₆₋₁₀-Aryloxy, 5- bis 10-gliedrigem Heteroaryl, 5- bis 10-gliedrigem Heteroaryloxy und -NR₁₃R₁₄, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Hydroxy, =O, Cyan, C₁₋₁₀-Alkyl, C₁₋₁₀-Alkoxy, C₃₋₁₂-Cycloalkyl, C₃₋₁₂-Cycloalkoxy, 3- bis 12-gliedrigem Heterocyclyl, 3- bis 12-gliedrigem Heterocyclyloxy, C₆₋₁₀-Aryl, C₆₋₁₀-Aryloxy, 5- bis 10-gliedrigem Heteroaryl, 5- bis 10-gliedrigem Heteroaryloxy und -NR₁₃R₁₄;
jedes R₁₃ und jedes R₁₄ jeweils unabhängig voneinander ausgewählt sind aus der Gruppe bestehend aus Wasserstoff, Deuterium, Hydroxy, C₁₋₁₀-Alkyl, C₂₋₁₀-Alkenyl, C₂₋₁₀-Alkinyl, C₃₋₁₂-Cycloalkyl, 3- bis 12-gliedrigem Heterocyclyl, C₆₋₁₀-Aryl, 5- bis 10-gliedrigem Heteroaryl, Sulfinyl, Sulfonyl, Methansulfonyl, Isopropylsulfonyl, Cyclopropylsulfonyl, p-Toluolsulfonyl, Aminosulfonyl, Dimethylaminosulfonyl und C₁₋₁₀-Alkanoyl, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Hydroxy, =O, C₁₋₁₀-Alkyl, C₂₋₁₀-Alkenyl, C₂₋₁₀-Alkinyl, C₁₋₁₀-Halogenalkyl, C₁₋₁₀-Deuterioalkyl, C₁₋₁₀-Alkoxy, C₃₋₁₂-Cycloalkyl, C₃₋₁₂-Cycloalkoxy, 3- bis 12-gliedrigem Heterocyclyl, 3- bis 12-gliedrigem Heterocyclyloxy, C₆₋₁₀-Aryl, C₆₋₁₀-Aryloxy, 5- bis 10-gliedrigem Heteroaryl, 5- bis 10-gliedrigem Heteroaryloxy, Amino, MonoC₁₋₁₀-Alkylamino, diC₁₋₁₀-Alkylamino und C₁₋₁₀-Alkanoyl; oder
R₁₃ und R₁₄ zusammen mit dem Stickstoffatom, an das sie direkt gebunden sind, ein 4- bis 10-gliedriges Heterocyclyl oder 5- bis 10-gliedriges Heteroaryl bilden und das 4- bis 10-gliedrige Heterocyclyl oder 5- bis 10-gliedrige Heteroaryl gegebenenfalls weiter substituiert ist durch einen oder mehrere Substituenten, ausgewählt aus der Gruppe bestehend aus Deuterium, Halogen, Hydroxy, =O, C₁₋₁₀-Alkyl, C₂₋₁₀-Alkenyl, C₂₋₁₀-Alkinyl, C₁₋₁₀-Halogenalkyl, C₁₋₁₀-Deuterioalkyl, C₁₋₁₀-Alkoxy, C₃₋₁₂-Cycloalkyl, C₃₋₁₂-Cycloalkoxy, 3- bis 12-gliedrigem Heterocyclyl, 3- bis 12-gliedrigem Heterocyclyloxy, C₆₋₁₀-Aryl, C₆₋₁₀-Aryloxy, 5- bis 10-gliedrigem Heteroaryl, 5- bis 10-gliedrigem Heteroaryloxy, Methansulfonylmethyl, Amino, MonoC₁₋₁₀-Alkylamino, diC₁₋₁₀-Alkylamino und C₁₋₁₀-Alkanoyl;
p 1 oder 2 ist, und
jedes r unabhängig 0, 1 oder 2 ist.

2. Verbindung der Formel (III), das Stereoisomer oder pharmazeutisch verträgliche Salz davon nach Anspruch 1, wobei die Verbindung der Formel (III) eine Verbindung der nachstehend gezeigten Formel (IV) ist: 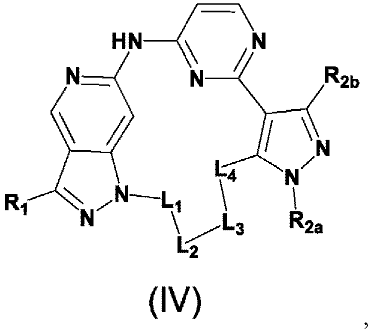 worin
L₁ CR₅ₐR_{5b} ist;
L₂ CR_{5c}R_{5d} ist;
L₃ CR₅ₑR_{5f} ist;
L₄ O ist;
R₁ ausgewählt ist aus der Gruppe bestehend aus Wasserstoff, Deuterium, Halogen, Cyan, C₁₋₄-Alkyl, C₂₋₄-Alkenyl, C₂₋₄-Alkinyl, C₃₋₆-Cycloalkyl, 3- bis 8-gliedrigem Heterocyclyl, C₆₋₈-Aryl, 5- bis 8-gliedrigem Heteroaryl und - SF₅, und die oben genannten Gruppen jeweils unabhängig voneinander gegebenenfalls weiter substituiert sind durch einen oder mehrere Substituenten ausgewählt aus der Gruppe bestehend aus Deuterium, Halogen, Cyan, C₁₋₄-Alkyl, C₂₋₄-Alkenyl, C₂₋₄-Alkinyl, C₃₋₆-Cycloalkyl, 3- bis 6-gliedrigem Heterocyclyl, C₁₋₄-Alkyl, das durch C₃₋₆-Cycloalkyl substituiert ist, C₁₋₄-Alkyl, das durch 3- bis 6-gliedriges Heterocyclyl substituiert ist, C₆₋₈-Aryl, 5- bis 8-gliedrigem Heteroaryl, =O, =S, -SF₅, -S(O)(=N-R₇)R₈, -N=S(O)R₈R₉-N=SR₈R₉-N(R₁₃)-S(O)₂R₁₀, -O-S(O)₂R₁₀, -S(O)rR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, - C(O)NR₁₃R₁₄und -N(R₁₃)-C(O)R₁₂, und die oben genannten Gruppen jeweils unabhängig voneinander gegebenenfalls weiter durch einen oder mehrere Substituenten substituiert sind, ausgewählt aus der Gruppe bestehend aus Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl, =O, =S, -SF₅, -S(O)(=N-R₇)R₈, -N=S(O)R₈R₉, - N=SR₈R₉, -N(R₁₃)-S(O)₂R₁₀, -O-S(O)₂R₁₀,-S(O)ᵣR₁₀, -O-R₁₁, -C(O)OR₁₁, - C(O)SR₁₁, -S-C(O)R₁₂, -C(O)R₁₂, -O-C(O)R₁₂,-P(O)(R₁₂)₂, -NR₁₃R₁₄, - C(=NR₁₃)R₁₂, -N(R₁₃)-C(=NR₁₄)R₁₂, -C(O)NR₁₃R₁₄ und -N(R₁₃)-C(O)R₁₂;
R₂ₐ ausgewählt ist aus der Gruppe bestehend aus Wasserstoff, Deuterium, Hydroxy, C₁₋₄-Alkyl, C₂₋₄-Alkenyl, C₂₋₄-Alkinyl, C₃₋₆-Cycloalkyl und 3- bis 6-gliedrigem Heterocyclyl, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3- bis 6-gliedrigem Heterocyclyl, =O, =S und -SF₅;
R_{2b} ausgewählt ist aus der Gruppe bestehend aus Wasserstoff, Deuterium, Halogen, Cyan, C₁₋₄-Alkyl, C₂₋₄-Alkenyl, C₂₋₄-Alkinyl, C₃₋₆-Cycloalkyl, 3- bis 6-gliedrigem Heterocyclyl und -SF₅, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3- bis 6-gliedrigem Heterocyclyl, =O, =S und -SF₅;
R₅ₐ und R_{5b} jeweils unabhängig voneinander ausgewählt sind aus der Gruppe bestehend aus Wasserstoff, Deuterium, Halogen, Cyan, C₁₋₄-Alkyl, C₃₋₆-Cycloalkyl und 3- bis 6-gliedrigem Heterocyclyl, oder R₅ₐ und R_{5b} zusammen mit dem Kohlenstoffatom, an das sie direkt gebunden sind, ein C₃₋₆-Cycloalkyl oder 4-bis 6-gliedriges Heterocyclyl, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuteroalkyl, C₂₋₄-Alkenyl, C₂₋₄-Alkinyl, C₃₋₆-Cycloalkyl, 3- bis 6-gliedrigem Heterocyclyl, =O, =S und -SF₅; und
R_{5c} und R_{5d} zusammen mit dem Kohlenstoffatom, an das sie direkt gebunden sind, ein C₃₋₆-Cycloalkyl oder 4- bis 6-gliedriges Heterocyclyl bilden, und die oben genannten Gruppen jeweils unabhängig voneinander gegebenenfalls zusätzlich durch einen oder mehrere Substituenten substituiert sind, ausgewählt aus der Gruppe bestehend aus Deuterium, Halogen, Cyan, C₁₋₄-Alkyl, C₁₋₄-Halogenalkyl, C₁₋₄-Deuteroalkyl, C₂₋₄-Alkenyl, C₂₋₄-Alkinyl, C₃₋₆-Cycloalkyl, 3- bis 6-gliedrigem Heterocyclyl, = O, =S und -SF₅;
R₅ₑ und R_{5f} jeweils unabhängig voneinander aus der Gruppe ausgewählt sind, bestehend aus Wasserstoff, Deuterium, Halogen, Cyan, C₁₋₄-Alkyl, C₃₋₆-Cycloalkyl und 3- bis 6-gliedrigem Heterocyclyl, oder R₅ₑ und R_{5f} zusammen mit dem Kohlenstoffatom, an das sie direkt gebunden sind, ein C₃₋₆-Cycloalkyl oder 4-bis 6-gliedriges Heterocyclyl bilden, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuteroalkyl, C₂₋₄-Alkenyl, C₂₋₄-Alkinyl, C₃₋₆-Cycloalkyl, 3- bis 6-gliedrigem Heterocyclyl, =O, =S und -SF₅; und
R₇, R₈, R₉, R₁₀, R₁₁, R₁₂, R₁₃, R₁₄ und r wie in Anspruch 1 definiert sind.

3. Verbindung der Formel (III), das Stereoisomer oder pharmazeutisch verträgliche Salz davon nach Anspruch 1, wobei:
L₁ CR₅ₐR_{5b} ist;
L₂ CR_{5c}R_{5d} ist;
L₃ CR₅ₑR_{5f} ist;
L₄ O ist;
R₅ₐ und R_{5b} jeweils unabhängig voneinander ausgewählt sind aus der Gruppe bestehend aus Wasserstoff, Deuterium, Fluor, Chlor, Brom, Cyan, Methyl, Ethyl, Propyl, Isopropyl, Cyclopropyl, Cyclobutyl, Oxacyclopropyl, Oxacyclobutyl, Azacyclopropyl und Azacyclobutyl, und die oben genannten Gruppen jeweils unabhängig voneinander gegebenenfalls weiter substituiert sind durch einen oder mehrere Substituenten, ausgewählt aus der Gruppe bestehend aus Deuterium, Fluor, Chlor, Brom, Cyan, Methyl, Ethyl, Propyl, Isopropyl, Monofluormethyl, Difluormethyl, Trifluormethyl, Monodeuteriomethyl, Dideuteriomethyl, Trideuteriomethyl, Vinyl, Ethinyl, Cyclopropyl, Cyclobutyl, Oxacyclopropyl, Oxacyclobutyl, Azacyclopropyl, Azacyclobutyl, =O, =S und -SF₅;
R_{5c} und R_{5d} zusammen mit dem Kohlenstoffatom, an das sie direkt gebunden sind, ein C₃₋₆-Cycloalkyl oder 4- bis 6-gliedriges Heterocyclyl, und die oben genannten Gruppen jeweils unabhängig voneinander gegebenenfalls weiter durch einen oder mehrere Substituenten substituiert sind, ausgewählt aus der Gruppe bestehend aus Deuterium, Fluor, Chlor, Brom, Cyan, Methyl, Ethyl, Propyl, Isopropyl, Monofluormethyl, Difluormethyl, Trifluormethyl, Monodeuteriomethyl, Dideuteriomethyl, Trideuteriomethyl, Vinyl, Ethinyl, Cyclopropyl, Cyclobutyl, Oxacyclopropyl, Oxacyclobutyl, Azacyclopropyl, Azacyclobutyl, =O, =S und -SF₅; und
R₅ₑ und R_{5f} jeweils unabhängig voneinander ausgewählt sind aus der Gruppe bestehend aus Wasserstoff, Deuterium, Fluor, Chlor, Brom, Cyan, Methyl, Ethyl, Propyl, Isopropyl, Cyclopropyl, Cyclobutyl, Oxacyclopropyl, Oxacyclobutyl, Azacyclopropyl und Azacyclobutyl, und die oben genannten Gruppen jeweils unabhängig voneinander gegebenenfalls weiter substituiert sind durch einen oder mehrere Substituenten, ausgewählt aus der Gruppe bestehend aus Deuterium, Fluor, Chlor, Brom, Cyan, Methyl, Ethyl, Propyl, Isopropyl, Monofluormethyl, Difluormethyl, Trifluormethyl, Monodeuteriomethyl, Dideuteriomethyl, Trideuteriomethyl, Vinyl, Ethinyl, Cyclopropyl, Cyclobutyl, Oxacyclopropyl, Oxacyclobutyl, Azacyclopropyl, Azacyclobutyl, =O, =S und -SF₅;
R₂ₐ ausgewählt ist aus der Gruppe bestehend aus Wasserstoff, Deuterium, Hydroxy, Methyl, Ethyl, Propyl, Isopropyl, Vinyl, Ethinyl, Cyclopropyl, Cyclobutyl, Oxacyclopropyl, Oxacyclobutyl, Azacyclopropyl und Azacyclobutyl, und die oben genannten Gruppen jeweils unabhängig voneinander gegebenenfalls weiter substituiert sind durch einen oder mehrere Substituenten, ausgewählt aus der Gruppe bestehend aus Deuterium, Fluor, Chlor, Brom, Cyan, Methyl, Ethyl, Propyl, Isopropyl, Monofluormethyl, Difluormethyl, Trifluormethyl, Monodeuteriomethyl, Dideuteriomethyl, Trideuteriomethyl, Vinyl, Ethinyl, Cyclopropyl, Cyclobutyl, Oxacyclopropyl, Oxacyclobutyl, Azacyclopropyl, Azacyclobutyl, =O, =S und -SF₅; und
R_{2b} ausgewählt ist aus der Gruppe bestehend aus Wasserstoff, Deuterium, Fluor, Chlor, Brom, Cyan, Methyl, Ethyl, Propyl, Isopropyl, Vinyl, Ethinyl, Cyclopropyl, Cyclobutyl, Oxacyclopropyl, Oxacyclobutyl, Azacyclopropyl, Azacyclobutyl und -SF₅, und die oben genannten Gruppen jeweils unabhängig voneinander gegebenenfalls weiter substituiert sind durch einen oder mehrere Substituenten, ausgewählt aus der Gruppe bestehend aus Deuterium, Fluor, Chlor, Brom, Cyan, Methyl, Ethyl, Propyl, Isopropyl, Monofluormethyl, Difluormethyl, Trifluormethyl, Monodeuteriomethyl, Dideuteriomethyl, Trideuteriomethyl, Vinyl, Ethinyl, Cyclopropyl, Cyclobutyl, Oxacyclopropyl, Oxacyclobutyl, Azacyclopropyl, Azacyclobutyl, =O, =S und -SF₅.

4. Verbindung der Formel (III), das Stereoisomer oder pharmazeutisch verträgliche Salz davon nach Anspruch 1, wobei die Verbindung der Formel (III) eine Verbindung der nachstehend gezeigten Formel (V) ist: 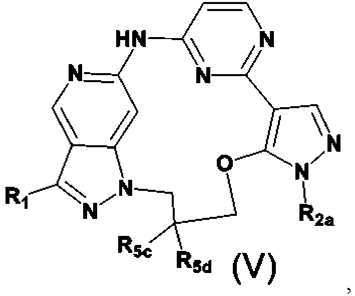 worin
R₁ ausgewählt ist aus C₂₋₄-Alkenyl, C₂₋₄-Alkinyl und 3- bis 8-gliedrigem Heterocyclyl, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₂₋₄-Alkenyl, C₂₋₄-Alkinyl, C₃₋₆-Cycloalkyl, 3- bis 6-gliedrigem Heterocyclyl, C₁₋₄-Alkyl , das durch C₃₋₆-Cycloalkyl substituiert ist, C₁₋₄-Alkyl, das durch 3- bis 6-gliedriges Heterocyclyl substituiert ist, =O, =S, -SF₅, -N(R₁₃)-S(O)₂R₁₀, -O-S(O)₂R₁₀,-S(O)ᵣR₁₀, - O-R₁₁, -C(O)OR₁₁, -S-C(O)R₁₂, -C(O)R₁₂, -O-C(O)R₁₂, -NR₁₃R₁₄, -C(O)NR₁₃R₁₄ und - N(R₁₃)-C(O)R₁₂, und die oben genannten Gruppen jeweils unabhängig voneinander gegebenenfalls noch weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuterioalkyl, C₂₋₄-Alkenyl, C₂₋₄-Alkinyl, C₃₋₆-Cycloalkyl, 3- bis 6-gliedrigem Heterocyclyl, C₆₋₈-Aryl, 5- bis 8-gliedrigem Heteroaryl, =O, =S, -SF₅, - N(R₁₃)-S(O)₂R₁₀, -O-S(O)₂R₁₀,-S(O)R₁₀, -O-R₁₁, -C(O)OR₁₁, -C(O)R₁₂, -O-C(O)R₁₂,-NR₁₃R₁₄, -C(O)NR₁₃R₁₄ und -N(R₁₃)-C(O)R₁₂;
R₂ₐ ausgewählt ist aus der Gruppe bestehend aus Wasserstoff, Deuterium, C₁₋₄-Alkyl, C₃₋₆-Cycloalkyl und 3- bis 6-gliedrigem Heterocyclyl, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuteroalkyl, C₃₋₆-Cycloalkyl und 3- bis 6-gliedrigem Heterocyclyl;
R_{5c} und R_{5d} zusammen mit dem Kohlenstoffatom, an das sie direkt gebunden sind, ein C₃₋₆-Cycloalkyl oder 4- bis 6-gliedriges Heterocyclyl bilden, und die oben genannten Gruppen jeweils unabhängig voneinander gegebenenfalls weiter durch einen oder mehrere Substituenten substituiert sind, ausgewählt aus der Gruppe bestehend aus Deuterium, Halogen, Cyan, C₁₋₄-Alkyl, C₁₋₄-Halogenalkyl, C₁₋₄-Deuteroalkyl, C₂₋₄-Alkenyl, C₂₋₄-Alkinyl, C₃₋₆-Cycloalkyl, 3- bis 6-gliedrigem Heterocyclyl, = O, =S und -SF₅;
wobei R₁₀, R₁₁, R₁₂, R₁₃, R₁₄ und r wie in Anspruch 1 definiert sind.

5. Verbindung der Formel (III), das Stereoisomer oder pharmazeutisch verträgliche Salz davon nach Anspruch 4, wobei R_{5c} und R_{5d} zusammen mit dem Kohlenstoffatom, an das sie direkt gebunden sind, ein C₃₋₆-Cycloalkyl oder 4- bis 6-gliedriges Heterocyclyl bilden; gegebenenfalls, wobei R_{5c} und R_{5d} zusammen mit dem Kohlenstoffatom, an das sie direkt gebunden sind, ein Cyclopropyl, Cyclobutyl, Cyclopentyl, Cyclohexyl, Oxacyclobutyl, Oxacyclopentyl oder Oxacyclohexyl bilden.

6. Verbindung der Formel (III), das Stereoisomer oder pharmazeutisch verträgliche Salz davon nach Anspruch 4, wobei:
R₁ C₂₋₄-Alkinyl ist und das C₂₋₄-Alkinyl gegebenenfalls weiter durch einen oder mehrere Substituenten substituiert ist, ausgewählt aus der Gruppe bestehend aus Deuterium, C₁₋₄-Alkyl, C₃₋₆-Cycloalkyl und 3- bis 6-gliedrigem Heterocyclyl und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Cyan, C₁₋₄-Alkyl, C₁₋₄-Halogenalkyl, C₁₋₄-Deuterioalkyl, C₃₋₆-Cycloalkyl und 3- bis 6-gliedrigem Heterocyclyl.

7. Verbindung der Formel (III), das Stereoisomer oder pharmazeutisch verträgliche Salz davon nach Anspruch 6, wobei:
R₁ Ethinyl ist, und das Ethinyl gegebenenfalls weiter substituiert ist durch einen oder mehrere Substituenten, ausgewählt aus der Gruppe bestehend aus Deuterium, Methyl, Ethyl, Propyl, Isopropyl, Butyl, Cyclopropyl, Cyclobutyl, Cyclopentyl, Cyclohexyl, Oxacyclopropyl, Oxacyclobutyl, Oxacyclopentyl, Oxacyclohexyl, Azacyclopropyl, Azacyclobutyl, Azacyclopentyl und Azacyclohexyl, und die oben genannten Gruppen jeweils unabhängig voneinander gegebenenfalls weiter substituiert sind durch einen oder mehrere Substituenten, ausgewählt aus der Gruppe bestehend aus Deuterium, Fluor, Brom, Cyan, Methyl, Ethyl, Propyl, Isopropyl, Butyl, Monofluormethyl, Difluormethyl, Trifluormethyl, Monodeuteriomethyl, Dideuteriomethyl, Trideuteriomethyl, Cyclopropyl, Cyclobutyl, Cyclopentyl, Cyclohexyl, Oxacyclopropyl, Oxacyclobutyl, Oxacyclopentyl, Oxacyclohexyl, Azacyclopropyl, Azacyclobutyl, Azacyclopentyl und Azacyclohexyl.

8. Verbindung der Formel (III), das Stereoisomer oder pharmazeutisch verträgliche Salz davon nach Anspruch 6, wobei:
R₁ Ethinyl ist, und das Ethinyl gegebenenfalls weiter substituiert ist durch einen oder mehrere Substituenten, ausgewählt aus der Gruppe bestehend aus Cyclopropyl, Cyclobutyl, Cyclopentyl, Cyclohexyl, Oxacyclopropyl, Oxacyclobutyl, Oxacyclopentyl, Oxacyclohexyl, Azacyclopropyl, Azacyclobutyl, Azacyclopentyl und Azacyclohexyl, und die oben genannten Gruppen jeweils unabhängig voneinander gegebenenfalls weiter substituiert sind durch einen oder mehrere Substituenten, ausgewählt aus der Gruppe bestehend aus Deuterium, Fluor, Brom, Cyan, Methyl, Ethyl, Propyl, Isopropyl, Monofluormethyl, Difluormethyl, Trifluormethyl, Monodeuteriomethyl, Dideuteriomethyl und Trideuteriomethyl.

9. Verbindung der Formel (III), das Stereoisomer oder pharmazeutisch verträgliche Salz davon nach Anspruch 6, wobei:
R₁ 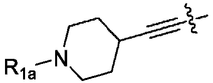 oder 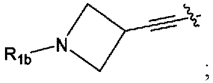 ist
R₁ₐ ausgewählt ist aus der Gruppe bestehend aus Wasserstoff, Methyl, Ethyl, Propyl, Isopropyl, Monofluormethyl, Difluormethyl, Trifluormethyl, Monodeuteriomethyl, Dideuteriomethyl und Trideuteriomethyl; und
R_{1b} augewählt ist aus der Gruppe bestehend aus Wasserstoff, Methyl, Ethyl, Propyl, Isopropyl, Monofluormethyl, Difluormethyl, Trifluormethyl, Monodeuteriomethyl, Dideuteriomethyl und Trideuteriomethyl.

10. Verbindung der Formel (III), das Stereoisomer oder pharmazeutisch verträgliche Salz davon nach Anspruch 4, wobei R₁ ein 4-bis 8-gliedriges stickstoffhaltiges Heterocyclyl ist und das 4- bis 8-gliedrige stickstoffhaltige Heterocyclyl gegebenenfalls weiter durch einen oder mehrere Substituenten substituiert ist, ausgewählt aus der Gruppe bestehend aus Deuterium, Halogen, Cyan, C₁₋₄-Alkyl, =O, -N(R₁₃)-S(O)₂R₁₀, -S(O)ᵣR₁₀, -O-R₁₁, -O-C(O)R₁₂, -NR₁₃R₁₄ und - N(R₁₃)-C(O)R₁₂, und das C₁₋₄-Alkyl gegebenenfalls weiter durch einen oder mehrere Substituenten substituiert ist, ausgewählt aus der Gruppe bestehend aus Deuterium, Halogen, Cyan, C₁₋₄-Alkyl, C₁₋₄-Halogenalkyl, C₁₋₄-Deuterioalkyl, -N(R₁₃)-S(O)₂R₁₀, -O-S(O)₂R₁₀, -S(O)ᵣR₁₀, -O-R₁₁, -O-C(O)R₁₂, -NR₁₃R₁₄ und -N(R₁₃)-C(O)R₁₂; und
R₁₀, R₁₁, R₁₂, R₁₃, R₁₄ sind wie in Anspruch 4 definiert.

11. Verbindung der Formel (III), das Stereoisomer oder pharmazeutisch verträgliche Salz davon nach Anspruch 10, wobei:
R₁ die folgende Struktur aufweist: 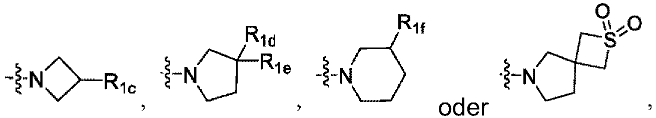
worin
R_{1c} ausgewählt ist aus der Gruppe bestehend aus Wasserstoff, Deuterium, Halogen, C₁₋₄-Alkyl, -N(R₁₃ₐ)-S(O)₂R₁₀ₐ, -O-R₁₁ₐ und -N(R₁₃ₐ)-C(O)R₁₂ₐ, und das C₁₋₄-Alkyl gegebenenfalls weiter durch einen oder mehrere Substituenten substituiert ist, ausgewählt aus der Gruppe bestehend aus Deuterium, C₁₋₄-Alkyl, C₁₋₄-Halogenalkyl, C₁₋₄-Deuterioalkyl, -S(O)ᵣR_{10b} und -O-R₁₁ₐ;
R_{1d} ausgewählt ist aus der Gruppe bestehend aus Wasserstoff, Deuterium, Halogen, C₁₋₄-Alkyl, -N(R₁₃ₐ)-S(O)₂R₁₀ₐ, -O-R₁₁ₐ und -N(R₁₃ₐ)-C(O)R₁₂ₐ;
R₁ₑ ausgewählt ist aus der Gruppe bestehend aus Wasserstoff, Deuterium, Halogen, C₁₋₄-Alkyl, -N(R₁₃ₐ)-S(O)₂R₁₀ₐ, -O-R₁₁ₐ und -N(R₁₃ₐ)-C(O)R₁₂ₐ, und das C₁₋₄-Alkyl gegebenenfalls weiter durch einen oder mehrere Substituenten substituiert ist, ausgewählt aus der Gruppe bestehend aus Deuterium, C₁₋₄-Alkyl, C₁₋₄-Halogenalkyl, C₁₋₄-Deuterioalkyl, -S(O)ᵣR_{10b} und -O-R₁₁ₐ;
R_{1f} aus der Gruppe ausgewählt ist, bestehend aus Wasserstoff, Deuterium, Halogen, C₁₋₄-Alkyl, -N(R₁₃ₐ)-S(O)₂R₁₀ₐ, -O-R₁₁ₐ und -N(R₁₃ₐ)-C(O)R₁₂ₐ, und das C₁₋₄-Alkyl gegebenenfalls weiter durch einen oder mehrere Substituenten substituiert ist, ausgewählt aus der Gruppe bestehend aus Deuterium, C₁₋₄-Alkyl, C₁₋₄-Halogenalkyl, C₁₋₄-Deuterioalkyl, -S(O)ᵣR_{10b} und -O-R₁₁ₐ;
jedes R₁₀ₐ unabhängig ausgewählt ist aus der Gruppe bestehend aus Wasserstoff, C₁₋₄-Alkyl, C₃₋₆-Cycloalkyl und 3- bis 6-gliedrigem Heterocyclyl;
jedes R_{10b} unabhängig ausgewählt ist aus der Gruppe bestehend aus Wasserstoff, C₁₋₄-Alkyl, C₃₋₆-Cycloalkyl und 3- bis 6-gliedrigem Heterocyclyl, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Hydroxy, C₁₋₄-Alkyl und C₁₋₄-Alkoxy;
jedes R₁₁ₐ unabhängig Wasserstoff oder C₁₋₄-Alkyl ist;
jedes R₁₂ₐ unabhängig ausgewählt ist aus der Gruppe bestehend aus Wasserstoff, C₁₋₄-Alkyl, C₁₋₄- Alkoxy und C₃₋₆-Cycloalkyl, und die oben genannten Gruppen jeweils unabhängig voneinander gegebenenfalls weiter substituiert sind durch einen oder mehrere Substituenten, ausgewählt aus der Gruppe bestehend aus Deuterium, Halogen, Hydroxy und Cyan;
jedes R₁₃ₐ unabhängig Wasserstoff oder C₁₋₄-Alkyl ist; und
jedes r unabhängig 0, 1 oder 2 ist.

12. Verbindung der Formel (III), das Stereoisomer oder pharmazeutisch verträgliche Salz davon nach Anspruch 10, wobei:
R₁ die folgende Struktur aufweist: 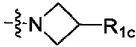 oder 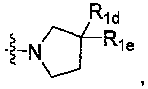 worin
R_{1c} ausgewählt ist aus der Gruppe bestehend aus Wasserstoff, Deuterium, Fluor, Brom, Methyl, Ethyl, Propyl, Isopropyl, Butyl, -N(R₁₃ₐ)-S(O)₂R₁₀ₐ, -O-R₁₁ₐ und - N(R₁₃ₐ)-C(O)R₁₂ₐ, und das Methyl, Ethyl, Propyl, Isopropyl oder Butyl jeweils unabhängig voneinander gegebenenfalls weiter substituiert sind durch einen oder mehrere Substituenten, ausgewählt aus der Gruppe bestehend aus Deuterium, Methyl, Ethyl, Propyl, Isopropyl, Butyl, Monofluormethyl, Difluormethyl, Trifluormethyl, Monodeuteriomethyl, Dideuteriomethyl, Trideuteriomethyl, -S(O)rR_{10b} und -O-R₁₁ₐ;
R_{1d} ausgewählt ist aus der Gruppe bestehend aus Wasserstoff, Deuterium, Fluor, Brom, Methyl, Ethyl, Propyl, Isopropyl und Butyl;
R₁ₑ ausgewählt ist aus der Gruppe bestehend aus Wasserstoff, Deuterium, Fluor, Brom, Methyl, Ethyl, Propyl, Isopropyl, Butyl, -N(R₁₃ₐ)-S(O)₂R₁₀ₐ, -O-R₁₁ₐ und - N(R₁₃ₐ)-C(O)R₁₂ₐ, und das Methyl, Ethyl, Propyl, Isopropyl oder Butyl jeweils unabhängig voneinander gegebenenfalls weiter substituiert sind durch einen oder mehrere Substituenten, ausgewählt aus der Gruppe bestehend aus Deuterium, Methyl, Ethyl, Propyl, Isopropyl, Butyl, Monofluormethyl, Difluormethyl, Trifluormethyl, Monodeuteriomethyl, Dideuteriomethyl, Trideuteriomethyl, -S(O)rR_{10b} und -O-R₁₁ₐ;
jedes R₁₀ₐ unabhängig ausgewählt ist aus der Gruppe bestehend aus Wasserstoff, Methyl, Ethyl, Propyl, Isopropyl, Butyl, Cyclopropyl, Cyclobutyl, Cyclopentyl, Cyclohexyl, Oxacyclopropyl, Oxacyclobutyl, Oxacyclopentyl, Oxacyclohexyl, Azacyclopropyl, Azacyclobutyl, Azacyclopentyl und Azacyclohexyl;
jedes R_{10b} unabhängig ausgewählt ist aus der Gruppe bestehend aus Wasserstoff, Methyl, Ethyl, Propyl, Isopropyl, Butyl, Cyclopropyl, Cyclobutyl, Cyclopentyl, Cyclohexyl, Oxacyclopropyl, Oxacyclobutyl, Oxacyclopentyl, Oxacyclohexyl, Azacyclopropyl, Azacyclobutyl, Azacyclopentyl und Azacyclohexyl, und die oben genannten Gruppen jeweils unabhängig voneinander gegebenenfalls weiter substituiert sind durch einen oder mehrere Substituenten, ausgewählt aus der Gruppe bestehend aus Deuterium, Fluor, Chlor, Hydroxy, Methyl, Ethyl, Propyl, Isopropyl, Butyl, Methoxy, Ethoxy und Propoxy;
jedes R₁₁ₐ unabhängig ausgewählt ist aus der Gruppe bestehend aus Wasserstoff, Methyl, Ethyl, Propyl, Isopropyl und Butyl;
jedes R₁₂ₐ unabhängig ausgewählt ist aus der Gruppe bestehend aus Wasserstoff, Methyl, Ethyl, Propyl, Isopropyl, Butyl, Methoxy, Ethoxy, Propoxy, tert-Butoxy, Cyclopropyl, Cyclobutyl, Cyclopentyl und Cyclohexyl, und die oben genannten Gruppen jeweils unabhängig voneinander gegebenenfalls weiter substituiert sind durch einen oder mehrere Substituenten, ausgewählt aus der Gruppe bestehend aus Deuterium, Fluor, Chlor, Hydroxy und Cyan;
jedes R₁₃ₐ unabhängig Wasserstoff ist; und
jedes r unabhängig 0, 1 oder 2 ist.

13. Verbindung der Formel (III), das Stereoisomer oder pharmazeutisch verträgliche Salz davon nach Anspruch 10, wobei:
R₁ die folgende Struktur aufweist: 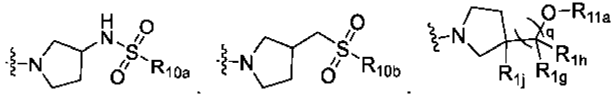 oder 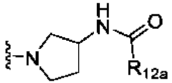
worin
R_{1g} Wasserstoff, Deuterium oder Methyl ist; R₁ₕ Wasserstoff, Deuterium oder Methyl ist; R₁ⱼ Wasserstoff, Deuterium oder Methyl ist; q 0,1 oder 2 ist;
R₁₀ₐ ausgewählt ist aus der Gruppe bestehend aus Wasserstoff, Methyl, Ethyl, Propyl, Isopropyl, Butyl, Cyclopropyl, Cyclobutyl, Cyclopentyl, Cyclohexyl, Oxacyclopropyl, Oxacyclobutyl, Oxacyclopentyl, Oxacyclohexyl, Azacyclopropyl, Azacyclobutyl, Azacyclopentyl und Azacyclohexyl;
R_{10b} ausgewählt ist aus der Gruppe bestehend aus Wasserstoff, Methyl, Ethyl, Propyl, Isopropyl, Butyl, Cyclopropyl, Cyclobutyl, Cyclopentyl, Cyclohexyl, Oxacyclopropyl, Oxacyclobutyl, Oxacyclopentyl, Oxacyclohexyl, Azacyclopropyl, Azacyclobutyl, Azacyclopentyl und Azacyclohexyl, und die oben genannten Gruppen jeweils unabhängig voneinander gegebenenfalls weiter substituiert sind durch einen oder mehrere Substituenten, ausgewählt aus der Gruppe bestehend aus Deuterium, Fluor, Chlor, Hydroxy, Methyl, Ethyl, Propyl, Isopropyl, Butyl, Methoxy, Ethoxy und Propoxy;
jedes R₁₁ₐ unabhängig ausgewählt ist aus der Gruppe bestehend aus Wasserstoff, Methyl, Ethyl, Propyl, Isopropyl und Butyl;
R₁₂ₐ ausgewählt ist aus der Gruppe bestehend aus Wasserstoff, Methyl, Ethyl, Propyl, Isopropyl, Butyl, Methoxy, Ethoxy, Propoxy, tert-Butoxy, Cyclopropyl, Cyclobutyl, Cyclopentyl und Cyclohexyl, und die oben genannten Gruppen jeweils unabhängig voneinander gegebenenfalls weiter substituiert sind durch einen oder mehrere Substituenten, ausgewählt aus der Gruppe bestehend aus Deuterium, Fluor, Chlor, Hydroxy und Cyan;

14. Verbindung der Formel (III), das Stereoisomer oder pharmazeutisch verträgliche Salz davon nach Anspruch 4, wobei R₂ₐ ausgewählt ist aus der Gruppe bestehend aus Wasserstoff, Deuterium, C₁₋₄-Alkyl, C₁₋₄-Halogenalkyl, C₁₋₄-Deuteroalkyl, C₃₋₆-Cycloalkyl und 3- bis 6-gliedrigem Heterocyclyl.

15. Verbindung der Formel (III), das Stereoisomer oder pharmazeutisch verträgliche Salz davon nach Anspruch 14, wobei R₂ₐ ausgewählt ist aus der Gruppe bestehend aus Wasserstoff, Deuterium, Methyl, Ethyl, Propyl, Isopropyl, Butyl, Monofluormethyl, Difluormethyl, Trifluormethyl, Monodeuteriomethyl, Dideuteriomethyl, Trideuteriomethyl, Cyclopropyl, Cyclobutyl, Oxacyclopropyl, Oxacyclobutyl, Azacyclopropyl und Azacyclobutyl.

16. Verbindung der Formel (III), das Stereoisomer oder ein pharmazeutisch verträgliches Salz davon nach Anspruch 1, wobei sie aus den folgenden Verbindungen ausgewählt ist: 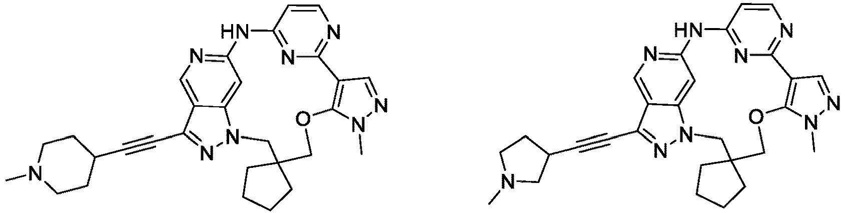 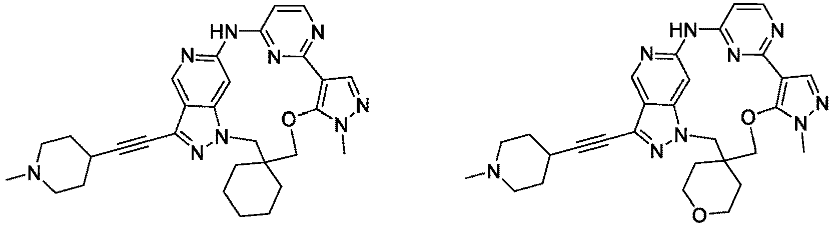 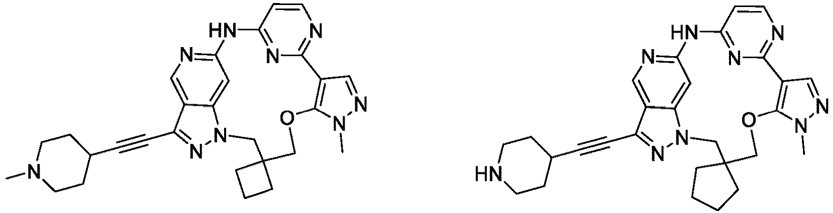 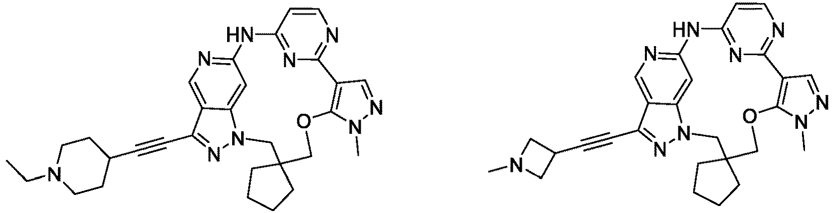 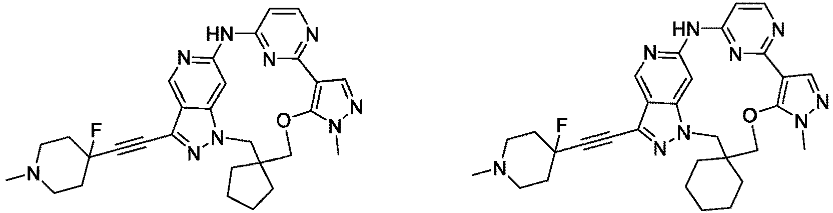 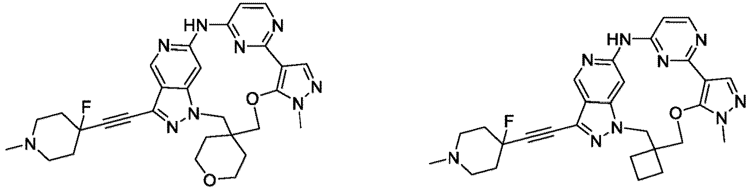 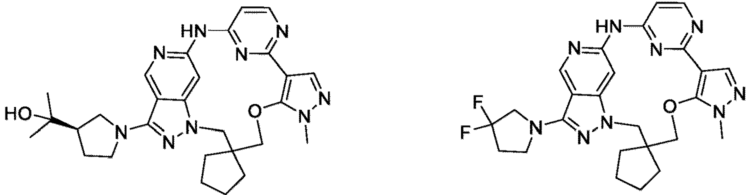 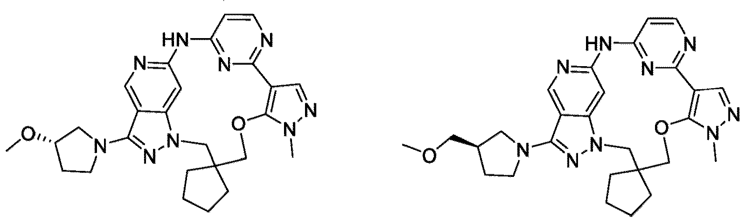 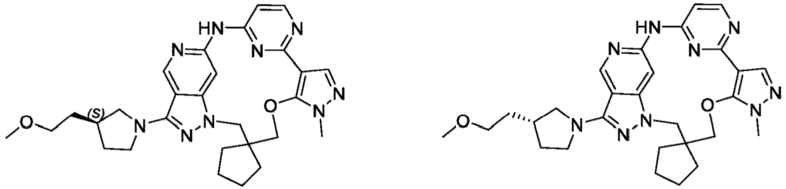 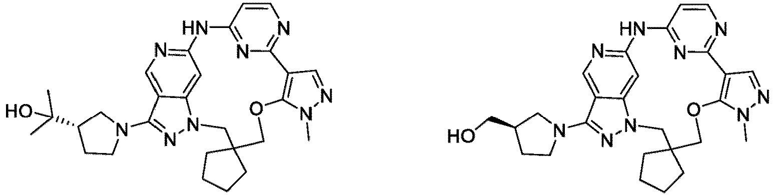 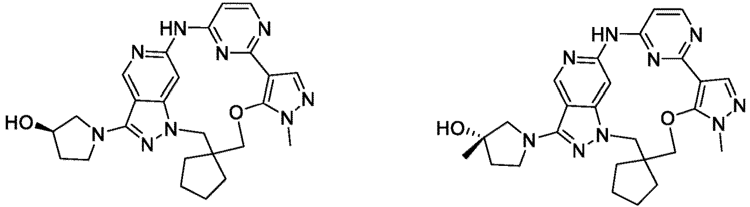 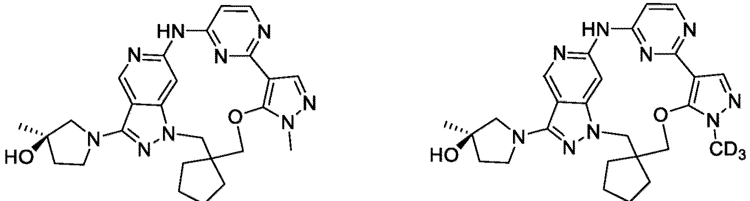 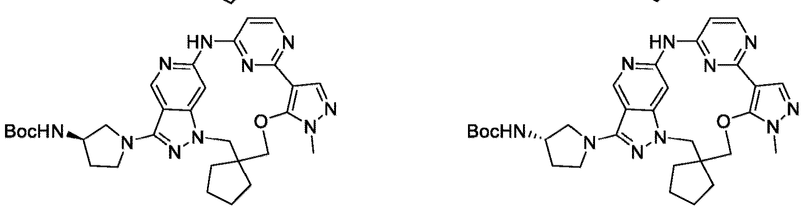 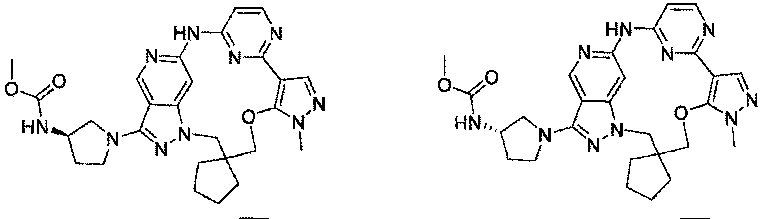 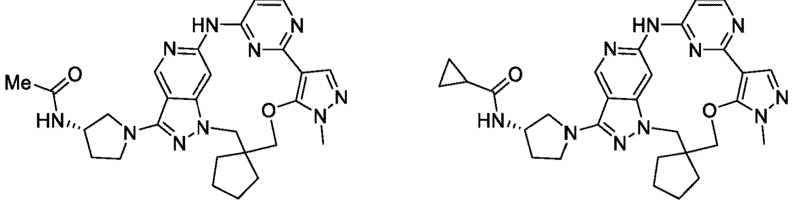 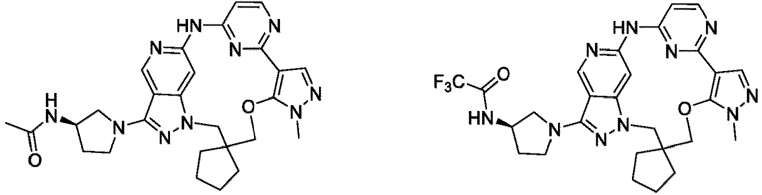 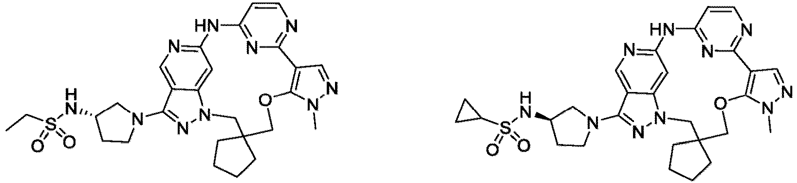 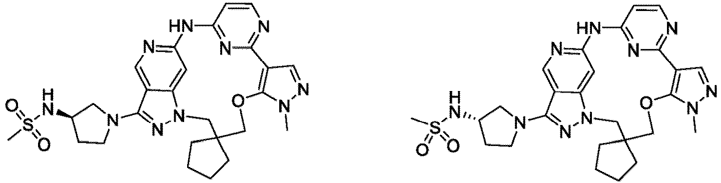 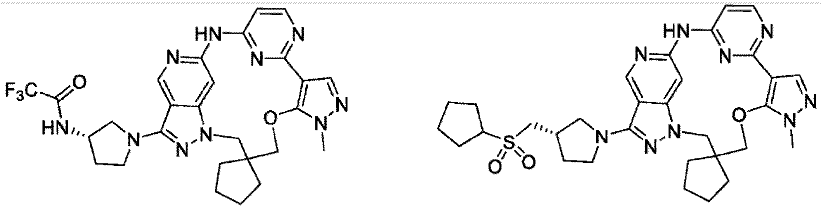 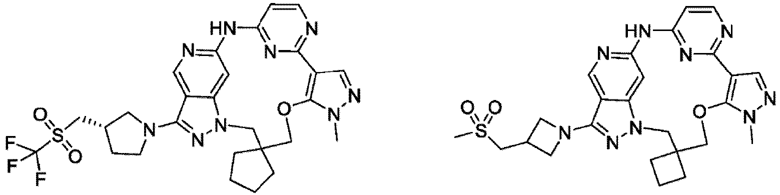 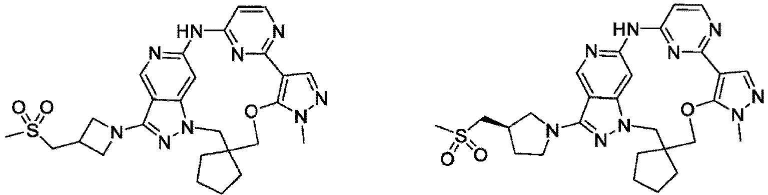 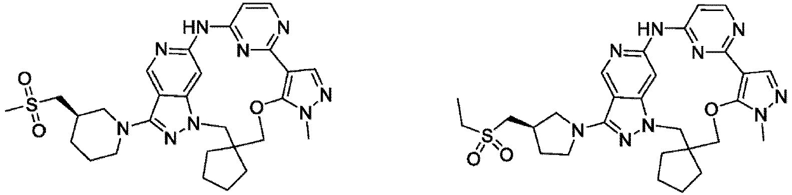 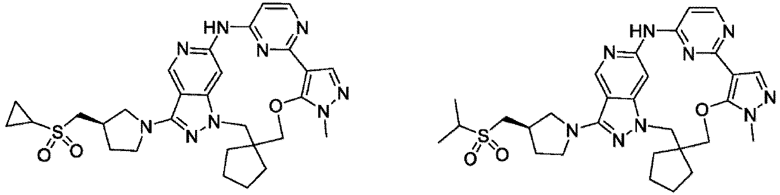 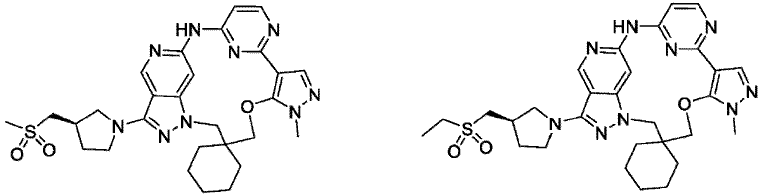 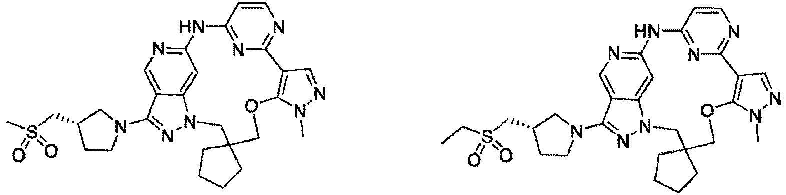 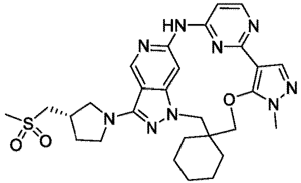 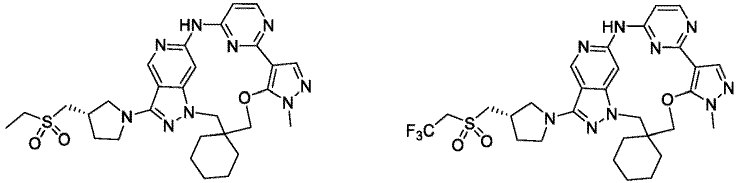 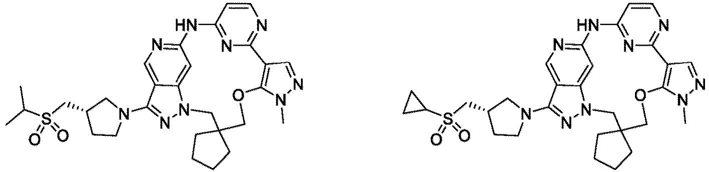 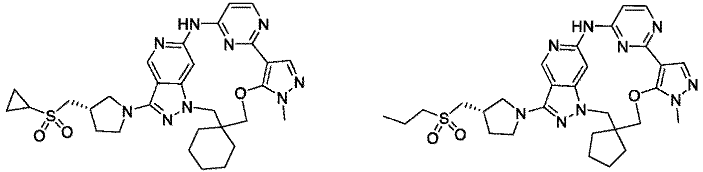 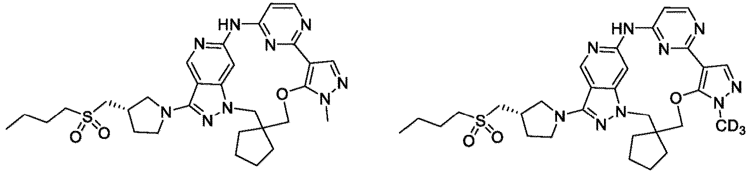 und

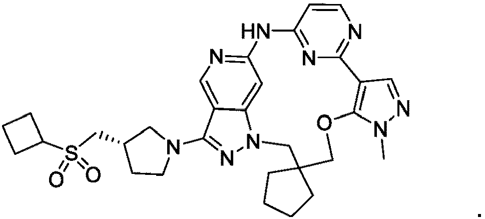

17. Pharmazeutische Zusammensetzung, umfassend die Verbindung der Formel (III), deren Stereoisomer oder ein pharmazeutisch verträgliches Salz davon nach einem der Ansprüche 1 bis 16 sowie einen pharmazeutisch verträglichen Träger.

18. Verbindung der Formel (III), das Stereoisomer oder pharmazeutisch verträgliches Salz davon nach einem der Ansprüche 1 bis 16 zur Verwendung beim Behandeln und/oder Vorbeugen von Tumoren oder metastasierenden Erkrankungen, die mindestens teilweise mit der EGFR-L858R/C797S-Doppelmutation, der EGFR-Del19/C797S-Doppelmutation, L858R/T790M/C797S-Dreifachmutation und der Del19/T790M/C797S-Dreifachmutation assoziiert sind; gegebenenfalls, wobei die Tumore oder metastasierenden Erkrankungen Tumore und/oder metastasierende Erkrankungen sind, die durch Hyperproliferation und eine Funktionsstörung bei der Zelltodinduktion verursacht werden.

19. Verbindung zur Verwendung nach Anspruch 18, wobei die Tumore oder metastatischen Erkrankungen ausgewählt sind aus der Gruppe bestehend aus Lungenkrebs, Darmkrebs, Bauchspeicheldrüsenkrebs, Kopf- und Halskrebs, Brustkrebs, Eierstockkrebs, Gebärmutterkrebs, Magenkrebs, nicht-kleinzelligem Lungenkrebs, Leukämie, myelodysplastischem Syndrom, malignem Lymphom, Kopf-Hals-Tumor, Thorax-Tumor, gastrointestinalem Tumor, endokrinem Tumor, Brusttumor und anderen gynäkologischen Tumoren, urologischem Tumor, Hauttumor, Sarkom, invertiertem sinonasalem Papillom oder Plattenepithelkarzinom der Nasennebenhöhlen, das mit einem invertierten sinonasalen Papillom assoziiert ist.

## Revendications

1. Composé de formule (III), stéréoisomère ou sel pharmaceutiquement acceptable de celui-ci : 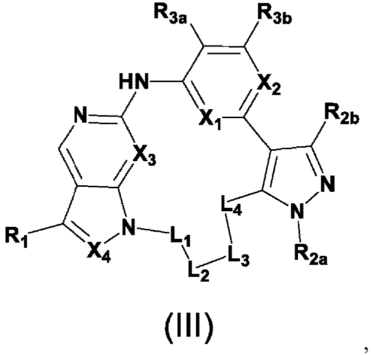 dans lequel,
X₁ est CRₐ ou N ;
X₂ est CR_{b} ou N ;
X₃ est CR_{c} ou N ;
X₄ est CR_{d} ou N ;
L₁ est CR₅ₐR_{5b} ;
L₂ est (CR_{5c}R_{5d})ₚ ;
L₃ est une liaison, CR₅ₑR_{5f} ou C(O) ;
L₄ est CR_{5g}R₅ₕ, O, NR_{6c} ou C(O) ;
R₁ est choisi dans le groupe constitué par hydrogène, deutérium, halogène, cyano, alkyle en C₁₋₄, alcényle en C₂₋₄, alcynyle en C₂₋₄, cycloalkyle en C₃₋₆, hétérocyclyle à 3-8 chaînons, aryle en C₆₋₈, hétéroaryle de 5 à 8 chaînons, - SF₅, -S(O)(=N-R₇)R₈, -N=S(O)R₈R₉, -N=SR₈R₉, -O-S(O)₂R₁₀, -S(O)ᵣR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂, -NR₁₃R₁₄, - C(=NR₁₃)R₁₂, -N(R₁₃)-C(=NR₁₄)R₁₂, -C(O)NR₁₃R₁₄ et -N(R₁₃)-C(O)R₁₂, et les groupes ci-dessus sont chacun indépendamment éventuellement substitués en outre par un ou plusieurs substituants choisis dans le groupe constitué par le deutérium, halogène, cyano, alkyle en C₁₋₄, alcényle en C₂₋₄, alcynyle en C₂₋₄, cycloalkyle en C₃₋₆, hétérocyclyle à 3-6 chaînons, alkyle en C₁₋₄ substitué par cycloalkyle en C₃₋₆, alkyle en C₁₋₄ substitué par un hétérocyclyle à 3-6 chaînons, aryle en C₆₋₈, hétéroaryle de 5 à 8 chaînons, =O, =S, -SF₅, -S(O)(=N-R₇)R₈, - N=S(O)R₈R₉, -N=SR₈R₉, -N(R₁₃)-S(O)₂R₁₀, -O-S(O)₂R₁₀, -S(O)R₁₀,-O-R₁₁, - C(O)OR₁₁, -C(O)SR₁₁, -S-C(O)R₁₂, -C(O)R₁₂, -O-C(O)R₁₂, -P(O)(R₁₂)₂,-NR₁₃R₁₄, - C(=NR₁₃)R₁₂, -N(R₁₃)-C(=NR₁₄)R₁₂, -C(O)NR₁₃R₁₄ et -N(R₁₃)-C(O)R₁₂, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle de 5 à 8 chaînons, =O, =S, -alkyl en C₀₋₄-SF₅, -alkyle en C₀₋₄-S(O)(=N-R₇)R₈, -alkyle en C₀₋₄-N=S(O)R₈R₉, -alkyle en C₀₋₄-N=SR₈R₉, -alkyle en C₀₋₄-N(R₁₃)-S(O)₂R₁₀, -alkyle en C₀₋₄-O-S(O)₂R₁₀, -alkyle en C₀₋₄-S(O)ᵣR₁₀, -alkyle en C₀₋₄-O-R₁₁, -alkyle en C₀₋₄-C(O)OR₁₁, -alkyle en C₀₋₄-C(O)SR₁₁, -alkyle en C₀₋₄-S-C(O)R₁₂, -alkyle en C₀₋₄-C(O)R₁₂, -alkyle en C₀₋₄-OC(O)R₁₂, -alkyle en C₀₋₄-P(O)(R₁₂)₂, -alkyle en C₀₋₄-NR₁₃R₁₄, -alkyle en C₀₋₄-C(=NR₁₃)R₁₂, -alkyle en C₀₋₄-N(R₁₃)-C(=NR₁₄)R₁₂, -alkyle en C₀₋₄-C(O)NR₁₃R₁₄ et - alkyle en C₀₋₄-N(R₁₃)-C(O)R_{12 ;}
R₂ₐ est choisi dans le groupe constitué par hydrogène, deutérium, hydroxy, alkyle en C₁₋₄, alcényle en C₂₋₄, alcynyle en C₂₋₄, cycloalkyle en C₃₋₆ et hétérocyclyle à 3-6 chaînons, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle de 5 à 8 chaînons, =O, =S, -SF₅, -S(O)(=N-R₇)R₈, -N=S(O)R₈R₉, - N=SR₈R₉, -O-S(O)₂R₁₀,-S(O)ᵣR₁₀, -O-R₁₁, -C(O)OR₁₁, -C(O)SR₁₁, -S-C(O)R₁₂, - C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, - C(O)NR₁₃R₁₄ et -N(R₁₃)-C(O)R₁₂ ;
R_{2b} est choisi dans le groupe constitué par hydrogène, deutérium, halogène, cyano, alkyle en C₁₋₄, alcényle en C₂₋₄, alcynyle en C₂₋₄, cycloalkyle en C₃₋₆, hétérocyclyle à 3-6 chaînons, C₆₋₈aryle, hétéroaryle à 5-8 chaînons et -SF₅, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle à 5-8 chaînons, =O, =S,-SF₅,-S(O)(=N-R₇)R₈,-N=S(O)R₈R₉,-N=SR₈R₉,-O-S(O)₂R₁₀,-S(O)ᵣR₁₀, -O-R₁₁, -C(O)OR₁₁, -C(O)SR₁₁, -S-C(O)R₁₂, -C(O)R₁₂, -O-C(O)R₁₂, -P(O)(R₁₂)₂, -NR₁₃R₁₄, -C(=NR₁₃)R₁₂, -N(R₁₃)-C(=NR₁₄)R₁₂, -C(O)NR₁₃R₁₄ et -N(R₁₃)-C(O)R₁₂ ;
R₃ₐ, R_{3b}, Rₐ et R_{b} sont chacun indépendamment choisis dans le groupe constitué par hydrogène, deutérium, halogène, cyano, alkyle en C₁₋₄, haloalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle à 5-8 chaînons et -SF₅ ;
R_{c} est choisi dans le groupe constitué par hydrogène, deutérium, halogène, cyano, alkyle en C₁₋₄, haloalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle à 5-8 chaînons et -SF₅ ;
R_{d} est choisi dans le groupe constitué par hydrogène, deutérium, halogène, cyano, alkyle en C₁₋₄, haloalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle à 5-8 chaînons et -SF₅ ;
R₅ₐ et R_{5b} sont chacun indépendamment choisis dans le groupe constitué par hydrogène, deutérium, halogène, cyano, alkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle de 5 à 8 chaînons et -SF₅, ou, R₅ₐ et R_{5b}, conjointement avec l'atome de carbone auquel ils sont directement liés, forment un C(O), cycloalkyle en C₃₋₆, hétérocyclyle à 4-6 chaînons, aryle en C₆₋₈ ou hétéroaryle à 5-8 chaînons, et les groupes ci-dessus sont chacun indépendamment éventuellement en outre substitués par un ou plusieurs substituants choisis dans le groupe constitué par le deutérium, un halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle de 5 à 8 chaînons, =O, =S, -SF₅, -S(O)(=N-R₇)R₈, -N=S(O)R₈R₉, -N=SR₈R₉, -O-S(O)₂R₁₀,-S(O)ᵣR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂,-NR₁₃R₁₄, - C(=NR₁₃)R₁₂, -N(R₁₃)-C(=NR₁₄)R₁₂, -C(O)NR₁₃R₁₄ et -N(R₁₃)-C(O)R₁₂ ;
lorsque p vaut 1, R_{5c} et R_{5d}, conjointement avec l'atome de carbone auquel ils sont directement liés, forment un cycloalkyle en C₃₋₆ ou hétérocyclyle à 4-6 chaînons, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle de 5 à 8 chaînons, =O, =S, -SF₅, -S(O)(=N-R₇)R₈, -N=S(O)R₈R₉, -N=SR₈R₉, -O-S(O)₂R₁₀,-S(O)ᵣR₁₀, - O-R₁₁, -C(O)OR₁₁, -C(O)SR₁₁, -S-C(O)R₁₂, -C(O)R₁₂, -O-C(O)R₁₂, -P(O)(R₁₂)₂, - NR₁₃R₁₄, -C(=NR₁₃)R₁₂, -N(R₁₃)-C(=NR₁₄)R₁₂, -C(O)NR₁₃R₁₄ et -N(R₁₃)-C(O)R₁₂, ou
lorsque p vaut 2, un groupe de R_{5c} et R_{5d}, conjointement avec l'atome de carbone auquel ils sont directement liés, forment un cycloalkyle en C₃₋₆ ou hétérocyclyle à 4-6 chaînons, et lorsqu'un groupe de R_{5c} et R_{5d}, conjointement avec l'atome de carbone auquel ils sont directement liés, forment un cycloalkyle en C₃₋₆ ou hétérocyclyle à 4-6 chaînons, l'autre groupe de R_{5c} et R_{5d} sont chacun indépendamment choisis dans le groupe constitué par l'hydrogène, le deutérium, un halogène, un cyano, alkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle de 5 à 8 chaînons, -SF₅, -S(O)(=N-R₇)R₈, -N=S(O)R₈R₉, -N=SR₈R₉, -O-S(O)₂R₁₀,-S(O)ᵣR₁₀, -O-R₁₁, -C(O)OR₁₁, -C(O)SR₁₁, -S-C(O)R₁₂, -C(O)R₁₂, -O-C(O)R₁₂, - P(O)(R₁₂)₂, -NR₁₃R₁₄, -C(=NR₁₃)R₁₂, -N(R₁₃)-C(=NR14)R₁₂, -C(O)NR₁₃R₁₄ et - N(R₁₃)-C(O)R₁₂ ;
R₅ₑ et R_{5f} sont chacun indépendamment choisis dans le groupe constitué par hydrogène, deutérium, halogène, cyano, alkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle à 5-8 chaînons et -SF₅, ou, R₅ₑ et R_{5f}, conjointement avec l'atome de carbone auquel ils sont directement liés, forment un cycloalkyle en C₃₋₆, hétérocyclyle à 4-6 chaînons, aryle en C₆₋₈ ou hétéroaryle à 5-8 chaînons, et les groupes ci-dessus sont chacun indépendamment éventuellement en outre substitués par un ou plusieurs substituants choisis dans le groupe constitué par le deutérium, un halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle de 5 à 8 chaînons, =O, =S, -SF₅, -S(O)(=N-R₇)R₈, -N=S(O)R₈R₉, -N=SR₈R₉, -O-S(O)₂R₁₀,-S(O)ᵣR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂, -NR₁₃R₁₄, - C(=NR₁₃)R₁₂, -N(R₁₃)-C(=NR₁₄)R₁₂, -C(O)NR₁₃R₁₄ et -N(R₁₃)-C(O)R₁₂ ;
R_{5g} et R₅ₕ sont chacun indépendamment choisis dans le groupe constitué par hydrogène, deutérium, halogène, cyano, alkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle à 5-8 chaînons et -SF₅, ou, R_{5g} et R₅ₕ, conjointement avec l'atome de carbone auquel ils sont directement liés, forment un cycloalkyle en C₃₋₆, hétérocyclyle à 4-6 chaînons, aryle en C₆₋₈ ou hétéroaryle à 5-8 chaînons, et les groupes ci-dessus sont chacun indépendamment éventuellement substitués en outre par un ou plusieurs substituants choisis dans le groupe constitué par deutérium, halogène, cyano, alkyle en C₁₋₄, haloalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle de 5 à 8 chaînons, =O, =S, -SF₅, -S(O)(=N-R₇)R₈, -N=S(O)R₈R₉,-N=SR₈R₉, -O-S(O)₂R₁₀,-S(O)ᵣR₁₀, -O-R₁₁, -C(O)OR₁₁, - C(O)SR₁₁, -S-C(O)R₁₂, -C(O)R₁₂, -O-C(O)R₁₂, -P(O)(R₁₂)₂, -NR₁₃R₁₄, - C(=NR₁₃)R₁₂, -N(R₁₃)-C(=NR₁₄)R₁₂, -C(O)NR₁₃R₁₄ et -N(R₁₃)-C(O)R₁₂ ;
R_{6c} est choisi dans le groupe constitué par hydrogène, deutérium, alkyle en C₁₋₄, cycloalkyle en C₃₋₆, hétérocyclyle à 3-6 chaînons et -O-R₁₁, et les groupes ci-dessus sont chacun indépendamment éventuellement en outre substitués par un ou plusieurs substituants choisis dans le groupe constitué par deutérium, halogène, cyano, hydroxy, =O, alkyle en C₁₋₄, halogénoalkyle en C₁₋₄, deutérioalkyle en C₁₋₄, alcényle en C₂₋₄, alcynyle en C₂₋₄, alcoxy en C₁₋₄, cycloalkyle en C₃₋₆, cycloalcoxy en C₃₋₆, hétérocyclyle à 3-6 chaînons, hétérocyclyloxy à 3-6 chaînons et -NR₁₃R₁₄ ;
chaque R₇ est indépendamment choisi dans le groupe consistant en hydrogène, deutérium, alkyle en C₁₋₁₀, alcényle en C₂₋₁₀, alcynyle en C₂₋₁₀, cycloalkyle en C₃₋₁₀, hétérocyclyle à 3-10 chaînons, aryle en C₆₋₁₀, hétéroaryle à 5-10 chaînons, -alkyle en C₀₋₈-S(O)ᵣR₁₀, -alkyle en C₀₋₈-C(O)OR₁₁, -alkyle en C₀₋₈-C(O)R₁₂ ou -alkyle en C₀₋₈-C(O)NR₁₃R₁₄, et les groupes ci-dessus sont éventuellement en outre substitués par un ou plusieurs substituants choisis dans le groupe constitué par deutérium, halogène, cyano, nitro, azido, alkyle en C₁₋₁₀, alcényle en C₂₋₁₀, alcynyle en C₂₋₁₀, halogénoalkyle en C₁₋₁₀, deutérioalkyle en C₁₋₁₀, cycloalkyle en C₃₋₁₀, hétérocyclyle à 3-10 chaînons, aryle en C₆₋₁₀, hétéroaryle à 5-10 chaînons, -alkyle en C₀₋₈-S(O)ᵣR₁₀, -alkyle en C₀₋₈-O-R₁₁, -alkyle en C₀₋₈-C(O)OR₁₁, -alkyle en C₀₋₈-C(O)R₁₂, -alkyle en C₀₋₈-O-C(O)R₁₂, -alkyle en C₀₋₈-NR₁₃R₁₄, -alkyle en C₀₋₈-C(=NR₁₃)R₁₂, -alkyle en C₀₋₈-N(R₁₃)-C(=NR₁₄)R₁₂, -alkyle en C₀₋₈-C(O)NR₁₃R₁₄ou -alkyle en C₀₋₈-N(R₁₃)-C(O)R₁₂ ;
chaque R₈ et chaque R₉ sont indépendamment choisis dans le groupe constitué par hydrogène, deutérium, hydroxy, alkyle en C₁₋₁₀, alcényle en C₂₋₁₀, alcynyle en C₂₋₁₀, cycloalkyle en C₃₋₁₀, hétérocyclyle à 3-10 chaînons, aryle en C₆₋₁₀ et hétéroaryle à 5-10 chaînons, ou, R₈ et R₉ conjointement avec l'atome de soufre auquel ils sont directement liés, forment un hétérocyclyle à 3-10 chaînons, et les groupes ci-dessus sont éventuellement en outre substitués par un ou plusieurs substituants choisis dans le groupe constitué par deutérium, halogène, cyano, nitro, azido, alkyle en C₁₋₁₀, alcényle en C₂₋₁₀, alcynyle en C₂₋₁₀, haloalkyle en C₁₋₁₀, deutérioalkyle en C₁₋₁₀, cycloalkyle en C₃₋₁₀, hétérocyclyle à 3-10 chaînons, aryle en C₆₋₁₀, hétéroaryle de 5 à 10 chaînons, -alkyle en C₀₋₈-S(O)ᵣR₁₀, -alkyle en C₀₋₈-O-R₁₁, - alkyle en C₀₋₈-C(O)OR₁₁, -alkyle en C₀₋₈-C(O)R₁₂, -alkyle en C₀₋₈-O-C(O)R₁₂, -alkyle en C₀₋₈-NR₁₃R₁₄, -alkyle en C₀₋₈-C(=NR₁₃)R₁₂, -alkyle en C₀₋₈-N(R₁₃)-C(=NR₁₄)R₁₂, - alkyle en C₀₋₈-C(O)NR₁₃R₁₄ et -alkyle en C₀₋₈-N(R₁₃)-C(O)R₁₂ ;
chaque R₁₀ est indépendamment choisi dans le groupe constitué par hydrogène, deutérium, hydroxy, alkyle en C₁₋₁₀, alcényle en C₂₋₁₀, cycloalkyle en C₃₋₁₂, hétérocyclyle à 3-12 chaînons, aryle en C₆₋₁₀, hétéroaryle de 5 à 10 chaînons et -NR₁₃R₁₄, et les groupes ci-dessus sont chacun indépendamment éventuellement en outre substitués par un ou plusieurs substituants choisis dans le groupe constitué par deutérium, halogène, hydroxy, =O, alkyle en C₁₋₁₀, alcoxy en C₁₋₁₀, cycloalkyle en C₃₋₁₂, cycloalcoxy en C₃₋₁₂, hétérocyclyle de 3 à 12 chaînons, hétérocyclyloxy de 3 à 12 chaînons, aryle en C₆₋₁₀, aryloxy en C₆₋₁₀, hétéroaryle à 5-10 chaînons, hétéroaryloxy à 5-10 chaînons et -NR₁₃R₁₄ ;
chaque R₁₁ est indépendamment choisi dans le groupe constitué par hydrogène, deutérium, alkyle en C₁₋₁₀, alcényle en C₂₋₁₀, cycloalkyle en C₃₋₁₂, hétérocyclyle à 3-12 chaînons, aryle en C₆₋₁₀ et hétéroaryle à 5-10 chaînons, et les groupes ci-dessus sont chacun indépendamment éventuellement en outre substitués par un ou plusieurs substituants choisis dans le groupe constitué par le deutérium, un halogène, hydroxy, =O, cyano, alkyle en C₁₋₁₀, alcoxy en C₁₋₁₀, cycloalkyle en C₃₋₁₂, cycloalcoxy en C₃₋₁₂, hétérocyclyle à 3-12 chaînons, hétérocyclyloxy à 3-12 chaînons, aryle en C₆₋₁₀, aryloxy en C₆₋₁₀, hétéroaryle à 5-10 chaînons, hétéroaryloxy à 5-10 chaînons et -NR₁₃R₁₄ ;
chaque R₁₂ est indépendamment choisi dans le groupe constitué par hydrogène, deutérium, hydroxy, alkyle en C₁₋₁₀, alcoxy en C₁₋₁₀, alcényle en C₂₋₁₀, alcynyle en C₂₋₁₀, cycloalkyle en C₃₋₁₂, cycloalcoxy en C₃₋₁₂, hétérocyclyle de 3 à 12 chaînons, hétérocyclyloxy de 3 à 12 chaînons, aryle en C₆₋₁₀, aryloxy en C₆₋₁₀, hétéroaryle à 5-10 chaînons, hétéroaryloxy à 5-10 chaînons et -NR₁₃R₁₄, et les groupes ci-dessus sont chacun indépendamment éventuellement en outre substitués par un ou plusieurs substituants choisis dans le groupe constitué par le deutérium, un halogène, hydroxy, =O, cyano, alkyle en C₁₋₁₀, alcoxy en C₁₋₁₀, cycloalkyle en C₃₋₁₂, cycloalcoxy en C₃₋₁₂, hétérocyclyle de 3 à 12 chaînons, hétérocyclyloxy de 3 à 12 chaînons, aryle en C₆₋₁₀, aryloxy en C₆₋₁₀, hétéroaryle à 5-10 chaînons, hétéroaryloxy à 5-10 chaînons et -NR₁₃R₁₄ ;
chaque R₁₃ et chaque R₁₄ sont indépendamment choisis dans le groupe constitué par hydrogène, deutérium, hydroxy, alkyle en C₁₋₁₀, alcényle en C₂₋₁₀, alcynyle en C₂₋₁₀, cycloalkyle en C₃₋₁₂, hétérocyclyle à 3-12 chaînons, aryle en C₆₋₁₀, hétéroaryle à 5-10 chaînons, sulfinyle, sulfonyle, méthanesulfonyle, isopropylsulfonyle, cyclopropylsulfonyle, p-toluènesulfonyle, aminosulfonyle, diméthylaminosulfonyle et alcanoyle en C₁₋₁₀, et les groupes ci-dessus sont chacun indépendamment éventuellement en outre substitués par un ou plusieurs substituants choisis dans le groupe constitué par deutérium, halogène, hydroxy, =O, alkyle en C₁₋₁₀, alcényle en C₂₋₁₀, alcynyle en C₂₋₁₀, haloalkyle en C₁₋₁₀, deutérioalkyle en C₁₋₁₀, alcoxy en C₁₋₁₀, cycloalkyle en C₃₋₁₂, cycloalcoxy en C₃₋₁₂, hétérocyclyle de 3 à 12 chaînons, hétérocyclyloxy de 3 à 12 chaînons, aryle en C₆₋₁₀, aryloxy en C₆₋₁₀, hétéroaryle à 5-10 chaînons, hétéroaryloxy à 5-10 chaînons, amino, monoalkylamino en C₁₋₁₀, diC₁₋₁₀alkylamino et alcanoyle en C₁₋₁₀ ; ou
R₁₃ et R₁₄, conjointement avec l'atome d'azote auquel ils sont directement liés, forment un hétérocyclyle à 4-10 chaînons ou un hétéroaryle à 5-10 chaînons et l'hétérocyclyle à 4-10 chaînons ou l'hétéroaryle à 5-10 chaînons est éventuellement en outre substitué par un ou plusieurs substituants choisis dans le groupe constitué par deutérium, halogène, hydroxy, =O, alkyle en C₁₋₁₀, alcényle en C₂₋₁₀, alcynyle en C₂₋₁₀, haloalkyle en C₁₋₁₀, deutérioalkyle en C₁₋₁₀, alcoxy en C₁₋₁₀, cycloalkyle en C₃₋₁₂, cycloalcoxy en C₃₋₁₂, hétérocyclyle de 3 à 12 chaînons, hétérocyclyloxy de 3 à 12 chaînons, aryle en C₆₋₁₀, aryloxy en C₆₋₁₀, hétéroaryle de 5 à 10 chaînons, hétéroaryloxy de 5 à 10 chaînons, méthanesulfonylméthyle, amino, mono alkylamino en C₁₋₁₀, dialkylamino en C₁₋₁₀ et alcanoyle en C₁₋₁₀ ;
p est 1 ou 2 ; et
chaque r est indépendamment 0, 1 ou 2.

2. Composé de formule (III), stéréoisomère ou sel pharmaceutiquement acceptable de celui-ci selon la revendication 1, dans lequel le composé de formule (III) est un composé de formule (IV) tel que représenté ci-dessous : 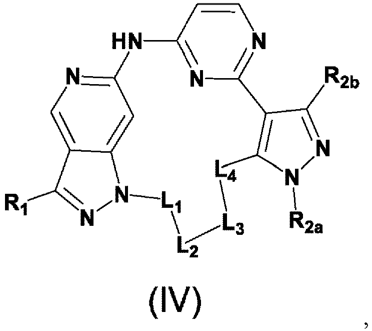 dans lequel,
L₁ est CR₅ₐR_{5b} ;
L₂ est CR_{5c}R_{5d} ;
L₃ est CR₅ₑR_{5f} ;
L₄ est O ;
R₁ est choisi dans le groupe constitué par hydrogène, deutérium, halogène, cyano, alkyle en C₁₋₄, alcényle en C₂₋₄, alcynyle en C₂₋₄, cycloalkyle en C₃₋₆, hétérocyclyle à 3-8 chaînons, aryle en C₆₋₈, hétéroaryle à 5-8 chaînons et - SF₅, et les groupes ci-dessus sont chacun indépendamment éventuellement en outre substitués par un ou plusieurs substituants choisis dans le groupe constitué par deutérium, halogène, cyano, alkyle en C₁₋₄, alcényle en C₂₋₄, alcynyle en C₂₋₄, cycloalkyle en C₃₋₆, hétérocyclyle à 3-6 chaînons, alkyle en C₁₋₄ substitué par cycloalkyle en C₃₋₆, alkyle en C₁₋₄ substitué par un hétérocyclyle à 3-6 chaînons, aryle en C₆₋₈, hétéroaryle de 5 à 8 chaînons, =O, =S, -SF₅, -S(O)(=N-R₇)R₈, - N=S(O)R₈R₉, -N=SR₈R₉, -N(R₁₃)-S(O)₂R₁₀, -O-S(O)₂R₁₀, -S(O)ᵣR₁₀, -O-R₁₁, - C(O)OR₁₁, -C(O)SR₁₁, -S-C(O)R₁₂, -C(O)R₁₂, -O-C(O)R₁₂, -P(O)(R₁₂)₂, -NR₁₃R₁₄, - C(=NR₁₃)R₁₂, -N(R₁₃)-C(=NR₁₄)R₁₂, -C(O)NR₁₃R₁₄ et -N(R₁₃)-C(O)R₁₂, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle de 5 à 8 chaînons, =O, =S, -SF₅, -S(O)(=N-R₇)R₈, -N=S(O)R₈R₉, -N=SR₈R₉, -N(R₁₃)-S(O)₂R₁₀, -O-S(O)₂R₁₀,-S(O)ᵣR₁₀, -O-R₁₁, -C(O)OR₁₁, -C(O)SR₁₁, -S-C(O)R₁₂, -C(O)R₁₂, -O-C(O)R₁₂,-P(O)(R₁₂)₂, -NR₁₃R₁₄, - C(=NR₁₃)R₁₂, -N(R₁₃)-C(=NR₁₄)R₁₂, -C(O)NR₁₃R₁₄ et -N(R₁₃)-C(O)R₁₂ ;
R₂ₐ est choisi dans le groupe constitué par hydrogène, deutérium, hydroxy, alkyle en C₁₋₄, alcényle en C₂₋₄, alcynyle en C₂₋₄, cycloalkyle en C₃₋₆ et hétérocyclyle à 3-6 chaînons, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, =O, =S et -SF₅ ;
R_{2b} est choisi dans le groupe constitué par hydrogène, deutérium, halogène, cyano, alkyle en C₁₋₄, alcényle en C₂₋₄, alcynyle en C₂₋₄, cycloalkyle en C₃₋₆, hétérocyclyle à 3-6 chaînons et -SF₅, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, =O, =S et -SF₅ ;
R₅ₐ et R_{5b} sont chacun indépendamment choisis dans le groupe constitué par hydrogène, deutérium, halogène, cyano, alkyle en C₁₋₄, cycloalkyle en C₃₋₆ et hétérocyclyle à 3-6 chaînons, ou, R₅ₐ et R_{5b}, conjointement avec l'atome de carbone auquel ils sont directement liés, forment un cycloalkyle en C₃₋₆ ou un hétérocyclyle à 4-6 chaînons, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, =O, =S et -SF₅ ; et
R_{5c} et R_{5d}, conjointement avec l'atome de carbone auquel ils sont directement liés, forment un cycloalkyle en C₃₋₆ ou un hétérocyclyle à 4-6 chaînons, et les groupes ci-dessus sont chacun indépendamment éventuellement en outre substitués par un ou plusieurs substituants choisis dans le groupe constitué par le deutérium, un halogène, un cyano, un alkyle en C₁₋₄, un halogénoalkyle en C₁₋₄, un deutérioalkyle en C₁₋₄, un alcényle en C₂₋₄, un alcynyle en C₂₋₄, un cycloalkyle en C₃₋₆, un hétérocyclyle à 3-6 chaînons, =O, =S et -SF₅ ;
R₅ₑ et R_{5f} sont chacun indépendamment choisis dans le groupe constitué par hydrogène, deutérium, halogène, cyano, alkyle en C₁₋₄, cycloalkyle en C₃₋₆ et hétérocyclyle à 3-6 chaînons, ou, R₅ₑ et R_{5f}, conjointement avec l'atome de carbone auquel ils sont directement liés, forment un cycloalkyle en C₃₋₆ ou un hétérocyclyle à 4-6 chaînons, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, =O, =S et -SF₅ ; et
R₇, R₈, R₉, R₁₀, R₁₁, R₁₂, R₁₃, R₁₄ et r sont tels que définis dans la revendication 1.

3. Composé de formule (III), stéréoisomère ou sel pharmaceutiquement acceptable de celui-ci selon la revendication 1, dans lequel :
L₁ est CR₅ₐR_{5b} ;
L₂ est CR_{5c}R_{5d} ;
L₃ est CR₅ₑR_{5f} ;
L₄ est O ;
R₅ₐ et R_{5b} sont chacun indépendamment choisis dans le groupe constitué par hydrogène, deutérium, fluor, chlore, brome, cyano, méthyle, éthyle, propyle, isopropyle, cyclopropyle, cyclobutyle, oxacyclopropyle, oxacyclobutyle, azacyclopropyle et azacyclobutyle, et les groupes ci-dessus sont chacun indépendamment éventuellement en outre substitués par un ou plusieurs substituants choisis dans le groupe constitué par deutérium, fluor, chlore, brome, cyano, méthyle, éthyle, propyle, isopropyle, monofluorométhyle, difluorométhyle, trifluorométhyle, monodeutériométhyle, dideutériométhyle, trideutériométhyle, vinyle, éthynyle, cyclopropyle, cyclobutyle, oxacyclopropyle, oxacyclobutyle, azacyclopropyle, azacyclobutyle, =O, =S et -SF₅ ;
R_{5c} et R_{5d}, conjointement avec l'atome de carbone auquel ils sont directement liés, forment un cycloalkyle en C₃₋₆ ou un hétérocyclyle à 4-6 chaînons, et les groupes ci-dessus sont chacun indépendamment éventuellement en outre substitués par un ou plusieurs substituants choisis dans le groupe constitué de deutérium, fluor, chlore, brome, cyano, méthyle, éthyle, propyle, isopropyle, monofluorométhyle, difluorométhyle, trifluorométhyle, monodeutériométhyle, dideutériométhyle, trideutériométhyle, vinyle, éthynyle, cyclopropyle, cyclobutyle, oxacyclopropyle, oxacyclobutyle, azacyclopropyle, azacyclobutyle, =O, =S et -SF₅ ; et
R₅ₑ et R_{5f} sont chacun indépendamment choisis dans le groupe constitué par hydrogène, deutérium, fluor, chlore, brome, cyano, méthyle, éthyle, propyle, isopropyle, cyclopropyle, cyclobutyle, oxacyclopropyle, oxacyclobutyle, azacyclopropyle et azacyclobutyle, et les groupes ci-dessus sont chacun indépendamment éventuellement en outre substitués par un ou plusieurs substituants choisis dans le groupe constitué par deutérium, fluor, chlore, brome, cyano, méthyle, éthyle, propyle, isopropyle, monofluorométhyle, difluorométhyle, trifluorométhyle, monodeutériométhyle, dideutériométhyle, trideutériométhyle, vinyle, éthynyle, cyclopropyle, cyclobutyle, oxacyclopropyle, oxacyclobutyle, azacyclopropyle, azacyclobutyle, =O, =S et -SF₅ ;
R₂ₐ est choisi dans le groupe constitué par hydrogène, deutérium, hydroxy, méthyle, éthyle, propyle, isopropyle, vinyle, éthynyle, cyclopropyle, cyclobutyle, oxacyclopropyle, oxacyclobutyle, azacyclopropyle et azacyclobutyle, et les groupes ci-dessus sont chacun indépendamment éventuellement en outre substitués par un ou plusieurs substituants choisis dans le groupe constitué par deutérium, fluor, chlore, brome, cyano, méthyle, éthyle, propyle, isopropyle, monofluorométhyle, difluorométhyle, trifluorométhyle, monodeutériométhyle, dideutériométhyle, trideutériométhyle, vinyle, éthynyle, cyclopropyle, cyclobutyle, oxacyclopropyle, oxacyclobutyle, azacyclopropyle, azacyclobutyle, =O, =S et -SF₅ ; et
R_{2b} est choisi dans le groupe constitué par hydrogène, deutérium, fluor, chlore, brome, cyano, méthyle, éthyle, propyle, isopropyle, vinyle, éthynyle, cyclopropyle, cyclobutyle, oxacyclopropyle, oxacyclobutyle, azacyclopropyle, azacyclobutyle et -SF₅, et les groupes ci-dessus sont chacun indépendamment éventuellement en outre substitués par un ou plusieurs substituants choisis dans le groupe constitué par deutérium, fluor, chlore, brome, cyano, méthyle, éthyle, propyle, isopropyle, monofluorométhyle, difluorométhyle, trifluorométhyle, monodeutériométhyle, dideutériométhyle, trideutériométhyle, vinyle, éthynyle, cyclopropyle, cyclobutyle, oxacyclopropyle, oxacyclobutyle, azacyclopropyle, azacyclobutyle, =O, =S et -SF₅.

4. Composé de formule (III), stéréoisomère ou sel pharmaceutiquement acceptable de celui-ci selon la revendication 1, dans lequel le composé de formule (III) est un composé de formule (V) tel que représenté ci-dessous : 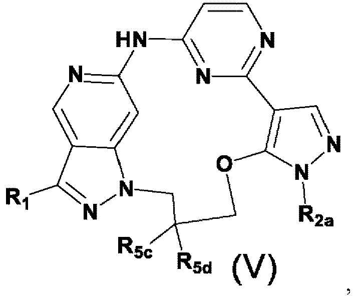 dans lequel,
R₁ est choisi parmi alcényle en C₂₋₄, alcynyle en C₂₋₄ et hétérocyclyle à 3-8 chaînons, et les groupes ci-dessus sont chacun indépendamment éventuellement substitués en outre par un ou plusieurs substituants choisis dans le groupe constitué par deutérium, halogène, cyano, alkyle en C₁₋₄, alcényle en C₂₋₄, alcynyle en C₂₋₄, cycloalkyle en C₃₋₆, hétérocyclyle à 3-6 chaînons, alkyle en C₁₋₄ substitué par cycloalkyle en C₃₋₆, alkyle en C₁₋₄ substitué par un hétérocyclyle à 3-6 chaînons, =O, =S, -SF₅, -N(R₁₃)-S(O)₂R₁₀, -O-S(O)₂R₁₀,-S(O)ᵣR₁₀, -O-R₁₁, - C(O)OR₁₁, -S-C(O)R₁₂, -C(O)R₁₂, -O-C(O)R₁₂, -NR₁₃R₁₄, -C(O)NR₁₃R₁₄ et -N(R₁₃)-C(O)R₁₂, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, aryle en C₆₋₈, hétéroaryle de 5 à 8 chaînons, =O, =S, -SF₅, -N(R₁₃)-S(O)₂R₁₀, -O-S(O)₂R₁₀,-S(O)R₁₀, -O-R₁₁, -C(O)OR₁₁, -C(O)R₁₂, -OC(O)R₁₂,-NR₁₃R₁₄, -C(O)NR₁₃R₁₄ et -N(R₁₃)-C(O)R₁₂ ;
R₂ₐ est choisi dans le groupe constitué par hydrogène, deutérium, alkyle en C₁₋₄, cycloalkyle en C₃₋₆ et hétérocyclyle à 3-6 chaînons, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, cycloalkyle en C₃₋₆ et hétérocyclyle à 3-6 chaînons ;
R_{5c} et R_{5d}, conjointement avec l'atome de carbone auquel ils sont directement liés, forment un cycloalkyle en C₃₋₆ ou un hétérocyclyle à 4-6 chaînons, et les groupes ci-dessus sont chacun indépendamment éventuellement en outre substitués par un ou plusieurs substituants choisis dans le groupe constitué par deutérium, halogène, cyano, alkyle en C₁₋₄, haloalkyle en C₁₋₄, deutérioalkyle en C₁₋₄, alcényle en C₂₋₄, alcynyle en C₂₋₄, cycloalkyle en C₃₋₆, hétérocyclyle à 3-6 chaînons, =O, =S et -SF₅ ;
R₁₀, R₁₁, R₁₂, R₁₃, R₁₄ et r sont tels que définis dans la revendication 1.

5. Composé de formule (III), stéréoisomère ou sel pharmaceutiquement acceptable de celui-ci selon la revendication 4, dans lequel R_{5c} et R_{5d}, conjointement avec l'atome de carbone auquel ils sont directement liés, forment un cycloalkyle en C₃₋₆ ou un hétérocyclyle à 4-6 chaînons ; éventuellement dans lequel R_{5c} et R_{5d}, conjointement avec l'atome de carbone auquel ils sont directement liés, forment un cyclopropyle, cyclobutyle, cyclopentyle, cyclohexyle, oxacyclobutyle, oxacyclopentyle ou oxacyclohexyle.

6. Composé de formule (III), stéréoisomère ou sel pharmaceutiquement acceptable de celui-ci selon la revendication 4, dans lequel :
R₁ est un alcynyle en C₂₋₄, et l'alcynyle en C₂₋₄ est éventuellement en outre substitué par un ou plusieurs substituants choisis dans le groupe constitué par deutérium, alkyle en C₁₋₄, cycloalkyle en C₃₋₆ et hétérocyclyle à 3-6 chaînons, et les groupes ci-dessus sont chacun indépendamment éventuellement en outre substitués par un ou plusieurs substituants choisis dans le groupe constitué par deutérium, halogène, cyano, alkyle en C₁₋₄, halogénoalkyle en C₁₋₄, deutérioalkyle en C₁₋₄, cycloalkyle en C₃₋₆ et hétérocyclyle à 3-6 chaînons.

7. Composé de formule (III), stéréoisomère ou sel pharmaceutiquement acceptable de celui-ci selon la revendication 6, dans lequel :
R₁ est un éthynyle, et l'éthynyle est éventuellement en outre substitué par un ou plusieurs substituants choisis dans le groupe constitué de deutérium, méthyle, éthyle, propyle, isopropyle, butyle, cyclopropyle, cyclobutyle, cyclopentyle, cyclohexyle, oxacyclopropyle, oxacyclobutyle, oxacyclopentyle, oxacyclohexyle, azacyclopropyle, azacyclobutyle, azacyclopentyle et azacyclohexyle, et les groupes ci-dessus sont chacun indépendamment éventuellement en outre substitués par un ou plusieurs substituants choisis dans le groupe constitué de deutérium, fluor, brome, cyano, méthyle, éthyle, propyle, isopropyle, butyle, monofluorométhyle, difluorométhyle, trifluorométhyle, monodeutériométhyle, dideutériométhyle, trideutériométhyle, cyclopropyle, cyclobutyle, cyclopentyle, cyclohexyle, oxacyclopropyle, oxacyclobutyle, oxacyclopentyle, oxacyclohexyle, azacyclopropyle, azacyclobutyle, azacyclopentyle et azacyclohexyle.

8. Composé de formule (III), stéréoisomère ou sel pharmaceutiquement acceptable de celui-ci selon la revendication 6, dans lequel :
R₁ est un éthynyle, et l'éthynyle est éventuellement en outre substitué par un ou plusieurs substituants choisis dans le groupe constitué par cyclopropyle, cyclobutyle, cyclopentyle, cyclohexyle, oxacyclopropyle, oxacyclobutyle, oxacyclopentyle, oxacyclohexyle, azacyclopropyle, azacyclobutyle, azacyclopentyle et azacyclohexyle, et les groupes ci-dessus sont chacun indépendamment éventuellement en outre substitués par un ou plusieurs substituants choisis dans le groupe constitué par deutérium, fluor, brome, cyano, méthyle, éthyle, propyle, isopropyle, monofluorométhyle, difluorométhyle, trifluorométhyle, monodeutériométhyle, dideutériométhyle et trideutériométhyle.

9. Composé de formule (III), stéréoisomère ou sel pharmaceutiquement acceptable de celui-ci selon la revendication 6, dans lequel :
R₁ est 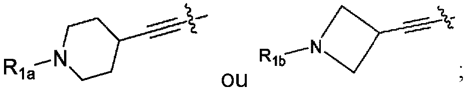
R₁ₐ est choisi dans le groupe constitué par hydrogène, méthyle, éthyle, propyle, isopropyle, monofluorométhyle, difluorométhyle, trifluorométhyle, monodeutériométhyle, dideutériométhyle et trideutériométhyle ; et
R_{1b} est choisi dans le groupe constitué par hydrogène, méthyle, éthyle, propyle, isopropyle, monofluorométhyle, difluorométhyle, trifluorométhyle, monodeutériométhyle, dideutériométhyle et trideutériométhyle.

10. Composé de formule (III), stéréoisomère ou sel pharmaceutiquement acceptable de celui-ci selon la revendication 4, dans lequel R₁ est un hétérocyclyle azoté à 4-8 chaînons, et l'hétérocyclyle azoté à 4-8 chaînons est éventuellement en outre substitué par un ou plusieurs substituants choisis dans le groupe constitué par deutérium, halogène, cyano, alkyle en C₁₋₄, =O, -N(R₁₃)-S(O)₂R₁₀, - S(O)ᵣR₁₀, -O-R₁₁, -O-C(O)R₁₂, -NR₁₃R₁₄ et -N(R₁₃)-C(O)R₁₂, et l'alkyle en C₁₋₄ est éventuellement en outre substitué par un ou plusieurs substituants choisis dans le groupe constitué par deutérium, halogène, cyano, alkyle en C₁₋₄, halogénoalkyle en C₁₋₄, deutérioalkyle en C₁₋₄, -N(R₁₃)-S(O)₂R₁₀, -O-S(O)₂R₁₀, -S(O)ᵣR₁₀, -O-R₁₁, - O-C(O)R₁₂, -NR₁₃R₁₄ et -N(R₁₃)-C(O)R₁₂ ; et
R₁₀, R₁₁, R₁₂, R₁₃, R₁₄ et r sont tels que définis dans la revendication 4.

11. Composé de formule (III), stéréoisomère ou sel pharmaceutiquement acceptable de celui-ci selon la revendication 10, dans lequel :
R₁ présente la structure suivante : 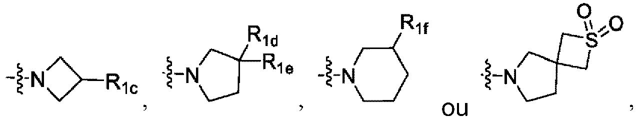
dans lequel,
R_{1c} est choisi dans le groupe constitué par hydrogène, deutérium, halogène, alkyle en C₁₋₄, -N(R₁₃ₐ)-S(O)₂R₁₀ₐ, -O-R₁₁ₐ et -N(R₁₃ₐ)-C(O)R₁₂ₐ, et l'alkyle en C₁₋₄ est éventuellement en outre substitué par un ou plusieurs substituants choisis dans le groupe constitué par deutérium, alkyle en C₁₋₄, haloalkyle en C₁₋₄, deutérioalkyle en C₁₋₄, -S(O)ᵣR_{10b} et -O-R₁₁ₐ ;
R_{1d} est choisi dans le groupe constitué par hydrogène, deutérium, halogène, alkyle en C₁₋₄, -N(R₁₃ₐ)-S(O)₂R₁₀ₐ, -O-R₁₁ₐ et -N(R₁₃ₐ)-C(O)R₁₂ₐ ;
R₁ₑ est choisi dans le groupe constitué par hydrogène, deutérium, halogène, alkyle en C₁₋₄, -N(R₁₃ₐ)-S(O)₂R₁₀ₐ, -O-R₁₁ₐ et -N(R₁₃ₐ)-C(O)R₁₂ₐ, et l'alkyle en C₁₋₄ est éventuellement en outre substitué par un ou plusieurs substituants choisis dans le groupe constitué par deutérium, alkyle en C₁₋₄, haloalkyle en C₁₋₄, deutérioalkyle en C₁₋₄, -S(O)ᵣR_{10b} et -O-R₁₁ₐ ;
R_{1f} est choisi dans le groupe constitué par hydrogène, deutérium, halogène, alkyle en C₁₋₄, -N(R₁₃ₐ)-S(O)₂R₁₀ₐ, -O-R₁₁ₐ et -N(R₁₃ₐ)-C(O)R₁₂ₐ, et l'alkyle en C₁₋₄ est éventuellement en outre substitué par un ou plusieurs substituants choisis dans le groupe constitué par deutérium, alkyle en C₁₋₄, haloalkyle en C₁₋₄, deutérioalkyle en C₁₋₄, -S(O)ᵣR_{10b} et -O-R₁₁ₐ ;
chaque R₁₀ₐ est indépendamment choisi dans le groupe constitué par hydrogène, alkyle en C₁₋₄, cycloalkyle en C₃₋₆ et hétérocyclyle à 3-6 chaînons ;
chaque R_{10b} est indépendamment choisi dans le groupe constitué par hydrogène, alkyle en C₁₋₄, cycloalkyle en C₃₋₆ et hétérocyclyle de 3 à 6 chaînons, et les groupes ci-dessus sont chacun indépendamment éventuellement en outre substitués par un ou plusieurs substituants choisis dans le groupe constitué par deutérium, halogène, hydroxy, alkyle en C₁₋₄ et alcoxy en C₁₋₄ ;
chaque R₁₁ₐ est indépendamment l'hydrogène ou un alkyle en C₁₋₄ ;
chaque R₁₂ₐ est indépendamment choisi dans le groupe constitué par hydrogène, alkyle en C₁₋₄, alcoxy en C₁₋₄ et cycloalkyle en C₃₋₆, et les groupes ci-dessus sont chacun indépendamment éventuellement en outre substitués par un ou plusieurs substituants choisis dans le groupe constitué par deutérium, halogène, hydroxy et cyano ;
chaque R₁₃ₐ est indépendamment l'hydrogène ou un alkyle en C₁₋₄ ; et
chaque r est indépendamment 0, 1 ou 2.

12. Composé de formule (III), stéréoisomère ou sel pharmaceutiquement acceptable de celui-ci selon la revendication 10, dans lequel :
R₁ a la structure suivante : 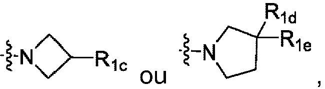 dans lequel,
R_{1c} est choisi dans le groupe constitué par hydrogène, deutérium, fluor, brome, méthyle, éthyle, propyle, isopropyle, butyle, -N(R₁₃ₐ)-S(O)₂R₁₀ₐ, -O-R₁₁ₐ et - N(R₁₃ₐ)-C(O)R₁₂ₐ, et le méthyle, éthyle, propyle, isopropyle ou butyle est indépendamment éventuellement en outre substitué par un ou plusieurs substituants choisis dans le groupe constitué par deutérium, méthyle, éthyle, propyle, isopropyle, butyle, monofluorométhyle, difluorométhyle, trifluorométhyle, monodeutériométhyle, dideutériométhyle, trideutériométhyle, -S(O)ᵣR_{10b} et -O-R₁₁ₐ ;
R_{1d} est choisi dans le groupe constitué par hydrogène, deutérium, fluor, brome, méthyle, éthyle, propyle, isopropyle et butyle ;
R₁ₑ est choisi dans le groupe constitué par hydrogène, deutérium, fluor, brome, méthyle, éthyle, propyle, isopropyle, butyle, -N(R₁₃ₐ)-S(O)₂R₁₀ₐ, -O-R₁₁ₐ et - N(R₁₃ₐ)-C(O)R₁₂ₐ, et méthyle, éthyle, propyle, isopropyle ou butyle est indépendamment éventuellement en outre substitué par un ou plusieurs substituants choisis dans le groupe constitué par deutérium, méthyle, éthyle, propyle, isopropyle, butyle, monofluorométhyle, difluorométhyle, trifluorométhyle, monodeutériométhyle, dideutériométhyle, trideutériométhyle, -S(O)ᵣR_{10b} et -O-R₁₁ₐ ;
chaque R₁₀ₐ est indépendamment choisi dans le groupe constitué par hydrogène, méthyle, éthyle, propyle, isopropyle, butyle, cyclopropyle, cyclobutyle, cyclopentyle, cyclohexyle, oxacyclopropyle, oxacyclobutyle, oxacyclopentyle, oxacyclohexyle, azacyclopropyle, azacyclobutyle, azacyclopentyle et azacyclohexyle ;
chaque R_{10b} est indépendamment choisi dans le groupe constitué par hydrogène, méthyle, éthyle, propyle, isopropyle, butyle, cyclopropyle, cyclobutyle, cyclopentyle, cyclohexyle, oxacyclopropyle, oxacyclobutyle, oxacyclopentyle, oxacyclohexyle, azacyclopropyle, azacyclobutyle, azacyclopentyle et azacyclohexyle, et les groupes ci-dessus sont chacun indépendamment éventuellement en outre substitués par un ou plusieurs substituants choisis dans le groupe constitué par deutérium, fluor, chlore, hydroxy, méthyle, éthyle, propyle, isopropyle, butyle, méthoxy, éthoxy et propoxy ;
chaque R₁₁ₐ est indépendamment choisi dans le groupe constitué par hydrogène, méthyle, éthyle, propyle, isopropyle et butyle ;
chaque R₁₂ₐ est indépendamment choisi dans le groupe constitué par hydrogène, méthyle, éthyle, propyle, isopropyle, butyle, méthoxy, éthoxy, propoxy, tert-butoxy, cyclopropyle, cyclobutyle, cyclopentyle et cyclohexyle, et les groupes ci-dessus sont chacun indépendamment éventuellement en outre substitués par un ou plusieurs substituants choisis dans le groupe constitué par deutérium, fluor, chlore, hydroxy et cyano ;
chaque R₁₃ₐ est indépendamment l'hydrogène ; et
chaque r est indépendamment 0, 1 ou 2.

13. Composé de formule (III), stéréoisomère ou sel pharmaceutiquement acceptable de celui-ci selon la revendication 10, dans lequel :
R₁ a la structure suivante : 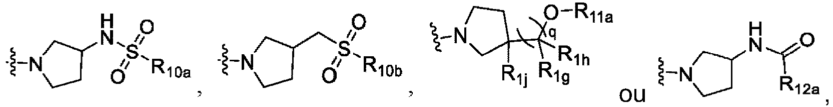
dans lequel,
R_{1g} est hydrogène, deutérium ou méthyle ; R₁ₕ est hydrogène, deutérium ou méthyle ; R₁ⱼ est hydrogène, deutérium ou méthyle ; q est 0, 1 ou 2 ;
R₁₀ₐ est choisi dans le groupe constitué par l'hydrogène, méthyle, éthyle, propyle, isopropyle, butyle, cyclopropyle, cyclobutyle, cyclopentyle, cyclohexyle, oxacyclopropyle, oxacyclobutyle, oxacyclopentyle, oxacyclohexyle, azacyclopropyle, azacyclobutyle, azacyclopentyle et azacyclohexyle ;
R_{10b} est choisi dans le groupe constitué par l'hydrogène, un méthyle, un éthyle, un propyle, un isopropyle, un butyle, un cyclopropyle, un cyclobutyle, un cyclopentyle, un cyclohexyle, un oxacyclopropyle, un oxacyclobutyle, un oxacyclopentyle, un oxacyclohexyle, un azacyclopropyle, un azacyclobutyle, un azacyclopentyle et un azacyclohexyle, et les groupes ci-dessus sont chacun indépendamment éventuellement en outre substitués par un ou plusieurs substituants choisis dans le groupe constitué par deutérium, fluor, chlore, hydroxy, méthyle, éthyle, propyle, isopropyle, butyle, méthoxy, éthoxy et propoxy ;
chaque R₁₁ₐ est indépendamment choisi dans le groupe constitué par hydrogène, méthyle, éthyle, propyle, isopropyle et butyle ;
R₁₂ₐ est choisi dans le groupe constitué par hydrogène, méthyle, éthyle, propyle, isopropyle, butyle, méthoxy, éthoxy, propoxy, tert-butoxy, cyclopropyle, cyclobutyle, cyclopentyle et cyclohexyle, et les groupes ci-dessus sont chacun indépendamment éventuellement en outre substitués par un ou plusieurs substituants choisis dans le groupe constitué par deutérium, fluor, chlore, hydroxy et cyano.

14. Composé de formule (III), stéréoisomère ou sel pharmaceutiquement acceptable de celui-ci selon la revendication 4, dans lequel R₂ₐ est choisi dans le groupe constitué par hydrogène, deutérium, alkyle en C₁₋₄, halogénoalkyle en C₁₋₄, deutérioalkyle en C₁₋₄, cycloalkyle en C₃₋₆ et hétérocyclyle à 3-6 chaînons.

15. Composé de formule (III), stéréoisomère ou sel pharmaceutiquement acceptable de celui-ci selon la revendication 14, dans lequel R₂ₐ est choisi dans le groupe constitué par hydrogène, deutérium, méthyle, éthyle, propyle, isopropyle, butyle, monofluorométhyle, difluorométhyle, trifluorométhyle, monodeutériométhyle, dideutériométhyle, trideutériométhyle, cyclopropyle, cyclobutyle, oxacyclopropyle, oxacyclobutyle, azacyclopropyle et azacyclobutyle.

16. Composé de formule (III), stéréoisomère ou sel pharmaceutiquement acceptable de celui-ci selon la revendication 1, dans lequel il est choisi parmi les composés suivants : 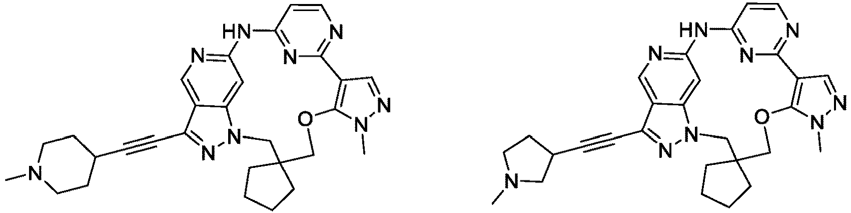 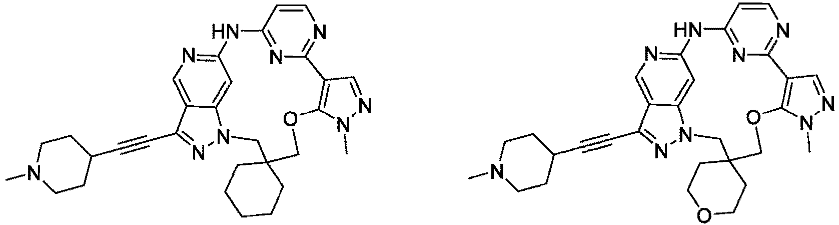 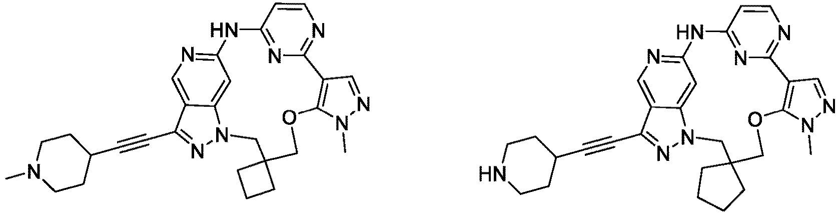 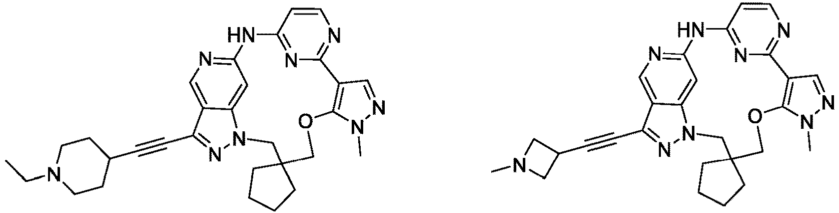 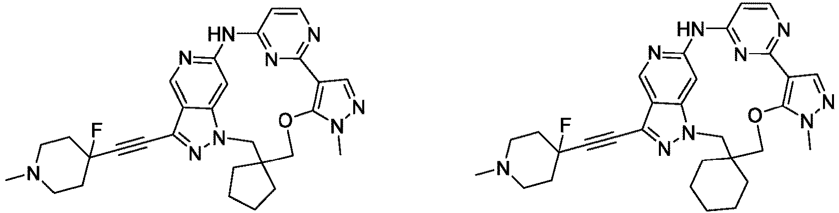 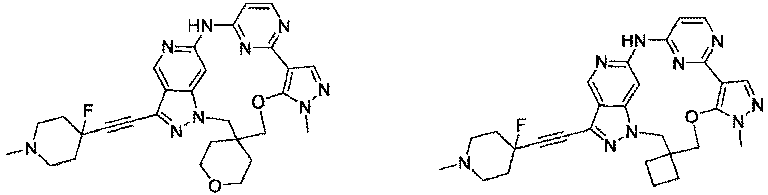 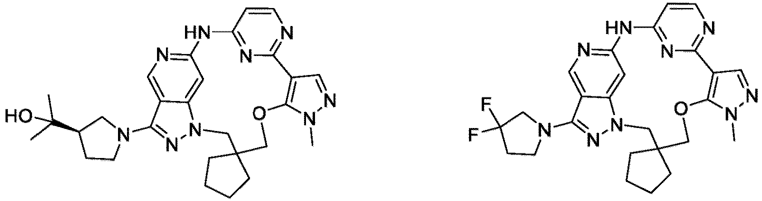 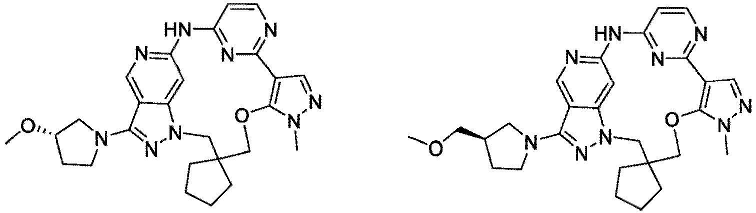 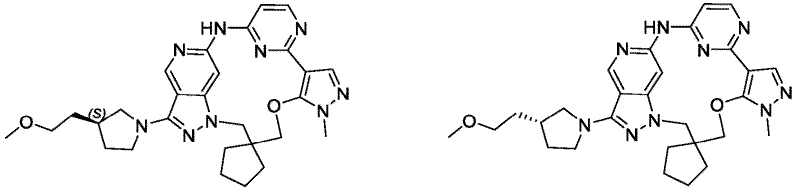 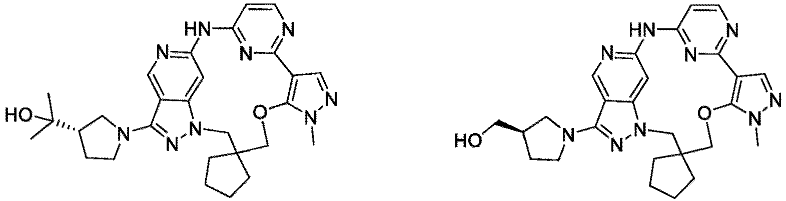 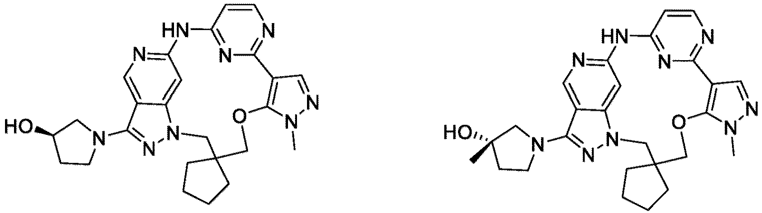 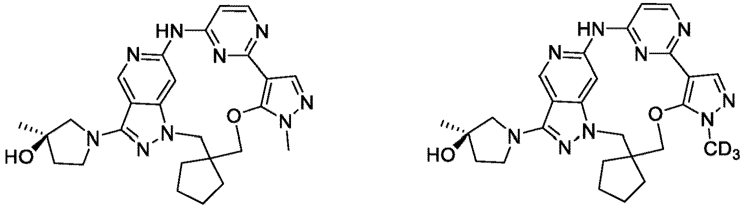 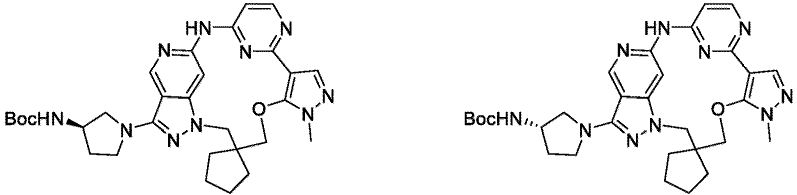 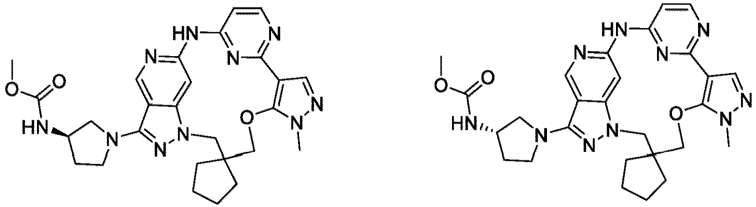 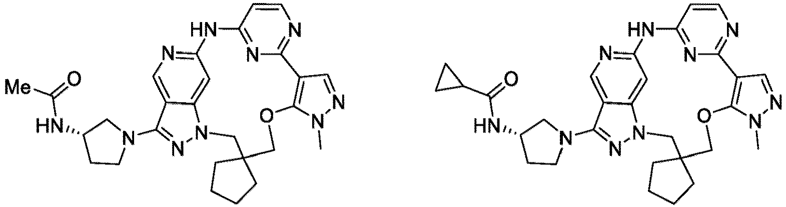 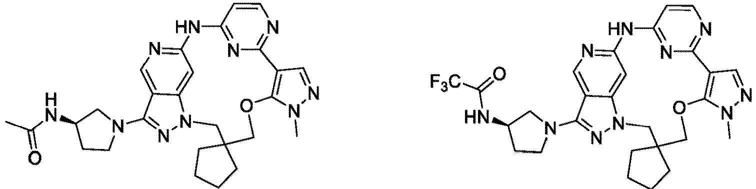 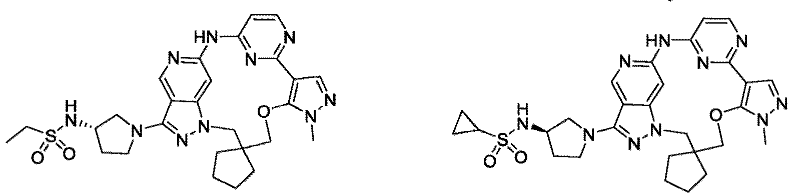 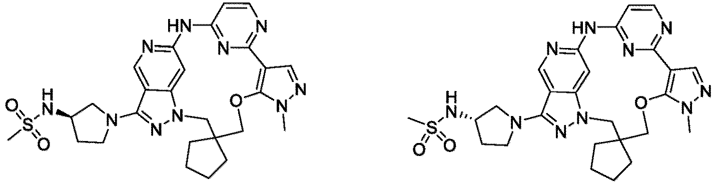 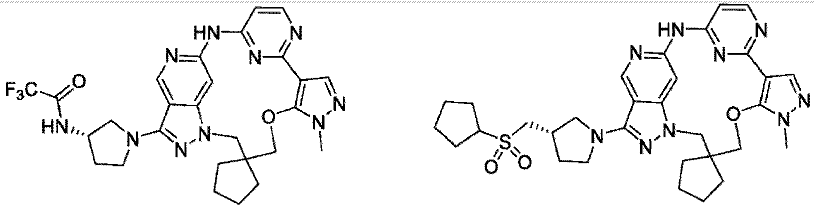 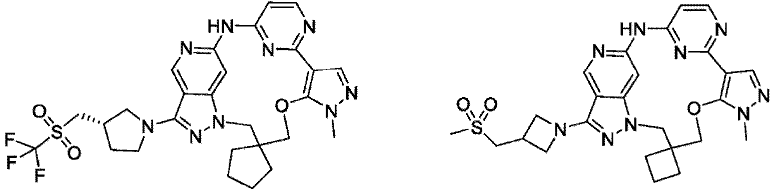 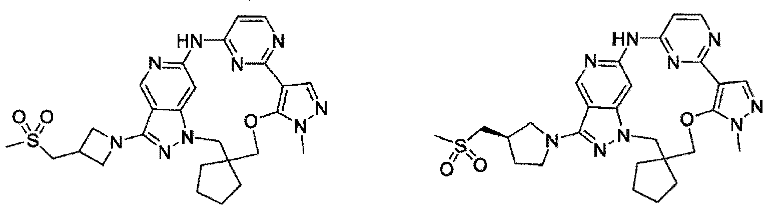 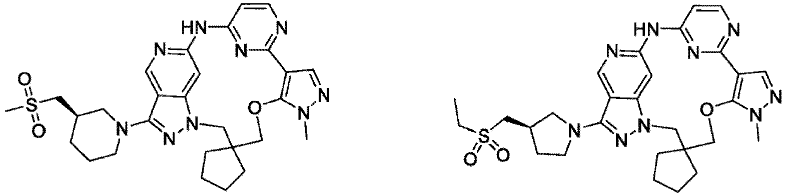 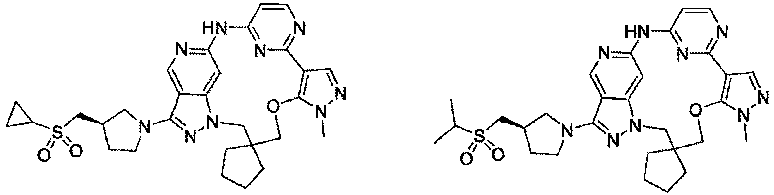 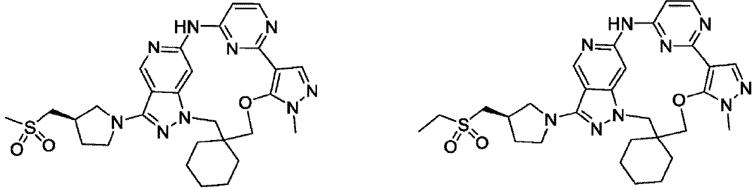 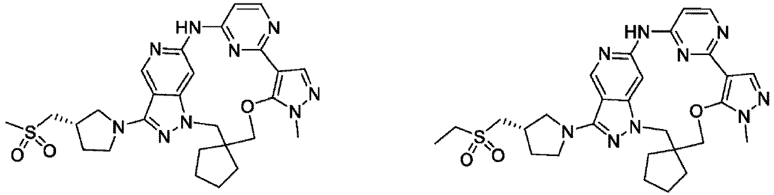 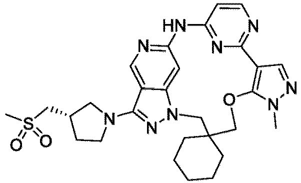 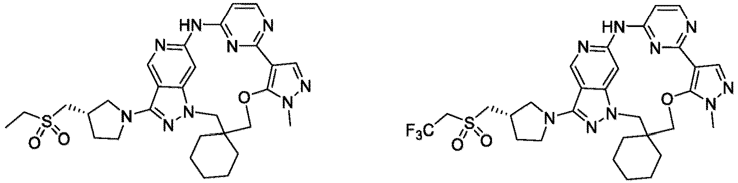 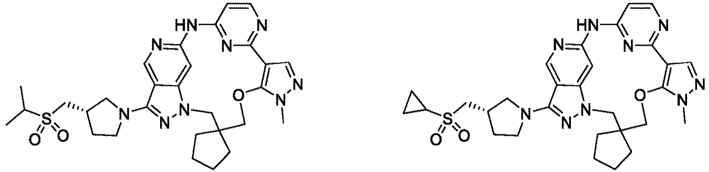 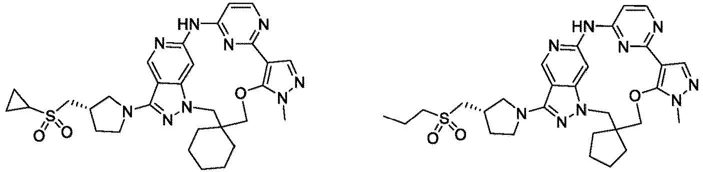 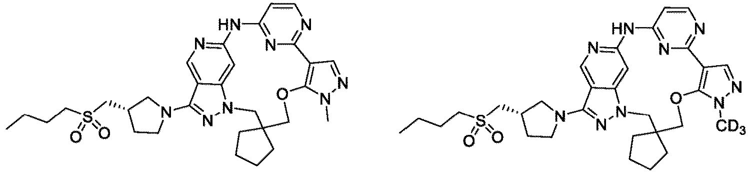 et

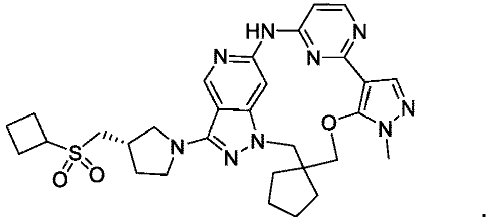

17. Composition pharmaceutique, comprenant le composé de formule (III), le stéréoisomère ou sel pharmaceutiquement acceptable de celui-ci selon l'une quelconque des revendications 1 à 16, et un support pharmaceutiquement acceptable.

18. Composé de formule (III), stéréoisomère ou sel pharmaceutiquement acceptable de celui-ci selon l'une quelconque des revendications 1 à 16 pour une utilisation dans le traitement et/ou la prévention de tumeurs ou de maladies métastatiques associées au moins partiellement à la double mutation L858R/C797S de l'EGFR, la double mutation Del19/C797S de l'EGFR, la triple mutation L858R/T790M/C797S et la triple mutation Del19/T790M/C797S ; éventuellement dans lequel les tumeurs ou les maladies métastatiques sont des tumeurs et/ou des maladies métastatiques causées par une hyperprolifération et un dysfonctionnement de l'induction de la mort cellulaire.

19. Composé pour une utilisation selon la revendication 18, dans lequel les tumeurs ou les maladies métastatiques sont choisies dans les groupes constitués par cancer du poumon, cancer du côlon, cancer du pancréas, cancer de la tête et du cou, cancer du sein, cancer de l'ovaire, cancer de l'utérus, cancer gastrique, cancer du poumon non à petites cellules, leucémie, syndrome myélodysplasique, lymphome malin, tumeur de la tête et du cou, tumeur thoracique, tumeur gastro-intestinale, tumeur endocrinienne, tumeur du sein et autre tumeur gynécologique, tumeur urologique, tumeur cutanée, sarcome, papillome inversé naso-sinusien ou carcinome épidermoïde naso-sinusien associé à un papillome inversé naso-sinusien.
